(19) Europäisches Patentamt
European Patent Office
Office européen des brevets

(11) EP 2 036 918 A1

(12) EUROPEAN PATENT APPLICATION
published in accordance with Art. 153(4) EPC

(43) Date of publication:
**18.03.2009 Bulletin 2009/12**

(21) Application number: **07745096.3**

(22) Date of filing: **12.06.2007**

(51) Int Cl.:
***C07K 5/02*** *(2006.01)* ***A61K 38/00*** *(2006.01)*
***A61P 25/04*** *(2006.01)*

(86) International application number:
**PCT/JP2007/061809**

(87) International publication number:
**WO 2007/145208 (21.12.2007 Gazette 2007/51)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **12.06.2006 JP 2006162379**
**27.12.2006 PCT/JP2006/326144**

(71) Applicants:
- **Vexon, Inc.**
**Shinagawa-ku**
**Tokyo 142-0062 (JP)**
- **Sakurada, Shinobu**
**Izumi-ku**
**Sendai-shi**
**Miyagi 981-3203 (JP)**

(72) Inventor: **SAKURADA, Shinobu**
**Sendai-shi, Miyagi 981-3203 (JP)**

(74) Representative: **Behnisch, Werner et al**
**Reinhard, Skuhra, Weise & Partner GbR**
**Patent- und Rechtsanwälte**
**Friedrichstrasse 31**
**80801 München (DE)**

# (54) PEPTIDE DERIVATIVE

(57) A pharmaceutical composition comprising a peptide derivative or its pharmaceutical equivalent salt having a general formula, $R^1N=C(R^2)-AA^1-AA^2-AA^3-AA^4-Y$, in which $R^1$ is such as a hydrogen, $R^2$ is such as a methyl group, Y is such as a methylamino group, $AA^1$ is such as a tyrosine group, $AA^2$ is such as a D-arginine group, $AA^3$ is such as a phenylalanine group, and $AA^4$ is such as a N-methyllysine group has analgesic activity against a variety of pains on both subcutaneous and oral administration.

EP 2 036 918 A1

## Description

### CROSS REFERENCE TO RELATED APPLICATIONS

This application claims priority from International App. Ser. No. PCT/JP2006/326144 filed December 27, 2006 and Japanese Patent App. Ser. No. 2006-162379 filed June 12, 2006, the entire contents of each of which are herein incorporated by reference.

### BACKGROUND OF THE INVENTION

#### 1. Field of Invention

The present invention relates to a peptide derivative. More specifically the present invention relates to a peptide derivative having particularly analgesic activity or anti-nociceptive activity.

#### 2. Description of the related art

One of the issues remained to be addressed for human being is "pain control". "Ache" i.e. a pain may be an important information of occurrence of trauma or illness, but it is difficult to quickly relieve such a pain, e.g. acute pain, chronic pain, fibromyalgia, neuropathic pain, diabetic neuropathic pain, cancer pain, MSU (urate) induced knee arthritis pain, knee osteoarthritis pain, and rheumatoid arthritis pain, by medical treatment under the present set of circumstances. Such a pain always excruciates the patient, enervates the patient to fight the disease, and even has the patient in despair.

From historical point of view, human beings have been fighting against such a pain by using a opium. Opium obtained from a soup of immature fruit of poppy is described on the clay slate left by Schmahl during Mesopotamia civilization five thousands year ago. Further it was used an anesthetic agent since before Christ in Greek. In other word, opium is the oldest drug in human history.

Since Serturner, a German pharmacist, discovered morphine obtained from the opium in 1803, until today morphine has been used as an excellent analgesic drug to control especially cancer pain and thus it is an important analgesic drug.

Morphine, however, is a habitual and narcotic drug, is physically and mentally addictive due to multiple administrations, and accordingly as it causes an chronic addiction, i.e. addiction to drug, the dosage must be increased to provide the analgesic effect. Thus, morphine must be controlled and administered with a very special care. Further as morphine is relatively not long lasting against serious pain, frequent administrations in a short period of time are required. Accordingly It results a big burden not only to the patient but also to medical staffs, patient's family members and/or care-givers, and an medical care of the patient at the last stage of cancer at home (or medical care in a hospice) is problematic to be realistic.

While pains on which morphine is not effective, e.g. neuropathic pain, are known and thus more effective analgesic drug than morphine has been investigated for such pains.

Thus there was need for an analgesic drug having equally to or more effective than morphine in a variety of pains without a narcotic property with a longer lasting activity.

Since morphine was discovered as noted above, herein researches of analgesic drugs have been focused on alkaloids related to the chemical structure of morphine. On the other hand, there have been approaches to apply peptide derivatives for an analgesic drug since peptides (opioid peptides), methionine enkephalin and leucine enkephalin, having morphine like activity were discovered in the swine brain in 1973.

While an analgesic drug is required to be easily administered and may provide fewer burdens to the patient, and when it is either orally or subcutaneously administered, it is required that it may also provide a longer lasting anti-nociceptive or analgesic activity. Both enkephalins as noted, however, are easily decomposed by enzymes in the body and thus could not provide any anti-nociceptive or analgesic effect on oral or subcutaneous administration.

Delmorphine isolated from the skin of the African frog, *Phyllomedusa sauvagel,* in 1980, includes a D-isomer amino acid residue which was considered not existing in nature except in a bacterium and resists to an enzymatic decomposition in the body. (Non-patent reference 1) Based on such discovery, a variety of developments for the synthetic peptide derivatives having D-isomer amino acid residue have been performed, but it was difficult to obtain especially high anti-nociceptive or analgesic effect on an oral administration.

For example, TAPA (Tyr-D-Arg-Phe-β-Ala) synthesized by the present inventors has nine times stronger anti-nociceptive activity than morphine on subcutaneous administration. (Non-patent reference 2 - 5) The anti-nociceptive activity of TAPA on oral administration was almost equal to the activity of morphine.

Following such basic examination the present inventors synthesized a series of peptide derivatives having amidine group at the amino end to improve the anti-nociceptive activity on oral administration. (Patent references 1 - 3)

Among them the peptide derivative ADAMB (Nα-amidine-[Tyr]-[D-Arg]-[Phe]-[N-Methyl-β-Ala]-OH) had about 23 times stronger anti-nociceptive activity than morphine on subcutaneous administration and about 3.8 times stronger anti-nociceptive activity than morphine on oral administration. (See non-patent reference 6 noted below and incorporated herein by reference)

When we amidated ADAMB at the carboxyl end to improve anti-nociceptive activity, the anti-nociceptive activity of the amide decreased to one seventh of the activity of morphine on subcutaneous administration and also to one fifth of the activity of morphine on oral administration. Further when we synthesized N-methyl-amide at the carboxyl end of ADAMB, the anti-nociceptive activity of the N-methyl-amide further decreased on both oral and subcutaneous administrations. (Non-patent reference 7) Thus it was obvious that either amide or N-methyl amide at the carboxyl end of ADAMB decreased anti-nociceptive activity rather than increased.

Further when the present inventors synthesized a series of peptide derivatives having 1-iminomethyl group at the amino end, tyrosine as the first amino acid residue, D-arginine or D-methionine sulfoxide as the second amino acid residue, and phenylalanine as the third amino acid residue in the basic peptide structure, even though the anti-nociceptive activity on oral administration increased more than the anti-nociceptive activity of ADAMB as noted above, it was not considered satisfactory. (Patent reference 4 and non-patent reference 8)

Thus the issues to provide the drug which has high and excellent both anti-nociceptive activity and analgesic activity on both oral and subcutaneous administrations allowing displace morphine or cap morphine without narcotic effect, or which is applicable to medical treatments on which morphine is not effective or adequate due to its narcotic drawback, have been remained to be addressed.

To address the objects noted above the present inventors further studied to modify the carboxyl end of the peptide derivative to increase the anti-nociceptive activity of the peptide derivative having 1-iminoethyl group at the amino end. ADAMB substituted with a low iminoalkyl group at the amino end was amidated in such study.

It was envisioned from the above experimental results of the amidated ADAMB at the carboxyl end that the ADAMB substituted with a low-iminoalkyl group at the amino would also decrease anti-nociceptive activity thereof.

However, contrary to expectation, when the carboxyl end of the peptide noted above was amidated, the anti-nociceptive activities on both oral and subcutaneous administration increased and additionally the anti-nociceptive activities were retained longer. The results were unexpected. Further when the carboxyl end was methyl-amidated, it was found that the anti-nociceptive activities on both oral and subcutaneous administrations increased more than when it was amidated. Thus according to the present invention the present inventors found as described hereinafter that the novel peptide derivatives had excellent anti-nociceptive activities on both oral and subcutaneous administrations.
The contents of the following documents are herein incorporated by reference in this disclosure in their entireties.

Patent reference 1: International Publication Wo95/24421
Patent reference 2: International Publication Wo97/10261
Patent reference 3: International Publication Wo97/10262
Patent reference 4: International Publication Wo99/33864
Non patent reference 1: Int. J. Peptide Protein Res. 17: 275 (1981)
Non patent reference 2: Br. J. Pharmacol., 95:15 (1988)
Non patent reference 3: Peptides, 11: 139 (1990)
Non patent reference 4: Neuropharmacol., 32: 689 (1993)
Non patent reference 5: Pharmacol. Biochem. Behav., 24:27 (1988)
Non patent reference 6: Chem. Pharm. Bull., 50: 771-780 (2002)
Non patent reference 7: J. Med. Chem., 45: 5081-5089 (2002)
Non patent reference 8: Chem. Pharm. Bull., 51: 759-771 (2003)

## OBJECT AND SUMMARY OF THE INVENTION

One proposed object of the present invention is to provide a peptide having a strong analgesic or anti-nociceptive activity on oral administration in addition to on subcutaneous administration. Another proposed object of the present invention is to provide a peptide having a longer lasting analgesic or anti-nociceptive activity on both oral administration and subcutaneous administration. Another proposed object of the present invention is to provide a drug including the peptide derivatives noted above as an active substance. Another proposed object of the present invention is to provide a therapeutic method for pains and other medical symptoms by the administration of the peptide derivatives noted above. Another proposed object of the present invention is to provide a drug including the peptide derivatives which may eb effective on pains, e.g. neuropathic pain, cancer pain, MSU (urate) induced knee arthritis pain, knee osteoarthritis pain, and chronic rheumatoid arthritis pain, by medical treatment under the present set of circumstances, and may treat other diseases and symptoms.

To address the objects noted above, in addition to the study noted above, the present inventors further studied

to modify the carboxyl end of the peptide derivative to increase the anti-nociceptive activity of the peptide derivative having 1-iminoethyl group at the amino end. ADAMB substituted with a low-iminoalkyl group at the amino end was amidated in such study.

It was envisioned from the above experimental results of the amidated ADAMB at the carboxyl end that the ADAMB substituted with a low-iminoalkyl group at the amino end would also decrease anti-nociceptive activity thereof.

However, contrary to expectation, when the carboxyl end of the peptide noted above was amidated, the anti-nociceptive activities on both oral and subcutaneous administration surprisingly increased and additionally the anti-nociceptive activities were retained longer. The results were unexpected. Further when the carboxyl end was methyl-amidated, it was found that the anti-nociceptive activities on both oral and subcutaneous administration increased more than when it was amidated. Thus according to the present invention the present inventors found as described hereinafter that the novel peptide derivatives had excellent anti-nociceptive activities on both oral and subcutaneous administration.

According to one embodiment of the present invention there is provided a compound or a pharmacologically acceptable salt thereof comprising: a general formula shown in chemical formula (1);

$$R^1N=C(R^2)\text{-}AA^1\text{-}AA^2\text{-}AA^3\text{-}AA^4\text{-}Y \qquad (1)$$

wherein $R^1$ is selected one of hydrogen atom, hydroxyl group, low-alkyl group, and low-alkoxyl group; wherein $R^2$ is low-alkyl group; and wherein Y is represented by chemical formula (2) below;

$$\text{-}n(R^3)R^4 \qquad (2);$$

in chemical formula (2) wherein $R^3$ and $R^4$ are respectively and independently selected one of hydrogen atom, hydroxyl group, low-alkyl group, and low-alkoxyl group; or five or six member nitrogen-containing heterocyclic group in which the nitrogen atom is connected to $R^3$ and $R^4$; wherein $AA^1$ noted above is an α-amino acid residue represented by the formula (3) below;

[C1]

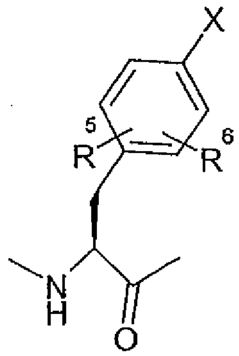

(3)

in formula (3) wherein $R^3$ and $R^4$ are respectively and independently selected one of hydrogen atom, halogen atom, low-alkyl group, and halogenated low-alkyl group; in chemical formula (3) wherein X is selected one of hydrogen atom, halogen atom, hydroxyl group, a group represented by chemical formula (4) below and by chemical formula (5);

$$\text{-O-CO-}R^7 \qquad (4)$$

$$\text{-O-CO-O-}R^8 \qquad (5)$$

in chemical formula (4) and in chemical formula (5) wherein $R^7$ and $R^8$ are respectively and independently selected one of $C_{1-16}$ alkyl group, hydroxy-$C_{1-16}$ alkyl group, amino-$C_{1-16}$ alkyl group, (mono-low-alkyl)-amino-$C_{1-16}$alkyl group, (di-low-alkyl)-amino-$C_{1-16}$ alkyl group, $C_{3-10}$ cycloalkyl group, $C_{3-10}$ cycloalkyl substituted low-alkyl group, $C_{2-16}$ alkenyl group, $C_{2-16}$ alkynyl group, heterocyclic group, aryl group, and aryl-substituted low-alkyl group; wherein $AA^2$ noted above is a D-α-amino acid residue represented by chemical formula (6) below, in formula (6) wherein $R^9$ is selected one of amino group, (mono-alkyl-low)-amino group, low-acylamino group, guanidino group, low

[C2]

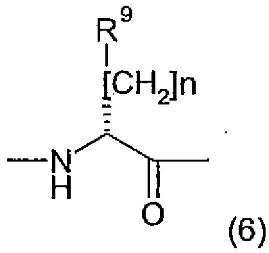

(6)

alkyl substituted guanidino group, imino low-alkyl group, ureide group, low-alkyl-substituted ureide group, low-alkylthio group, low-alkylsulfinyl group, low-alkylsulfonyl group, low-acyl group, and hydroxy low-alkyl group, wherein n is an integer of 1 thorough 4, wherein $AA^3$ is selected one of non-substituted phenylalanine residue, substituted phenylalanine residue, non-substituted D-phenylalanine residue and substituted D-phenylalanine residue; wherein $AA^4$ is an $\alpha$-amino acid residue represented by chemical formula (7) below or

$-N(R^{10})-CH(R^{11})-CO-$ (7)

a $\beta$-amino acid residue represented by chemical formula (8) below;

$-N(R^{10})-CH(R^{11})-CH(R^{12})-CO-$ (8)

in chemical formula (7) and (8) wherein $R^{10}$ and $R^{12}$ are respectively and independently selected one of hydrogen, low-alkyl group, low-alkenyl group, low-alkenyl group, aryl group, and aryl-substituted low-alkyl group; wherein $R^{11}$ is hydrogen atom or a group represented by chemical formula (9) below,

$-Z-N(R^{13})-R^{14}$ (9)

in chemical formula (9) wherein Z is selected one of low-alkylene group, low-alkenylene group, and low-alkynylene group, and wherein $R^{13}$ and $R^{14}$ are respectively and independently selected one of hydrogen atom, low-alkyl group, aryl group, and aryl-substituted-low-alkyl group, or $R^{13}$ and $R^{14}$ are five or six member nitrogen-containing heterocyclic group in which nitrogen atom is connected to $R^{13}$ and $R^{14}$.

According to another embodiment of the present invention there is provided the compound or the pharmacologically acceptable salt thereof, further comprising: $R^1$ (noted above) which is a hydrogen atom.

According to another embodiment of the present invention there is provided the compound or the pharmacologically acceptable salt thereof further comprising: $R^2$ (noted above) which is a methyl group or a ethyl group.

According to another claim of the present invention there is provided the compound or the pharmacologically acceptable salt thereof further comprising: $AA^1$ (noted above) which is an $\alpha$-amino acid residue represented by chemical formula (10)

[C3]

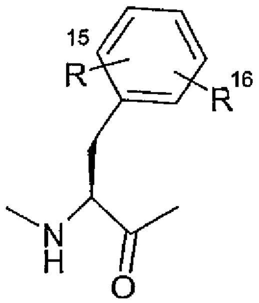

(10)

or a D-α-amino acid residue represented by chemical formula (11);

[C4]

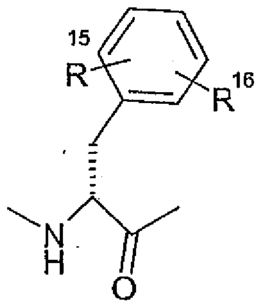

(11)

in formula (10) and (11) wherein $R^{15}$ and $R^{16}$ are respectively and independently selected one of hydrogen atom, halogen atom, low-alkyl group, and halogenated low-alkyl group.

According another embodiment of the present invention there is provided the compound or the pharmacologically acceptable salt thereof further comprising: $AA^3$ (noted above) which is selected one of phenylalanine residue, D-phenylalanine residue, p-fluorophenylalanine residue, D-p-fluorophenylalanine residue, o-trifluoromethylphenylalanine residue, D-o-trifluoromethylphenylalanine residue, and 2,6-dimethylphenylalanine residue

According to another embodiment of the present invention there is provided the compound or the pharmacologically acceptable salt thereof further comprising: $AA^4$ (noted above) which is N-methyllysine residue or N-methyl-β-alanine residue.

According to another embodiment of the present invention there is provided the compound or the pharmacologically acceptable salt thereof further comprising: X in chemical formula (3) which is hydroxy group.

According to another embodiment of the present invention there is provided the compound or the pharmacologically acceptable salt thereof further comprising: X in chemical formula (3) which is hydrogen atom or halogen atom.

According to another embodiment of the present invention there is provided the compound or the pharmacologically acceptable salt thereof further comprising: X in chemical formula (3) which is represented by chemical formula (4) or (5) wherein $R^7$ in chemical formula (4) and $R^8$ in chemical formula (5) are respectively and independently selected one of $C_{1-16}$ alkyl group, hydroxy-$C_{1-16}$ alkyl group, amino-$C_{1-16}$ alkyl group, (mono-low-alkyl)-amino-$C_{1-16}$ alkyl group, (di-low-alkyl)-amino-$C_{1-16}$ alkyl group, $C_{3-10}$ cycloalkyl group, $C_{3-10}$ cycloalkyl substituted low-alkyl group, $C_{2-16}$ alkenyl group, $C_{2-16}$ alkynyl group, aryl group, heterocyclic group, and aryl-substituted low-alkyl group.

According to another embodiment of the present invention there is provided the compound or the pharmacologically acceptable salt thereof further comprising: $AA^1$ (noted above) which is selected one of tyrosine residue, 2,6-dimethyltyrosine residue, o-acyltyrosine residue, o-alkoxycarbonyl tyrosine residue, o-phenoxycarbonyl tyrosine residue, o-acetyl tyrosine residue, and 2,6-dimethylphenyl alanine residue.

According to another embodiment of the present invention there is provided the compound or the pharmacologically acceptable salt thereof further comprising: $AA^2$ (noted above) which is selected one of D-methionine sulfoxide residue, D-arginine residue, and D-citrulline residue.

According to another embodiment of the present invention there is provided the compound or the pharmacologically acceptable salt thereof further comprising: $AA^2$ (noted above) which is selected one of D-$N^5$-acetylornithine

residue, D-5-oxonorleucine residue, and D-5-hydroxynorleucine residue.

According to another embodiment of the present invention there is provided the compound or the pharmacologically acceptable salt thereof further comprising: $R^3$ and $R^4$ which are independently selected as at least one of a hydrogen atom, hydroxy group, low-alkyl group and low-alkoxy group.

According to another embodiment of the present invention there is provided the compound or the pharmacologically acceptable salt thereof further comprising: $R^1$ which is hydrogen atom, $R^2$ which is methyl group, $AA^1$ noted above which is o-acetyltyrosine residue, $AA^2$ noted above which is D-methionine sulfoxide residue or D-arginine residue, $AA^3$ noted above which is phenylalanine residue, $AA^4$ noted above which is N-methyllysine residue or N-methyl-β-alanine residue, and Y which is $-NH_3$ or $-NH-CH_3$.

According to another embodiment of the present invention there is provided the compound or the pharmacologically acceptable salt thereof wherein: $R^1$ is hydrogen atom, $R^2$ is methyl group, $AA^1$ noted above which is tyrosine residue, $AA^2$ noted above which is D-methionine sulfoxide residue or D-arginine residue, $AA^3$ noted above which is phenylalanine, $AA^4$ noted above which is N-methyllysine residue or N-methyl-β-alanine residue, Y noted above which is $-NH_2$ or $-NH-CH_3$.

According to another embodiment of the present invention there is provided the compound or the pharmacologically acceptable salt thereof further comprising: $R^1$ which is hydrogen atom, $R^2$ which is methyl group, $AA^1$ noted above which is o-acetyltyrosine residue, $AA^2$ noted above which is D-methionine sulfoxide residue or D-arginine residue, $AA^3$ noted above which is phenylalanine, $AA^4$ noted above which is N-methyllysine residue or N-methyl-β-alanine residue, Y noted above which is $-NH_2$ or $-NH-CH_3$.

According to another embodiment of the present invention there is provided the compound or the pharmacologically acceptable salt thereof further comprising: $R^1$ which is hydrogen atom, $R^2$ which is methyl group, $AA^1$ noted above which is tyrosine residue, $AA^2$ noted above which is D-5-hydroxynorleucine residue, $AA^3$ noted above which is phenylalanine residue, $AA^4$ noted above which is N-methyllysine residue or N-methyl-β-alanine residue, Y noted above which is $-NH_2$ or $-NH-CH_3$.

According to another embodiment of the present invention there is provided the compound or the pharmacologically acceptable salt thereof further comprising: $R^1$ which is hydrogen atom, $R^2$ which is methyl group, $AA^1$ noted above which is tyrosine residue, $AA^2$ noted above which is D-methionine sulfoxide residue, $AA^3$ noted above which is phenylalanine, $AA^4$ noted above which is N-methyllysine residue or N-methyl-β-alanine residue, Y noted above which is $-NH_2$ or $-NH-CH_3$.

According to another embodiment of the present invention there is provided the compound or the pharmacologically acceptable salt thereof further comprising: $R^1$ which is hydrogen atom, $R^2$ which is methyl group, $AA^1$ noted above which is 2,6-dimethyltyrosine residue, $AA^2$ noted above which is D-methionine sulfoxide residue or D-arginine, $AA^3$ noted above which is phenylalanine residue, $AA^4$ noted above which is N-methyllysine residue or N-methyl-β-alanine residue, Y noted above which is $-NH_2$ or $-NH-CH_3$.

According to another embodiment of the present invention there is provided a pharmaceutical composition comprising at least one of the compound or the pharmacologically acceptable salt thereof noted earlier as an active substance.

According to another embodiment of the present invention there is provided a pharmaceutical composition comprising at least one of the compound or the pharmacologically acceptable salt thereof noted earlier as one of an active substance and a pharmacologically acceptable carrier.

According to another embodiment of the present invention there is provided a pharmaceutical composition, the pharmacological composition according to the above noted compositions being the pharmaceutical composition and enabling to prevent pains and/or to be used to treat pains.

According to another embodiment of the present invention there is provided a pharmaceutical composition, the pharmacological composition according to the above being a pharmaceutical composition enabling the prevention of pains and/or use in the treatment of pains; wherein the pains are cancer pains.

According to another embodiment of the present invention there is provided a pharmaceutical composition, the pharmacological composition enabling the prevention of pain and/or the use in treatment of pain wherein the pain is a neuropathic pain.

According to another embodiment of the present invention there is provided a pharmaceutical composition, the pharmacological composition enabling the prevention of pain and/or the use of a treatment of pain, wherein the pain is a knee osteoarthritis pain.

According to another embodiment of the present invention there is provided a pharmaceutical composition, the pharmacological composition enabling the prevention of pain and/or the use of a treatment of pain; wherein the pain is rheumatoid arthritis pain.

Thus the compounds or the pharmacologically acceptable salts thereof of the present invention may provide a strong analgesic activity or an anti-nociceptive activity on not only subcutaneous administration but on also oral administration, and the analgesic activity or the anti-nociceptive activity on both oral administration and subcutaneous

administration are longer lasting.
According to the present invention the pharmaceutical composition may provide a strong analgesic effect or an anti-nociceptive effect against extremely broad range of pains including heat-stimulated pains, neuropathic pains, and pressure-stinging pains; may be employed as a general pain killer better than morphine with the high and longer lasting effects and, in addition, without narcotic property; and may eliminate or alleviate burdens of a patient suffering from pains, medical staffs and the patient's family taking care of the patient. As the pharmaceutical composition provided the extremely effective anti-nociceptive activity against three different pains, it may be most likely applied to other pains due to such as collagenosis, pains of unknown cause at present; pains to which morphine is not applicable due to narcotic property, and pains on which morphine is not effective.
The above, and other objects, features and advantages of the present invention will become apparent from the following description read in conduction with the accompanying drawings.

**BRIEF DESCRIPTION OF THE DRAWINGS**

Fig. 1 is a graph of time-course variation of anti-nociceptive activity of a compound according to example 1 after subcutaneously administered.
Fig. 2 is a graph of dose-response curve of the compound according to example 1 after subcutaneously administered.
Fig. 3 is a graph of time-course variation of anti-nociceptive activity of a compound according to example 2 after subcutaneously administered.
Fig. 4 is a graph of dose-response curve of the compound according to example 2 after subcutaneously administered.
Fig. 5 is a graph of time-course variation of anti-nociceptive activity of a compound according to example 2 after orally administered.
Fig. 6 is a graph of dose-response curve of the compound according to example 2 after orally administered.
Fig. 7 is a graph of time-course variation of anti-nociceptive activity of a compound according to example 3 after subcutaneously administered.
Fig. 8 is a graph of dose-response curve of the compound according to example 3 after subcutaneously administered.
Fig. 9 is a graph of time-course variation of anti-nociceptive activity of a compound according to example 4 after subcutaneously administered.
Fig. 10 is a graph of dose-response curve of the compound according to example 4 after subcutaneously administered.
Fig. 11 is a graph of time-course variation of anti-nociceptive activity of a compound according to example 4 after orally administered.
Fig. 12 is a graph of dose-response curve of the compound according to example 4 after orally administered.
Fig. 13 is a graph of time-course variation of anti-nociceptive activity of a compound according to example 5 after subcutaneously administered.
Fig. 14 is a graph of dose-response curve of the compound according to example 5 after subcutaneously administered.
Fig. 15 is a graph of time-course variation of anti-nociceptive activity of a compound according to example 6 after subcutaneously administered.
Fig. 16 is a graph of dose-response curve of the compound according to example 6 after subcutaneously administered.
Fig. 17 is a graph of time-course variation of anti-nociceptive activity of a compound according to example 6 after orally administered.
Fig. 18 is a graph of dose-response curve of the compound according to example 6 after orally administered.
Fig. 19 is a graph of time-course variation of anti-nociceptive activity of a compound according to example 7 after subcutaneously administered.
Fig. 20 is a graph of dose-response curve of the compound according to example 7 after subcutaneously administered.
Fig. 21 is a graph of time-course variation of anti-nociceptive activity of a compound according to example 7 after orally administered.
Fig. 22 is a graph of dose-response curve of the compound according to example 7 after orally administered.
Fig. 23 is a graph of time-course variation of anti-nociceptive activity of a compound according to example 8 after subcutaneously administered.
Fig. 24 is a graph of dose-response curve of the compound according to example 8 after subcutaneously administered.
Fig. 25 is a graph of time-course variation of anti-nociceptive activity of a compound according to comparison

example 1 after subcutaneously administered.

Fig. 26 is a graph of dose-response curve of the compound according to comparison example 1 after subcutaneously administered.

Fig. 27 is a graph of time-course variation of anti-nociceptive activity of a compound according to comparison example 1 after orally administered.

Fig. 28 is a graph of dose-response curve of the compound according to comparison example 1 after orally administered.

Fig. 29 is a graph of time-course variation of anti-nociceptive activity of a compound according to comparison example 2 after subcutaneously administered.

Fig. 30 is a graph of dose-response curve of the compound according to comparison example 2 after subcutaneously administered.

Fig. 31 is a graph of time-course variation of anti-nociceptive activity of a compound according to comparison example 2 after orally administered.

Fig. 32 is a graph of dose-response curve of the compound according to comparison example 2 after orally administered.

Fig. 33 is a graph of time-course variation of anti-nociceptive activity of a compound according to comparison example 3 after subcutaneously administered.

Fig. 34 is a graph of dose-response curve of the compound according to comparison example 3 after subcutaneously administered.

Fig. 35 is a graph of time-course variation of anti-nociceptive activity of a compound according to comparison example 4 after subcutaneously administered.

Fig. 36 is a graph of dose-response curve of the compound according to comparison example 4 after subcutaneously administered.

Fig. 37 is a graph of a correlation between oral dosages and AUC.

Fig. 38 is a graph of a correlation between subcutaneous dosages and AUC.

Fig. 39 is a graph of a correlation between subcutaneous dosage and AUC of SS8225-04 on the nerve damage model animal.

Fig. 40 is a graph of a time-course variation from a stimulative threshold (gram weight) on 0.125 mg/Kg of dosage of SS8225-04 on the nerve damage model animal.

Fig. 41 is a graph of a time-course variation from a stimulative threshold (gram weight) on 0.25 mg/Kg of dosage of SS8225-04 on the nerve damage model animal.

Fig. 42 is a graph of a time-course variation from a stimulative threshold (gram weight) on 0.5 mg/Kg of dosage of SS8225-04 on the nerve damage model animal.

Fig. 43 is a graph of a time-course variation from a stimulative threshold (gram weight) on 0.7 mg/Kg of dosage of SS8225-04 on the nerve damage model animal.

Fig. 44 is a graph of a correlation between subcutaneous dosages of SS8225-07 and AUC on a nerve damage model animal.

Fig. 45 is a graph of a time-course variation from a stimulative threshold (gram weight) on 0.125 mg/Kg of dosage of SS8225-07 on the nerve damage model animal.

Fig. 46 is a graph of a time-course variation from a stimulative threshold (gram weight) on 0.25 mg/Kg of dosage of SS8225-07 on the nerve damage model animal.

Fig. 47 is a graph of a time-course variation from a stimulative threshold (gram weight) on 0.5 mg/Kg of dosage of SS8225-07 on the nerve damage model animal.

Fig. 48 is a graph of a time-course variation from a stimulative threshold (gram weight) on 1.0 mg/Kg of dosage of SS8225-07 on the nerve damage model animal.

Fig. 49 is a graph of a correlation between subcutaneous dosages of morphine and AUC on a nerve damage model animal.

Fig. 50 is a graph of a time-course variation from a stimulative threshold (gram weight) on 2.5 mg/Kg of dosage of morphine on the nerve damage model animal.

Fig. 51 is a graph of a time-course variation from a stimulative threshold (gram weight) on 3.5 mg/Kg of dosage of morphine on the nerve damage model animal.

Fig. 52 is a graph of a time-course variation from a stimulative threshold (gram weight) on 5 mg/Kg of dosage of morphine on the nerve damage model animal.

Fig. 53 is a graph of a time-course variation from a stimulative threshold (gram weight) on 7 mg/Kg of dosage of morphine on the nerve damage model animal.

Fig. 54 is a graph of a time-course variation from a stimulative threshold (gram weight) on 10 mg/Kg of dosage of morphine on the nerve damage model animal.

Fig. 55 is a graph of a time course variation of anti-nociceptive effect of SS8225-04 after subcutaneously administered

on a tail-pressure test.

Fig. 56 is a graph of a dose-response curve of SS8225-04 on a subcutaneous administration.

Fig. 57 is a graph of a time course variation of anti-nociceptive effect of SS8225-04 after orally administered on a tail-pressure test.

Fig. 58 is a graph of a dose-response curve of SS8225-04 on an oral administration.

Fig. 59 is a graph of a time course variation of anti-nociceptive effect of SS8225-07 after subcutaneously administered on a tail-pressure test.

Fig. 60 is a graph of a dose-response curve of SS8225-07 on a subcutaneous administration.

Fig. 61 is a graph of a time course variation of anti-nociceptive effect of SS8225-07 after orally administered on a tail-pressure test.

Fig. 62 is a graph of a dose-response curve of SS8225-07 on an oral administration.

Fig. 63 is a graph of a time course variation of anti-nociceptive effect of morphine after subcutaneously administered on a tail-pressure test.

Fig. 64 is a graph of a dose-response curve of morphine on a subcutaneous administration.

Fig. 65 is a graph of a time course variation of anti-nociceptive effect of morphine after orally administered on a tail-pressure test.

Fig. 66 is a graph of a dose-response curve of morphine on an oral administration.

Fig. 67 is a graph of a correlation between oral dosages and AUC.

Fig. 68 is a graph of a correlation between subcutaneous dosages and AUC.

Fig. 69 is a graph of a time course variation of anti-nociceptive effect of morphine after orally administered on a knee osteoarthritis model.

Fig. 70 is a graph of a time course variation of anti-nociceptive effect of oxycodone after orally administered on a knee osteoarthritis model.

Fig. 71 is a graph of a time course variation of anti-nociceptive effect of SS8225-04 after orally administered on a knee osteoarthritis model.

Fig. 72 is a graph of a time course variation of anti-nociceptive effect of SS8225-07 after orally administered on a knee osteoarthritis model.

Fig. 73 is a graph of a correlation between morphine dosages and AUC.

Fig. 74 is a graph of a correlation between oxycodone dosages and AUC.

Fig. 75 is a graph of a correlation between SS8225-04 dosages and AUC.

Fig. 76 is a graph of a correlation between SS8225-07 dosages and AUC.

Fig. 77 is a graph of a correlation between respective drugs and AUC on knee osteoarthritis model.

Fig. 78 is a graph of a correlation between respective drugs and % MPU on knee osteoarthritis model.

Fig. 79 is a graph of a time-course variation of stimulation threshold (gram weight) on oral administration of morphine after administration on a rheumatoid arthritis pain model. (Results on CFA non-administered leg)

Fig. 80 is a graph of a time-course variation of stimulation threshold (gram weight) on oral administration of morphine after administration on a rheumatoid arthritis pain model. (Results on CFA administered leg)

Fig. 81 is a graph of a time-course variation of stimulation threshold (gram weight) on oral administration of morphine after administration on a rheumatoid arthritis pain model. (Results on CFA non-administered leg)

Fig. 82 is a graph of a time-course variation of stimulation threshold (gram weight) on oral administration of SS8225-04 after administration on a rheumatoid arthritis pain model. (Results on CFA administered leg)

Fig. 83 is a graph of a correlation between oral dosages and AUC.

Fig. 84 is a graph of a correlation between oral dosages and AUC per unit time.

## DETAIL DESCRIPTION OF THE PREFFERED EXAMPLES

The present invention there may provide a compound or a pharmacologically acceptable salt thereof comprising: a general formula shown.

$$R^1N=C(R^2)\text{-}AA^1\text{-}AA^2\text{-}AA^3\text{-}AA^4\text{-}Y \qquad (1)$$

In chemical formula (1), $R^1$ is selected one of hydrogen atom, hydroxyl group, low-alkyl group, and low-alkoxyl group; wherein $R^2$ is low-alkyl group.

Y is represented by chemical formula (2) below. In chemical formula (2) $R^3$ and $R^4$ are respectively and independently selected one of hydrogen atom, hydroxyl group, low-alkyl group, and low-alkoxyl group; or five or six member nitrogen-containing heterocyclic group in which the nitrogen atom is connected to $R^3$ and $R^4$.

$-N(R^3)R^4$ (2).

$AA^1$ is an α-amino acid residue represented by the formula (3) below.

[C5]

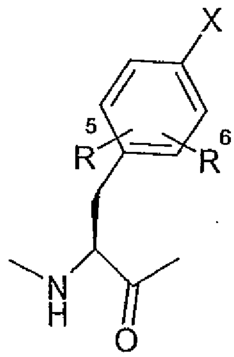

(3)

In chemical formula (3) wherein $R^5$ and $R^6$ are respectively and independently selected one of hydrogen atom, halogen atom, low-alkyl group, and halogenated low-alkyl group.

In chemical formula (3) X is selected one of hydrogen atom, halogen atom, hydroxyl group, a group represented by chemical formula (4) below and by chemical formula (5).

$-O-CO-R^7$ (4)

$-O-CO-O-R^8$ (5)

In chemical formula (4) and in chemical formula (5) wherein $R^7$ and $R^8$ are respectively and independently selected one of $C_{1-16}$ alkyl group, hydroxy-$C_{1-16}$ alkyl group, amino-$C_{1-16}$ alkyl group, (mono-low-alkyl)-amino-$C_{1-16}$ alkyl group, (di-low-alkyl)-amino-$C_{1-16}$ alkyl group, $C_{3-10}$ cycloalkyl group, $C_{3-10}$ cycloalkyl substituted low-alkyl group, $C_{2-16}$ alkenyl group, $C_{2-16}$ alkynyl group, heterocyclic group, aryl group, and aryl-substituted low-alkyl group.

$AA^2$ noted above is a D-α-amino acid residue represented by chemical formula (6) below.

[C6]

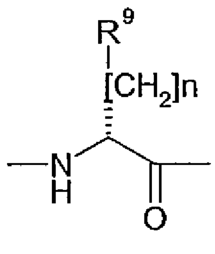

(6)

In chemical formula (6) $R^9$ is selected one of amino group, (mono-alkyl-low)-amino group, low-acylamino group, guanidino group, low-alkyl-substituted guanidino group, imino low-alkyl group, ureide group, low-alkyl-substituted ureide group, low-alkylthio group, low-alkylsulfinyl group, low-alkylsulfonyl group, low-acyl group, and hydroxy low-alkyl group, wherein n is an integer of 1 thorough 4.

$AA^3$ is selected one of non-substituted phenylalanine residue, substituted phenylalanine residue, non-substituted D-phenylalanine residue and substituted D-phenylalanine residue.

$AA^4$ is an α-amino acid residue represented by chemical formula (7) below or a β-amino acid residue represented by chemical formula (8) below.

$$-N(R^{10})-CH(R^{11})-CO- \qquad (7)$$

$$-N(R^{10})-CH(R^{11})-CH(R^{12})-CO- \qquad (8)$$

In chemical formula (7) and (8) wherein $R^{10}$ and $R^{12}$ are respectively and independently selected one of hydrogen, low-alkyl group, low-alkenyl group, low-alkynyl group, aryl group, and aryl-substituted low-alkyl group; wherein $R^{11}$ is hydrogen atom or a group represented by chemical formula (9) below.

$$-Z-N(R^{13})-R^{14} \qquad (9)$$

In chemical formula (9) wherein Z is selected one of low-alkylene group, low-alkenylene group, and low-alkynylene group, and wherein $R^{13}$ and $R^{14}$ are respectively and independently selected one of hydrogen atom, low-alkyl group, aryl group, and aryl-substituted alkyl group, or $R^{13}$ and $R^{14}$ are five or six member nitrogen-containing heterocyclic group in which nitrogen atom is connected to $R^{13}$ and $R^{14}$.

The compound of the present invention comprises a non-substituted or substituted imino-low-alkyl group at the amino end, specifically represented as $[R^1N=C(R^2)-]$ in formula (1), and a non-substituted or substituted amide group at the carboxyl end, specifically represented as $[-N(R^3)R^4]$ in formula (2). It was not envisioned at all from related arts that the peptides in which the functional groups were introduced would have strong and lasting analgesic activity and anti-) nociceptive activity on oral and subcutaneous administration.

The preferred example of $R^1$ in chemical formula (1) is hydrogen but the present invention is not limited to the example. The preferred example of $R^2$ is low-alkyl group but the present invention is not limited to the example. The further preferred example of $R^2$ is methyl group or ethyl group.

The preferred example of $R^3$ and $R^4$ in chemical formula (2) representing Y are both hydrogen, or a combination of one hydrogen and one methyl group, and specifically the preferred example of Y is $-NH_2$ or $-NH-CH_3$ but the present invention is not limited to the example.

The position of $R^5$ and $R^6$ in the benzene ring in chemical formula (3) representing $AA^1$and the position of $R^{15}$ and $R^{16}$ in the benzene ring in chemical formula (10) or (11) representing $AA^1$ is not particularly specified.

The preferred example of $AA^1$ in chemical formula (1) is one of such tyrosine residue, 2,6-dimethyltyrosine residue, o-acyl-tyrosine residue, o-alkoxycarbonyl tyrosine residue, o-phenoxycarbonyl tyrosine residue, o-acetyltyrosine residue and 2,6-dimethylphenyl alanine, but the present invention is not limited to the example.

The preferred example of $AA^2$ in chemical formula (1) is one of such D-arginine residue, D-methionine sulfoxide group, D-$N^5$-acetyl ornithine residue, D-5-oxonor leucine residue, D-citrulline residue, and D-hydroxy-nor-leucine, but the present invention is not limited to the example.

$AA^3$ may be an α-amino acid residue or a D-α-amino acid residue represented as chemical formula (10) or (11).

[C7]

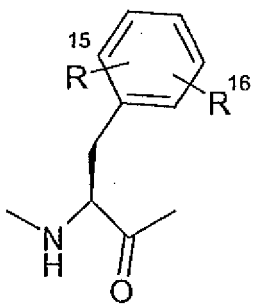

(10)

[C8]

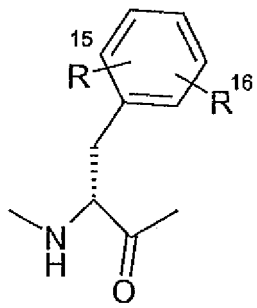

(11)

In formula (10) and (11) $R^{15}$ and $R^{16}$ are respectively and independently selected one of hydrogen atom, halogen atom, low-alkyl group, and halogenated low-alkyl group.

The preferred example of $AA^3$ in chemical formula (1) is one of such phenylalanine residue, o-trifluoromethylphenylalanine residue, 2,6-dimethylphenylalanine residue, D-phenylalanine residue, D-p-fluorophenylalanine residue, D-o-trifluoromethylphenylalanine residue, but the present invention is not limited to the example.

The preferred example of $AA^4$ in chemical formula (1) is one of such N-methyllysine residue and N-methyl-β-alanine residue, but the present invention is not limited to the example.

Analgesic activity and anti-nociceptive activity in the present description are employed in meaning pain relief. The different point of two activities is that the former is mainly used for human subject and the later is mainly used for experimental animal, and the two activities in the present description are used in exchangeable related to effects of the present invention.

If there are D-form and L-form for an amino acid or the residue thereof of the present description and the amino acid, and the residue thereof are not specified as D-form; the amino acid and the residue thereof are L-form.

The term "low" in "Low-alkyl", "low-alkoxyl", "low-acyl" and "low-alkylene" of the present description means that that each group includes 1, 2, 3, 4, 5 or 6 carbon(s). The term "low" in "Low-alkenyl", "low-alkynyl", "low-alkenylene" and "low-alkynylene" of the present description means that that each group includes 2, 3, 4, 5 or 6 carbons.

"Alkyl", "alkoxyl", "acryl", "alkylene", "alkenyl", "alkynyl", "alkenylene" and "alkynylene" may be either straight-chain isomer or branched-chain isomer. A branched-chain isomer may be either the branched-chain isomer including secondary carbon or the branched-chain isomer including tertiary carbon.

The preferred examples of the low-alkyl group noted above are methyl, ethyl, n-propyl, isopropyl, n-butyl, sec-butyl, tert-butyl, n-pentyl, neo-penty, n-hexyl but not limited to the examples. Further the preferred examples of $C_{1-16}$ alkyl group of such "$C_{1-16}$ alkyl", "hydroxy $C_{1-16}$ alkyl", "amino $C_{1-16}$ alkyl", "(mono-low-alkyl)amino $C_{1-16}$ alkyl", and "(di-low-alkyl)amino $C_{1-16}$ alkyl in the present description include straight-chain or branched-chain heptyl, octyl, nonyl, decyl, undecyl, dodecyl, tridecyl, tetradecyl, pentadcyl and hexadecyl in addition to the low-alkyl group noted above, but not limited to the examples. The preferred example of $C_{1-16}$ alkyl is $C_{6-12}$ alkyl group, further the preferred example of $C_{1-16}$ alkyl is $C_{8-10}$, and the most preferred example is straight-chain or branched-chain alkyl group of $C_8$ or $C_{10}$.

The "low-alkoxyl group" in the present description includes an alkoxyl group having 1, 2, 3, 4, 5 or 6 carbon atom(s). The preferred examples of the low-alkoxyl group noted above are methoxy and ethoxyl, but not limited to the examples.

"$C_{3-10}$ cycloalkyl group" of "$C_{3-10}$ cycloalkyl" and "cycloalkyl substituted $C_{3-10}$ low-alkyl group" is a cycloalkyl group having 3, 4, 5, 6, 7, 8, 9 or 10 carbon atoms. The preferred examples of $C_{3-10}$ alkyl group noted above are cyclopropyl, cyclobutyl, cyclopentyl, cyclohexyl and adamantyl, but not limited to the examples. Any carbon atom of the low-alkyl group of "$C_{3-10}$ cycloalkyl group of the cycloalkyl substituted $C_{3-10}$ low-alkyl group in the present description may be substituted and plural carbons may be substituted. For example, one or four, preferably one or two, and further preferably any carbon atom is substituted with one $C_{3-10}$ cycloalkyl group.

"$C_{2-16}$ alkenyl group" and "$C_{2-16}$ alkynyl group" are an alkenyl group and an alkynyl group, respectively, having respective carbon numbers are between 2 and 16, and include straight-chain type or branched-chain type. A position and/or number of double bond and triple bond are not specified. The preferred examples of the alkenyl group noted above are vinyl i.e. ethenyl, 2-propenyl, cis-1-propenyl, trans-1-propenyl group, but not limited to the examples. The preferred examples of the alkynyl group noted above are ethynyl, 2-propynyl, but the present invention is not limited to the examples.

The "heterocyclic group" in the present description includes saturated, unsaturated or partially saturated cyclic compound having selected at least one of groups comprising nitrogen atom, oxygen atom and sulfur atom, and carbon atoms. The preferred examples of the heterocyclic groups noted above are pyrazyl group, furanyl group and thiophenyl group, but not limited to the examples. $R^3$ and $R^4$ in chemical formula (2) and $R^{13}$ and $R^{14}$ in chemical formula (9) may construct a 5- or 6-member nitrogen-containing heterocyclic group with the nitrogen which is respectively connecting thereto. The nitrogen-containing heterocyclic group, e.g., may be 5- or 6-member saturated or unsaturated heterocyclic group including 1 or more than 2 nitrogen atoms constructing the ring. The preferred examples of the nitrogen-containing heterocyclic group noted above are pyrrolidino, piperidino, 3,4-dihydropyrrolidino and pyridinio group, but not limited to the examples.

"Aryl group" of "aryl group" and "aryl-substituted low-alkyl group" in the present description is an aromatic substitution group including 1 or more than 2 rings. The preferred examples of the aryl group noted above are phenyl group, naphthyl group, anthracenilyl group, phenanthryl group, but not limited to the examples. "Aryl group" of "aryl-substituted low-alkyl group" in the present description may substitute any carbon atom of low-alkyl group and also may substitute any number of carbon atoms. One or 4, e.g., preferably 1 or 2 and further preferable 1 aryl group may substitute any positioned carbon. The preferred examples of the aryl-substituted low-alkyl group noted above are benzyl group and phenethyl group, but not limited to the examples.

Both "low-acyl group" of "low-acyl group" and "low-acyl-amino group" in the present description is an acyl group having 1, 2, 3, 4, 5 or 6 carbon atom(s), i.e. alkanoacyl group. The preferred examples of the alkanoacyl group noted above are formyl group and acetyl group, but not limited to the examples."Low-acyl-amino group" of "low-acyl-amino group" in the present basic description is a group, in which one of hydrogen is or both hydrogens of amino group are substituted. The preferred examples of the acylamino group noted above is menoacetylamino group and diacetylyamino group, but not limited to the examples.

Halogen in the present description may be any selected one of fluoride, chloride, bromide or iodide. The position, number and kind of halogen substituted to halogenated low-alkyl group are not specifically limited, and thus any halogenated alkyl group in the range of from mono-halogenated low-alkyl group to per-halogenated low-alkyl group may be applied. If there is more than 2 halogen atoms, these may be the same or different. The preferred examples of the halogenated low-alkyl group noted above are chloromethyl group, bromomethyl group, fluoromethyl group, trifluoromethyl group, 2,2,2-triflurometthyl group, but not limited to the examples.

"Hydroxy group" of "hydroxy low-alkyl group" and "hydroxy $C_{1-16}$ alkyl group" in the present description may substitute any position of carbon atom of alkyl group and any number of carbon atoms. One or 4, e.g., preferably 1 or 2 and further preferably 1 hydroxy group may substitute any positioned carbon. The preferred examples of "hydroxy low-alkyl group" and "hydroxy $C_{1-16}$ alkyl group" noted above are hydroxymethyl group and 2-hydroxymethyl group, but not limited to the examples.

"Amino group" of "amino $C_{1-16}$, alkyl group" in the present description may substitute any position of carbon atom of alkyl group and any number of carbon atoms. One or 4, e.g., preferably 1 or 2 and further preferably 1 amino group may substitute any positioned carbon. The preferred examples of "amino $C_{1-16}$ alkyl group" noted above are aminomethyl and 2 aminoethyl, but not limited to the examples.

"(Mono-low-alkyl)-amino group" of "(mono-low-alkyl)-amino group" and "(mono-low-alkyl)-amino $C_{1-16}$ alkyl group" in the present description is a group in which respective alkyl group having 1, 2, 3, 4, 5 or 6 carbon atom(s) substitutes a hydrogen of the amino group. The preferred examples of "(mono-low-alkyl)-amino group" and ""(mono-low-alkyl)-amino $C_{1-16}$ alkyl group" of "(mono-low-alkyl)-amino group" noted above are methylamino group and ethylamino group, but not limited to the examples. "(Di-low-alkyl)-amino group" of "(di-low-alkyl)-amino $C_{1-16}$ alkyl group" in the present description is a group in which respective alkyl group having 1, 2, 3, 4, 5 or 6 carbon atom(s) substitutes a hydrogen of the amino group. The low-alkyl group substituting respective hydrogen atom may be the same alkyl group or the different alkyl group. The examples of "(di-low-alkyl)-amino group" noted above is dimethylamino group and diethylamino group, but not limited to the examples. "(Mono-low-alkyl)-amino group" may substitute any carbon atom of $C_{1-16}$ alkyl group and the amino group is substituted with one low-alkyl group noted above. The preferred examples of "(mono-low-alkyl)-amino $C_{1-16}$ alkyl group" noted above is e.g. 3-(methylamino)-n-propyl group and 2-(ethylamino)-n-pentyl group, but not limited to the examples. "(Di-low-alkyl)-amino group" may substitute any carbon atom of $C_{1-16}$ alkyl group and the amino group is substituted with two low-alkyl groups noted above. The preferred examples of "(di-low-alkyl)amino $C_{1-16}$ alkyl group" noted above is e.g. 3-(dimethylamino)ethyl group and 2-(diethylamino)ethyl group, but not limited to the examples.

$R^1$ of the compound of the present invention in chemical formula (1) is hydrogen atom and $R^2$ is methyl group; and if Y is $-NH_2$, the compound is represented as 1-iminoethyl-$AA^1$-$AA^2$-$AA^3$-$AA^4$-amido or 1-iminoethyl-$AA^1$-$AA^2$-$AA^3$-$M^4$-$NH_2$. Both $R^1$ and $R^2$ of the compound of the present invention in chemical formula (1) are hydrogen atoms and if Y is $-NH_2$, the compound is represented as 1-iminomethyl-$AA^1$-$AA^2$-$AA^3$-$AA^4$-amido or 1-iminomethyl-$AA^1$-$AA^2$-$AA^3$-$AA^4$-$NH_2$. $R^1$ of the compound of the present invention in chemical formula (1) is hydrogen atom and $R^2$ is methyl group; and if Y is $-NH-CH_3$, the compound is represented as 1-iminoethyl-$AA^1$-$AA^2$-$AA^3$-$AA^4$-methylamido or

1-iminoethyl-$AA^1$-$AA^2$-$AA^3$-$AA^4$-NH-$CH_3$. If there is no "$R^1N=C(R^2)$-", "iminomethyl" or "1-iminoethyl" at the left end of the peptide derivative of the present description, an amino group of the amino acid of the amino end is not substituted.

The term of "amino acid residue" is generally used in the field of peptide chemistry, and more specifically it is the residual structure of α-amino acid without respective the hydrogen atom and the hydroxy group, in the amino group and the carboxyl group located on α-position of α-amino acid; or on β-position of β-amino acid. The notation of amino acid is directed to Seikagaku Jiten (version 3, issued October 8, 1998; Tokyo Kagaku Doujin), WIPO Standard ST. 25, and the "Guideline for Notation of Base Sequence or Amino Acid Sequence in the Description" published by Japanese Patent Office, July 2002. If D-amino acid is included, it will be specified. Specifically, if the number i amino acid residue represented as "-$AA_i$-" (i is an integer of 1 through 4 is tyrosine residue, it is expressed as "-[Tyr]-"; if D-arginine, it is expressed as "-[D-Arg]-", having a short form with three alphabetical letters; if it is a modified amino acid residue e.g. 2,6-dimethyltyrosine residue, it is expressed as "-[2,6-dimethyl-Tyr]-"; if N-methyllysine, it is expressed as "-[N-MeLys]-"; if N-methyl β-alanine residue, it is expressed as "-[N-MeβAla]-"; if D-methionine sulfoxide residue, it is expressed as "-[D-Met(O)]-".

N-Methyllysine residue or N-MeLys in the description is the structure having chemical formula (12) below.

[C9]

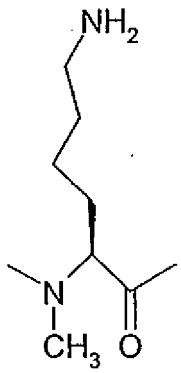

(12)

N-Methyl-β-alanine residue or N-MeβAla in the description is the structure having chemical formula (13) below.

[C10]

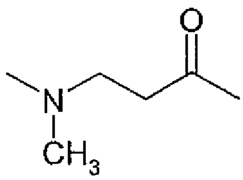

(13)

N-Methionine sulfoxide residue or D-Met(O) in the description is the structure having chemical formula (14) below.

[C11]

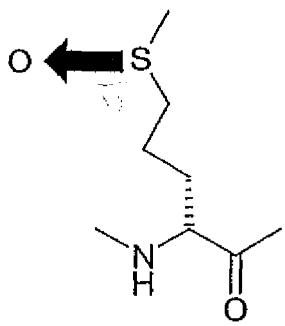

(14)

The compound of the present invention may have imino ethyl group or imino propyl group. The compound of the present invention may comprise; $R^1$ which is hydrogen atom and $R^2$ which is methyl or ethyl group in chemical formula (1); $R^3$ and $R^4$ in chemical formula (2) which are respectively and independently selected one of groups including hydrogen atom, hydroxyl group, low-alkyl group, low-alkoxyl group, and five or six member nitrogen-containing heterocyclic group in which the nitrogen atom is connected to $R^3$ and $R^4$; $R^5$ and $R^6$ in formula (3) which are respectively and independently selected one of groups including hydrogen atom, halogen atom, low-alkyl group, and halogenated low-alkyl group; X in chemical formula (3) which is selected one of groups including hydrogen atom, halogen atom, hydroxyl group, a group represented by chemical formula (4) and a group represented by chemical formula (5); $R^7$ and $R^8$ in chemical formula (4) and in chemical formula (5) which are respectively and independently selected one of groups including $C_{1-16}$ alkyl group, hydroxy-$C_{1-16}$ alkyl group, amino- $C_{1-16}$ alkyl group, (mono-low-alkyl)amino-$C_{1-16}$ alkyl group, (di-low-alkyl)amino- $C_{1-16}$ alkyl group, $C_{3-10}$ cycloalkyl group, $C_{3-10}$ cycloalkyl substituted low-alkyl group, $C_{2-16}$ alkenyl group, $C_{2-16}$ alkynyl group, aryl group, aryl-substituted low-alkyl group and heterocyclic group; $R^9$ in chemical formula (6) which is selected one of groups including amino group, (mono-low-alkyl)amino group, low-acylamino group, guanidino group, low-alkyl-substituted guanidino group, imino low-alkyl group, ureide group, low-alkyl-substituted ureide group, low-alkylthio group, low-alkylsulfinyl group, low-alkylsulfonyl group, low-acyl group, and hydroxy-low-alkyl group; n in chemical formula (6) which is an integer of 1 thorough 4; $AA^3$ chemical formula (1) which is non-substituted phenylalanine residue or substituted phenylalanine residue, non-substituted D-phenylalanine residue or substituted D-phenylalanine residue; $R^{10}$ and $R^{12}$ in chemical formula (7) and (8) which are respectively and independently selected one of groups including hydrogen, low-alkyl group, low-alkenyl group, low-alkynyl group, $C_{6-10}$-aryl group, and aryl-substituted-low-alkyl group; and $R^{11}$ in chemical formula (7) and (8) which is hydrogen atom or a group represented by chemical formula (9); Z in chemical formula (9) which is selected one of groups including low-alkylene group, low-alkenylene group, and low-alkynylene group; $R^{13}$ and $R^{14}$ in chemical formula (9) which are respectively and independently selected one of groups including hydrogen atom, low-alkyl group, aryl group, and aryl-substituted low-alkyl group, or $R^{13}$ and $R^{14}$ which are five or six member nitrogen-containing heterocyclic group in which nitrogen atom is connected to $R^{13}$ and $R^{14}$. The present invention may provide these compounds and pharmacologically acceptable salt thereof.

The compound of the present invention may have amide group or methyl amide group at the carboxyl end. The compound of the present invention may comprise; $R^1$ in chemical formula (1) which is selected one of groups including hydrogen atom, hydroxy group, low-alkyl group and low-alkoxyl group; and $R^2$ in chemical formula (1) which is selected one of groups including low-alkyl group; $R^3$ and $R^4$ in chemical formula (2) which are both hydrogen atoms, or one hydrogen and one methyl group; respectively and independently selected one of groups including hydrogen atom, hydroxyl group, low-alkyl group, low-alkoxyl group, and five or six member nitrogen-containing heterocyclic group in which the nitrogen atom is connected to $R^3$ and $R^4$; X in chemical formula (3) which is selected one of groups including hydrogen atom, halogen atom, hydroxyl group, a group represented by chemical formula (4) and a group represented by chemical formula (5); $R^5$ and $R^6$ in formula (3) which are respectively and independently selected one of groups including hydrogen atom, halogen atom, low-alkyl group, and halogenated low-alkyl group; $R^7$ and $R^8$ in chemical formula (4) and in chemical formula (5) which are respectively and independently selected one of groups including $C_{1-16}$ alkyl group, hydroxy-$C_{1-16}$ alkyl group, amino- $C_{1-16}$ alkyl group, (mono-low-alkyl)amino-$C_{1-16}$ alkyl group, (di-low-alkyl)amino-$C_{1-16}$ alkyl group, $C_{3-10}$ cycloalkyl group, $C_{3-10}$ cycloalkyl substituted-low-alkyl group, $C_{2-16}$ alkenyl group, $C_{2-16}$ alkynyl group, aryl group, aryl-substituted low-alkyl group and heterocyclic group; $R^9$ in chemical formula (6) which is selected one of groups including amino group, (mono-low-alkyl)amino group, low-acylamino group, guanidino group, low-alkyl-substituted guanidino group, imino low-alkyl group, ureide group, low-alkyl-substituted ureide group, low-alkylthio group, low-alkylsulfinyl group, low-alkylsulfonyl group, low-acyl group, and hydroxy-low-alkyl group; n in chemical formula (6) which is an integer of 1 thorough 4; $AA^3$ chemical formula (1) which is non-substituted phenylalanine residue or substituted

phenylalanine residue, non-substituted D-phenylalanine residue or substituted D-phenylalanine residue; $R^{10}$ and $R^{12}$ in chemical formula (7) and (8) which are respectively and independently selected one of groups including hydrogen, low-alkyl group, low-alkenyl group, low-alkynyl group, $C_{6-10}$-aryl group, and aryl-substituted-low-alkyl group; and $R^{11}$ in chemical formula (7) and (8) which is hydrogen atom or a group represented by chemical formula (9); Z in chemical formula (9) which is selected one of groups including low-alkylene group, low-alkenylene group, and low-alkynylene group; $R^{13}$ and $R^{14}$ in chemical formula (9) which are respectively and independently selected one of groups including hydrogen atom, low-alkyl group, aryl group, and aryl-substituted-low-alkyl group, or $R^{13}$ and $R^{14}$ which are five or six member nitrogen-containing heterocyclic group in which nitrogen atom is connected to $R^{13}$ and $R^{14}$. The present invention may provide these compounds and pharmacologically acceptable salt thereof.

The compound of the present invention may have X in chemical formula (3) which is hydroxy group. Specifically, the compound of the present invention may comprise; $R^1$ chemical formula (1) which is selected one of groups including hydrogen atom, hydroxy group, low-alkyl group and low-alkoxyl group; and $R^2$ in chemical formula (1) selected one of groups including low-alkyl group; $R^3$ and $R^4$ in chemical formula (2) which are respectively and independently selected one of groups including hydrogen atom, hydroxyl group, low-alkyl group, low-alkoxyl group, and five or six member nitrogen-containing heterocyclic group in which the nitrogen atom is connected to $R^3$ and $R^4$; X in chemical formula (3) which is hydroxyl group; $R^5$ and $R^6$ in formula (3) which are respectively and independently selected one of groups including hydrogen atom, halogen atom, low-alkyl group, and halogenated low-alkyl group; $R^9$ in chemical formula (6) which is selected one of groups including amino group, (mono-low-alkyl)amino group, low-acylamino group, guanidino group, low-alkyl-substituted guanidino group, imino low-alkyl group, ureide group, low-alkyl-substituted ureide group, low-alkylthio group, low-alkylsulfinyl group, low-alkylsulfonyl group, low-acyl group, and hydroxy-low-alkyl group; n in chemical formula (6) which is an integer of 1 thorough 4; $AA^3$ chemical formula (1) which is non-substituted phenylalanine residue or substituted phenylalanine residue, non-substituted D-phenylalanine residue or substituted D-phenylalanine residue; $R^{10}$ and $R^{12}$ in chemical formula (7) and (8) which are respectively and independently selected one of groups including hydrogen, low-alkyl group, low-alkenyl group, low-alkynyl group, $C_{6-10}$-aryl group, and aryl-substituted-low-alkyl group; and $R^{11}$ in chemical formula (7) and (8) which is hydrogen atom or a group represented by chemical formula (9); Z in chemical formula (9) which is selected one of groups including low-alkylene group, low-alkenylene group, and low-alkynylene group; $R^{13}$ and $R^{14}$ in chemical formula (9) which are respectively and independently selected one of groups including hydrogen atom, low-alkyl group, aryl group, and aryl-substituted-low-alkyl group, or $R^{13}$ and $R^{14}$ which are five or six member nitrogen-containing heterocyclic group in which nitrogen atom is connected to $R^{13}$ and $R^{14}$. The present invention may provide these compounds and pharmacologically acceptable salt thereof.

The compound of the present invention may have X in chemical formula (3) which is hydrogen atom or halogen atom. Specifically, the compound of the present invention may comprise; $R^1$ chemical formula (1) which is selected one of groups including hydrogen atom, hydroxy group, low-alkyl group and low-alkoxyl group; and $R^2$ in chemical formula (1) which is selected one of groups including low-alkyl group; $R^3$ and $R^4$ in chemical formula (2) which are respectively and independently selected one of groups including hydrogen atom, hydroxyl group, low-alkyl group, low-alkoxyl group, and five or six member nitrogen-containing heterocyclic group in which the nitrogen atom is connected to $R^3$ and $R^4$; X in chemical formula (3) which is hydrogen atom or halogen atom; $R^5$ and $R^6$ in formula (3) which are respectively and independently selected one of groups including hydrogen atom, halogen atom, low-alkyl group, and halogenated low-alkyl group; $R^9$ in chemical formula (6) which is selected one of groups including amino group, (mono-low-alkyl)amino group, low-acylamino group, guanidino group, low-alkyl-substituted guanidino group, imino low-alkyl group, ureide group, low-alkyl-substituted ureide group, low-alkylthio group, low-alkylsulfinyl group, low-alkylsulfonyl group, low-acyl group, and hydroxy-low-alkyl group; n in chemical formula (6) which is an integer of 1 thorough 4; $AA^3$ chemical formula (1) which is non-substituted phenylalanine residue or substituted phenylalanine residue, non-substituted D-phenylalanine residue or substituted D-phenylalanine residue; $R^{10}$ and $R^{12}$ in chemical formula (7) and (8) which are respectively and independently selected one of groups including hydrogen, low-alkyl group, low-alkenyl group, low-alkynyl group, $C_{6-10}$-aryl group, and aryl-substituted-low-alkyl group; and $R^{11}$ in chemical formula (7) and (8) which is hydrogen atom or a group represented by chemical formula (9); Z in chemical formula (9) which is selected one of groups including low-alkylene group, low-alkenylene group, and low-alkynylene group; $R^{13}$ and $R^{14}$ in chemical formula (9) which are respectively and independently selected one of groups including hydrogen atom, low-alkyl group, aryl group, and aryl-substituted-low-alkyl group, or $R^{13}$ and $R^{14}$ which are five or six member nitrogen-containing heterocyclic group in which nitrogen atom is connected to $R^{13}$ and $R^{14}$. The present invention may provide these compounds and pharmacologically acceptable salt thereof.

The compound of the present invention may have $AA^1$ in chemical formula (1) which is tyrosine residue or dimethyl tyrosine residue. Specifically, the compound of the present invention may comprise; $R^1$ chemical formula (1) which is selected one of groups including hydrogen atom, hydroxy group, low-alkyl group and low-alkoxyl group; and $R^2$ in chemical formula (1) is which selected one of groups including low-alkyl group; $R^3$ and $R^4$ in chemical formula (2) which are respectively and independently selected one of groups including hydrogen atom, hydroxyl group, low-alkyl group, low-alkoxyl group, and five or six member nitrogen-containing heterocyclic group in which the nitrogen atom is

connected to $R^3$ and $R^4$; $AA^1$ in chemical formula (1) which is tyrosine residue or dimethyl tyrosine residue; $R^9$ in chemical formula (6) which is selected one of groups including amino group, (mono-low-alkyl)amino group, low-acylamino group, guanidino group, low-alkyl-substituted guanidino group, imino low-alkyl group, ureide group, low-alkyl-substituted ureide group, low-alkylthio group, low-alkylsulfinyl group, low-alkylsulfonyl group, low-acyl group, and hydroxy-low-alkyl group; n in chemical formula (6) which is an integer of 1 thorough 4; $AA^3$ chemical formula (1) which is non-substituted phenylalanine residue or substituted phenylalanine residue, non-substituted D-phenylalanine residue or substituted D-phenylalanine residue; $R^{10}$ and $R^{12}$ in chemical formula (7) and (8) which are respectively and independently selected one of groups including hydrogen, low-alkyl group, low-alkenyl group, low-alkynyl group, $C_{6-10}$-aryl group, and aryl-substituted-low-alkyl group; and $R^{11}$ in chemical formula (7) and (8) which is hydrogen atom or a group represented by chemical formula (9); Z in chemical formula (9) which is selected one of groups including low-alkylene group, low-alkenylene group, and low-alkynylene group; $R^{13}$ and $R^{14}$ in chemical formula (9) which are respectively and independently selected one of groups including hydrogen atom, low-alkyl group, aryl group, and aryl-substituted-low-alkyl group, or $R^{13}$ and $R^{14}$ which are five or six member nitrogen-containing heterocyclic group in which nitrogen atom is connected to $R^{13}$ and $R^{14}$. The present invention may provide these compounds and pharmacologically acceptable salt thereof.

The compound of the present invention may have $AA^1$ in chemical formula (1) which is o-acyltyrosine residue, o-alkoxycarbonyl tyrosine residue, o-phenoxycarbony tyrosine residue, o-acetyltyrosine residue, or 2,6-dimethylphenyl alanine residue. Specifically, the compound of the present invention may comprise; $R^1$ chemical formula (1) which is selected one of groups including hydrogen atom, hydroxy group, low-alkyl group and low-alkoxyl group; and $R^2$ in chemical formula (1) which is selected one of groups including low-alkyl group; $R^3$ and $R^4$ in chemical formula (2) which are respectively and independently selected one of groups including hydrogen atom, hydroxyl group, low-alkyl group, low-alkoxyl group, and five or six member nitrogen-containing heterocyclic group in which the nitrogen atom is connected to $R^3$ and $R^4$; $AA^1$ in chemical formula (1) which is o-acyltyrosine residue, o-alkoxycarbonyl tyrosine residue, o-phenoxycarbony tyrosine residue, o-acetyltyrosine residue, or 2,6-dimethylphenyl alanine residue; $R^9$ in chemical formula (6) which is selected one of groups including amino group, (mono-low-alkyl)amino group, low-acylamino group, guanidino group, low-alkyl-substituted guanidino group, imino low-alkyl group, ureide group, low-alkyl-substituted ureide group, low-alkylthio group, low-alkylsulfinyl group, low-alkylsulfonyl group, low-acyl group, and hydroxy-low-alkyl group; n in chemical formula (6) which is an integer of 1 thorough 4; $AA^3$ chemical formula (1) which is non-substituted phenylalanine residue or substituted phenylalanine residue, non-substituted D-phenylalanine residue or substituted D-phenylalanine residue; $R^{10}$ and $R^{12}$ in chemical formula (7) and (8) which are respectively and independently selected one of groups including hydrogen, low-alkyl group, low-alkenyl group, low-alkynyl group, $C_{6-10}$-aryl group, and aryl-substituted-low-alkyl group; and $R^{11}$ in chemical formula (7) and (8) which is hydrogen atom or a group represented by chemical formula (9); Z in chemical formula (9) which is selected one of groups including low-alkylene group, low-alkenylene group, and low-alkynylene group; $R^{13}$ and $R^{14}$ in chemical formula (9) which are respectively and independently selected one of groups including hydrogen atom, low-alkyl group, aryl group, and aryl-substituted-low-alkyl group, or $R^{13}$ and $R^{14}$ which are five or six member nitrogen-containing heterocyclic group in which nitrogen atom is connected to $R^{13}$ and $R^{14}$. The present invention may provide these compounds and pharmacologically acceptable salts thereof.

The compound of the present invention may have $AA^2$ in chemical formula (1) which is D-arginine residue or D-methionine sulfoxide residue. Specifically, the compound of the present invention may comprise; $R^1$ chemical formula (1) which is selected one of groups including hydrogen atom, hydroxy group, low-alkyl group and low-alkoxyl group; and $R^2$ in chemical formula (1) which is selected one of groups including low-alkyl group; $R^3$ and $R^4$ in chemical formula (2) which are respectively and independently selected one of groups including hydrogen atom, hydroxyl group, low-alkyl group, low-alkoxyl group, and five or six member nitrogen-containing heterocyclic group in which the nitrogen atom is connected to $R^3$ and $R^4$; X in chemical formula (3) which is hydrogen atom, halogen atom or hydroxy group, or the functional group represented by chemical formula (4) or chemical formula (5); $R^5$ and $R^6$ in formula (3) which are respectively and independently selected one of groups including hydrogen atom, halogen atom, low-alkyl group, and halogenated low-alkyl group; $R^7$ and $R^8$ in chemical formula (4) and in chemical formula (5) which are respectively and independently selected one of groups including $C_{1-16}$ alkyl group, hydroxy-$C_{1-16}$ alkyl group, amino-$C_{1-16}$ alkyl group, (mono-low-alkyl)amino-$C_{1-16}$ alkyl group, (di-low-alkyl)amino-$C_{1-16}$ alkyl group, $C_{3-10}$ cycloalkyl group, $C_{3-10}$ cycloalkyl substituted-low-alkyl group, $C_{2-16}$ alkenyl group, $C_{2-16}$ alkynyl group, aryl group, aryl-substituted low-alkyl group and heterocyclic group $AA^2$ in chemical formula (1) which is D-arginine residue or D-methionine sulfoxide residue; $AA^3$ chemical formula (1) which is non-substituted phenylalanine residue or substituted phenylalanine residue, non-substituted D-phenylalanine residue or substituted D-phenylalanine residue; $R^{10}$ and $R^{12}$ in chemical formula (7) and (8) which are respectively and independently selected one of groups including hydrogen, low-alkyl group, low-alkenyl group, low-alkynyl group, $C_{6-10}$-aryl group, and aryl-substituted-low-alkyl group; and $R^{11}$ in chemical formula (7) and (8) which is hydrogen atom or a group represented by chemical formula (9); Z in chemical formula (9) which is selected one of groups including low-alkylene group, low-alkenylene group, and low-alkynylene group; $R^{13}$ and $R^{14}$ in chemical formula (9) which are respectively and independently selected one of groups including hydrogen atom, low-alkyl group, aryl group, and aryl-substituted-low-alkyl group, or $R^{13}$ and $R^{14}$ which are five or six member nitrogen-containing heterocyclic group

in which nitrogen atom is connected to $R^{13}$ and $R^{14}$. The present invention may provide these compounds and pharmacologically acceptable salts thereof.

The compound of the present invention may have $AA^2$ in chemical formula (1) which is D-$N^5$-acetylornithine residue or D-5-oxonorleucine residue. D-citrulline residue or D-5-hydroxy-norleucine. Specifically, the compound of the present invention may comprise; $R^1$ chemical formula (1) which is selected one of groups including hydrogen atom, hydroxy group, low-alkyl group and low-alkoxyl group; and $R^2$ in chemical formula (1) which is selected one of groups including low-alkyl group; $R^3$ and $R^4$ in chemical formula (2) which are respectively and independently selected one of groups including hydrogen atom, hydroxyl group, low-alkyl group, low-alkoxyl group, and five or six member nitrogen-containing heterocyclic group in which the nitrogen atom is connected to $R^3$ and $R^4$; X in chemical formula (3) which is hydrogen atom, halogen atom or hydroxy group, or the functional group represented by chemical formula (4) or chemical formula (5); $R^5$ and $R^6$ in formula (3) which are respectively and independently selected one of groups including hydrogen atom, halogen atom, low-alkyl group, and halogenated low-alkyl group; $R^7$ and $R^8$ in chemical formula (4) and in chemical formula (5) which are respectively and independently selected one of groups including $C_{1-16}$ alkyl group, hydroxy-$C_{1-16}$ alkyl group, amino-$C_{1-16}$ alkyl group, (mono-low-alkyl)amino-$C_{1-16}$ alkyl group, (di-low-alkyl)amino-$C_{1-16}$ alkyl group, $C_{3-10}$ cycloalkyl group, $C_{3-10}$ cycloalkyl substituted-low-alkyl group, $C_{2-16}$ alkenyl group, $C_{2-16}$ alkynyl group, aryl group, aryl-substituted low-alkyl group and heterocyclic group; $AA^2$ in chemical formula (1) which is D-$N^5$-acetylarnithine residue or D-5-oxonorleucine residue. D-citrulline residue or D-5-hydroxy-norleucirie; $AA^3$ chemical formula (1) which is non-substituted phenylalanine residue or substituted phenylalanine residue, non-substituted D-phenylalanine residue or substituted D-phenylalanine residue; $R^{10}$ and $R^{12}$ in chemical formula (7) and (8) which are respectively and independently selected one of groups including hydrogen, low-alkyl group, low-alkenyl group, low-alkynyl group, $C_{6-10}$-aryl group, and aryl-substituted-low-alkyl group; and $R^{11}$ in chemical formula (7) and (8) which is hydrogen atom or a group represented by chemical formula (9); Z in chemical formula (9) which is selected one of groups including low-alkylene group, low-alkenylene group, and low-alkynylene group; $R^{13}$ and $R^{14}$ in chemical formula (9) which are respectively and independently selected one of groups including hydrogen atom, low-alkyl group, aryl group, and aryl-substituted-low-alkyl group, or $R^{13}$ and $R^{14}$ which are five or six member nitrogen-containing heterocyclic group in which nitrogen atom is connected to $R^{13}$ and $R^{14}$. The present invention may provide these compounds and pharmacologically acceptable salts thereof.

The compound of the present invention may have $AA^3$ in chemical formula (1) which is phenylalanine. Specifically, the compound of the present invention may comprise; $R^1$ chemical formula (1) which is selected one of groups including hydrogen atom, hydroxy group, low-alkyl group and low-alkoxyl group; and $R^2$ in chemical formula (1) which is selected one of groups including low-alkyl group; $R^3$ and $R^4$ in chemical formula (2) which are respectively and independently selected one of groups including hydrogen atom, hydroxyl group, low-alkyl group, low-alkoxyl group, and five or six member nitrogen-containing heterocyclic group in which the nitrogen atom is connected to $R^3$ and $R^4$; X in chemical formula (3) which is hydrogen atom, halogen atom or hydroxy group, or the functional group represented by chemical formula (4) or chemical formula (5); $R^5$ and $R^6$ in formula (3) which are respectively and independently selected one of groups including hydrogen atom, halogen atom, low-alkyl group, and halogenated low-alkyl group; $R^7$ and $R^8$ in chemical formula (4) and in chemical formula (5) which are respectively and independently selected one of groups including $C_{1-16}$ alkyl group, hydroxy-$C_{1-16}$ alkyl group, amino-$C_{1-16}$ alkyl group, (mono-low-alkyl)amino-$C_{1-16}$ alkyl group, (di-low-alkyl)amino-$C_{1-16}$ alkyl group, $C_{3-10}$ cycloalkyl group, $C_{3-10}$ cycloalkyl substituted-low-alkyl group, $C_{2-16}$ alkenyl group, $C_{2-16}$ alkynyl group, aryl group, aryl-substituted low-alkyl group and heterocyclic group; $AA^2$ in chemical formula (1) which is D-$N^5$-acetylornithine residue or D-5-oxonorleucine residue. D-citrulline residue or D-5-hydroxy-norleucine; $R^9$ in chemical formula (6) which is selected one of groups including amino group, (mono-low-alkyl)amino group, low-acylamino group, guanidino group, low-alkyl-substituted guanidino group, imino low-alkyl group, ureide group, low-alkyl-substituted ureide group, low-alkylthio group, low-alkylsulfinyl group, low-alkylsulfonyl group, low-acyl group, and hydroxy-low-alkyl group; n in chemical formula (6) which is an integer of 1 thorough 4; $AA^3$ chemical formula (1) which is non-substituted phenylalanine residue; $R^{10}$ and $R^{12}$ in chemical formula (7) and (8) which are respectively and independently selected one of groups including hydrogen, low-alkyl group, low-alkenyl group, low-alkynyl group, $C_{6-10}$-aryl group, and aryl-substituted-low-alkyl group; and $R^{11}$ in chemical formula (7) and (8) which is hydrogen atom or a group represented by chemical formula (9); Z in chemical formula (9) which is selected one of groups including low-alkylene group, low-alkenylene group, and low-alkynylene group; $R^{13}$ and $R^{14}$ in chemical formula (9) which are respectively and independently selected one of groups including hydrogen atom, low-alkyl group, aryl group, and aryl-substituted-low-alkyl group, or $R^{13}$ and $R^{14}$ which are five or six member nitrogen-containing heterocyclic group in which nitrogen atom is connected to $R^{13}$ and $R^{14}$. The present invention may provide these compounds and pharmacologically acceptable salts thereof.

The compound of the present invention may have $AA^3$ in chemical formula (1) which is p-fluoroalanine residue, o-trifluroromethylphenyl alanine residue, 2,6-dimentylphenylalanine residue, D-phenylalanine residue, D-p-fluorophenylalanine residue or D-o-trifluoromethylphenyl alanine. Specifically, the compound of the present invention may comprise; $R^1$ chemical formula (1) which is selected one of groups including hydrogen atom, hydroxy group, low-alkyl group

and low-alkoxyl group; and $R^2$ in chemical formula (1) which is selected one of groups including low-alkyl group; $R^3$ and $R^4$ in chemical formula (2) which are respectively and independently selected one of groups including hydrogen atom, hydroxyl group, low-alkyl group, low-alkoxyl group, and five or six member nitrogen-containing heterocyclic group in which the nitrogen atom is connected to $R^3$ and $R^4$; X in chemical formula (3) which is hydrogen atom, chloride, fluoride or hydroxy group, or the functional group represented by chemical formula (4) or chemical formula (5); $R^5$ and $R^6$ in formula (3) which are respectively and independently selected one of groups including hydrogen atom, halogen atom, low-alkyl group, and halogenated low-alkyl group; $R^7$ and $R^8$ in chemical formula (4) and in chemical formula (5) which are respectively and independently selected one of groups including $C_{1-16}$ alkyl group, hydroxy-$C_{1-16}$ alkyl group, amino-$C_{1-16}$ alkyl group, (mono-low-alkyl)amino-$C_{1-16}$ alkyl group, (di-low-alkyl)amino-$C_{1-16}$ alkyl group, $C_{3-10}$ cycloalkyl group, $C_{3-10}$ cycloalkyl substituted-low-alkyl group, $C_{2-16}$ alkenyl group, $C_{2-16}$ alkynyl group, aryl group, aryl-substituted low-alkyl group and heterocyclic group; $R^9$ in chemical formula (6) which is selected one of groups including amino group, (mono-low-alkyl)amino group, low-acylamino group, guanidino group, low-alkyl-substituted guanidino group, imino low-alkyl group, ureide group, low-alkyl-substituted ureide group, low-alkylthio group, low-alkylsulfinyl group, low-alkylsulfonyl group, low-acyl group, and hydroxy-low-alkyl group; n in chemical formula (6) which is an integer of 1 thorough 4; $AA^3$ chemical formula (1) which is D-phenylalanine residue, p-fluoroalanine residue, o-trifluroromethylphenyl alanine residue, 2,6-dimentylphenylalanine residue, D-p-fluorophenylalanine residue or D-o-trifluoromethylphenyl alanine; $R^{10}$ and $R^{12}$ in chemical formula (7) and (8) which are respectively and independently selected one of groups including hydrogen, low-alkyl group, low-alkenyl group, low-alkynyl group, $C_{6-10}$-aryl group, and aryl-substituted-low-alkyl group; and $R^{11}$ in chemical formula (7) and (8) which is hydrogen atom or a group represented by chemical formula (9); Z in chemical formula (9) which is selected one of groups including low-alkylene group, low-alkenylene group, and low-alkynylene group; $R^{13}$ and $R^{14}$ in chemical formula (9) which are respectively and independently selected one of groups including hydrogen atom, low-alkyl group, aryl group, and aryl-substituted-low-alkyl group, or $R^{13}$ and $R^{14}$ which are five or six member nitrogen-containing heterocyclic group in which nitrogen atom is connected to $R^{13}$ and $R^{14}$. The present invention may provide these compounds and pharmacologically acceptable salts thereof.

The compound of the present invention may have $AA^4$ in chemical formula (1) which is N-methyllysine residue or n-methyl-β-alanine residue. Specifically, the compound of the present invention may comrise; $R^1$ chemical formula (1) which is selected one of groups including hydrogen atom, hydroxy group, low-alkyl group and low-alkoxyl group; and $R^2$ in chemical formula (1) which is selected one of groups including low-alkyl group; $R^3$ and $R^4$ in chemical formula (2) which are respectively and independently selected one of groups including hydrogen atom, hydroxyl group, low-alkyl group, low-alkoxyl group, and five or six member nitrogen-containing heterocyclic group in which the nitrogen atom is connected to $R^3$ and $R^4$; X in chemical formula (3) which is hydrogen atom, chloride, fluoride or hydroxy group, or the functional group represented by chemical formula (4) or chemical formula (5); $R^5$ and $R^6$ in formula (3) which are respectively and independently selected one of groups including hydrogen atom, halogen atom, low-alkyl group, and halogenated low-alkyl group; $R^7$ and $R^8$ in chemical formula (4) and in chemical formula (5) which are respectively and independently selected one of groups including $C_{1-16}$ alkyl group, hydroxy-$C_{1-16}$ alkyl group, amino-$C_{1-16}$ alkyl group, (mono-low-alkyl)amino-$C_{1-16}$ alkyl group, (di-low-alkyl)amino-$C_{1-16}$ alkyl group, $C_{3-10}$ cycloalkyl group, $C_{3-10}$ cycloalkyl substituted-low-alkyl group, $C_{2-16}$ alkenyl group, $C_{2-16}$ alkynyl group, aryl group, aryl-substituted low-alkyl group and heterocyclic group; $R^9$ in chemical formula (6) which is selected one of groups including amino group, (mono-low-alkyl) amino group, low-acylamino group, guanidino group, low-alkyl-substituted guanidino group, imino low-alkyl group, ureide group, low-alkyl-substituted ureide group, low-alkylthio group, low-alkylsulfinyl group, low-alkylsulfonyl group, low-acyl group, and hydroxy-low-alkyl group; n in chemical formula (6) which is an integer of 1 thorough 4; $AA^3$ chemical formula (1) which is non-substituted phenylalanine residue or substituted phenylalanine, residue; $R^{10}$ and $R^{12}$ in chemical formula (7) and (8) which are respectively and independently selected one of groups including hydrogen, low-alkyl group, low-alkenyl group, low-alkynyl group, $C_{6-10}$-aryl group, and aryl-substituted-low-alkyl group; and $AA^4$ in chemical formula (1) which is N-methyllysine residue or n-methyl-β-alanine residue. The present invention may provide these compounds and pharmacologically acceptable salts thereof.

The compound of the present invention is represented by general formula (1). The compound represented as chemical formula (1) may comprise; $R^1$ chemical formula (1) which is selected one of groups including hydrogen atom, hydroxy group, low-alkyl group and low-alkoxyl group; $R^2$ in chemical formula (1) which is selected one of groups including low-alkyl group; $R^3$ and $R^4$ in chemical formula (2) which are both hydrogen atom; or one is hydrogen and the other one is methyl group; $AA^1$ which is tyrosine residue, 2,6-dimethyltyrosine residue, o-acyltyrosine residue, o-alkoxycarbonyl-tyrosine residue, o-phenoxycarbony tyrosine residue, o-acetyltyrosine residue, 2,6-dimethylphenylalanine residue; $AA^2$ which is D-methionine sulfoxide residue, D-arginine residue, D-$N^5$-acetylornitine residue, D-5-oxonorleucine residue or D-5-hydroxynorleucine residue, $AA^3$ which is D-phenylalanine residue, p-fluoroalanine residue, D-p-fluorophenylalanine residue o-trifluroromethylphenyl alanine residue, or D-o-trifluoromethylphenyl alanine; and $AA^4$ which is N-methyllycine residue or N-methyl-β-alanine residue. The present invention may provide these compounds and pharmacologically acceptable salts thereof.

The preferred examples of the present invention are; 1-iminoethyl-[2,6-dimethyl-Tyr]-[D-Arg]-[Phe]-[N-MeβAla]-amide, 1-iminoethyl-[Tyr]-[D-Arg]-[Phe]-[N-MeβAla]-amide, 1-iminoethyl-[Tyr]-[D-Met(O)]-[Phe]-[N-MeβAla]-amide, 1-iminoethyl-[2,6-dimethyl-Tyr]-[D-Met(O)]-[Phe]-[N-MeβAla]-amide, 1-imino-[Tyr]-[D-Met(O)]-[Phe]-[N-MeβAla]-amide, 1-iminoethyl-[2,6-dimethyl-Tyr]-[D-Met(O)]-[Phe]-[N-MeLys]-amide, 1-iminoethyl-[Tyr]-[D-Met(O)]-[Phe]-[N-MeβAla]-methylamide, 1-iminoethyl-[Tyr]-[[D-Met(O)]-[Phe]-[N-MeβAla]-methylamide, but not limited to the examples. The present invention may provide these compounds and pharmacologically acceptable salts thereof.

The preferred examples of the present invention, in which the carboxyl end substituted with methylamide are; 1-iminoethyl-[2,6-dimethyl-Tyr]-[D-Arg]-[Phe]-[N-MeβAla]-methylamide, 1-iminoethyl-[Tyr]-[D-Arg]-[Phe]-[N-MeβAla]-methylamide, 1-iminoethyl-[2,6-dimethyl-Tyr]-[D-Met(O)]-[Phe]-[N-MeβAla]-methylamide, and 1-iminoethyl-[2,6-dimethyl-Tyr]-[D-Met(O)]-[Phe]-[N-MeLys]-methylamide, but not limited to the examples. The present invention may provide these compounds and pharmacologically acceptable salts thereof.

The preferred examples of the present invention, in which the 1-iminoethyl group substituted with methyl amide at the amino end are; iminopropyl-[2,6-dimethyl-Tyr]-[D-Arg]-[Phe]-[N-MeβAla]-amide, iminopropyl-[Tyr]-[D-Arg]-[Phe]-[N-MeβAla]-amide, iminopropyl-[D-Arg]-[Phe]-[N-MeβAla]-amide, iminopropyl-[2,6-dimethyl-Tyr]-[D-Met(O)]-[Phe]-[N-MeβAla]-amide, iminopropyl-[Tyr]-[D-Met(O)]-[Phe]-[N-MeβAla]-amide, iminopropyl-[2,6-dimethyl-Tyr]-[D-Met(O)]-[Phe]-[N-MeLys]-amide, iminopropyl-[Tyr]-[D-Met(O)]-[Phe]-[N-MeLys]-amdie, iminopropyl-[D-Met(O)]-[Phe]-[N-MeβAla]-methylamide, iminopropyl-[Tyr]-[D-Met(O)]-[Phe]-[N-MeLys]-mehylamide, iminopropyl-[2,6-dimethyl-Tyr]-[D-Arg]-[Phe]-[N-Me□Ala]-[N-MeβAla]-methylamide, iminopropyl-[Tyr]-[D-Arg]-[N-MeβAla]-methylamide, iminopropyl-[2,6-dimethyl-Tyr]-[D-Met(O)]-[Phe]-[N-MeβAla]-methylamide and iminopropyl-[2,6-dimethyl-Tyr]-[D-Met(O)]-[Phe]-[N-MeLys]-methylamide, but not limited to the examples. The present invention may provide these compounds and pharmacologically acceptable salts thereof.

The preferred examples of the present invention, in which the amino end is substituted with 1-iminoethyl group or iminopropyl group and the carbonyl end is substituted with ethyl amide are; 1-iminoethyl-[2,6-dimethyl-Tyr]-[D-Arg]-[Phe]-[N-MeβAla]-ethylamide, 1-iminoethyl-[Tyr]-[D-Arg]-[Phe]-[N-MeβAla]-ethylamide, 1-iminoethyl-[2,6-dimethyl-Tyr]-[D-Met(O)]-[Phe]-[N-MeβAla]-ethylamide, 1-iminoethyl-[Tyr]-[D-Met(O)]-[Phe]-[N-MeβAla]-ethylamide, 1-iminoethyl-[2,6-dimethyl-Tyr]-[D-Met(O)]-[Phe]-[N-MeLys]-ethylamide, 1-iminoethyl-[Tyr]-[D-Met(O)]-[Phe]-[N-MeLys]-ethylamide, iminopropyl-[2,6-dimethyl-Tyr]-[D-Arg]-[Phe]-[N-MeβAla]-ethylamide, iminopropyl-[Tyr]-[D-Arg]-[Phe]-[N-MeβAla]-ethylamideiminopropyl-[2,6-dimethyl-Tyr]-[D-Met(O)]-[Phe]-[N-MeβAla]-ethylamide, iminopropyl-[Tyr]-[D-Met (O)]-[Phe]-[N-MeβAla]-ethylamide, iminopropyl-[2,6-dimethyl-Tyr]-[D-Met(O)]-[Phe]-[N-MeLys]-ethylamide and iminopropyl-[Tyr]-[D-Met(O)]-[Phe]-[N-MeLys]-ethylamide, but not limited to the examples. The present invention may provide these compounds and pharmacologically acceptable salts thereof.

The preferred examples of the present invention, in which the amino end is substituted with 1-iminoethyl group or iminopropyl group and the carbonyl end is substituted with diethyl amide are; 1-iminoethyl-[2,6-dimethyl-Tyr]-[D-Arg]-[Phe]-[N-MeβAla]-dimethylamide, 1-iminoethyl-[Tyr]-[D-Arg]-[Phe]-[N-MeβAla]-dimethylamide, 1-iminoethyl-[2,6-dimethyl-Tyr]-[D-Met(O)]-[Phe]-[N-MeβAla]-dimethylamide, 1-iminoethyl-[Tyr]-[D-Met(O)]-[Phe]-[N-MeβAla]-dimethylamide, 1-iminoethyl-[2,6-dimethyl-Tyr]-[D-Met(O)]-[Phe]-[N-MeLys]-dimethylamide, 1-iminoethyl-[Tyr]-[D-Met(O)]-[Phe]-[N-MeLys]-dimethylamide, iminopropyl-[2,6-dimethyl-Tyr]-[D-Arg]-[Phe]-[N-MeβAla]-dimethylamide, iminopropyl-[Tyr]-[D-Arg]-[Phe]-[N-MeβAla]-dimethylamide, iminopropyl-[2,6-dimethyl-Tyr]-[D-Met(O)]-[Phe]-[N-MeβAla]-dimethylamide, iminopropyl-[Tyr]-[D-Met(O)]-[Phe]-[N-MeβAla]-dimethylamide, iminopropyl-[2,6-dimethyl-Tyr]-[D-Met(O)]-[Phe]-[N-MeLys]-dimethylamide and iminopropyl-[Tyr]-[D-Met(O)]-[Phe]-[N-MeLys]-dimethylamide, but not limited to the examples. The present invention may provide these compounds and pharmacologically acceptable salts thereof.

The compound of the present invention may include any optically active compound, any racemic compound, any diastereomer and any mixture thereof. The preferred examples of the pharmacologically acceptable salts of the compound of the present invention are an acid addition salt including hydrochloride, acetate salt and p-toluene sulfonic acid salt; a base addition salt including ammonium salt and organic amine; a hydrate and a solvate of a free form peptide derivative and a salt form peptide derivative, but not limited to the examples. The compound of the present invention may include a dimeric compound and a polymeric compound of the peptide derivative represented in chemical formula (1); and a cyclic compound in which the carbonyl end and the amino end of these peptide derivatives are connected. At present, a compound of the present invention is herein considered to have an analgesic activity and an anti-nociceptive activity expressed by binding to μ opioid receptor of the nervous system although the detail of mechanism is under experiments.

The present invention provides a pharmaceutical composition to be used to prevent and/or treat aches, particularly pains. The present invention provides a use of the compound or pharmacologically acceptable salt thereof of the present invention to produce a pharmaceutical to be used to prevent and/or treat pains. The present invention provides a method to prevent and/or treat pains, comprising a step to administer an effective dose of the compound of the present invention and/or pharmacologically acceptable salt to an animal including a human.

A pharmaceutical composition includes at least one of the compounds of the present invention or the pharmacologically acceptable salts thereof as an active substance. The pharmaceutical composition of the present invention may include at least one of the compounds of the present invention or the pharmacologically acceptable salts thereof

and at least one of pharmacologically acceptable carriers. A pharmaceutical composition of the present invention comprising a compound of the present invention or a pharmacologically acceptable salt thereof may be applied not only to prevent and/or treat pains in general but also to prevent and/or treat neuropathic pains and to prevent and/or treat cancer pains; and allows not only non-oral administration including intravenous administration, subcutaneous administration, intrarectal administration but also oral administration, transmucosal administration or transdermal administration. A variety of pharmaceutical preparations adaptable to these administration routes is known to one skilled in the art; one skilled in the art may select a pharmaceutical preparation case by case in adaptable desired manner, and according to need one skilled in the art may produce a pharmaceutical preparation in a pharmaceutical composition manner using available one or two pharmacologically acceptable carriers or additives for the pharmaceutical preparation. For example, a pharmaceutical preparation including a nasal drop and a nasal spray to be administered into nasal cavity, and a sublingual preparation to be administered into oral cavity, is preferred to transmucosal administration. A hydrate or a solvate of the compound of the present invention or a pharmacologically acceptable salt thereof may be used as an active drug substance of the pharmaceutical drug of the present invention. Although a dose is not particularly specified, the dose for two or three times administrations a day may be in the range of 0.1 through 10 mg per single administration on transdermal administration or transmucosal administration, and in the range of 1 through 100 mg per single administration on oral administration. Or approximately 0.1 through 1,000 mg and further preferably approximately 1 through 300 mg a day per adult may be administered. In addition, the dose may be set associated with parameters including body weight, age, gene type and symptom of the patient.

A pharmaceutical composition of the present invention may include a tablet preparation, a granular (fine particles) preparation, a capsular preparation, a injection preparation (intravenous drip preparation), an adhesive preparation, a suppository preparation, a suspension or emulsion solution, a paste preparation, an ointment preparation, a cream preparation, a lotion preparation, a nasal drop preparation, a eye drop preparation, but not limited to these. A pharmaceutical composition of the present invention may be sustained to maintain the retaining time for a long period of time.

A pharmacologically acceptable carrier or additive for the pharmaceutical composition of the present invention includes a stabilizer, a surfactant, a solubilizer, and an absorbent, but not limited to the examples. A pharmacologically acceptable carrier or additive for the pharmaceutical composition of the present invention may be selected in accordance with a dose form of the pharmaceutical composition of the present invention noted above.

A manufacturing method of the compound of the present invention is not particularly specified and the compound may be synthesized by a solid phase method and a liquid phase method commonly employed in general peptide syntheses. The examples of the present description disclose specifically and in details manufacturing methods about the representative compounds of the present invention. Accordingly one skilled in the art may, referring to the examples according to need, select adequate raw materials and reagents, modify and change case by case reaction conditions and reaction processes to produce readily the compounds of the present invention. A variety of excellent protection groups for amino groups and excellent condensation agents for condensation reaction are well known, and referring to the examples below and in addition, e.g. "Protein Technology - Basic and Application" Ed. Koichi Suzuki, Maruzen, 1992 and references therein; "Peptide Synthesis" M. Bondanszky et.al., John Wiley & Sons, NY, 1976, "Solid Phase Peptide Synthesis", M. Stewart and D. J. Young, W. H. Freeman and Co., San Francisco, 1969, these may be selected and used case by case. In case, a variety of commercial peptide synthesis apparatuses for solid phase synthesis, e.g. Moder 430A, Palkin Elmer Japan or PSSM-8, Shimazu, may be more convenient to be used. Resins and reagents to be used in the syntheses are readily available commercially.

An anti-nociceptive activity of the present invention may be evaluated quantitatively using animal experiments including such as tail flick method and tail pressure method. The tail flick method will be set forth in detail in examples of the present description. The tail pressure method is referred to Patent References 3 and 4. Briefly, pressure stimulation was added on mouse's tail root increased in 10 mmHg/sec ratio and then the pressure at which the mouse showed such flounce and biting the stimulated part was measured, which was considered a threshold for the pain reaction. The maximum pressure was 100 mmHg. The mice used for the experiment reacted to the pressure range between 40 mmHg and 50 mmHg in pre-selective experiment. A few mice or a few dozen of mice under the same condition were stimulated by adding pressure and respective threshold values for the pain reaction were measured in constant intervals after administration in accordance with different drugs administered and the different dosage thereof. According to the measurement value of the pain reaction threshold value, a percent of maximum possible effect, (% of MPE) was calculated using the following formula to quantify the anti-nociceptive activity.

$$\% \text{ of MPE} = [(Pt - P0) / (Pc - Po)] \times 100$$

(in the formula, Po is a pain reaction threshold value before drug administration; Pt is a pain reaction threshold value t minute after drug administration; and Pc is a maximum stimulative pressure)

The present invention will be further specifically described in the following examples, but the present invention is not limited to such examples. A compound of the present invention represented in chemical formula (1) is readily produced by referring to examples, modifying and changing the method disclosed in examples, or selecting case by case raw materials or reaction agents. Meaning of amino acid group in examples is the same as conventionally used one. Amino acid having D-form and L-form is discussed, if it is not specified as D-form, the amino acid is L-form. In addition, the following abbreviations may be used even if not specified. Specifically notations such as iminomethyl-[Phe]- and Boc-[Phe]-, means that nitrogen atom at the amino end of phenylalanine is modified with iminomethyl group or t-butoxycarbonyl group respectively.
Abbreviations used in the following examples stand for;

Boc: t-butoxycarbonyl
BrZ: 2-bromobenzyloxycarbonyl
Bzl: benzyl
ClZ: 2-Chlorobenzyloxycarbonyl
D-Arg: D-Arginine
D-Met(O): D-Methionine sulfoxide (a group in which one oxygen atom is binding to sulfur atom of D-methionine)
DMF: N,N-dimethylformamide
DMT: 2,6-Dimethyltyrosine
HF: Anhydrous hydrogen fluoride
HOBt: 1-Hydroxybenzotriazole
HPLC: High performance liquid chromatography
N-MeβAla: N-Methyl-β-alanine (a group in which a methyl group is binding to nitrogen atom of α-amino group of β-alanine)
N-MeLys: N-Methyllysine (a group in which a methyl group is binding to nitrogen atom of α-amino group of lysine)
N-MeLys(ClZ): ClZ-N-Methyllysine (a group in which a methyl group is binding to nitrogen atom of α-amino group of lysine and a ClZ group is binding to nitrogen atom of ε-amino group)
Phe: Phenylalanine
TFA: Trifluoroacetic acid
Tos: p-Tolune sulfonyl
WSCD: Water soluble carbodiimide, i.e. 1-ethyl-3-(3-dimethylaminopropyl)carbodiimide

1. SS8225-Peptide

Peptide derivatives synthesized herein as examples and comparison examples of the present description have serial numbers as SS8225-1, 2, 3.... (Hereinafter called SS8225-Peptide) The relationship between respective SS8225-peptide of examples and comparison examples of the present description and chemical structures are shown in Table 1. Comparison example 1 and 2 are morphine and oxycodone respectively. Peptide derivative SS8225-22 is shown as reference example because it was used to explain the synthetic method.
Table 1.

| | | |
|---|---|---|
| Example 1 | SS8225-01 | Iminoethyl-[DMT]-[D-Arg]-[Phe]-[N-MeβAla]-$NH_2$ |
| Example 2 | SS8225-02 | Iminoethyl-[Tyr]-[D-Arg]-[Phe]-[N-MeβAla]-$NH_2$ |
| Example 3 | SS8225-03 | Iminoethyl-[DMT]-[D-Met(O)]-[Phe]-[N-MeβAla]-$NH_2$ |
| Example 4 | SS8225-04 | Iminoethyl-[Tyr]-[D-Met(O)]-[Phe]-[N-MeβAla]-$NH_2$ |
| Example 5 | SS8225-05 | Iminoethyl-[DMT]-[D-Met(O)]-[Phe]-[N-MeLys]-$NH_2$ |
| Example 6 | SS8225-06 | Iminoethyl-[Tyr]-[D-Met(O)]-[Phe]-[N-MeLys]-$NH_2$ |
| Example 7 | SS8225-07 | Iminoethyl-[Tyr]-[D-Met(O)]-[Phe]-[N-MeβAla]-$NHCH_3$ |
| Example 8 | SS8225-08 | Iminoethyl-[Tyr]-[D-Met(O)]-[Phe]-[N-MeLys]-$NHCH_3$ |
| Comparison Example 1 | Morphine | |
| Comparison Example 2 | Oxycodone | |
| Comparison Example 3 | SS8225-09 | [DMT]-[D-Arg]-[Phe]-[N-MeLys]-$NH_2$ |

(continued)

| | | |
|---|---|---|
| Comparison Example 4 | SS8225-10 | [DMT]-[D-Met(O)]-[Phe]-[N-MeLys]-$NH_2$ |
| Comparison Example 5 | SS8225-11 | Iminoethyl-[Tyr]-[D-Met(O)]-[Phe]-[N-MeLys]-OH |
| Comparison Example 6 | SS8225-12 | Iminoethyl-[DMT]-[D-Arg]-[Phe]-[N-MeLys]-OH |
| (Reference) | SS8225-22 | Iminoethyl-[Tyr]-[D-Arg]-[Phe]-[N-MeLys]-$NH_2$ |

Further, the compounds according to example 1 through 8 of the present invention are represented as shown in chemical formulae (15) through (22).

[C12]

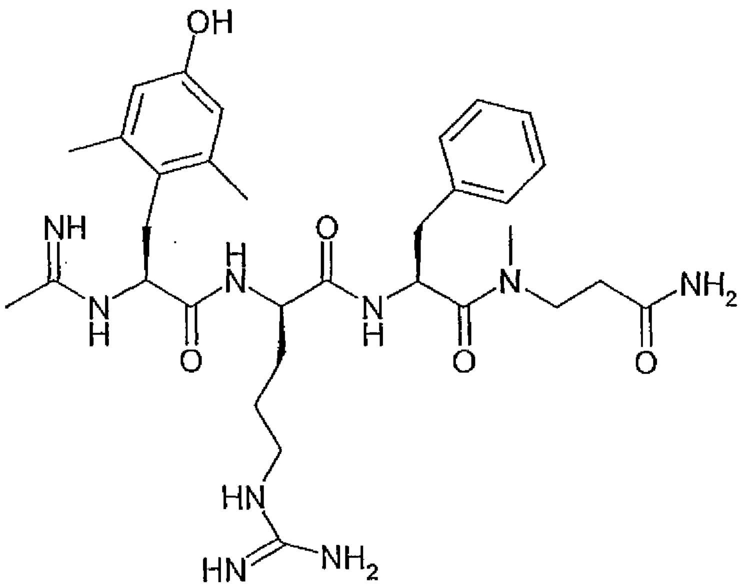

(15)

[C13]

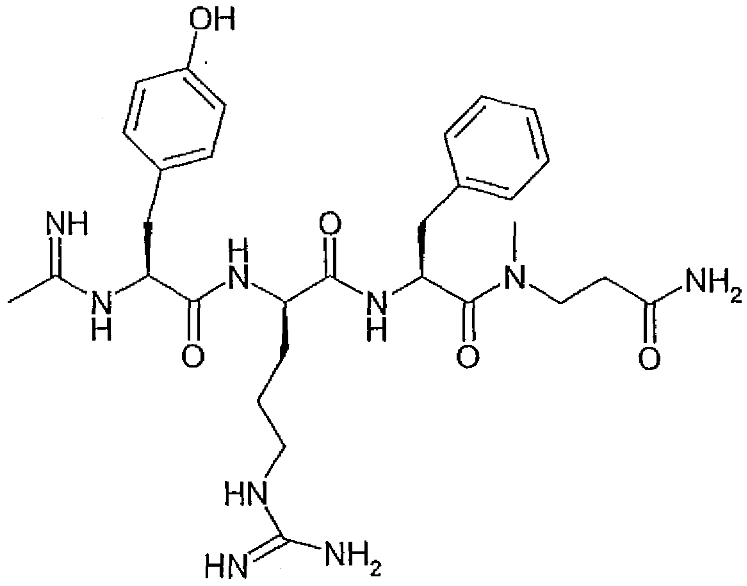

(16)

[C14]

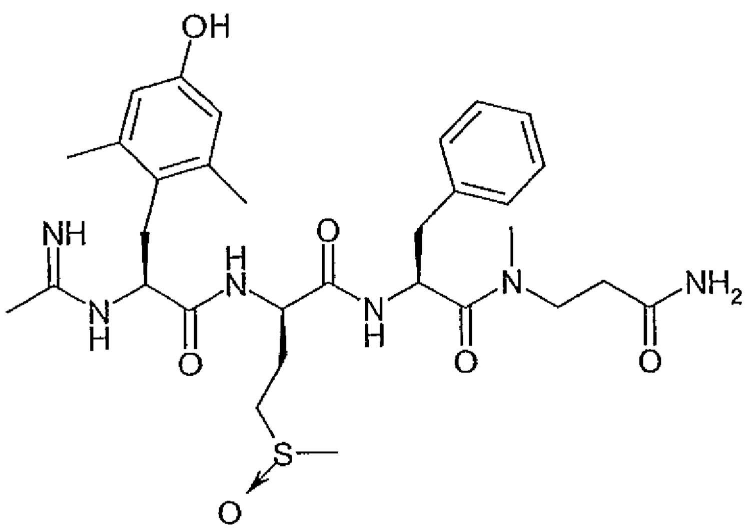

(17)

[C15]

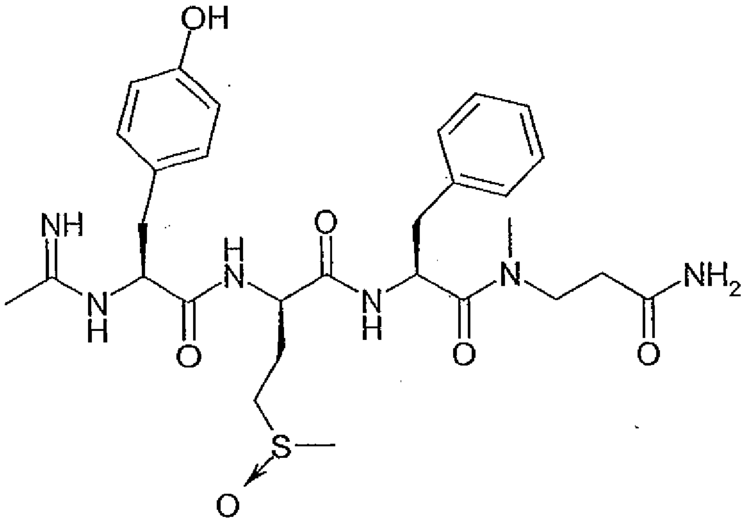

(18)

[C16]

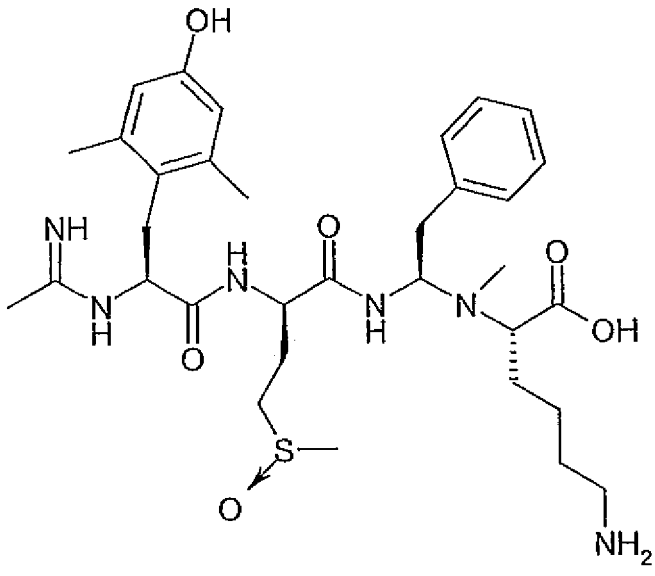

(19)

[C17]

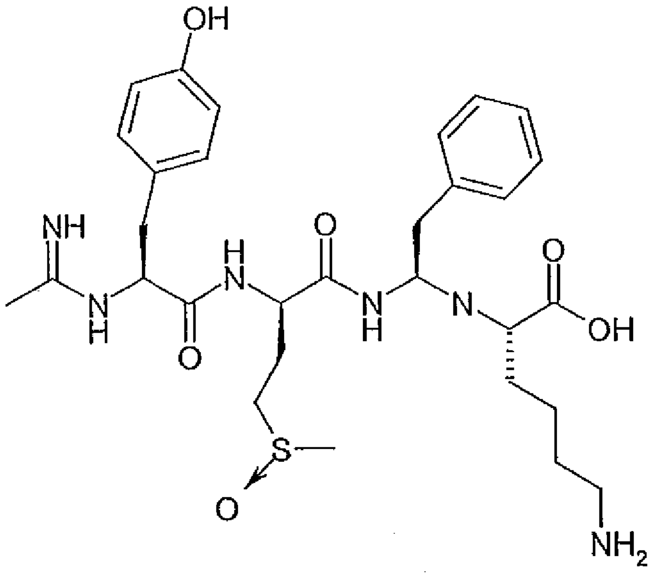

(20)

[C18]

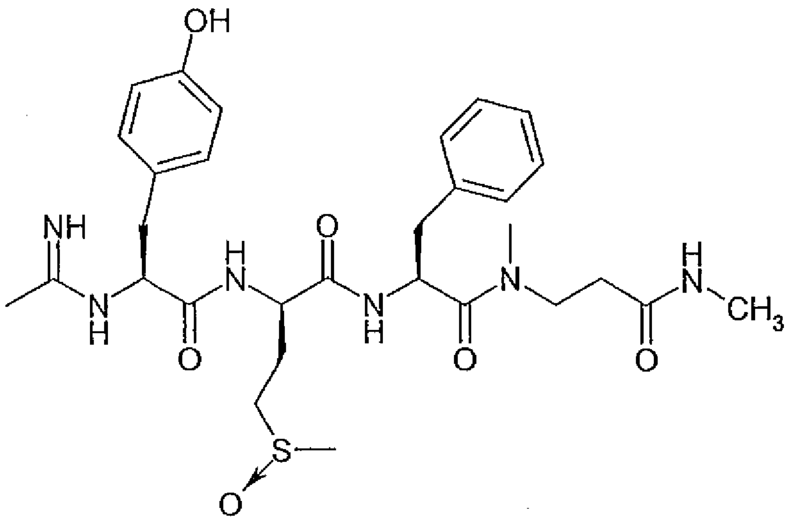

(21)

[C19]

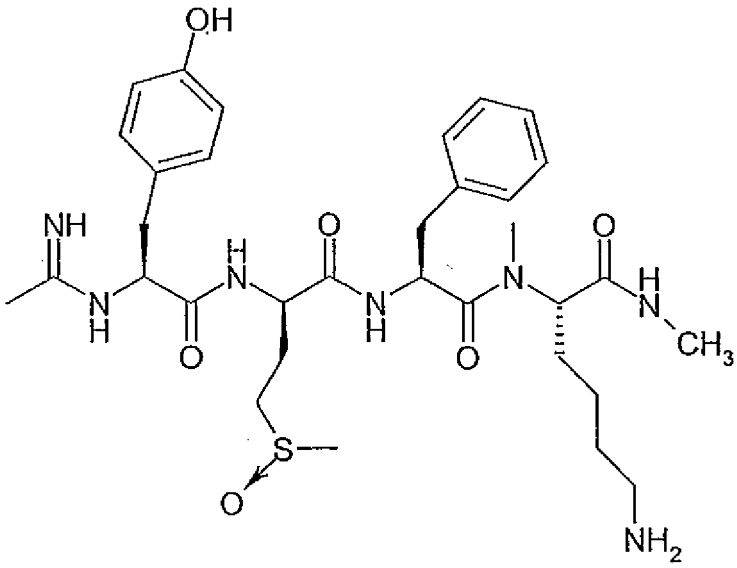

(22)

2. Synthesis of starting amino acid derivatives

(1) Availability of starting amino acid derivatives

Reagents used to synthesize the peptide derivatives noted below are commercially available or readily prepared by one skilled in the art. Boc-[N-MeLys(ClZ)]-OH used in a peptide synthesis reaction as a starting amino acid derivative, for example, can obtained from Anaspec, Inc.; Boc-[N-MeβAla]-OH can be obtained from Watanabe Chemical Co., Ltd.; and such Boc-[Phe]-OH, Boc-[D-Arg(Tos)]-OH, Boc-[Tyr(BrZ)]-OH and Boc-[D-Met]-OH can be obtained from Peptide Institute Co. Ltd..

(2) Synthesis of starting amino acid, Boc-[N-MeLys(ClZ)]-$NH_2$

Boc-[N-MeLys(ClZ)]-OH, HOBt and $NH_4Cl$ were dissolved in DMF, WSCD was added under cooling and then the solution was stirred overnight at room temperature. Ethyl acetate was added to the reaction solution which was then washed with 1 N-hydrogen chloride, saturated aqueous sodium bicarbonate solution and then saturated saline solution. The organic layer was dried with magnesium sulfate and then the solvent was concentrated under vacuum to give Boc-[N-MeLys(ClZ)]-$NH_2$ as an oil.

(3) Synthesis of starting amino acid, Boc-[N-MeLys(ClZ)]-o-Bzl

Boc-[N-MeLys(ClZ)]-OH and Bzl-Br were dissolved in DMF and triethylamine was added under cooling and the solution was stirred overnight at room temperature. Ethyl acetate was added to the reaction solution which was then washed with 1N-hydrogen chloride, saturated aqueous sodium bicarbonate solution and then saturated saline solution. The organic layer was dried with magnesium sulfate and then the solvent was concentrated under vacuum to give Boc-[N-MeLys(ClZ)]-o-Bzl as an oil.

(4) Synthesis of starting amino acid, Boc-[N-MeβAla]-o-Bzl

Boc-[N-MeβAla]--OH and Bzl-Br were dissolved in DMF and triethylamine was added under cooling and the solution was stirred overnight at room temperature. Ethyl acetate was added to the reaction solution which was then washed with 1 N-hydrogen chloride, saturated aqueous sodium bicarbonate solution and then saturated saline solution. The organic layer was dried with magnesium sulfate and then the solvent was concentrated under vacuum to give Boc-[N-MeβAla]-o-Bzl as an oil.

(5) Synthesis of starting amino acid, Boc-[N-MeβAla]-$NH_2$

Boc-[N-MeβAla]-OH, HOBt and $NH_4Cl$ were dissolved in DMF, WSCD was added under cooling and then the solution was stirred overnight at room temperature. Ethyl acetate was added to the reaction solution which was then washed with 1N-hydrogen chloride, saturated aqueous sodium bicarbonate solution and then saturated saline solution. The organic layer was dried with magnesium sulfate and then the solvent was concentrated under vacuum to give Boc-[N-MeβAla]-$NH_2$ as colorless solid.

(6) Synthesis of starting amino acid, Boc-[N-MeβAla]-$NH_2$

Boc-[N-MeβAla]-OH, HOBt and $NH_3CH_3 \cdot Cl$ were dissolved in DMF, WSCD was added under cooling and then the solution was stirred overnight at room temperature. Ethyl acetate was added to the reaction solution which was then washed with 1N-hydrogen chloride, saturated aqueous sodium bicarbonate solution and then saturated saline solution. The organic layer was dried with magnesium sulfate and then the solvent was concentrated under vacuum to give Boc-[N-MeβAla]-$NHCH_3$ as colorless oil.

(7) Synthesis of starting amino acid, Boc-[N-MeLys(ClZ)]-$NHCH_3$

Boc-[N-MeLys(ClZ)]-OH, HOBt and $NH_3CH_3 \cdot Cl$ were dissolved in DMF, WSCD was added under cooling and then the solution was stirred overnight at room temperature. Ethyl acetate was added to the reaction solution which was then washed with 1 N-hydrogen chloride, saturated aqueous sodium bicarbonate solution and then saturated saline solution. The organic layer was dried with magnesium sulfate and then the solvent was concentrated under vacuum to give Boc-[N-MeLys(ClZ)]-$NHCH_3$ as colorless oil.

3. Syntheses of SS8225-peptides

(1) Synthesis of SS8225-22

SS8225-09, 01, 02 and 12 of the examples and comparison example of the present description were synthesized by the method used for synthesis of SS8225-22 as the reference, and thus we set forth first the synthesis of SS8225-22. Boc group of the amino end of the starting material Boc-[N-MeLys]ClZ)]-$NH_2$ was eliminated using Tos-OH, TFA and hydrogen chloride dioxane solution to give Tos-OH·N-MeLys(ClZ)-$NH_2$. Then, Tos-OH·N-MeLys(ClZ)-$NH_2$, Boc-[Phe]-OH and HOBt were dissolved in DMF, WSCD was added under cooling and then the solution was stirred overnight at room temperature. Ethyl acetate was added to the reaction solution which was then washed with 1 N-hydrogen chloride, saturated aqueous sodium bicarbonate solution and then saturated saline solution. The organic layer was dried with magnesium sulfate and then the solvent was concentrated under vacuum to give Boc-[Phe]-[N-MeLys (ClZ)]-$NH_2$. Then as well as and one by one Boc-[D-Arg(Tos)]-OH and Boc-[Tyr(BrZ)]-OH were condensed to give Boc-[Tyr(BrZ)]-[D-Arg(Tos)]-[Phe]-[N-MeLys]-$NH_2$. Then, TFA·Tyr(BrZ)-[D-Arg(Tos)]-[Phe]-[N-MeLys]-$NH_2$ obtained by elimination of Boc of the amino end with TFA, acetoimidic acid ethyl hydrochloride and triethylamine were dissolved in DMF, and the solution were stirred for 2.5 hours at room temperature. The reaction solution was condensed under vacuum; cold water was added to precipitate the precipitate which was filtered and dried to give 1-iminoethyl-[Tyr(BrZ)]-[D-Arg(Tos)]-[Phe]-[N-MeLys]-$NH_2$. 1-iminoethyl-[Tyr(BrZ)]-[D-Arg(Tos)]-[Phe]-[N-MeLys]-$NH_2$ was treated with HF/p-cresol(85/15) solution for one hour under between -3°C and -5°C. After removal of HF under vacuum, the residue was dissolved in water and loaded onto ion exchange resin, eluted with 1% of aqueous acetic acid solution to give crude 1-iminoethyl-[Tyr]-[D-Arg]-[Phe]-[N-MeLys]-$NH_2$·acetic acid. Then the crude product was purified with reverse-phase HPLC and then was freeze-dried to give 1-iminoethyl-[Tyr]-[D-Arg]-[Phe]-[N-MeLys]-$NH_2$·acetic acid.

(2) Syntheses of SS8225-09, 01, 02 and 12

SS8225-9 (comparison example 3) from Boc-[N-MeLys(C1Z)]-$NH_2$ as a starting amino acid derivative, SS8225-12 (comparison example 6) from Boc-[N-MeLys(C1 Z)]-o-Bz1 as a starting amino acid derivative, SS8225-01 and -02 (example 1 and 2) from Boc-[N-MeβAla]-$NH_2$ as a starting amino acid derivative, were synthesized by the same method for SS8225-22, respectively.

(3) Synthesis of SS8225-06

Boc group of the amino end of the starting material Boc-[N-MeLys]ClZ)]-$NH_2$was eliminated using Tos-OH, TFA and hydrogen chloride dioxane solution to give Tos-OH·N-MeLys(ClZ)-$NH_2$. Then, Tos-OH·N-MeLys(ClZ)-$NH_2$,

Boc-[Phe]-OH and HOBt were dissolved in DMF, WSCD was added under cooling and then the solution was stirred overnight at room temperature. Ethyl acetate was added to the reaction solution which was then washed with 1N-hydrogen chloride, saturated aqueous sodium bicarbonate solution and then saturated saline solution. The organic layer was dried with magnesium sulfate and then the solvent was concentrated under vacuum to give Boc-[Phe]-[N-MeLys (ClZ)]-$NH_2$. Then as well as and one by one Boc-[D-Met]-OH and Boc-[Tyr(BrZ)]-OH were condensed to give Boc-[Tyr (BrZ)]-[D-Met]-[Phe]-[N-MeLys]-$NH_2$. Then, TFA·Tyr(BrZ)-[D-Met)]-[Phe]-[N-MeLys]-$NH_2$ obtained by elimination of Boc of the amino end with TFA, acetoimidic acid ethyl hydrochloride and triethylamine were dissolved in DMF, and the solution were stirred for 2.5 hours at room temperature. The reaction solution was condensed under vacuum; cold water was added to precipitate the precipitate which was filtered and dried to give 1-iminoethyl-[Tyr(BrZ)]-[D-Met]-[Phe]-[N-MeLys]-$NH_2$. 1-iminoethyl-[Tyr(BrZ)]-[D-Met]-[Phe]-[N-MeLys]-$NH_2$ was treated with HF/p-cresol(85/15) solution for one hour under between -3°C and -5°C. After removal of HF under vacuum, the residue was dissolved in water and loaded onto ion exchange resin, eluted with 1% of aqueous acetic acid solution to give crude 1-iminoethyl-[Tyr]-[D-Met]-[Phe]-[N-MeLys]-$NH_2$·acetic acid. Then the crude 1-iminoethyl-[Tyr]-[D-Met]-[Phe]-[N-MeLys]-$NH_2$·acetic acid was dissolved in aqueous acetic acid solution and after addition of hydrogen peroxide the solution was stirred for 1.5 hours. Then the reaction solution was fractionally purified with reverse-phase HPLC and then was freeze-dried to give SS8225-06 (example 6) acetic acid.

In addition, when D-Met was changed to D-Met(O), hydrogen peroxide was used so that the present derivatives were a mixture of D-methionine-(S)-sulfoxide and D-methionine-(R)-sulfoxide.

(4) Synthesis of SS8225-10, 03, 04, 11, 05, 07 and 08

SS8225-10 and 05 (comparison example 4 and example 5) from Boc-[N-MeLys(C1Z)]-$NH_2$ as a starting amino acid derivative, SS8225-11 (comparison example 5) from Boc-[N-MeLys(G1Z)]-o-Bz1 as a starting amino acid derivative, SS8225-03 and -04 (example 3 and 4) from Boc-[N-MeβAla]-$NH_2$ as a starting amino acid derivative, SS8225-07 (example 7) from Boc-[N-MeβAla]-$NHCH_3$ as a starting amino acid derivative, SS8225-08 (example 8) from Boc-[N-MeLys (C1Z)]-$NHCH_3$ as a starting amino acid derivative were synthesized by the same method for SS8225-06, respectively.

4. NMR Spectra of SS8225-peptides

NMR spectra of respective SS8225-peptides were measured using Nihon Denshi JEOL JNM-EX270, 270MHz, at room temperature.

NMR Spectra of SS8225-01 (example 1)

$^1$H-NMR ($D_2O$) δ: 7.30-7.10 (5H, m), 6.49 (2H,s), 5.10-4.88 (1 H, m),4.20 (1 H, q, J=5.3Hz), 4.02-3.87 (1 H, m), 3.68-3.32 (2H, m), 3.25-2.67 (9H, m), 2.35 (2H, t J=6.8Hz), 2.16 (2.6H, s) 2.11 (6H, s) 1.92 (0.4H, s), 1.24-0.64 (4H, m)

NMR Spectra of SS8225-02 (example 2)

$^1$H-NMR ($D_2O$) δ: 7.30-7.10 (5H, m), 7.01 (2H, d, J=8.0Hz), 6.73 (2H, d, J=8.0Hz), 5.09-4.89 (1 H, m), 4.34 (1 H, t, J=7.7Hz), 3.99 (1 H, q, J=6.0Hz), 3.54-3.45 (2H, m), 3.08-2.67 (9H, m), 2.34 (2H, t J=6.9Hz), 2.11 (2.6H, s),1.96 (0.4H, m)

NMR Spectra of SS8225-03 (example 3)

$^1$H-NMR ($D_2O$) δ: 7.32-7.13 (5H, m), 6.51 (2H,s), 5.10-4.90 (1 H, m),4.30-4.13 (1 H, m), 4.13-3.96 (1 H, m), 3.68-3.35 (2H, m), 3.27-2.71 (7H, m), 2.48 (3H, s), 2.34 (2H, t J=6.8Hz), 2.16 (3H, s) 2.11 (6H, s) 2.01-1.90 (2H, m), 1.67-1.30 (2H, m)

NMR Spectra of SS8225-04 (example 4)

$^1$H-NMR ($D_2O$) δ: 7.32-7.10 (5H, m), 7.03 (2H, d, J=8.0Hz), 6.75 (2H, d, J=8.0Hz), 5.08-4.90 (1 H, m), 4.34 (1 H, t, J=7.6Hz), 4.20-4.00 (1 H, m), 3.65-3.33 (2H, m), 3.06-2.72 (7H, m), 2.49 (3H, s), 2.34 (2H, t J=6.5Hz), 2.27-1.95 (5H, m), 1.78-1.40 (2H, m)

NMR Spectra of SS8225-05 (example 5)

$^1$H-NMR ($D_2O$) δ: 7.35-7.13 (5H, m), 6.53 (2H, s), 5.10-4.96 (1 H, m), 4.85-4.76 (1 H, m), 4.39-3.90 (1 H, m), 3.24-2.78 (9H, m), 2.51 (3H, s), 2.18 (3H, s), 2.13 (6H, s), 1.98-1.36 (8H, m), 1.30-0.85 (2H, m)

NMR Spectra of SS8225-06 (example 6)

$^1$H-NMR ($D_2O$) δ: 7.40-7.13 (5H, m), 7.05 (2H, d, J=8.0Hz), 6.77 (2H, d, J=8.0Hz), 5.04 (1 H, t, J=7.6Hz), 4.80-4.60 (1 H, m), 4.36 (1 H, t, J=7.6Hz), 4.24-4.00 (1 H, m), 3.15-2.68 (9H, m), 2.52 (3H, s), 2.35-1.45 (11 H, m), 1.26-0.85 (2H, m)

NMR Spectra of SS8225-07 (example 7)

$^1$H-NMR ($D_2O$) δ: 7.24-7.05 (5H, m), 6.97 (2H, d, J=8.4Hz), 6.68 (2H, d, J=8.4Hz), 4.98-4.83 (1 H, m), 4.27 (1 H, t, J=7.6Hz), 4.13-3.93 (1 H, m), 3.58-3.25 (2H, m), 3.00-2.63 (7H, m), 2.49 (3H, s), 2.42 (3H, s), 2.30-1.86 (7H, m), 1.67-1.32 (2H, m)

NMR Spectra of SS8225-08 (example 8)

$^1$H-NMR ($D_2O$) δ: 7.35-7.06 (5H, m), 7.01 (2H, d, J=8.2Hz), 6.73 (2H, d, J=8.2Hz), 4.97 (1 H, t, J=7.4Hz), 4.80-4.60 (1 H, m), 4.31 (1 H, t, J=7.4Hz), 4.20-3.95 (1 H, m), 3.08-2.73 (9H, m), 2.65-2.43 (6H, m), 2.31-1.92 (5H, m), 1.75-1.38 (6H, m), 1.25-0.78 (2H, m)

5. Evaluation of anti-nociceptive activities by a tail-flick method - Part 1 -

(1) Experimental animal, male ddy mice (Japan SLC), weighing the body weight in the range of 22 to 25g, were used for the studies. The animals had been kept under constant conditions (room temperature 22 ± 2°C, humidity 55 ± 5%, 12 hours light-and-dark cycle having light time 9:00 to 21:00 and dark time 21:00 to 9:00) until used for an experiment. The animals were fed the mouse solid diet (F2, Funabashi farm. Funabashi, Japan) with unrestricted tap water intake.

(2) Administered drug and administration method SS8225-01, 02, 03 04, 05, 06, 07 and 08 as examples and SS8225-09, 10 , 11 and 12 as comparison examples were used as the administered drugs. In addition, morphine and oxycodone were used as the administered drugs to be compared. The administered solutions were prepared respectively to be 0.1 mL/10g of mouse body weight by adjusting and diluting with Ringer's solution to provide the drug by weight in the range of 0.3125 through 20 mg/ Kg of mouse body weight for SS8225-01, 02, 03 04, 05, 06, 07, 08, 09, 10, 11 and 12, which were comparable with administration conditions for the administered morphine and oxycodone by weight in the range of 0.89 through 67.5 mg/ Kg of mouse body weight. In addition only Liger solution was administered as an experimental control. The animals were left for 60 minutes in the plastic cage to become familiar to the environment prior to the test in which measured a latent time for each mouse before drug administration in accordance with a tail-flick method noted below. Heat was radiated to a part of each mouse tail which is 2 cm from the end of its tail. Each of heat radiation periods before beginning of tail-flick was measured as a false pain threshold. Mice which responded to the stimulation within 2.5 to 3.5 seconds were pre-selected for the test. Administration solution under one administration condition for one kind of drug was administered to one experimental animal group including 10 mice. After the body weight of each mouse was measured, each volume of the administration solution was adjusted to be 0.1 mL/10 g of mouse body weight and then the solution was administered. The administration methods were two methods of subcutaneous administration (s. c.) and oral administration (p. o.). On subcutaneous administration, the administration solution of 0.1 mL per 10 g of body weight (0.1 mL/10 g) was injected into the back of the mouse using 27 gauge needle. On oral administration, the administration solution of 0.1 mL per 10 g of body weight (0.1 mL/10 g) was administered using an oral sonde.

(3) Evaluation method for anti-nociceptive activity
A tail-flick method with a heat nociceptive stimulation as a benchmark of anti-nociceptive activity was used to evaluate quantitatively anti-nociceptive activity of respective drug every respective administration method and every respective administration condition. Heat was radiated to a part of each mouse's tail of respective experimental group, which is 2 cm from the end of its tail, at 15, 30 and 60 minutes after the drug administration according to the preset measurement interval and a latent time until respective mouse swung its tail was measured as a false pain threshold value. The maximum stimulation period (cut-off-time) was set as 10 seconds to minimize the damage to the stimulation part. The anti-nociceptive effect was evaluated quantitatively by calculating %MPE (% of maximum possible effect) according to the following formula.

[M 1]

$$\%MPE = \frac{T2 - T1}{Tc - T1} \times 100 \text{ ------ Formula (1)}$$

T1: A latent time until beginning of tail-flick before respective drug administration (seconds)
T2: A latent time until beginning of tail-flick after respective drug administration (seconds)
Tc: A maximum stimulation period (cut-off-time)

The graphs (Fig. 1, Fig. 3, Fig. 5, Fig. 7, Fig. 9, Fig. 11, Fig. 13, Fig. 15, Fig. 17, Fig. 19, Fig. 21, Fig. 23, Fig. 25, Fig. 27, Fig. 29, Fig. 31, Fig. 33 and Fig. 35) showing a time-course variation of %MPE were created, in which a vertical axis is an average value with a standard error (means±S. E. M.) obtained by calculation of %MPE of respective animals of 10 mice every administration condition of respective drugs and a horizontal axis is a time after administration. After treating %MPE data of each animal of experimental groups with respective doses and %MPE data of each animal of the experimental group with a control Ringer's solution at the same measurement time every drugs using the two-way layout dispersion analysis (two-way ANOVA), the hazard-ratio of $P<0.05$ was decided to be a significant difference according to Bonferroni post-test.

The lasting time of anti-nociceptive activity under respective administration conditions according to the present description is the period from the time when the significant difference of anti-nociceptive activity became first less than 5% in comparison with the control administered the Liger solution to the time when the significant difference of anti-nociceptive activity was no longer less than 5%, according to the curve showing the time-course variation of %MPE of each dose condition referring to Fig. 1, Fig. 3, Fig. 5, Fig. 7, Fig. 9, Fig. 11, Fig. 13, Fig. 15, Fig. 17, Fig. 19, Fig. 21, Fig. 23, Fig. 25, Fig. 27, Fig. 29, Fig. 31, Fig. 33 and Fig. 35) which were showing the time-course variation of %MPE. A lasting time of anti-nociceptive activity of each drug in the present description is the lasting time of anti-nociceptive activity under the dose condition providing the longest lasting time.

A dose-response curve graphs (Fig. 1 through 12, B and D) were obtained by analyzing the time-course variation data of %MPE every dose conditions using the curve analysis program (GraphPad Prism Software Version 3.0: GraphPad Software, San Diego, CA U.S.A.), and calculating 50% effective dose ($ED_{50}$) and 95% confidence limit thereof. $ED_{50}$ is represented in the unit of mg/Kg. $ED_{50}$ of respective drugs every respective dose conditions in the present description is the $ED_{50}$ value under the dose condition showing the strongest pharmacological effect associated with respective drugs/administration methods, and specifically is the minimum value among $ED_{50}$s under each dose condition. When the level of anti-nociceptive activity of each drug is compared in the present description, it is represented as $ED_{50}$ value of a drug is how many times higher than $ED_{50}$ value of the other drug.

(4) Results of anti-nociceptive activity test

The results of anti-nociceptive activity test of examples 1 through 8 and comparison examples 1 through 6 using the tail-flick method are shown in the following Table 2; and Fig. 1, Fig. 3, Fig. 5, Fig. 7, Fig. 9, Fig. 11, Fig. 13, Fig. 15, Fig. 17, Fig. 19, Fig. 21, Fig. 23, Fig. 25, Fig. 27, Fig. 29, Fig. 31, Fig. 33 and Fig. 35. If there is * sign on the upper limit of the standard deviation plotted every dose conditions and every times after administration in the graphs showing the time-course variation of these %MPE, it means that the hazard ratio of the significant difference of the anti-nociceptive activities in comparison with the control having the Ringer's solution under the dose condition is not more than 5%. If there is ** sign on the upper limit of the standard deviation plotted every dose conditions and every times after administration in the graphs showing the time-course variation of these %MPE, it means that the hazard ratio of the siginificant difference of the anti-nociceptive activities in comparison with the control having the Ringer's solution under the dose condition is not more than 1%. The time when the time-course variation curve of anti-nociceptive activity is maximal on subcutaneous administration and oral administration of respective drugs is obtained from these graphs as a peak-activity time. In columns of subcutaneous $ED_{50}$ and oral $ED_{50}$ of examples and comparison examples in Table 2, a value not in the parenthesis is $ED_{50}$ at the time of activity-peak for respective drugs, specifically 50% effective dose, and a range of values not in the parenthesis is a 95% confidence limit of $ED_{50}$ at the time of activity-peak. When $ED_{50}$ on subcutaneous administration is larger than 0.5 mg/Kg is expressed as >0.5, and when $ED_{50}$ on oral administration is larger than 10 mg/Kg is expressed as >0.5, because of weak anti-nociceptive activity thereof.

[Table 2]

| | | Subcutaneous $ED_{50}$ (mg/Kg) | Oral $ED_{50}$ (mg/Kg) |
|---|---|---|---|
| Example 1 | SS8225-01 | 0.12 (0.08-0.20) | >10 |
| Example 2 | SS8225-02 | 0.06 (0.03-0.10) | 4.0 (3.97-4.10) |
| Example 3 | SS8225-03 | 0.08 (0.06-0.12) | >10 |

(continued)

| | | Subcutaneous $ED_{50}$ (mg/Kg) | Oral $ED_{50}$ (mg/Kg) |
|---|---|---|---|
| Example 4 | SS8225-04 | 0.05 (0.01-0.11) | 2.53 (2.17-2.95) |
| Example 5 | SS8225-05 | 0.10 (0.04-0.22) | >10 |
| Example 6 | SS8225-06 | 0.18 (0.07-0.43) | 6.02 (4.37-8.28) |
| Example 7 | SS8225-07 | 0.05 (0.03-0.09) | 2.51 (2.23-2.83) |
| Example 8 | SS8225-08 | 0.10 (0.05-0.24) | >10 |
| Comparison Example 1 | Morphine | 2.51 (1.48-4.26) | 29.9 (26.37-33.95) |
| Comparison Example 2 | Oxycodone | 1.21 (0.95-1.54) | 7.82 (6.46-9.47) |
| Comparison Example 3 | SS8225-09 | 0.44 (0.33-0.59) | >10 |
| Comparison Example 4 | SS8225-10 | 0.28 (0.21-0.39) | >10 |
| Comparison Example 5 | SS8225-11 | >0.5 | >10 |
| Comparison Example 6 | SS8225-12 | >0.5 | >10 |

According to graphs noted above; Fig. 1 - Fig. 4, Fig. 7 - Fig. 10, Fig. 13 - Fig. 16, Fig. 19, Fig. 20, Fig. 23 - Fig. 26, Fig. 29, Fig. 30, and Fig. 33 - Fig. 36 showed the results on subcutaneous administration test; and Fig. 5, Fig. 6, Fig. 11, Fig. 12, Fig. 17, Fig. 18, Fig. 21, Fig. 22, Fig. 27, Fig. 28, Fig. 31 and Fig. 32 showed the results on oral administration test. According to these graphs, the odd number graphs show the time-course variation of anti-nociceptive activity after administration, in which the vertical axis is %MPE (without unit) and the horizontal axis is the time (in minute). B and D show the dose-response curves, in which the vertical axis is %MPE and the horizontal axis is the dose (mg/Kg unit).

Example 1

Fig. 1 and Fig. 2 show the results of the anti-nociceptive activity test on subcutaneous administration of example 1 (SS8225-11: 1-iminoethyl-[DMT]-[D-Arg]-[Phe]-[N-MeβAla]-$NH_2$). Dose-responsible and significant anti-nociceptive activity was expressed by the subcutaneous administration of peptide derivative SS8225-11. The $ED_{50}$ value at 90 minutes after the subcutaneous administration, which is the activity-peak time, was 0.12 mg/Kg; the 95% confidence limit was 0.08-0.20 mg/Kg; and the lasting time with the doses of 0.5 mg/Kg and 0.25 mg/Kg on the subcutaneous administration was not less than 7 hours.

Example 2

Fig. 3 and Fig. 4 show the results of the anti-nociceptive activity test on subcutaneous administration of example 2 (SS8225-1: 1-iminoethyl-[Tyr]-[D-Arg]-[Phe]-[N-MeβAla]-$NH_2$). Dose-responsible and significant anti-nociceptive activity was expressed by the subcutaneous administration of peptide derivative SS8225-12. The $ED_{50}$ value at 90 minutes after the subcutaneous administration, which is the activity-peak time, was 0.06 mg/Kg; the 95% confidence limit was 0.03-0.10 mg/Kg; and the lasting time with the dose of 0.125 mg/Kg on the subcutaneous administration was 6 hours. Fig. 5 and Fig. 6. show the results of the anti-nociceptive activity test on oral administration of example 2. Dose-responsible anti-nociceptive activity was expressed also by the oral administration. The $ED_{50}$ value at 4 hours after the oral administration, which is the activity-peak time, was 4.0 mg/Kg; the 95% confidence limit was 3.97-4.10 mg/Kg; and the lasting time with the dose of 10 mg/Kg a on the oral administration was 10 hours.

Example 3

Fig. 7 and Fig. 8 show the results of the anti-nociceptive activity test on subcutaneous administration of example 3 (SS8225-13: 1-iminoethyl-[DMT]-[D-Met(O)]-[Phe]-[N-MeβAla]-$NH_2$). Dose-responsible and significant anti-nociceptive activity was expressed by the subcutaneous administration of peptide derivative SS8225-13. The $ED_{50}$ value at 90 minutes after the subcutaneous administration, which is the activity-peak time, was 0.08 mg/Kg; the 95% confidence limit was 0.06-0.12 mg/Kg; and the lasting time with the doses of 0.5 mg/Kg and 0.25 mg/Kg on the subcutaneous administration was 7 hours.

Example 4

Fig. 9 and Fig. 10 show the results of the anti-nociceptive activity test on subcutaneous administration of example 4 (SS8225-14: 1-iminoethyl-[Tyr]-[D-Met(O)]-[Phe]-[N-MeβAla]-$NH_2$). Dose-responsible and significant anti-nociceptive activity was expressed by the subcutaneous administration of peptide derivative SS8225-14. The $ED_{50}$ value at 90 minutes after the subcutaneous administration, which is the activity-peak time, was 0.05 mg/Kg; the 95% confidence limit was 0.01-0.11 mg/Kg; and the lasting time with the dose of 0.125 mg/Kg on the subcutaneous administration was 7 hours. Fig. 11 and Fig. 12 show the results of the anti-nociceptive activity test on oral administration of example 4. Dose-responsible and significant anti-nociceptive activity was expressed also by the oral administration. The $ED_{50}$ value at 180 hours after the oral administration, which is the activity-peak time, was 2.53 mg/Kg; the 95% confidence limit was 2.17-2.95 mg/Kg; and the lasting time with the dose of 10 mg/Kg a on the oral administration was not less than 10 hours.

Example 5

Fig. 13 and Fig. 14 show the results of the anti-nociceptive activity test on subcutaneous administration of example 5 (SS8225-19: 1-iminoethyl-[DMT]-[D-Arg]-[Phe]-[N-MeLys]-$NH_2$). Dose-responsible and significant anti-nociceptive activity was expressed by the subcutaneous administration of peptide derivative SS8225-19. The $ED_{50}$ value at 120 minutes after the subcutaneous administration, which is the activity-peak time, was 0.10 mg/Kg; the 95% confidence limit was 0.04-0.22 mg/Kg; and the lasting time with the doses of 0.25 mg/Kg on the subcutaneous administration was 7 hours.

Example 6

Fig. 15 and Fig. 16 show the results of the anti-nociceptive activity test on subcutaneous administration of example 6 (SS8225-20: 1-iminoethyl-[Tyr]-[D-Met(O)]-[Phe]-[N-MeLys]-$NH_2$). Dose-responsible and significant anti-nociceptive activity was expressed by the subcutaneous administration of peptide derivative SS8225-20. The $ED_{50}$ value at 90 minutes after the subcutaneous administration, which is the activity-peak time, was 0.18 mg/Kg; the 95% confidence limit was 0.07-0.43 mg/Kg; and the lasting time with the dose of 0.25 mg/Kg on the subcutaneous administration was 5 hours. Fig. 17 and Fig. 18 show the results of the anti-nociceptive activity test on oral administration of example 6. Dose-responsible and significant anti-nociceptive activity was expressed also by the oral administration. The $ED_{50}$ value at 120 minutes after the oral administration, which is the activity-peak time, was 6.02 mg/Kg; the 95% confidence limit was 4.37-8.28 mg/Kg; and the lasting time with the dose of 10 mg/Kg a on the oral administration was not less than 17 hours.

Example 7

Fig. 19 and Fig. 20 show the results of the anti-nociceptive activity test on subcutaneous administration of example 7 (SS8225-23: 1-iminoethyl-[Tyr]-[D-Met(O)]-[Phe]-[N-MeLys]-$NHCH_3$). Dose-responsible and significant anti-nociceptive activity was expressed by the subcutaneous administration of peptide derivative SS8225-23. The $ED_{50}$ value at 60 minutes after the subcutaneous administration, which is the activity-peak time, was 0.05 mg/Kg; the 95% confidence limit was 0.03-0.09 mg/Kg; and the lasting time with the dose of 0.125 mg/Kg on the subcutaneous administration was 5 hours. Fig. 21 and Fig. 22 show the results of the anti-nociceptive activity test on oral administration of example 7. Dose-responsible and significant anti-nociceptive activity was expressed also by the oral administration. The $ED_{50}$ value at 180 minutes after the oral administration, which is the activity-peak time, was 2.51 mg/Kg; the 95% confidence limit was 2.23-2.83 mg/Kg; and the lasting time with the dose of 10 mg/Kg a on the oral administration was 8 hours.

Example 8

Fig. 23 and Fig. 24 show the results of the anti-nociceptive activity test on subcutaneous administration of example 8 (SS8225-24: 1-iminoethyl-[Tyr]-[D-Met(O)]-[Phe]-[N-MeLys]-$NHCH_3$). Dose-responsible and significant anti-nociceptive activity was expressed by the subcutaneous administration of peptide derivative SS8225-24. The $ED_{50}$ value at 90 minutes after the subcutaneous administration, which is the activity-peak time, was 0.1 mg/Kg; the 95% confidence limit was 0.05-0.24 mg/Kg; and the lasting time with the dose of 0.25 mg/Kg on the subcutaneous administration was 5 hours.

Comparison Example 1

Fig. 25 and Fig. 26 show the results of the anti-nociceptive activity test on subcutaneous administration of comparison example 1 (morphine). Dose-responsible and significant anti-nociceptive activity was expressed by the

subcutaneous administration of morphine. The $ED_{50}$ value at 30 minutes after the subcutaneous administration, which is the activity-peak time, was 2.51 mg/Kg; the 95% confidence limit was 1.48-4.26 mg/Kg; and the lasting time with the dose of 5 mg/Kg on the subcutaneous administration was 90 minutes. Fig. 27 and Fig. 28 show the results of the anti-nociceptive activity test on oral administration of comparison example 1 (morphine). Dose-responsible and significant anti-nociceptive activity was expressed also by the oral administration. The $ED_{50}$ value at 45 minutes after the oral administration, which is the activity-peak time, was 29.92 mg/Kg; the 95% confidence limit was 26.37-33.95 mg/Kg; and the lasting time with the dose of 67.5 mg/Kg a on the oral administration was 5 hours.

Comparison Example 2

Fig. 29 and Fig. 30 show the results of the anti-nociceptive activity test on subcutaneous administration of comparison example 2 (oxycodone). Dose-responsible and significant anti-nociceptive activity was expressed by the subcutaneous administration of oxycodone. The $ED_{50}$ value at 30 minutes after the subcutaneous administration, which is the activity-peak time, was 1.21 mg/Kg; the 95% confidence limit was 0.95-1.54 mg/Kg; and the lasting time with the dose of 1.75 mg/Kg on the subcutaneous administration was 90 minutes. Fig. 31 and Fig. D show the results of the anti-nociceptive activity test on oral administration of comparison example 2 (oxycodone). Dose-responsible and significant anti-nociceptive activity was expressed also by the oral administration. The $ED_{50}$ value at 45 minutes after the oral administration, which is the activity-peak time, was 7.82 mg/Kg; the 95% confidence limit was 6.46-9.47 mg/Kg; and the lasting time with the dose of 20 mg/Kg a on the oral administration was 5 hours.

Comparison Example 3

Fig. 33 and Fig. 34 show the results of the anti-nociceptive activity test on subcutaneous administration of comparison example 3 (SS8225-1: [DMT]-[D-Arg]-[Phe]-[N-MeLys]-$NH_2$). Dose-responsible and significant anti-nociceptive activity was expressed by the subcutaneous administration of peptide derivative SS8225-1. The $ED_{50}$ value at 90 minutes after the subcutaneous administration, which is the activity-peak time, was 0.44 mg/Kg; the 95% confidence limit was 0.33-0.59 mg/Kg; and the lasting time with the dose of 1 mg/Kg on the subcutaneous administration was 5 hours. When peptide derivative SS8225-1 was orally administered, the $ED_{50}$ value of anti-nociceptive activity was over 10 mg/Kg.

Comparison Example 4

Fig. 35 and Fig. 36 show the results of the anti-nociceptive activity test on subcutaneous administration of comparison example 4 (SS8225-5: [DMT]-[D-Met(O)]-[Phe]-[N-MeLys]-$NH_2$). Dose-responsible and significant anti-nociceptive activity was expressed by the subcutaneous administration of peptide derivative SS8225-5. The $ED_{50}$ value at 90 minutes after the subcutaneous administration, which is the activity-peak time, was 0.28 mg/Kg; the 95% confidence limit was 0.21-0.39 mg/Kg; and the lasting time with the dose of 0.5 mg/Kg on the subcutaneous administration was 6 hours. When peptide derivative SS8225-5 was orally administered, the $ED_{50}$ value of anti-nociceptive activity was over 10 mg/Kg.

Comparison Example 5

When comparison example 5 (SS8225-16: 1-iminoethyl-[Tyr]-[D-Met(O)]-[Phe]-[N-MeLys]-OH) was administered orally and subcutaneously, the results of anti-nociceptive activity test showed that the $ED_{50}$ value of anti-nociceptive activity on the subcutaneous administration of SS8225-16 was over 0.5 mg/Kg and the $ED_{50}$ value of anti-nociceptive activity on the oral administration was over 10 mg/Kg. (No Fig. is showed.)

Comparison Example 6

When comparison example 6 (SS8225-17: 1-iminoethyl-[Tyr]-[D-Arg]-[Phe]-[N-MeLys]-OH) was administered orally and subcutaneously, the results of anti-nociceptive activity test showed that the $ED_{50}$ value of anti-nociceptive activity on the subcutaneous administration of SS8225-17 was over 0.5 mg/Kg and the $ED_{50}$ value of anti-nociceptive activity on the oral administration was over 10 mg/Kg. (No Fig. is showed.)

According to the results of the anti-nociceptive activity test (a tail-flick method), example peptide derivatives includes a compound having 2.5 mg/Kg of $ED_{50}$ value on oral administration, which is 10 times more active than the anti-nociceptive activity of morphine on the oral administration and several times more active than the anti-nociceptive activities of oxycodone and peptides according to comparison example. The $ED_{50}$ values of the compounds of the present invention at the activity-peak-time was not more than 0.12 mg/Kg on the subcutaneous administration, which is

20 times more active than the anti-nociceptive activity of morphine on the subcutaneous administration and 10 times more active than the anti-nociceptive activities of oxycodone. Some peptide of comparison examples is more active than the anti-nociceptive activity of oxycodone on the subcutaneous administration, but is less active than the anti-nociceptive activity of oxycodone on the oral administration and is only one several part of examples. The lasting periods of the anti-nociceptive activity of the compounds of the present invention are not shorter than one of morphine.., in some case it is 10 hours which is twice longer than one of morphine. Accordingly it is obvious that the compounds of the present invention have strong anti-nociceptive activity, especially on oral administration. Therefore the present invention may be adaptive to a drug to prevent and/or treat pains, especially as an excellent oral drug.

Then AUC (the area surrounded by the baseline and the time-course reaction curve in a graph showing the relationship between time after administration (elapsed time) and % MPE) calculated using the results obtained by oral and subcutaneous administrations of such drugs, and the correlatgion between the dosage and the AUC are shown in Fig. 37 and 38 respectively, in which AUC is the longitudinal axis and the logarithm of the dosage is the horizontal axis.

It is obvious from these figures that SS8225-04 and SS8225-07 of the present invention may provide the same AUC with a half through one fiftieth dosage of morphine dosage on either oral or subcutaneous administration, and further when the dosage is increased relatively, a very strong anti-nociceptive activity which may be never obtained with normal dosage of morphine is provided.

6. Anti-nociceptive activity test - Part 2 - (Allodynia Experiment)

Further the anti-nociceptive activity test using a nerve damage model envisioning a neuropathic pain was performed.

Nerve-damage Model

Preparation of the nerve-damage model was conducted according to the model created by Seltzer (partial sciatic nerve ligation: RSL model).

Specifically the amount of 0.1 mL/10g of Nembutal solution 7 times diluted with Ringer's solution (Fuso Pharmaceutical Kogyo) was injected into the peritoneal cavity of mouse using 26 gauge needle (Termo) to give anesthetic. The animal was lain on its stomach, its femoral skin was cut open to incise through the fascia, the part between the heads of the biceps femoris was cut and then its sciatic nerve was fixed. The 1/3 to 1/2 part of the right lower leg around the sciatic nerve detached without damaging was completely ligated with a nylon suture thread No. 9 (Natsume Seisakusho) using a vascular needle 000 spring hole (Natsume Seisakusho). Further the cut skin was sutured with the silk thread No.2 (Natsume Seisakusho) using a surgical needle 1 spring needle (Natsume Seisakusho). After the operation, the mice had been kept until the testing date under the above conditions.

Measurement method

The measurement of allodynia effect on the nerve-damage model animals was conducted according to the Von Frey filament method 7 days after the nerve ligation.

Specifically a model animal was put one by one into the compartment having the net floor and after confirmed that the spontaneous movement disappeared after 1 hour or 2 hours, a predetermined amount of each drug solution (morphine, SS8225-04 or SS8225-07) was subcutaneously administered.

Then a few kinds of filaments having different strength of stimulus (Touch-test Sensory Evaluator Instruction North Coast Medical Inc.) were pushed perpendicularly on the sole of the hinder leg until the filament was bent. The threshold value at which the animal showed escape reaction was measured as a gram weight (in Fig. "gram" (1 gram weight is 0.00981 N)).

Further, for comparison, the left lower leg of the animal without nerve-damage was also stimulated and the threshold values were measured. AUCs (the area surrounded with the time-reaction curve and the base line of a graph showing the relationship between the time after administration (time-course) and %MPE) were calculated from the obtained results. Antiallodynia activity obtained from the AUC divided by the measuring time was compared as strength of activity per unit of the time period.

Test Results

Allodynia measurements of respective doses of Morphine (2.5 to 10 mg, S.C.), SS8225-04 (0.125 to 0.7 mg/Kg S.C.) and SS8225-07 (0.125 to 1.0 mg/Kg, S. C.) were conducted.
Herein the results when SS8225-04 was used are shown in Fig. 39 (the relationship between the administration of SS8225-04 on the subcutaneous administration and AUC); the results when 0.125 mg/Kg (body weight), 0.25 mg/Kg

(body weight), 0.5 mg/Kg (body weight) or 0.7 mg/Kg (body weight) of SS8225-04 were subcutaneously administered respectively are shown in Fig. 40 through Fig. 43; the results when SS8225-07 was used are shown in Fig. 44 (the relationship between the administration of SS8225-07 on the subcutaneous administration and AUC); the results when 0.125 mg/Kg (body weight), 0.25 mg/Kg (body weight), 0.5 mg/Kg (body weight) or 0.7 mg/Kg (body weight) of SS8225-07 were subcutaneously administered respectively are shown in Fig. 45 through Fig. 48; the results when morphine was used are shown in Fig. 49 (the relationship between the administration of morphine on the subcutaneous administration and AUC); and the results when 2.5 mg/Kg (body weight), 3.5 mg/Kg (body weight), 5 mg/Kg (body weight) , 7 mg/Kg (body weight) or 10 mg/Kg (body weight) of morphine were subcutaneously administered respectively are shown in Fig. 50 through Fig. 53.

It is confirmed from these graphs that peptide derivatives of the present invention, SS8225-04 and SS8225-07 have high anti-nociceptive activities, the dose of these peptides to provide the same level of anti-nociceptive activity as morphine are 1/10 through 1/20 amount of morphine, further at that level the lasting period of anti-nociceptive activities of the peptides are about twice longer than the lasting period of morphine.

Neuropathic pain has allodynia as a main symptom and resists to Morphine as an opioid. According to this experiment, anti-allodynia effect due to S. C. administration of Morphine to the animals of which sciatic nerve was ligated significantly decreased at the left hinder leg. In contrast, when SS8225-04 and SS8225-07 were administered, decrease of anti-allodynia effect was not observed at any doses. Specifically, it was obvious that anti-allodynia effect did not decrease in accordance with an administration of SS8225-04 and SS822-07 and these results were different from the result obtained on Morphine administration.

Accordingly it was confirmed that peptide derivatives of the present invention, SS8225-04 and SS8225-07, had high anti-nociceptive activity on the nerve damage model envisioning a neuropathic pain and non-diminishing antiallodynia activity.

7. Anti-nociceptive activity test - Part 3 - Tail-pressure test)

A pressure-stinging test to investigate further application to other anti-nociceptive activity in addition to the tail-flick test and the neuropathic pain test using the nerve damage model noted above was conducted.

Measurement method

The predetermined amount of the peptide derivatives SS8225-04 and SS8225-07 of the present invention and morphine as a related art were subcutaneously or orally administered to mice of which the tail part was pressured with an increasing pressure ratio of 133 Pa (10 mmHg)/sec. The pressure when the mouse showed flounce or biting the pressured part was measured as a pain-reaction threshold value. The mouse which reacted to 532 through 665 Pa (40 - 50 mmHg) was reselected for the test. In addition the maximal stimulating pressure was set as 1333 Pa (100 mmHg). Then %MPE at this point was calculated from formula (I) as well as noted above.

Test results

The time-course variation graph of anti-nociceptive activity on the tail-pressure test after the subcutaneous administration of SS8225-04 is shown in Fig. 56; the dose-response curve graph thereon is shown in Fig. 57; the time-course variation graph of anti-nociceptive activity on the tail-pressure test after the oral administration of SS8225-04 is shown in Fig. 55; and the dose-response curve graph thereon is shown in Fig. 58, respectively.

The time-course variation graph of anti-nociceptive activity on the tail-pressure test after the subcutaneous administration of SS8225-07 is shown in Fig. 59; the dose-response curve graph thereon is shown in Fig. 60; the time-course variation graph of anti-nociceptive activity on the tail-pressure test after the oral administration of SS8225-07 is shown in Fig. 61; and the dose-response curve graph thereon is shown in Fig. 62, respectively.

The time-course variation graph of anti-nociceptive activity on the tail-pressure test after the subcutaneous administration of morphine is shown in Fig. 63; the dose-response curve graph thereon is shown in Fig. 64; the time-course variation graph of anti-nociceptive activity on the tail-pressure test after the oral administration of morphine is shown in Fig. 65; and the dose-response curve graph thereon is shown in Fig. 66, respectively.

It is confirmed from these graphs that peptide derivatives of the present invention, SS8225-04 and SS8225-07 have high anti-nociceptive activities, the dose of these peptides to provide the same level of anti-nociceptive activity as morphine are 1/10 through 1/20 amount of morphine, further the lasting period of anti-nociceptive activities more than 6 hours (s. c.) or more than 12 hours (p. o.); and respective lasting periods are far longer than the lasting period of anti-nociceptive activity of morphine.

Then AUC calculated using the results obtained by oral and subcutaneous administrations of such drugs, and the correlation between the dosage and the AUC are shown in Fig. 67 and 68 respectively, in which AUC is the longitudinal

axis and the logarithm of the dosage is the horizontal axis.

It is obvious from these figures that SS8225-04 and SS8225-07 of the present invention may provide the same AUC with one hundredth through one twentieth dosage of morphine dosage on either oral or subcutaneous administration, and further when the dosage is increased relatively, a very strong anti-nociceptive activity which may be never obtained with normal dosage of morphine is provided.

8. Anti-nociceptive activity test - part 4 - (Anti-nociceptive activity test when MSU induced knee osteoarthritis pain generated by artificial means envisioning a pain due to knee osteoarthritis)

MSU induced knee osteoarthritis pain generated by artificial means as an knee osteoarthritis pain model was used to evaluate anti-nociceptive activity on this pain in comparison with anti-nociceptive activity of morphine and oxycodone.

Experimental Animal

Experimental animal, male SD type rats, weighing the body weight in the range of 170 to 190g, were used for the studies. The animals had been kept under constant conditions (room temperature 23 ± 1°C, humidity 52 ± 2%, 12 hours light-and-dark cycle having light time 7:00 to 19:00 and dark time 19:00 to 7:00) until used for an experiment. The animals were fed the mouse solid diet (F2, Funabashi farm. Funabashi, Japan) with unrestricted tap water intake, and kept in captivity for at least more than two days in a laboratory.

Test Drugs

Morphine (Sankyo), oxycodone (Mallinckrodt), SS8226-04 and SS8226-07 were tested. Any test drugs were dissolved in Ringer's solution (Fuso Yakuhin) to be used.

Administration of induction substance (sodium urate needle crystal: MSU)

MSU was suspended with the suspension solution (10% of polyoxyethylene sorbitan mono-oleate (Tween 80: Nakarai Tesk) as 0.1 g per 1 mL. The animal was anesthetized with diethyl ether and after the surface of its left hind leg knee joint was wiped with 70% ethanol cotton, a porous administration of the suspension solution above noted was conducted into the cavity of let hind leg knee joint.

Measurement of both hind leg paw-pressure and calculation of both hind leg paw-pressure ratio

Pressures to both hind leg paw were measured using a paw-pressure measurement apparatus and recorded. Measurement time at respective times was 4.5 seconds. Calculated paw-pressure ratio (ppr) is a value calculated by dividing the pressure on the left hind leg by the pressure on the right hind leg according to the measured pressures on both hind legs.

The anti-nociceptive effect was evaluated quantitatively by calculating %MPE (% of maximum possible effect) according to the following formula.

[M 2]

$$\%MPE = \frac{ppr\ 1 - ppr\ 0}{(1 - ppr\ 0)} \times 100 \text{ ------ Formula (II)}$$

In the formula, ppr 0 and ppr 1 are respective paw-pressure ratios on both hind legs before and after administration. $ED_{50}$ value of anti-nociceptive activity was calculated using GraphPad Prism data-graph processing software version 4.0, San Diego, California, USA.

Calculation of area under curve: AUC

The strength of anti-nociceptive activity was represented by calculating the time-course variation of anti-nociceptive

activity AUC using GraphPad Prism noted above. In addition, $ED_{50}$ of anti-nociceptive activity was calculated using GraphPad Prism as the theoretical maximum AUC was [100% (theoretical maximum reaction of each measurement) x a period of measurement time (minute)] every medication.

Paw-pressure was measured twice at 4 hours after administration to provide ppr by calculation and the average of those values was both hind leg paw-pressure ratio before the drug administration. After the measurement of both hind leg paw-pressure before ther drug administration, 0.1 mL of Ringer's solution or a test drug in Ringer's solution per 100g rat body weight was orally administered, and then a paw-pressure was measured once every unit time after the oral administration.

Statistic analysis

Statistical processing was conducted using GraphPad Prism. After the two-way layout dispersion analysis, the hazard-ratio of P<0.05 was decided to be a significant difference according to Bonferroni post-test. The hazard-ratios were distinguished into less than 5% (P<0.05) and less than 1% (P<0.01) and represented as "*" and "**" in the same manner conducted for the Tail-flick method.

Test results

The results of each test drug's effect on pains induced by administration of MSU into the hind leg knee joint cavity of a rat are shown in Fig, 69 through Fig. 78. Specifically the time-course variation of %MPE after oral administration and the results of respective dosages of morphine, oxycodone, SS8225-04 and SS8225-07 are shown in Fig. 69 through Fig. 72 respectively, and the AUS results of morphine, oxycodone, SS8225-04 and SS8225-07 are shown in Fig. 73 through Fig. 76. Further AUC is longitudinal axis and dosage is horizontal axis in the graph of Fig. 77, %MPE is longitudinal axis and dosage is horizontal axis in the graph of Fig. 78.

According to Fig. 69 through 72, activity lasting-times when morphine, oxycodone, SS822504 and SS8225 were orally administered were 300, 120, 420 and 360 minutes respectively. Respective activity lasting-times for oxycodone, SS8225-04 and SS8225-07 were 0.4, 1.4 and 1.2 times longer than for morphine. Activity peaks for morphine, oxycodone, SS8225-04 and SS8225-07 were 90, 60, 180 and 120 minutes respectively, and $ED_{50}$ values at respective activity peaks were 37.23 (17.65 - 78.56) mg/Kg, 10.35 (8.09 -13.25) mg/Kg, 15.84 (11.24 - 22.32) mg/kg and 16.95 (3.17 - 90.59) mg/kg respectively. Respective anti-nociceptive activity lasting-times for oxycodone, SS8225-04 and SS8225-07 were 3.6, 2.4 and 2.2 times longer than for morphine.

According to Fig. 73 through 76, AUC of time-course variation of anti-nociceptive activity were studied. AUC for Ringer's solution oral administration was 1283; AUCs for 15, 30 and 60 mg/kg of morphine were 1724, 4485 and 13883; AUCs for 7.5, 15 and 30 mg/kg of oxycodone were 2525, 6363 and 10562; AUCs for 7.5, 15 and 30 mg/kg of SS8225-04 were 6410, 8133 and 24702; AUCs for 7.5, 15 and 30 mg/kg of SS8225-07 were 2919, 7894 and 20162. $ED_{50}$ of AUCs for morphine, oxycodone, SS8225-04 and SS8225-07 were 86.38 (48.89 - 152.6) mg/kg, 49.35 (9.98-243.9) mg/kg, 34.10 (3.36 - 345.8) mg/kg and 40.38 (31.08 - 52.48) mg/kg. Accordingly, anti-nociceptive activities for oxycodone, SS8225-04 and SS8225-07 were approximately 1.8, 2.5 and 2.1 times stronger than for morphine.

In addition, according to Fig. 77 and 78 anti-nociceptive activity of SS8225-04 and SS8225-07 were stronger than morphine, and stronger than or equal to oxycodone thereof on model experiments for knee osteoarthritis.

9. Anti-nociceptive activity test - part 5 - (Anti-nociceptive activity test when rheumatoid arthritis pain generated by artificial means envisioning a pain due to inflammatory pain)

Anti-nociceptive activity of SS8225-04 of the present invention against an inflammatory pain generated for mice using an adjuvant on oral administration were studied in comparison with morphine.

Experimental Animal

Experimental animal, male ddy type mice (Nihon SLC), weighing the body weight in the range of 15 to 20g, were used for the studies. The animals had been kept under constant conditions (room temperature 23 ± 1 °C, humidity 52 ± 2%, 12 hours light-and-dark cycle having light time 7:00 to 19:00 and dark time 19:00 to 7:00) until used for an experiment. The animals were fed the mouse solid diet (F2, Funabashi farm. Funabashi, Japan) with unrestricted tap water intake, and kept in captivity for at least more than two days in a laboratory.

Test Drugs

**[0231]** [00214] Complete Freund's Adjuvant hereinafter CFA (Sigma), morphine and SS8226-04 were used. Morphine

and SS8225-04 were dissolved in Ringer's solution to be orally administered.

Inflammatory pain model

A mouse was firmly fixed without anesthesia and 25 μL of Complete Freund's Adjuvant (CFA) using a number 30 guage needle (Termo) was subcutaneously administered into the dorsum of left hind foot to provide an inflammatory pain model. The model was kept under the conditions noted above until used for the text.

Measurement of anti-nociceptive activity on the inflammatory pain model was conducted according to Von Fray filament method as well as on never damaged model 5 days after CFA administration. The drugs were orally administered.

Time-course variation of threshold value after the drug administration was measured and then AUC was calculated from the obtained result. The anti-nociceptive activity is represented as a unit AUC value (AUC / min) which is the total AUC value or AUC divided by the measurement time and compared to study. $ED_{50}$ of the anti-nociceptive activity was calculated by reducing the measurement value to a percentage of the upper limit which was set as 2.0 for the unit AUC.

Test results

Inflammatory pain model

The pain threshold of CFA non-administered foot was 0.50 gram weight at 5 days after CFA administration versus approximately 0.25 gram weight as the pain threshold of CFA administered foot. It was obvious that an inflammatory allodynia was provided.

Anti-nociceptive activity of morphine

The anti-nociceptive activities of morphine were shown in Fig. 79 (on CFA non-administered foot) and in Fig. 80 (on CFA administered foot). (However, the results at between 30 and 90 min after administration were very varied, and especially the data for 100 mg/kg of morphine at 60 min after administration was extremely varied so that this data was not shown in Fig. 79 and Fig. 80.)

An anti-nociceptive activity of morphine on the oral administration (35, 70 or 100 mg/Kg) was observed dose-dependently with the peak at 30 minutes after administration, and its lasting time was 360 minutes. The total AUC value and the unit-time AUC value of the CFA administered foot was approximately 60% of the total AUC value and the unit-time AUC value of the CFA non-administered foot in comparison of the anti-nociceptive activities between CFA non-administered foot and CFA administered foot. The total AUC values of the group which 100 mg/Kg was orally administered to were 556 for the CFA non-administered foot and 328 for the CFA administered foot. On the other hand when $ED_{50}$ of the anti-nociceptive activity was calculated using the unit-time AUC value (AUC/min), $ED_5$- values for CFA non-administered foot and CFA administered foot were 67.88 mg/Kg and 113.0 mg/Kg respectively. In addition the unit-time AUC values for the group which 100 mg/Kg were orally administered 1.545 for the CFA.non-administered foot and 0.910 for the CFA administered foot.

The anti-nociceptive activities of SS8825-04 were shown in Fig. 81 (on CFA non-administered foot) and in Fig. 81 (on CFA administered foot).

An anti-nociceptive activity of SS8225-04 on the oral administration (7, 10 or 14 mg/Kg) was observed dose-dependently with the peak at 300 minutes after administration, and its lasting time was 720 minutes which was extremely longer lasting than for morphine. The total AUC value and the unit-time AUC value of the CFA administered foot was approximately 60% of the total AUC value and the unit-time AUC value of the CFA non-administered foot in comparison of the anti-nociceptive activities between CFA non-administered foot and CFA administered foot. The total AUC values of the group which 14 mg/Kg was orally administered to were 1058 for the CFA non-administered foot and 577 for the CFA administered foot. Respective values were twice of the values for 100 mg/Kg of morphine administered. On the other hand when $ED_{50}$ of the anti-nociceptive activity was calculated using the unit-time AUC value (AUC/min), $ED_5$-values for CFA non-administered foot and CFA administered foot were 9.849 mg/Kg and 17.04 mg/Kg respectively. The anti-nociceptive activities were in the range of 6.6 and 6.9 times stronger than morphine. In addition the unit-time AUC values for the group which 14 mg/Kg were orally administered 1.470 for the CFA non-administered foot and 0.814 for the CFA administered foot.

According to the results of SS8225-04 and morphine on the oral administration, AUC and AUC per unit-time (unit-time AUC) were calculated. The relationship between the dosage and the AUC was shown in Fig. 83 in which AUC was the longitudinal axis and dosage was the horizontal axis; and the relationship between the dosage and the unit-time AUC was shown in Fig. 84 in which unit-time AUC was the longitudinal axis and the logarithm of dosage was the horizontal axis.

According to Fig. 83, SS8225-04 in the range of one hundredth and one fiftieth dosage of morphine dosage may provide satisfactorily the same AUC; and in addition when the dosage of SS8255-04 is relatively increased, an extremely strong anti-nociceptive activity, which morphine can never provide, may be obtained. In addition, SS8225-04 in the range of one eighth and one fourth dosage of morphine dosage may provide satisfactorily the same unit-time AUC.
In addition, although the inventors compared the anti-nociceptive activity of the present invention with morphine and other drugs in anti-nociceptive activity comparison, the present invention may be applied to other areas where none of traditional drug has not been used, and such applications naturally should be involved in the present invention in addition to the areas where a traditional drug has been used.

10. Evaluation of acute toxicity

Since the $ED_{50}$ of SS8225-04 of the present invention was 0.05 mg/Kg (s.c.) in Table 2, 100 mg/Kg, 50mg/Kg, 25 mg/Kg and 6.25 mg/Kg corresponding to 2000, 1000, 500 and 125 times more amount than the $ED_{50}$ value were administered to evaluate acute toxicity thereof.
Mice weighing in the range of 22 to 25 g, male ddy mouse, were employed. Number of mice out of five mice in each group that died within 72 to 96 hours recorded. Also, since the $ED_{50}$ of morphine was 2.51 mg/Kg (s.c.) in Table 2, 1000 mg/Kg and 500 mg/Kg corresponding to 400 and 200 times more amount than the $ED_{50}$ value were administered to study death number. The result is shown in Table 3.
Table 3:

| Dosage of SS8225-04 (mg/Kg) | Number of mice died (out of five) | Lethality (%) |
|---|---|---|
| 100 | 1 | 20 |
| 50 | 1 | 20 |
| 25 | 0 | 0 |
| 6.25 | 0 | 0 |

Table 4:

| Dosage of morphine (mg/Kg) | Number of mice died (out of five) | Lethality (%) |
|---|---|---|
| 1000 | 51 | 100 |
| 500 | 01 | 0 |

According to Table 4, when 2000 and 1000 times more amount of the $ED_{50}$ of SS8225-04 of the present invention were administered (S.C.); the lethality was 20%; and when 500 times more amount was administered, the lethality was 0%. When 400 times more amount of the $ED_{50}$ of morphine was administered (S.C.), the lethality was 100%. Accordingly, it was confirmed that the acute toxicity of SS8225-04 of the present invention was satisfactorily low. Having described at least one of the preferred embodiments of the present invention with reference to the accompanying drawings, it is to be understood that the invention is not limited to those precise embodiments, and that various changes, modifications, and adaptations may be effected therein by one skilled in the art without departing from the scope or spirit of the invention as defined in the appended claims.

## Claims

**1.** At least one of a compound and a pharmacologically acceptable salt thereof, said at least one comprising:

a general formula shown in chemical formula (1);

$$R^1N=C(R^2)\text{-}AA^1\text{-}AA^2\text{-}AA^3\text{-}AA^4\text{-}Y \qquad (1)$$

wherein said $R^1$ is selected one of hydrogen atom, hydroxyl group, low alkyl group, and low-alkoxyl group;
wherein said $R^2$ is a low-alkyl group; and
wherein said Y is represented by chemical formula (2) below;

$$-n(R^3)R^4 \qquad (2);$$

wherein said $R^3$ and said $R^4$ in chemical formula (2) are respectively and independently selected one of hydrogen atom, hydroxyl group, low-alkyl group, and low-alkoxyl group; or five or six member nitrogen-containing heterocyclic group in which the nitrogen atom is connected to $R^3$ and $R^4$ ;
wherein said $AA^1$ is an α-amino acid residue represented by chemical formula (3) below;

[C1]

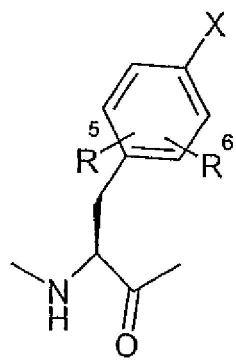

(3)

wherein said $R^3$ and said $R^4$ in chemical formula (3) are respectively and independently selected one of hydrogen atom, halogen atom, low-alkyl group, and halogenated low-alkyl group;
wherein X in chemical formula (3) is selected one of hydrogen atom, halogen atom, hydroxyl group, a group represented by chemical formula (4) below and by chemical formula (5);

$$-O-CO-R^7 \qquad (4)$$

$$-O-CO-O-R^8 \qquad (5)$$

wherein said $R^7$ and said $R^8$ in chemical formula (4) and in chemical formula (5) are respectively and independently selected one of $C_{1-16}$ alkyl group, hydroxy-$C_{1-16}$ alkyl group, amino- $C_{1-16}$ alkyl group, (mono-low-alkyl)-amino-$C_{1-16}$ alkyl group, (di-low-alkyl)-amino- $C_{1-16}$ alkyl group, $C_{3-10}$ cycloalkyl group, $C_{3-10}$ cycloalkyl substituted low-alkyl group, $C_{2-16}$ alkenyl group, $C_{2-16}$ alkynyl group, heterocyclic group, aryl group, and aryl-substituted low-alkyl group;
wherein said $AA^2$ is a D-α-amino acid residue represented by chemical formula (6) below;
wherein said $R^9$ in chemical formula (6) is selected one of amino group, (mono-alkyl-low)-amino group, low-acylamino group, guanidino group, low-alkyl substituted guanidino group, imino low-alkyl group, ureide group, low-alkyl-substituted ureide group, low-alkylthio group, low-alkylsulfinyl group, low-alkylsulfonyl group, low-acyl group, and hydroxy low-alkyl group, wherein n is an integer of 1 thorough 4,

[C2]

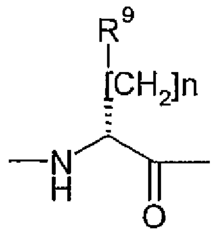

(6)

wherein said $AA^3$ is selected one of non-substituted phenylalanine residue, substituted phenylalanine residue, non-substituted D-phenylalanine residue and substituted D-phenylalanine residue;
wherein said $AA^4$ is an $\alpha$-amino acid residue represented by chemical formula (7) below or

$$-N(R^{10})-CH(R^{11})-CO- \qquad (7)$$

a $\beta$-amino acid residue represented by chemical formula (8) below;

$$-N(R^{10})-CH(R^{11})-CH(R^{12})-CO- \qquad (8)$$

wherein said $R^{10}$ and said $R^{12}$ in chemical formula (7) and (8) are respectively and independently selected one of hydrogen, low-alkyl group, low-alkenyl group, low-alkynyl group, aryl group, and aryl-substituted low-alkyl group;
wherein said $R^{11}$ is hydrogen atom or a group represented by chemical formula (9) below,

$$-Z-N(R^{13})-R^{14} \qquad (9)$$

wherein said Z in chemical formula (9) is selected one of low-alkylene group, low-alkenylene group, and low-alkynylene group, and
wherein said $R^{13}$ and said $R^{14}$ are respectively and independently selected one of hydrogen atom, low-alkyl group, aryl group, and aryl-substituted-low-alkyl group, or said $R^{13}$ and said $R^{14}$ are five or six member nitrogen-containing heterocyclic group in which nitrogen atom is connected to said $R^{13}$ and said $R^{14}$.

**2.** At least one of a compound and a pharmacologically acceptable salt thereof, according to claim 1, wherein:

said $R^1$ which is a hydrogen atom.

**3.** At least one of a compound and a pharmacologically acceptable salt thereof, according to claim 1, wherein:

said $R^2$ which is a methyl group or an ethyl group.

**4.** At least one of a compound and a pharmacologically acceptable salt thereof, according to claim 1, wherein:

said $AA^3$ which is an $\alpha$-amino acid residue represented by chemical formula (10)

[C3]

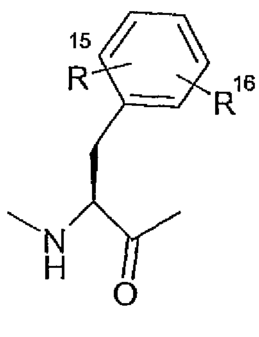

(10)

or D-α-amino acid residue represented by chemical formula (11);

[C4]

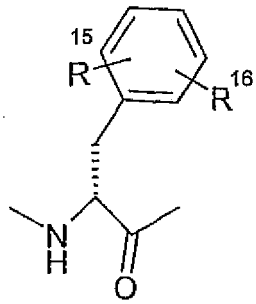

(11)

wherein said $R^{15}$ and said $R^{16}$ in formula (10) and (11) are respectively and independently selected one of hydrogen atom, halogen atom, low-alkyl group, and halogenated low-alkyl group.

**5.** At least one of a compound and a pharmacologically acceptable salt thereof, according to claim 1, wherein:

said $AA^3$ which is selected one of phenylalanine residue, D-phenylalanine residue, p-fluorophenylalanine residue, D-p-fluorophenylalanine residue, o-trifluoromethylphenylalanine residue, D-o-trifluoromethylphenylalanine residue, and 2,6-dimethylphenylalanine residue

**6.** At least one of a compound and a pharmacologically acceptable salt thereof, according to claim 1, wherein:

said $AA^4$ which is N-methyllysine residue or N-methyl-β-alanine residue.

**7.** At least one of a compound and a pharmacologically acceptable salt thereof, according to claim 1, wherein:

said X which in chemical formula (3) is hydroxy group.

**8.** At least one of a compound and a pharmacologically acceptable salt thereof, according to claim 1, wherein:

said X in chemical formula (3) which is hydrogen atom or halogen atom.

**9.** At least one of a compound and a pharmacologically acceptable salt thereof, according to claim 1, wherein:

said X in chemical formula (3) which is represented by chemical formula (4) or (5); and

wherein said $R^7$ in chemical formula (4) and said $R^8$ in chemical formula (5) which are respectively and independently selected one of $C_{1-16}$ alkyl group, hydroxy-$C_{1-16}$ alkyl group, amino- $C_{1-16}$ alkyl group, (mono-low-alkyl)-amino-$C_{1-16}$ alkyl group, (di-low-alkyl)-amino- $C_{1-16}$ alkyl group, $C_{3-10}$ cycloalkyl group, $C_{3-10}$ cycloalkyl substituted low-alkyl group, $C_{2-16}$ alkenyl group, $C_{2-16}$ alkynyl group, aryl group, heterocyclic group, and aryl-substituted low-alkyl group.

**10.** At least one of a compound and a pharmacologically acceptable salt thereof, according to claim 1, wherein:

said $AA^1$ which is selected one of tyrosine residue, 2,6-dimethyltyrosine residue, o-acyltyrosine residue, o-alkoxycarbonyl tyrosine residue, o-phenoxycarbonyl tyrosine residue, o-acetyl tyrosine residue, and 2,6-dimethylphenyl alanine residue.

**11.** At least one of a compound and a pharmacologically acceptable salt thereof, according to claim 1, wherein:

said $AA^2$ which is selected one of D-methionine sulfoxide residue, D-arginine residue, and D-citrulline residue.

**12.** At least one of a compound and a pharmacologically acceptable salt thereof, according to claim 1, wherein:

said $AA^2$ which is selected one of D-$N^5$-acetylornithine residue, D-5-oxonorleucine residue, and D-5-hydroxynorleucine residue.

**13.** At least one of a compound and a pharmacologically acceptable salt thereof, according to claim 1, wherein:

said $R^3$ and said $R^4$ which are independently selected one of hydrogen atom, hydroxy group, low-alkyl group and low-alkoxy group.

**14.** At least one of a compound and a pharmacologically acceptable salt thereof, according to claim 1, further comprising:

said $R^1$ which is hydrogen atom, said $R^2$ which is methyl group, said $AA^1$ which is o-acetyltyrosine residue, said $AA^2$ which is D-methionine sulfoxide residue or D-arginine residue, said $AA^3$ which is phenylalanine residue, said $AA^4$ which is N-methyllysine residue or N-methyl-β-alanine residue, and Y is $-NH_2$ or $-NH-CH_3$.

**15.** At least one of a compound and a pharmacologically acceptable salt thereof, according to claim 1, further comprising:

said $R^1$ which is hydrogen atom, said $R^2$ which is methyl group, said $AA^1$ which is tyrosine residue, said $AA^2$ which is D-methionine sulfoxide residue or D-arginine residue, said $AA^3$ which is phenylalanine, said $AA^4$ which is N-methyllysine residue or N-methyl-β-alanine residue, said Y which is $-NH_2$ or $-NH-CH_3$.

**16.** At least one of a compound and a pharmacologically acceptable salt thereof, according to claim 1, further comprising:

said $R^1$ which is hydrogen atom, said $R^2$ which is methyl group, said $AA^1$ which is o-acetyltyrosine residue, said $AA^2$ which is D-methionine sulfoxide residue or D-arginine residue, said $AA^3$ which is phenylalanine, said $AA^4$ which is N-methyllysine residue or N-methyl-β-alanine residue, said Y which is $-NH_2$ or $-NH-CH_3$.

**17.** At least one of a compound and a pharmacologically acceptable salt thereof, according to claim 1, further comprising
said $R^1$ which is hydrogen atom, said $R^2$ which is methyl group, said $AA^1$ which is tyrosine residue, said $AA^2$ which is D-5-hydroxynorleucine residue, said $AA^3$ which is phenylalanine, said $AA^4$ which is N-methyllysine residue or N-methyl-β-alanine residue, said Y is $- NH_2$ or $-NH-CH_3$.

**18.** At least one of a compound and a pharmacologically acceptable salt thereof, according to claim 1, further comprising:

said $R^1$ which is hydrogen atom, said $R^2$ which is methyl group, said $AA^1$ which is tyrosine residue, said $AA^2$ which is D-methionine sulfoxide residue, said $AA^3$ which is phenylalanine, said $AA^4$ which is N-methyllysine residue or N-methyl-β-alanine residue, said Y which is $-NH_2$ or $-NH-CH_3$.

**19.** At least one of a compound and a pharmacologically acceptable salt thereof, according to claim 1, further comprising:

said $R^1$ which is hydrogen atom, said $R^2$ which is methyl group, said $AA^1$ which is 2,6-dimethyltyrosine residue, said $AA^2$ which is D-methionine sulfoxide residue or D-arginine, said $AA^3$ which is phenylalanine, said $AA^4$ which is N-methyllysine residue or N-methyl-β-alanine residue, said Y which is $-NH_2$ or$-NH-CH_3$.

**20.** A pharmaceutical composition, comprising:

at least one of a compound and a pharmacologically acceptable salt thereof, according to claim 1, wherein:

said pharmaceutical composition is at least one of an active substance and a pharmacologically acceptable carrier.

**21.** A pharmacological composition, comprising:

at least one of a compound and a pharmacologically acceptable salt thereof, according to claim 1, wherein: :

said pharmaceutical composition enables at least one of the prevention of pain and a use in a treatment of pain

**23.** A pharmaceutical composition, according to claim 22, wherein:

said pain is a cancer pain.

**24.** A pharmaceutical composition, according to claim 22, wherein:

said pain is a neuropathic pain.

**25.** A pharmaceutical composition, according to claim 22, wherein:

said pain is a knee osteoarthritis pain.

**26.** A pharmaceutical composition, according to claim 22, wherein:

said pain is a rheumatoid arthritis pain.

Fig. 1

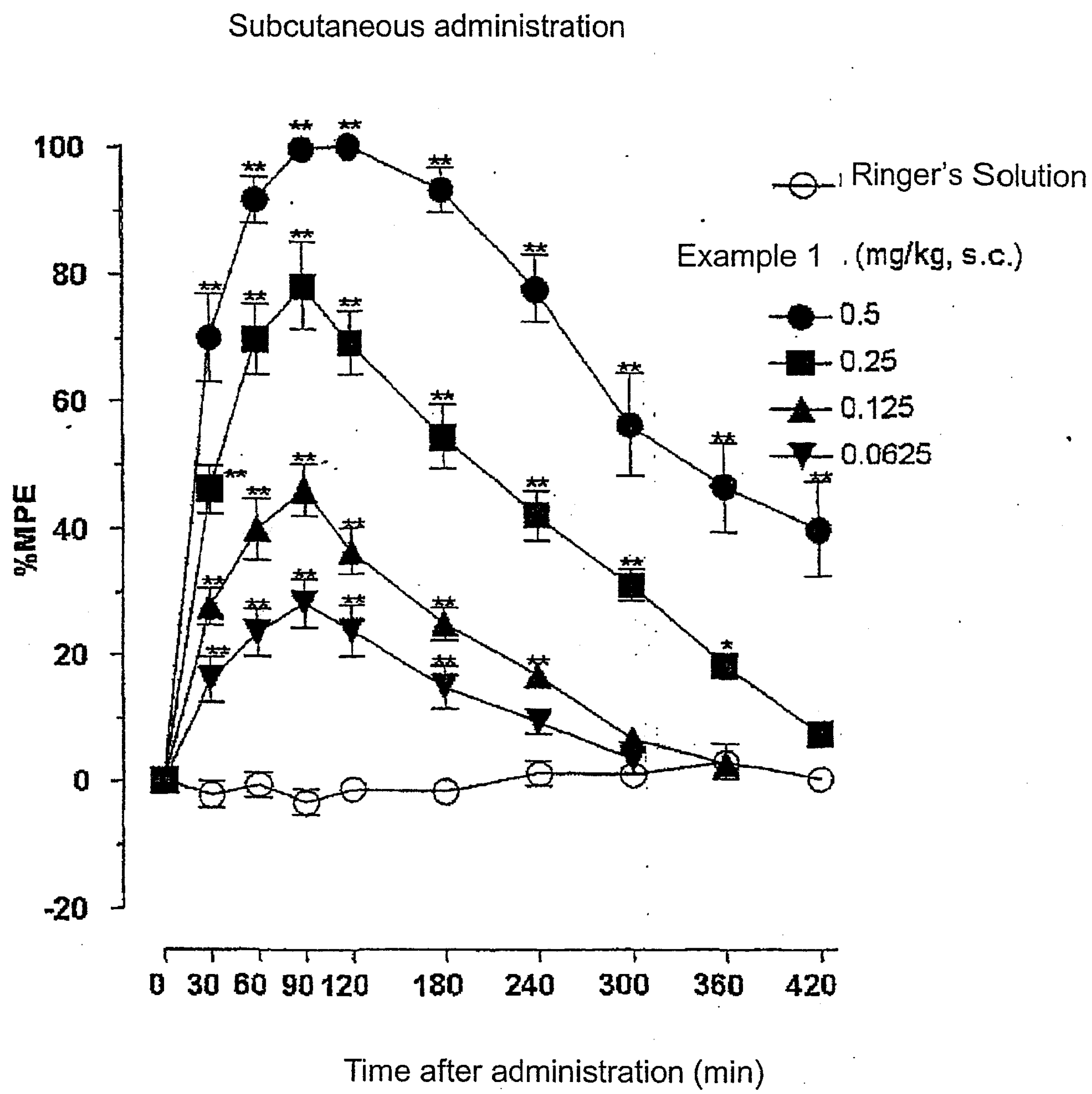
Subcutaneous administration
%MPE
100
80
60
40
20
0
-20
0 30 60 90 120 180 240 300 360 420
Time after administration (min)
Ringer's Solution
Example 1 (mg/kg, s.c.)
0.5
0.25
0.125
0.0625

Fig. 2

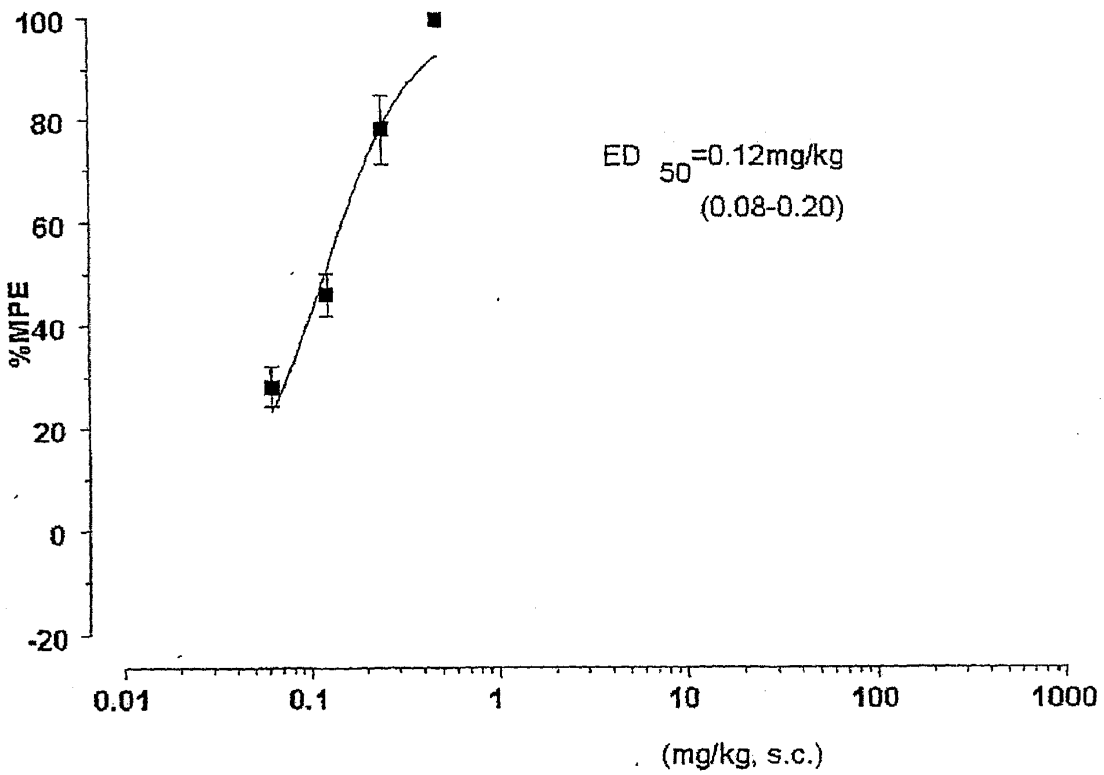


Example 1: Dosage on subcutaneous administration

Fig. 3

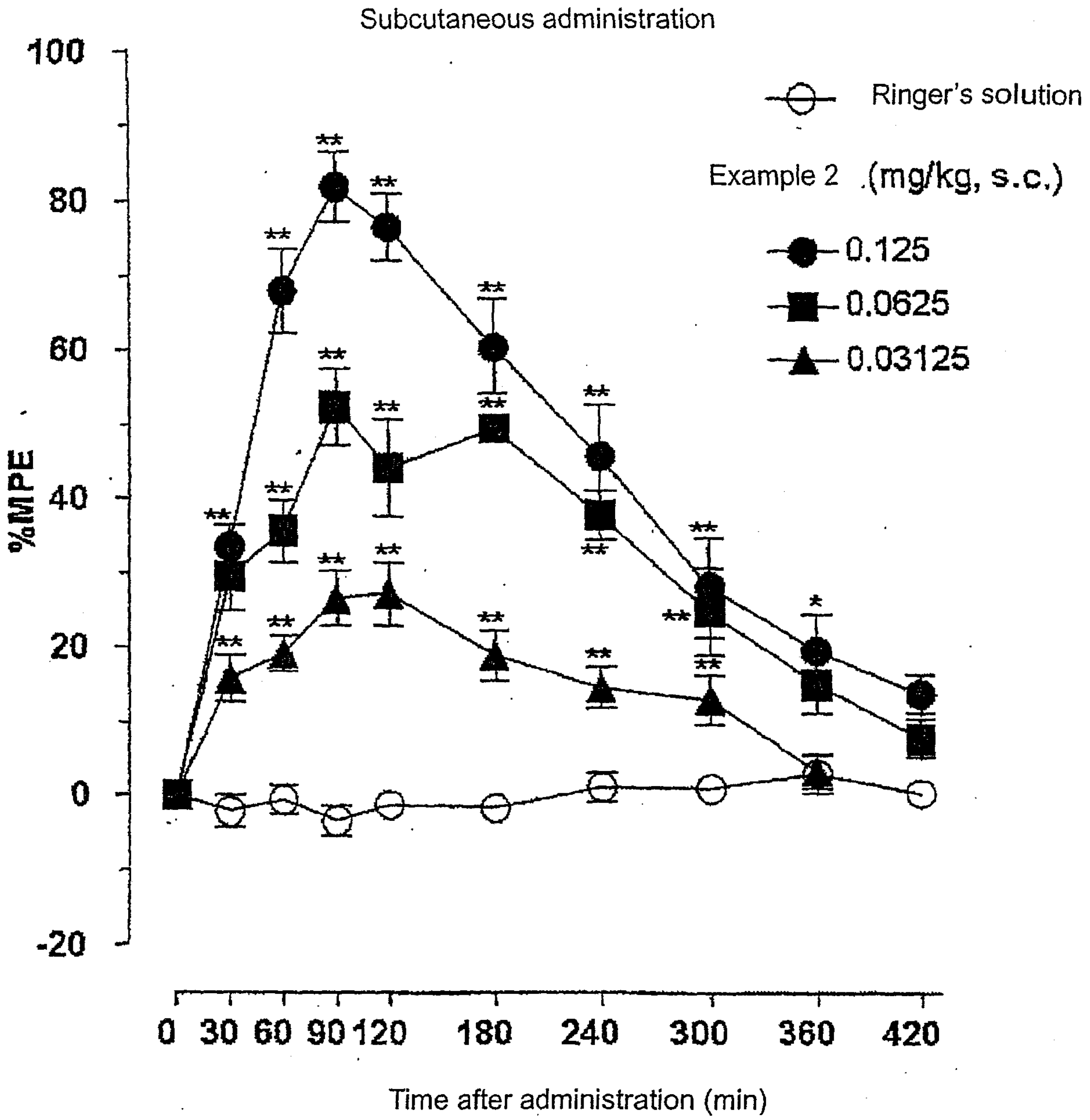
Subcutaneous administration
100
80
60
40
20
0
-20
%MPE
Ringer's solution
Example 2 (mg/kg, s.c.)
0.125
0.0625
0.03125
0 30 60 90 120 180 240 300 360 420
Time after administration (min)

Fig. 4

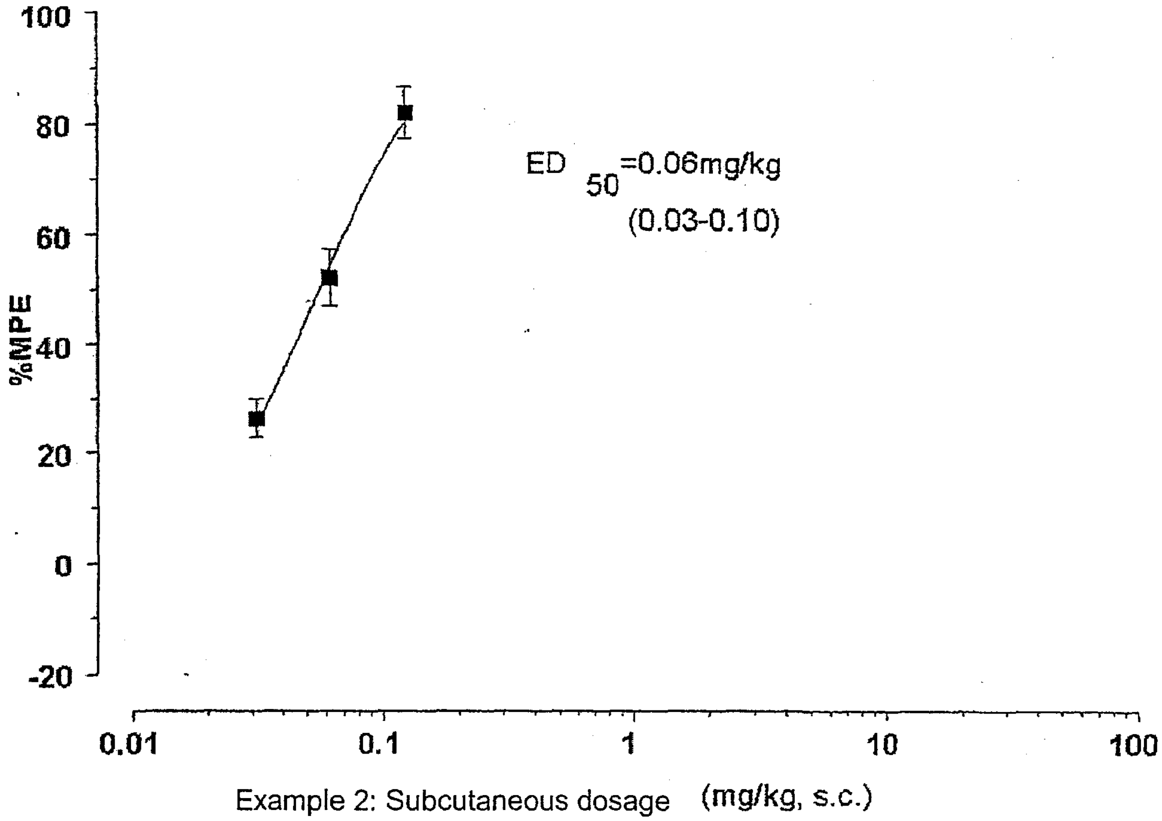
100
80
60
40
20
0
-20
%MPE
ED 50=0.06mg/kg
(0.03-0.10)
0.01
0.1
1
10
100
Example 2: Subcutaneous dosage (mg/kg, s.c.)

Fig. 5

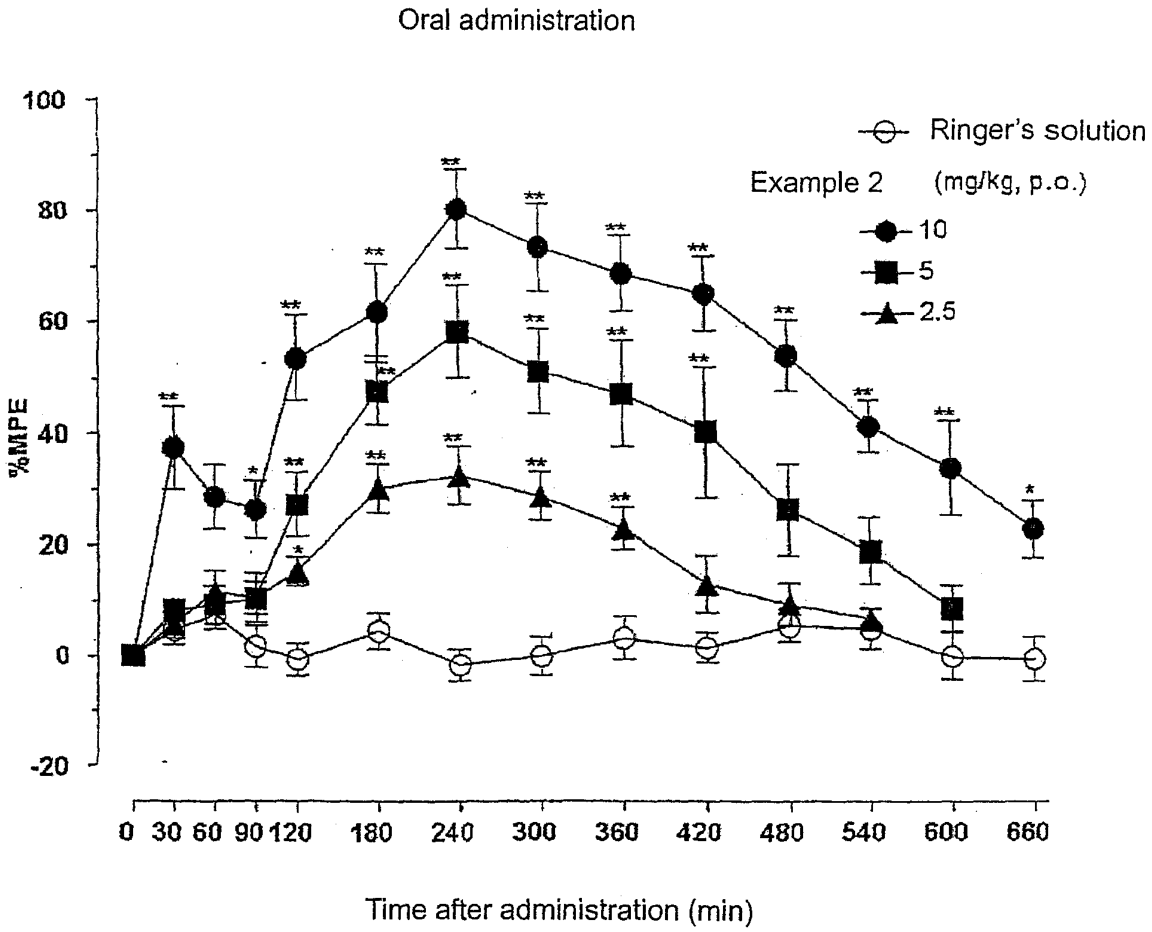
Oral administration
100
80
60
40
20
0
-20
%MPE
Ringer's solution
Example 2 (mg/kg, p.o.)
10
5
2.5
0 30 60 90 120 180 240 300 360 420 480 540 600 660
Time after administration (min)

Fig. 6

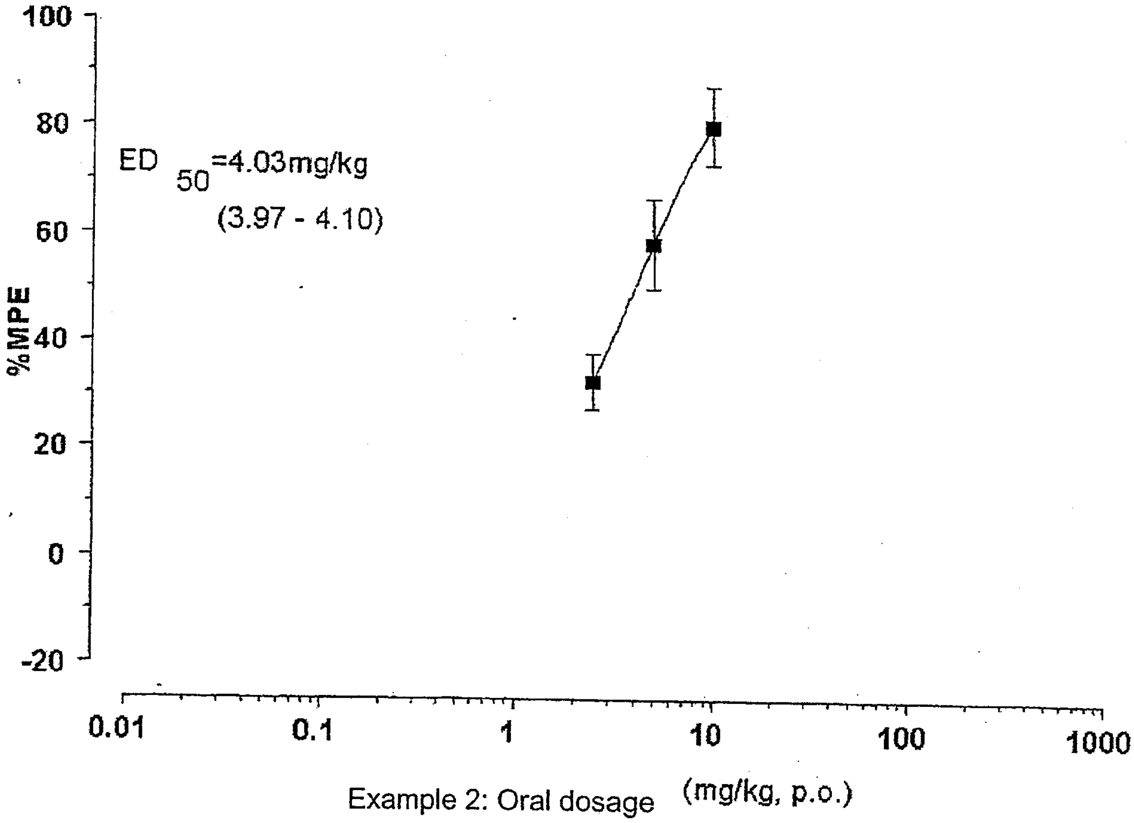
100
80
60
40
20
0
-20
%MPE
ED 50=4.03mg/kg
(3.97 - 4.10)
0.01
0.1
1
10
100
1000
Example 2: Oral dosage (mg/kg, p.o.)

Fig. 7

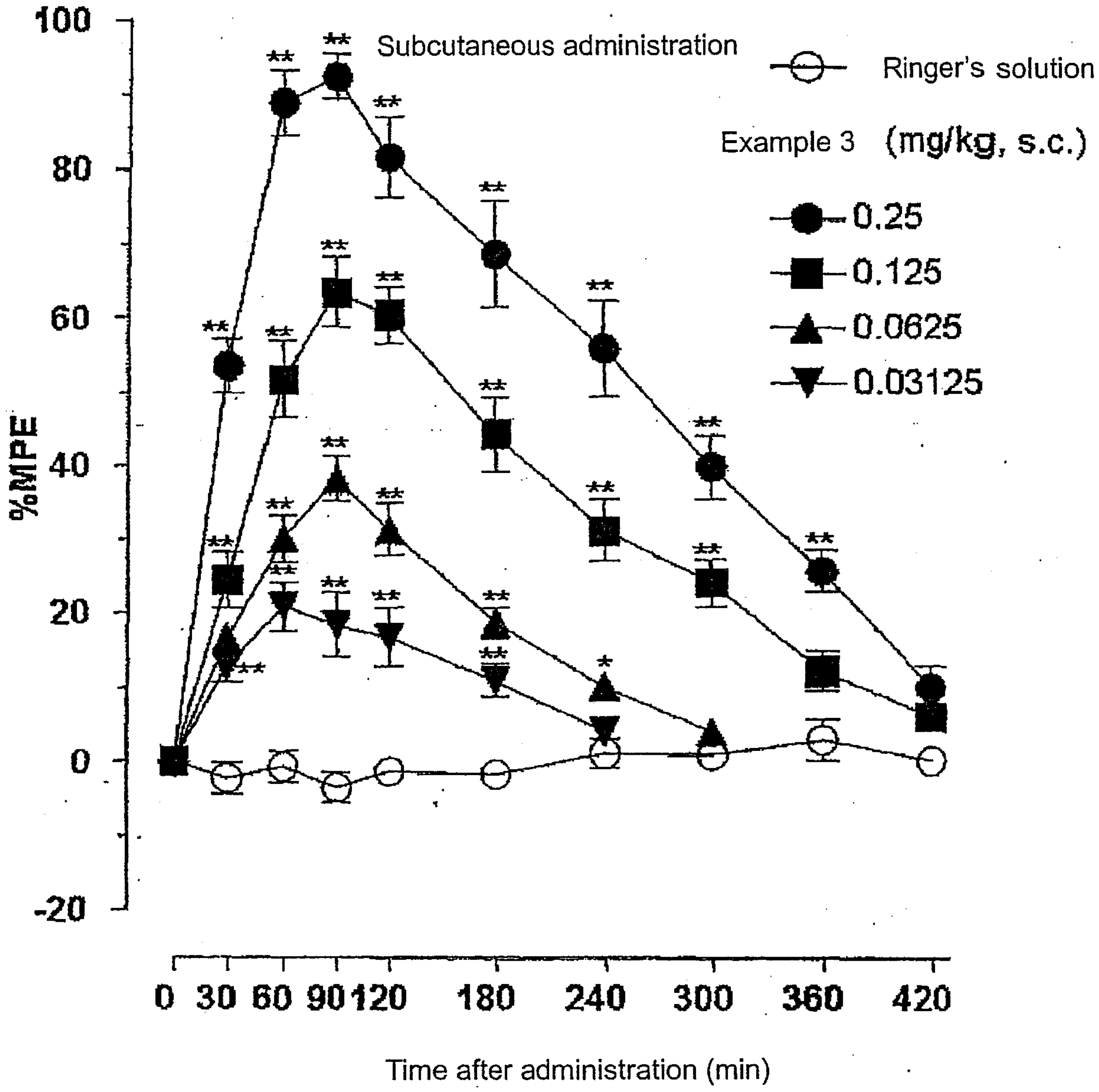
Subcutaneous administration
Ringer's solution
Example 3 (mg/kg, s.c.)
0.25
0.125
0.0625
0.03125
%MPE
100
80
60
40
20
0
-20
0 30 60 90 120 180 240 300 360 420
Time after administration (min)

Fig. 8

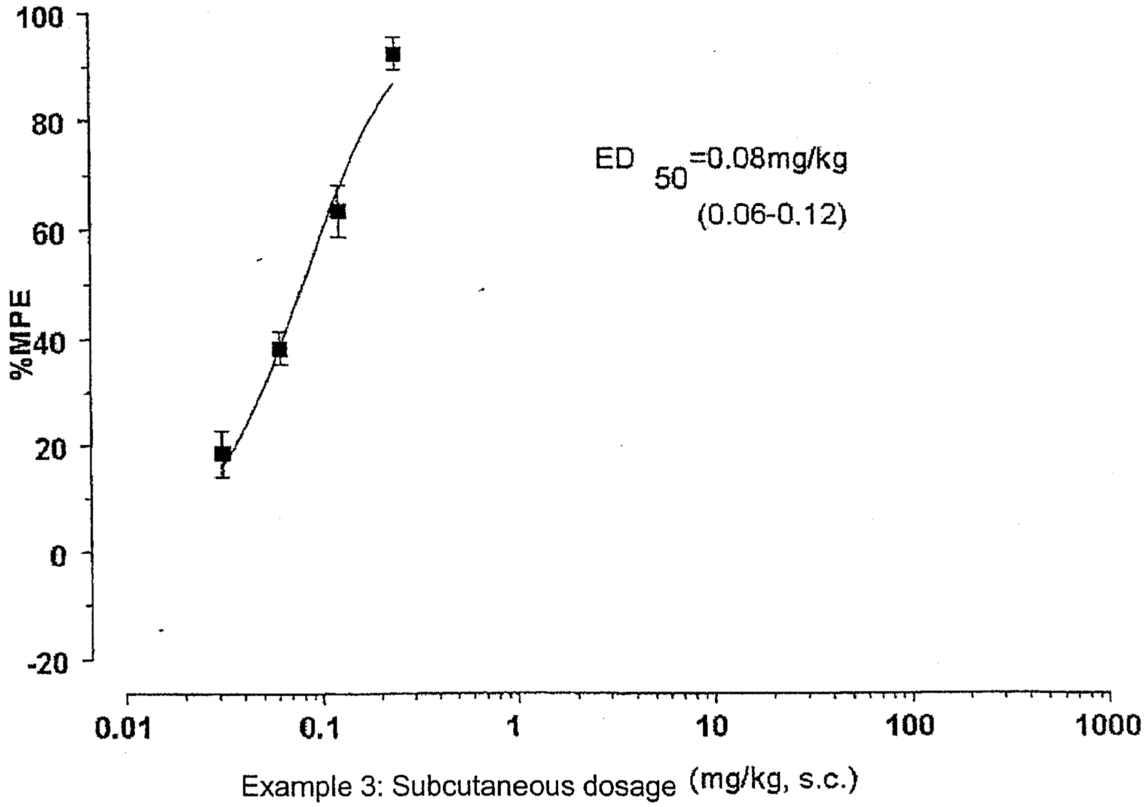
100
80
60
40
20
0
-20
%MPE
ED 50=0.08mg/kg
(0.06-0.12)
0.01
0.1
1
10
100
1000
Example 3: Subcutaneous dosage (mg/kg, s.c.)

Fig. 9

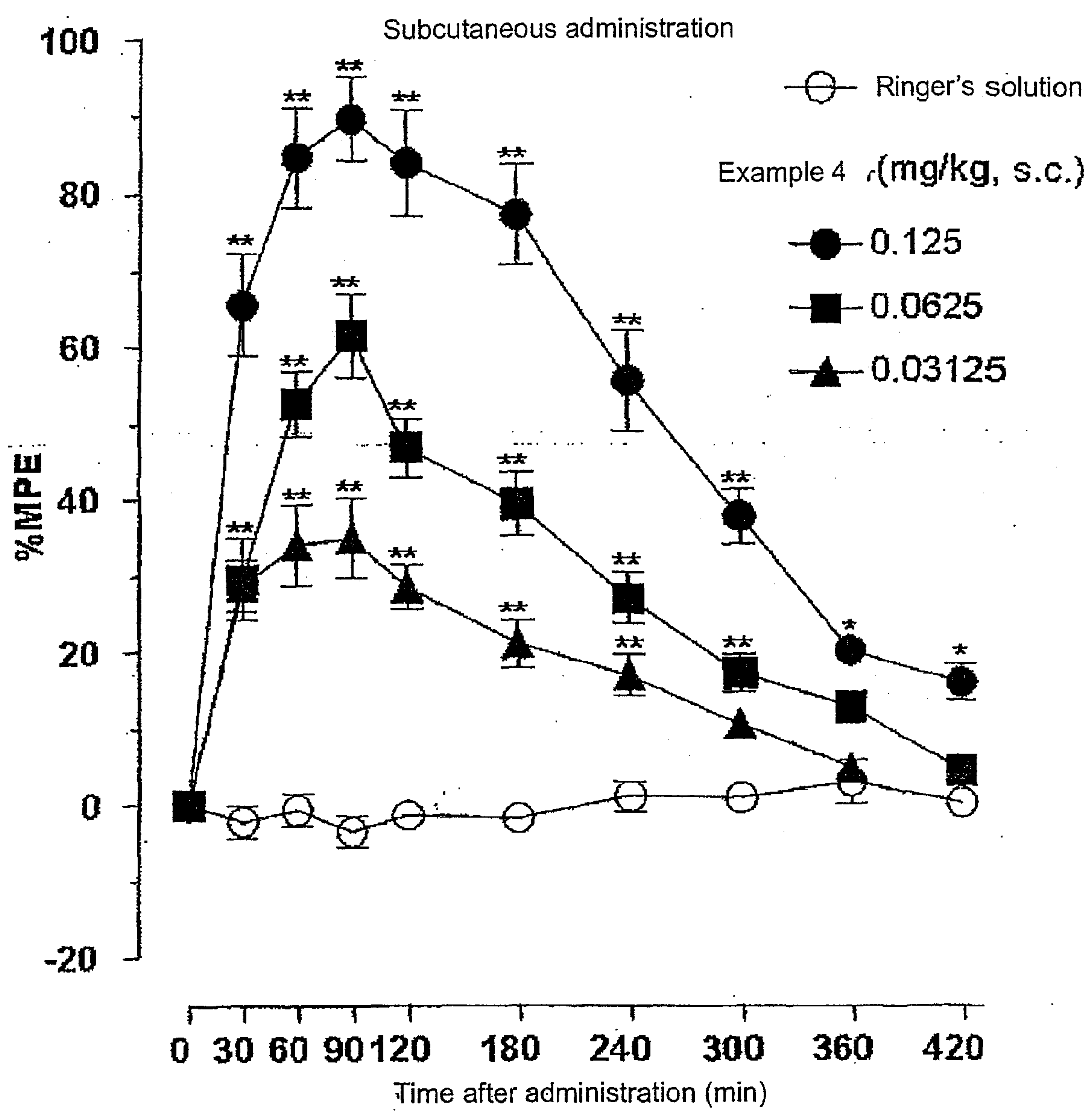
Subcutaneous administration
Ringer's solution
Example 4 (mg/kg, s.c.)
0.125
0.0625
0.03125
%MPE
100
80
60
40
20
0
-20
0 30 60 90 120 180 240 300 360 420
Time after administration (min)

Fig. 10

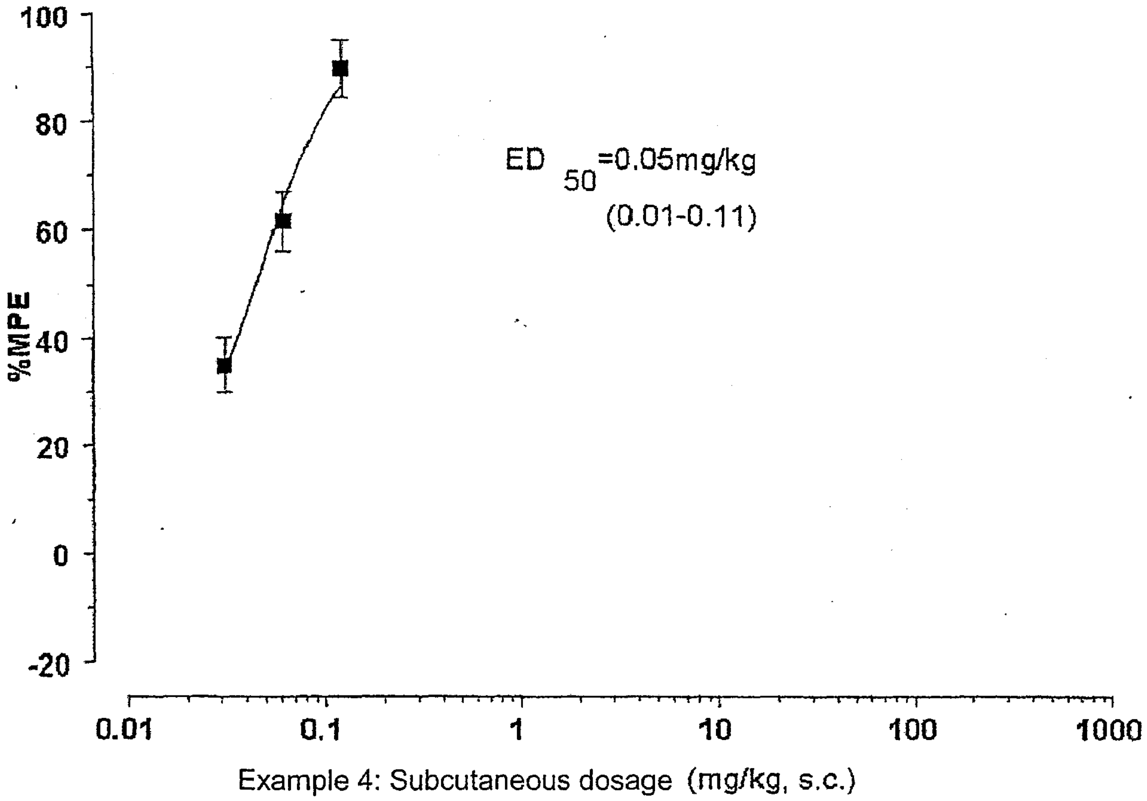
100
80
60
40
20
0
-20
%MPE
ED 50=0.05mg/kg
(0.01-0.11)
0.01
0.1
1
10
100
1000
Example 4: Subcutaneous dosage (mg/kg, s.c.)

Fig. 11

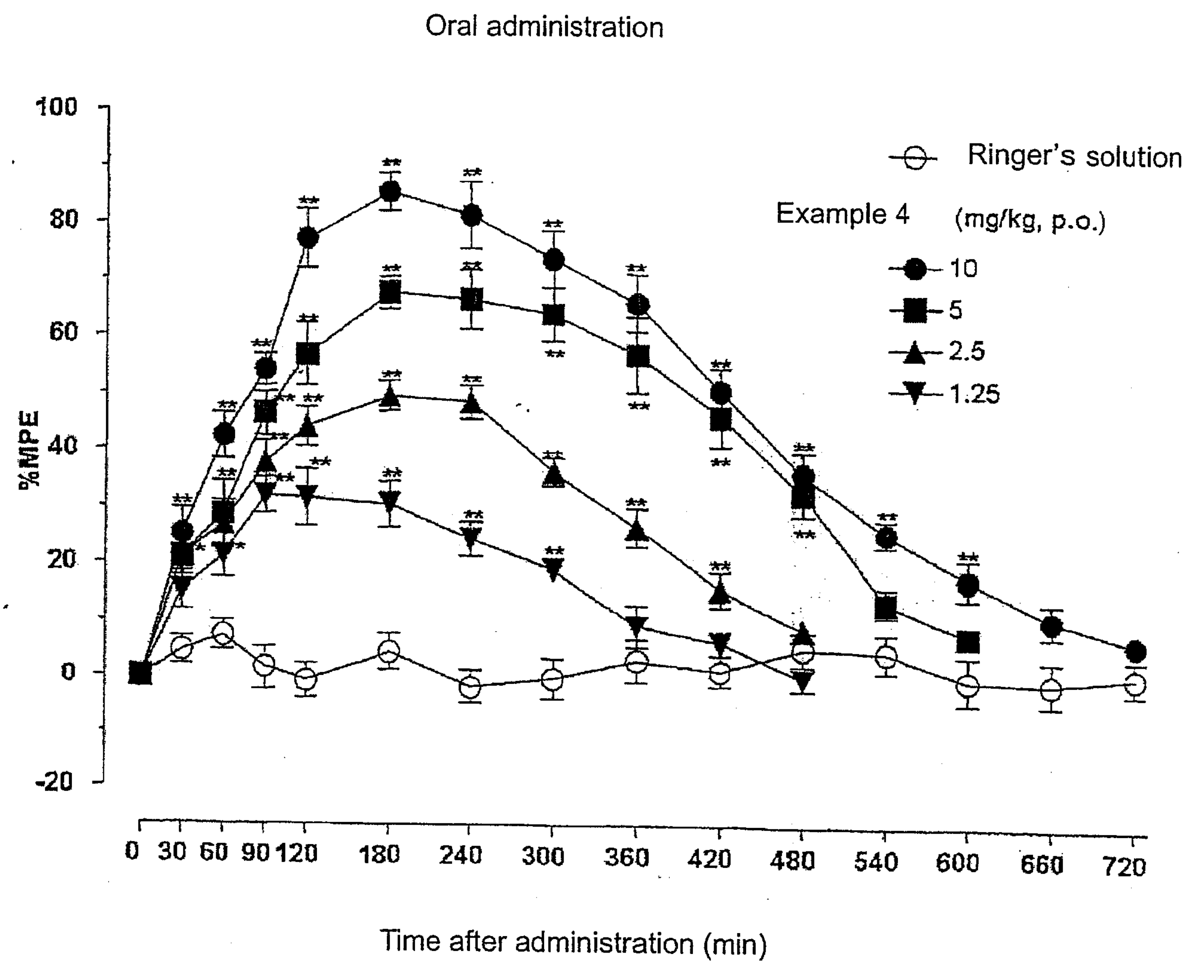
Oral administration
Ringer's solution
Example 4 (mg/kg, p.o.)
10
5
2.5
1.25
%MPE
100
80
60
40
20
0
-20
0 30 60 90 120 180 240 300 360 420 480 540 600 660 720
Time after administration (min)

Fig. 12

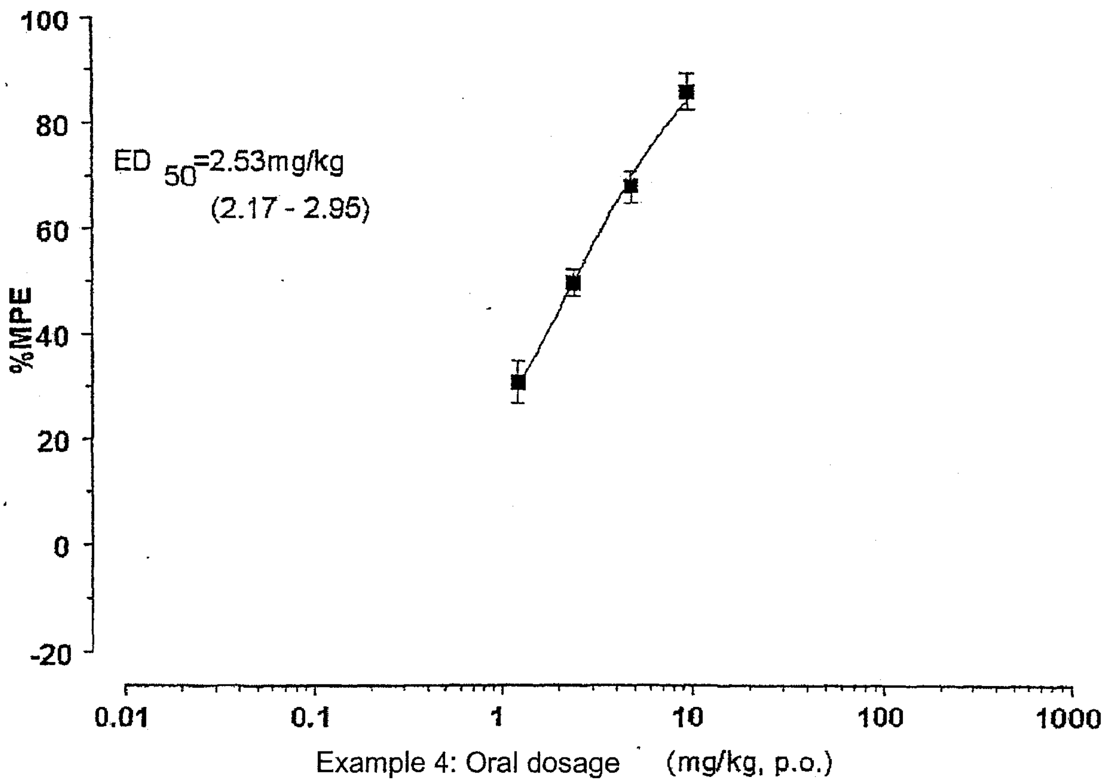
100
80
60
40
20
0
-20
%MPE
ED 50=2.53mg/kg
(2.17 - 2.95)
0.01
0.1
1
10
100
1000
Example 4: Oral dosage (mg/kg, p.o.)

Fig. 13

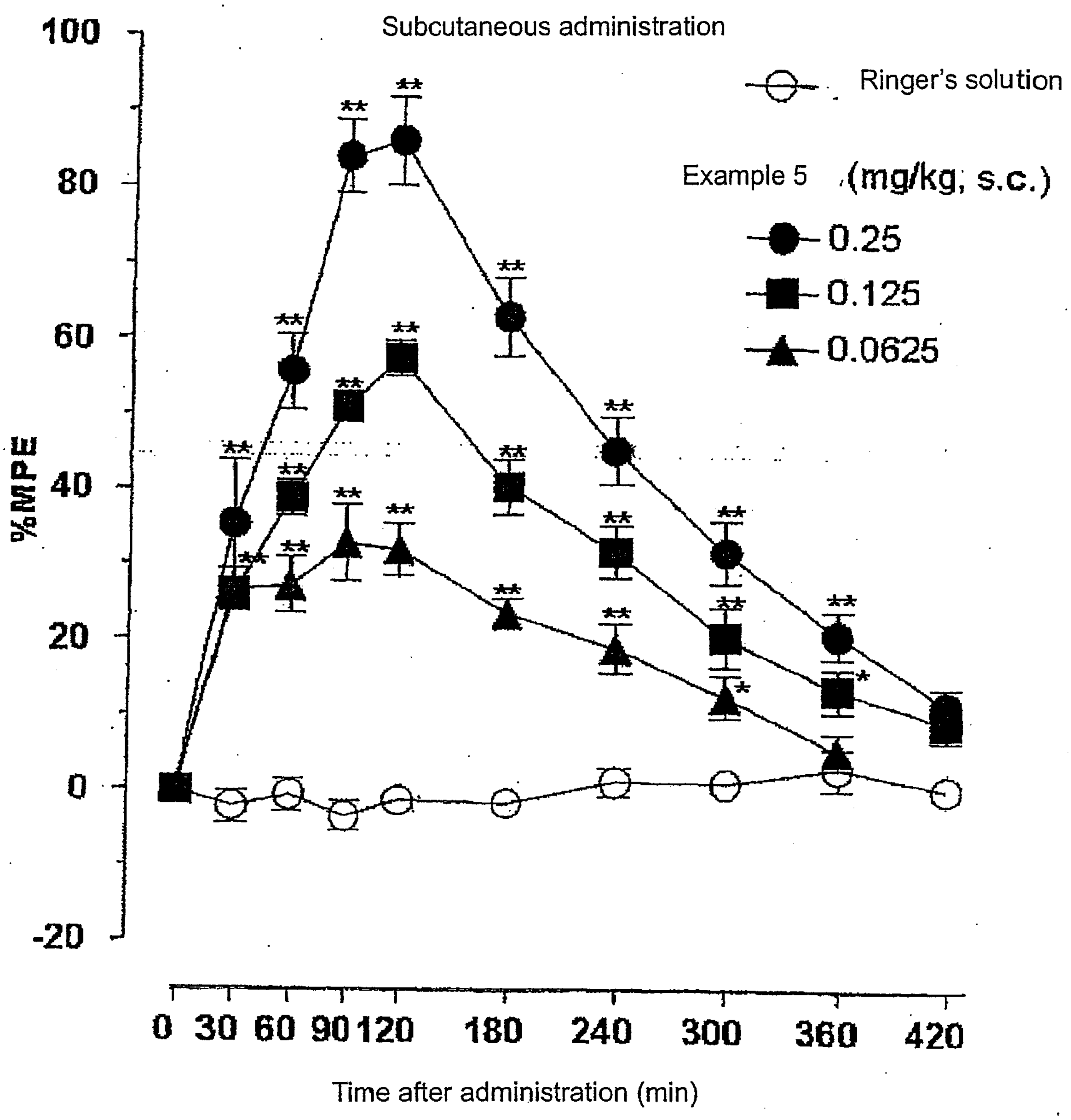
Subcutaneous administration
Ringer's solution
Example 5 (mg/kg; s.c.)
0.25
0.125
0.0625
%MPE
100
80
60
40
20
0
-20
0 30 60 90 120 180 240 300 360 420
Time after administration (min)

Fig. 14

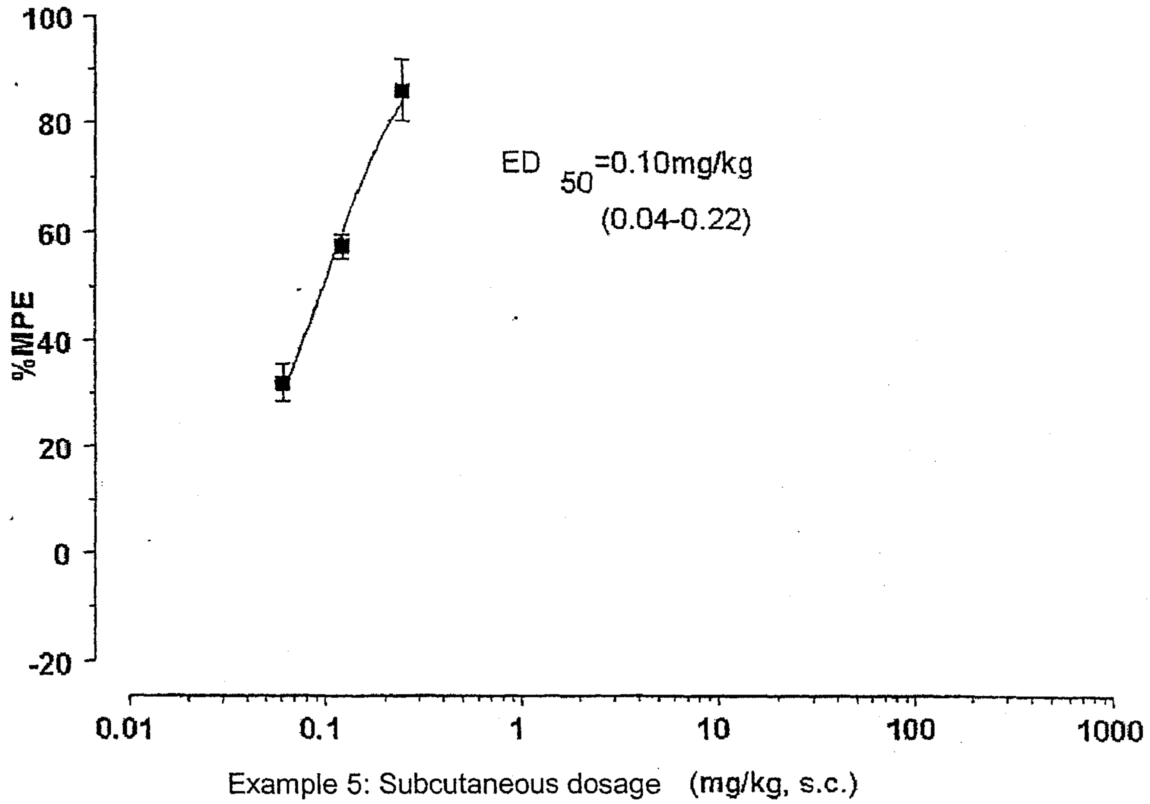
100
80
60
40
20
0
-20
%MPE
ED 50=0.10mg/kg
(0.04-0.22)
0.01
0.1
1
10
100
1000
Example 5: Subcutaneous dosage (mg/kg, s.c.)

Fig. 15

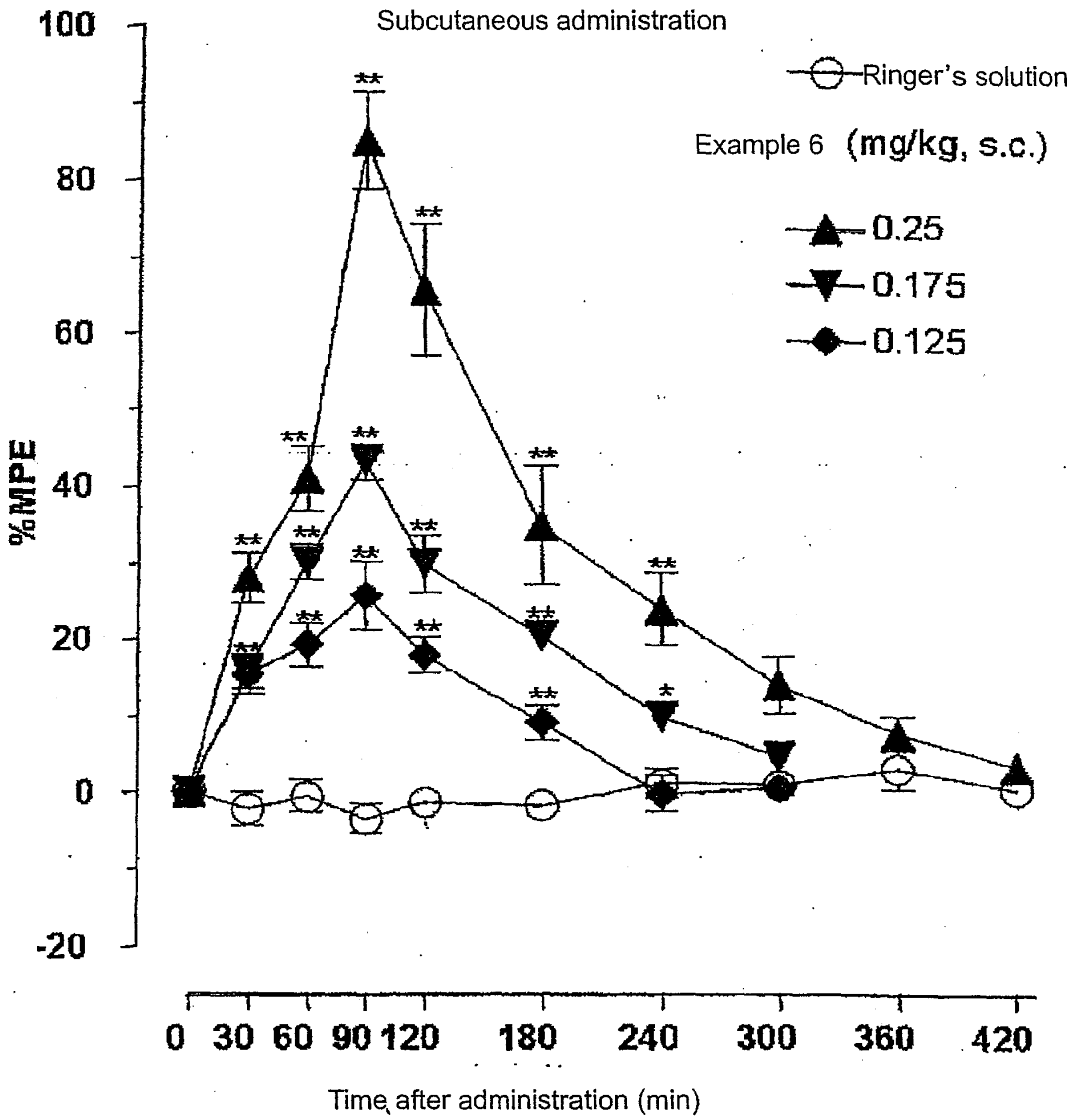
Subcutaneous administration
Ringer's solution
Example 6 (mg/kg, s.c.)
0.25
0.175
0.125
100
80
60
40
20
0
-20
%MPE
0 30 60 90 120 180 240 300 360 420
Time after administration (min)

Fig. 16

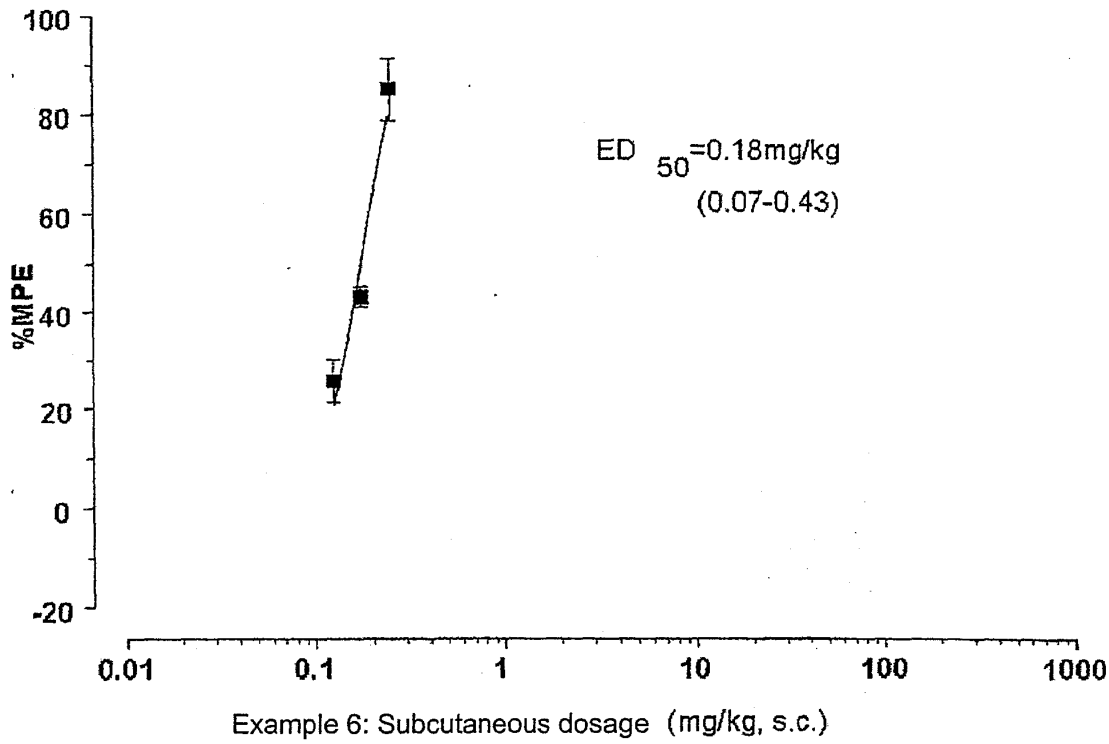
100
80
60
40
20
0
-20
%MPE
ED 50=0.18mg/kg
(0.07-0.43)
0.01
0.1
1
10
100
1000
Example 6: Subcutaneous dosage (mg/kg, s.c.)

Fig. 17

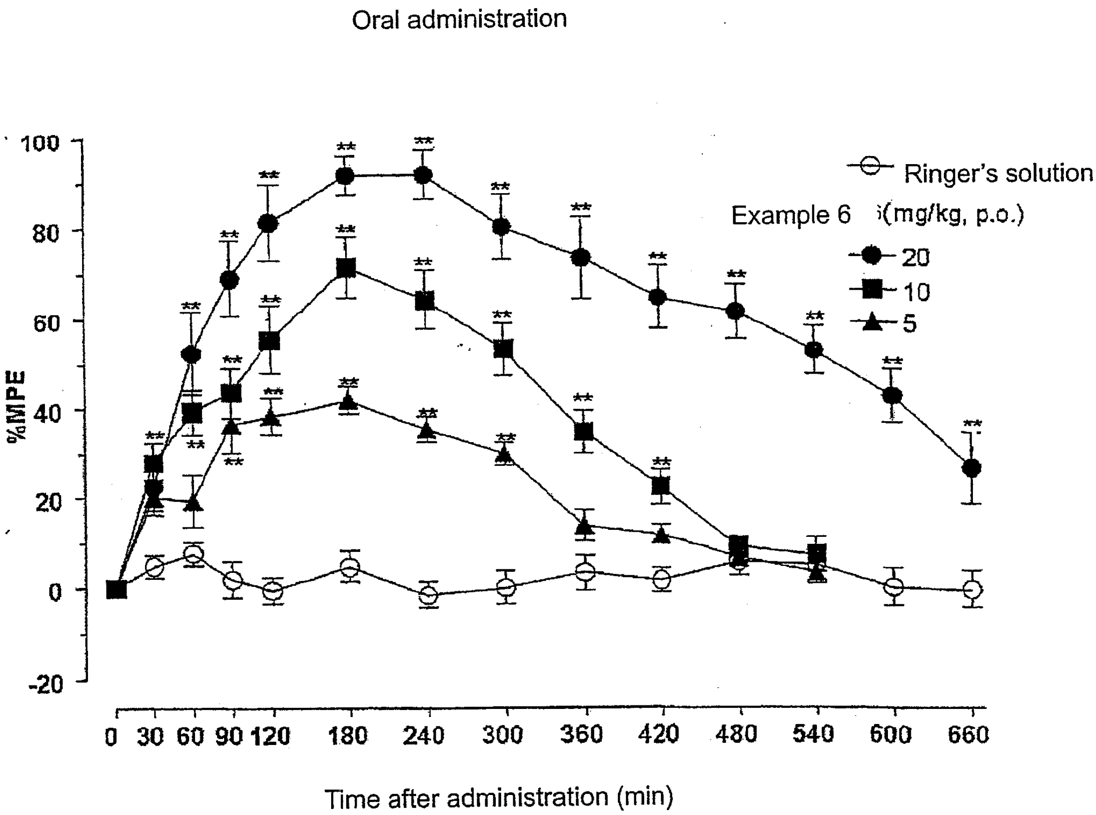
Oral administration
%MPE
100
80
60
40
20
0
-20
Ringer's solution
Example 6 (mg/kg, p.o.)
20
10
5
**
0 30 60 90 120 180 240 300 360 420 480 540 600 660
Time after administration (min)

Fig. 18

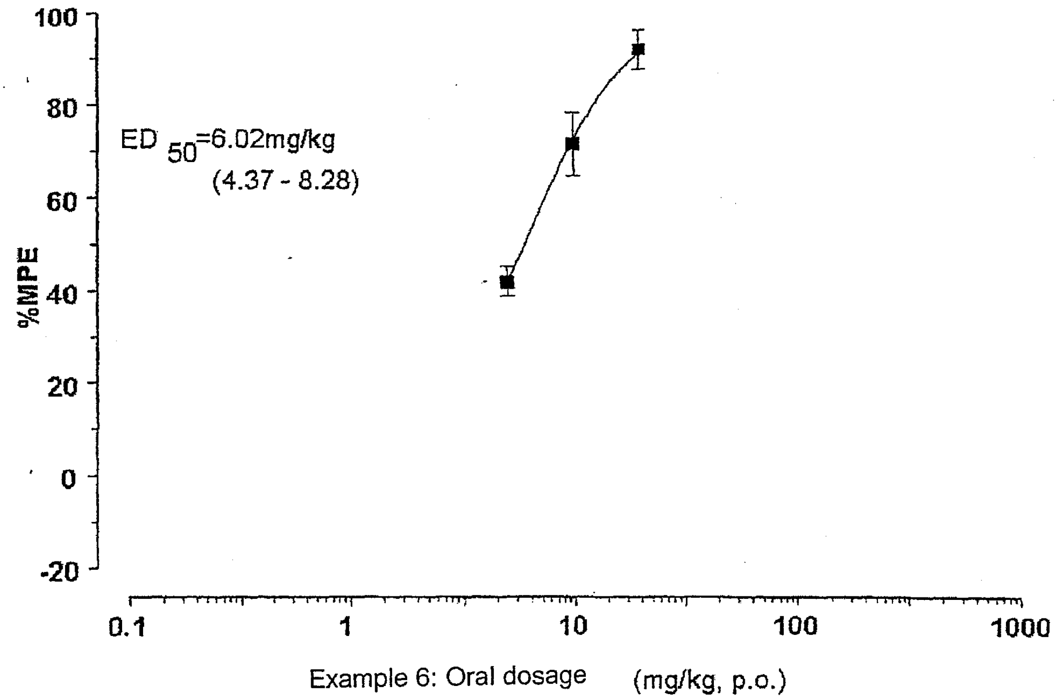
100
80
60
40
20
0
-20
%MPE
ED 50=6.02mg/kg
(4.37 - 8.28)
0.1
1
10
100
1000
Example 6: Oral dosage (mg/kg, p.o.)

Fig. 19

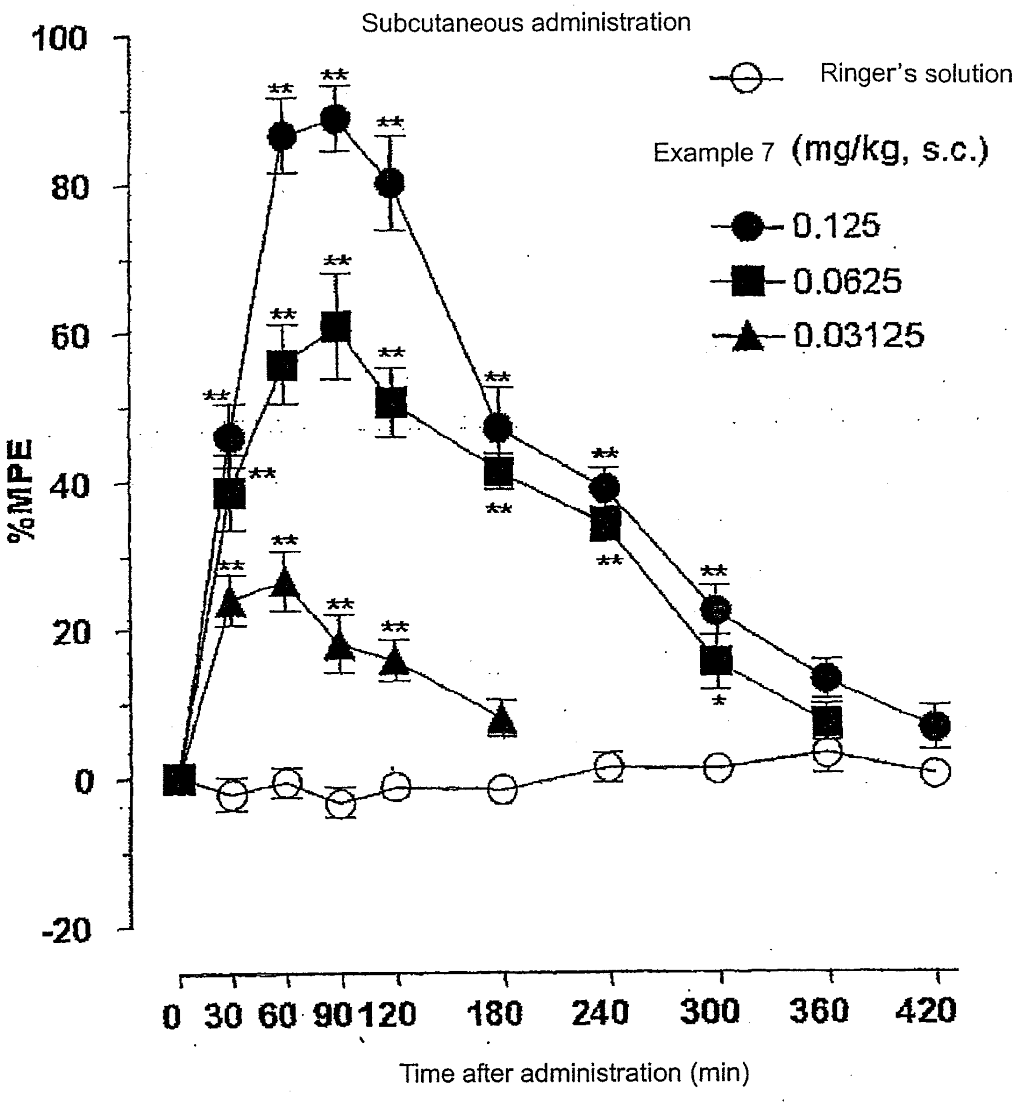
Subcutaneous administration
Ringer's solution
Example 7 (mg/kg, s.c.)
0.125
0.0625
0.03125
%MPE
100
80
60
40
20
0
-20
0 30 60 90 120 180 240 300 360 420
Time after administration (min)

Fig. 20

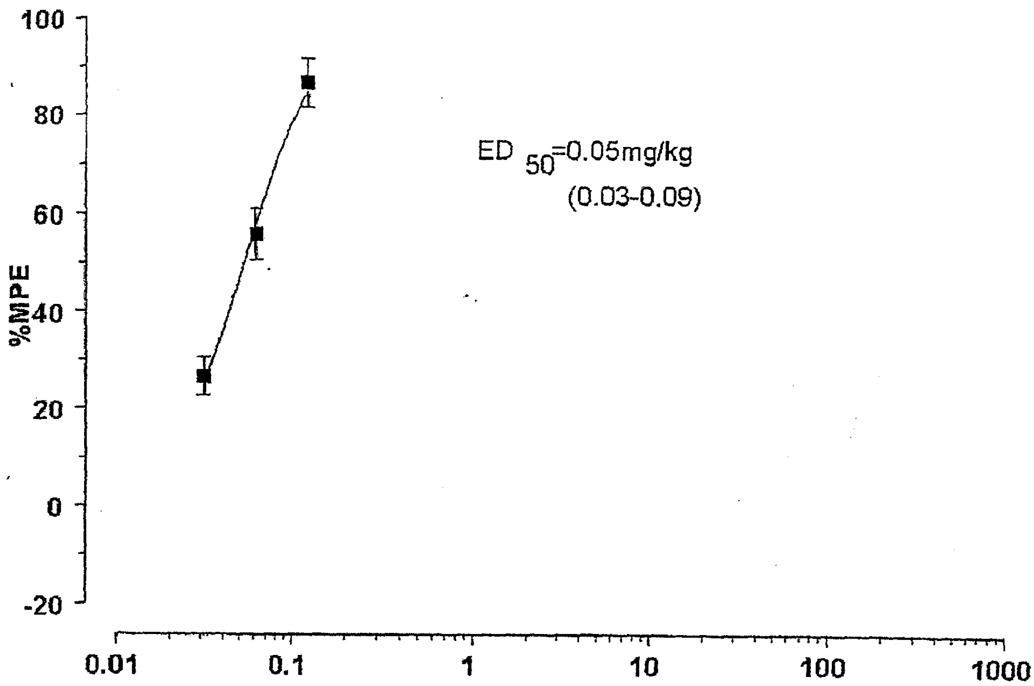


Example 7: Subcutaneous dosage

Fig. 21

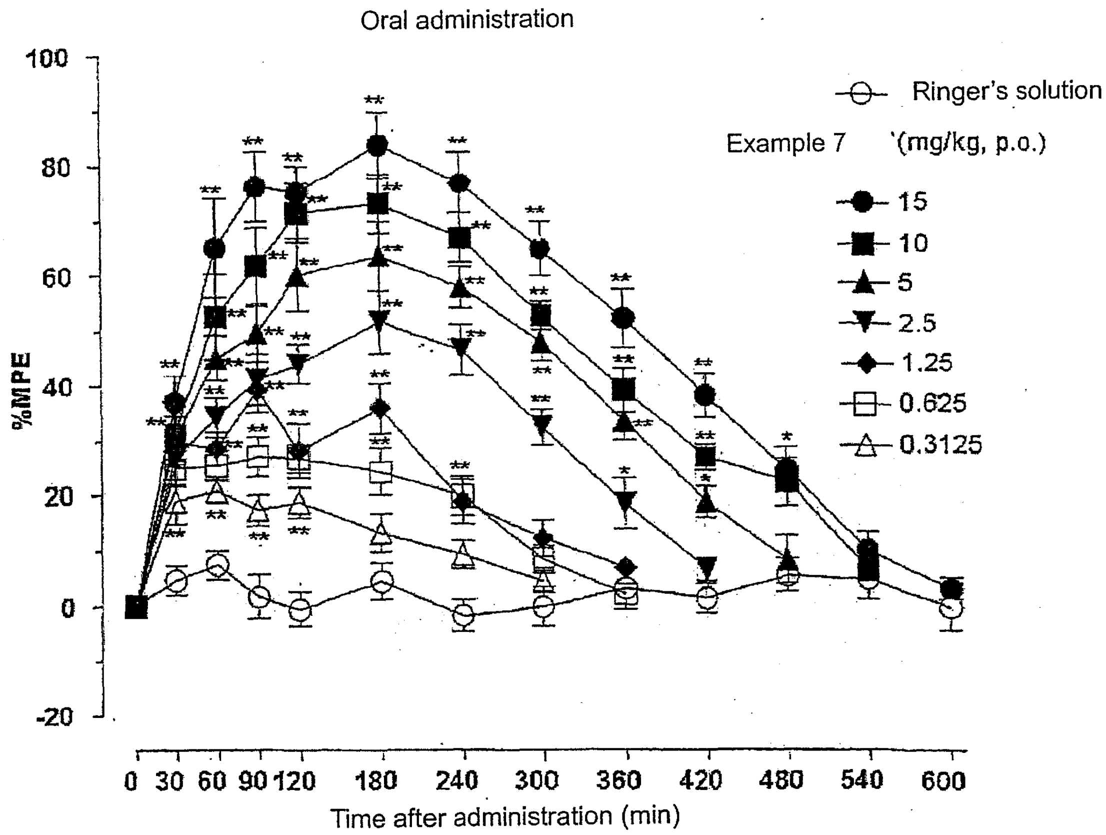
Oral administration
%MPE
100
80
60
40
20
0
-20
0 30 60 90 120 180 240 300 360 420 480 540 600
Time after administration (min)
Ringer's solution
Example 7 (mg/kg, p.o.)
15
10
5
2.5
1.25
0.625
0.3125

Fig. 22

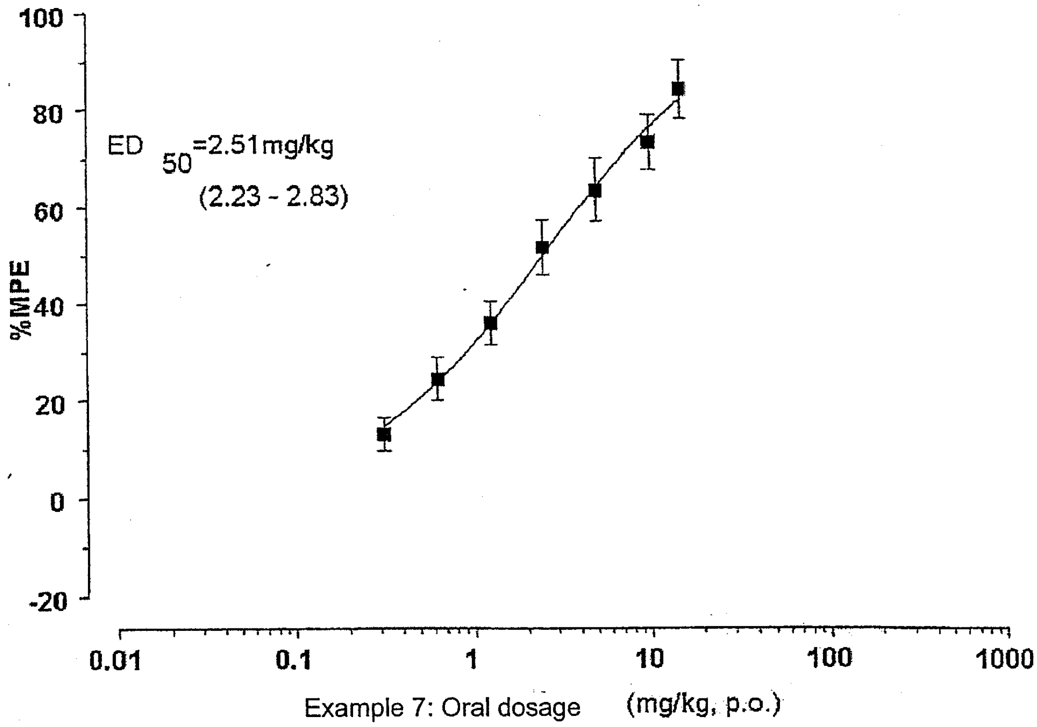
100
80
60
40
20
0
-20
%MPE
ED 50=2.51mg/kg
(2.23 - 2.83)
0.01
0.1
1
10
100
1000
Example 7: Oral dosage
(mg/kg, p.o.)

Fig. 23

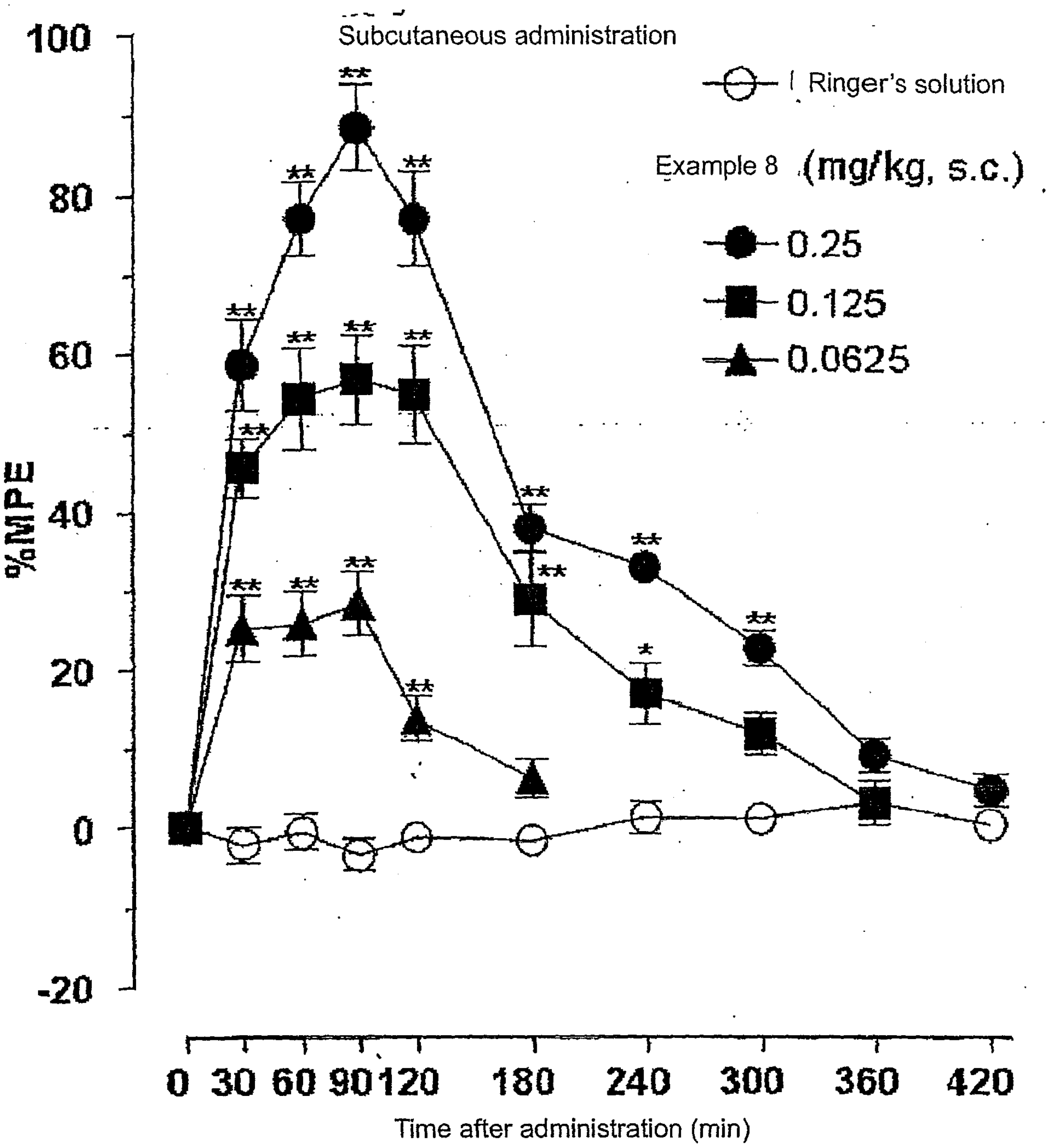
Subcutaneous administration
Ringer's solution
Example 8 (mg/kg, s.c.)
0.25
0.125
0.0625
%MPE
100
80
60
40
20
0
-20
0 30 60 90 120 180 240 300 360 420
Time after administration (min)

Fig. 24

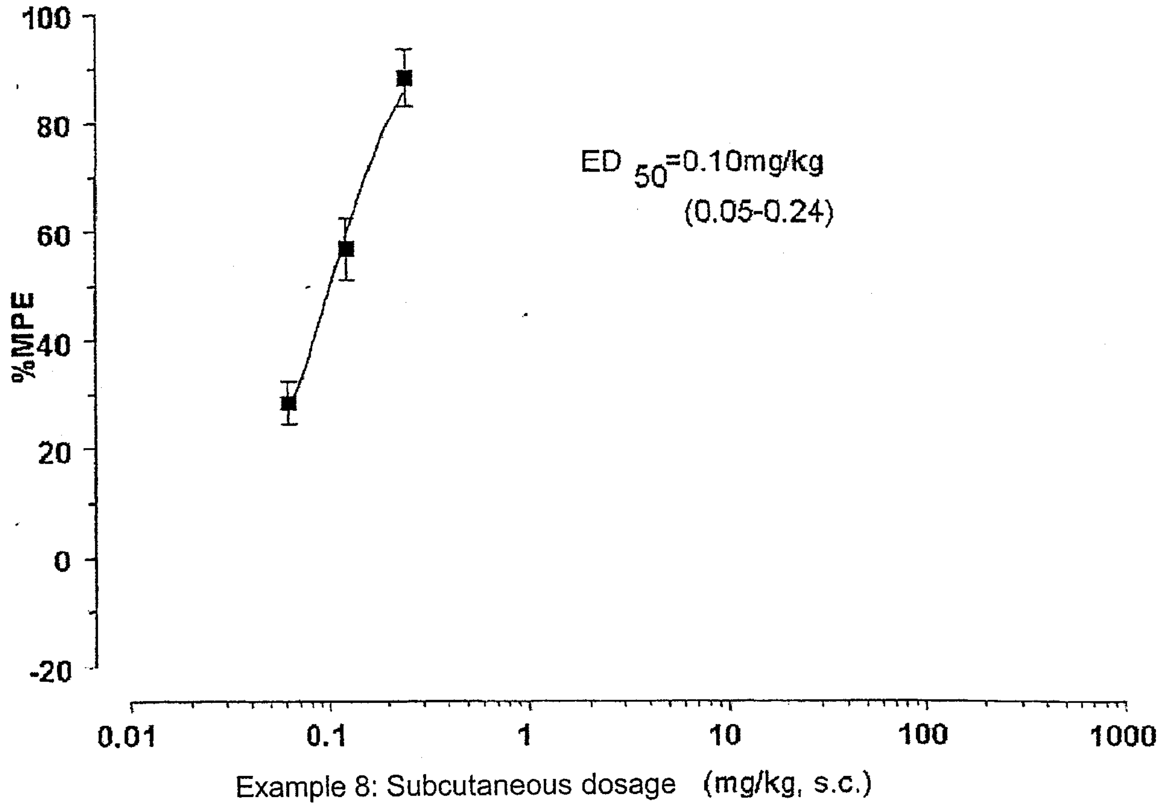
100
80
60
40
20
0
-20
%MPE
ED 50=0.10mg/kg
(0.05-0.24)
0.01
0.1
1
10
100
1000
Example 8: Subcutaneous dosage (mg/kg, s.c.)

Fig. 25

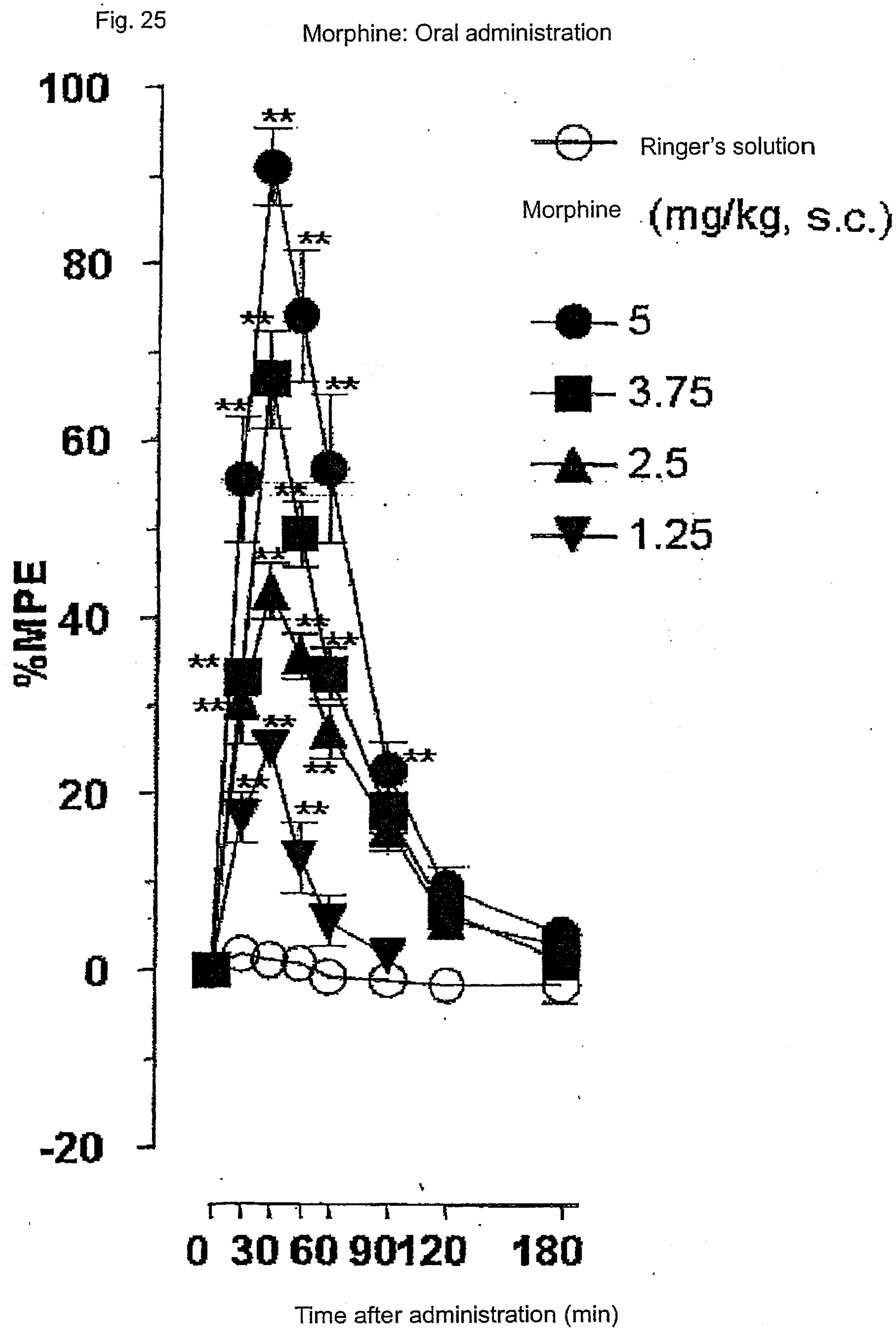
Morphine: Oral administration
100
80
60
40
20
0
-20
%MPE
Ringer's solution
Morphine (mg/kg, s.c.)
5
3.75
2.5
1.25
0 30 60 90 120 180
Time after administration (min)

Fig. 26

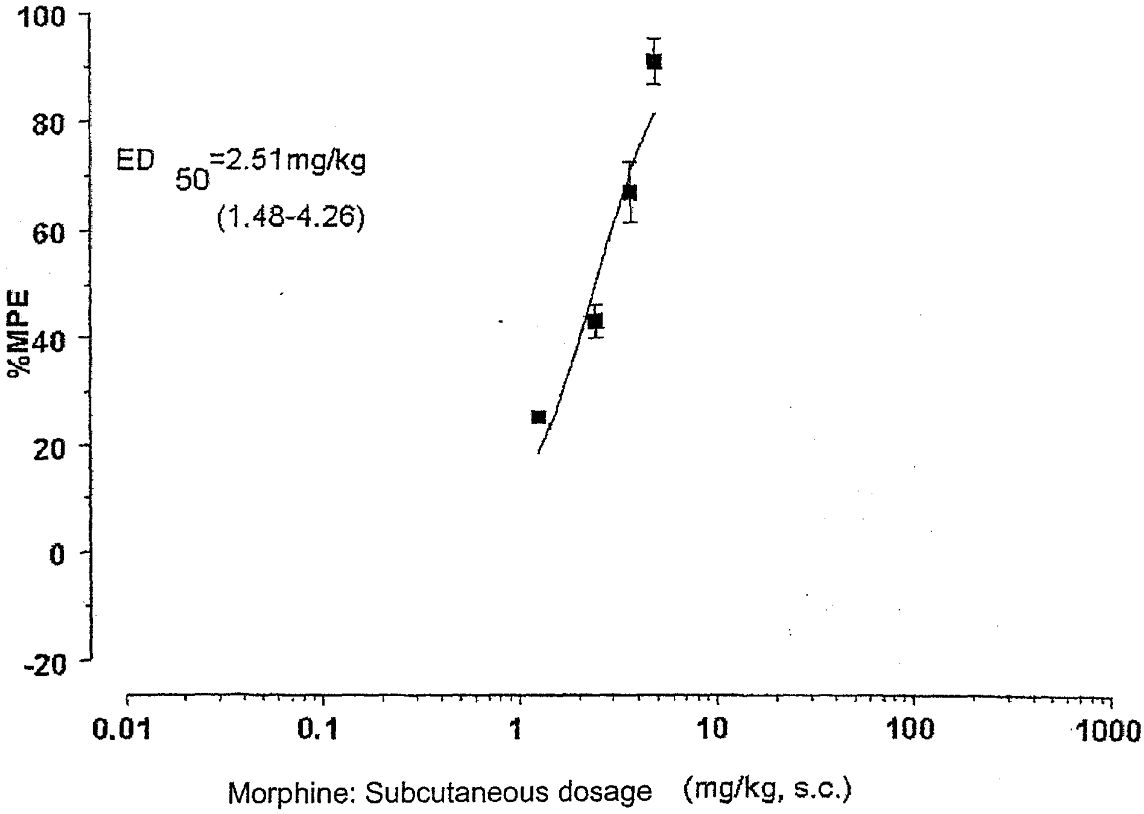
100
80
60
40
20
0
-20
%MPE
ED 50=2.51mg/kg
(1.48-4.26)
0.01
0.1
1
10
100
1000
Morphine: Subcutaneous dosage (mg/kg, s.c.)

Fig. 27

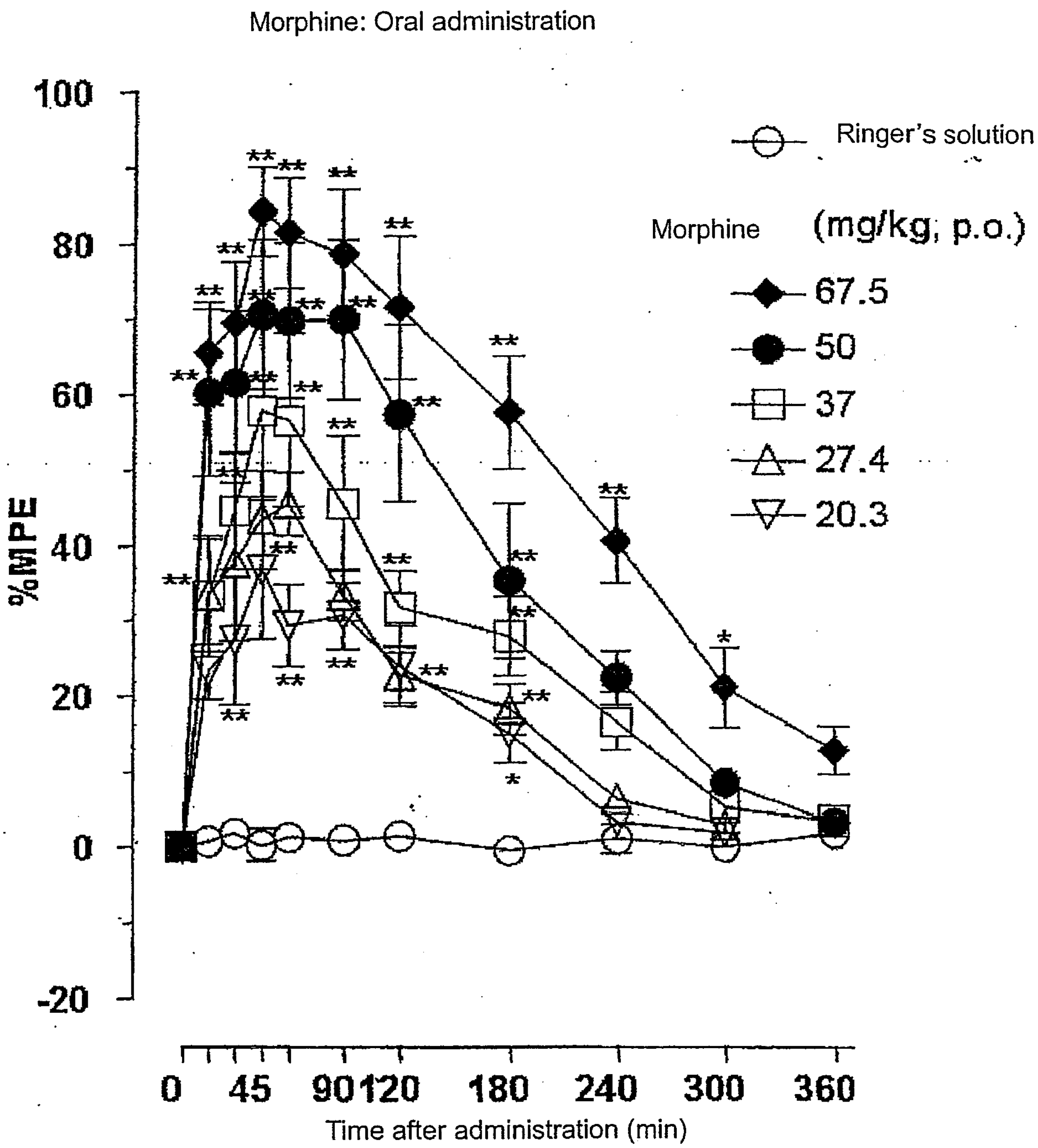
Morphine: Oral administration
%MPE
100
80
60
40
20
0
-20
0
45
90
120
180
240
300
360
Time after administration (min)
Ringer's solution
Morphine (mg/kg, p.o.)
67.5
50
37
27.4
20.3

Fig. 28

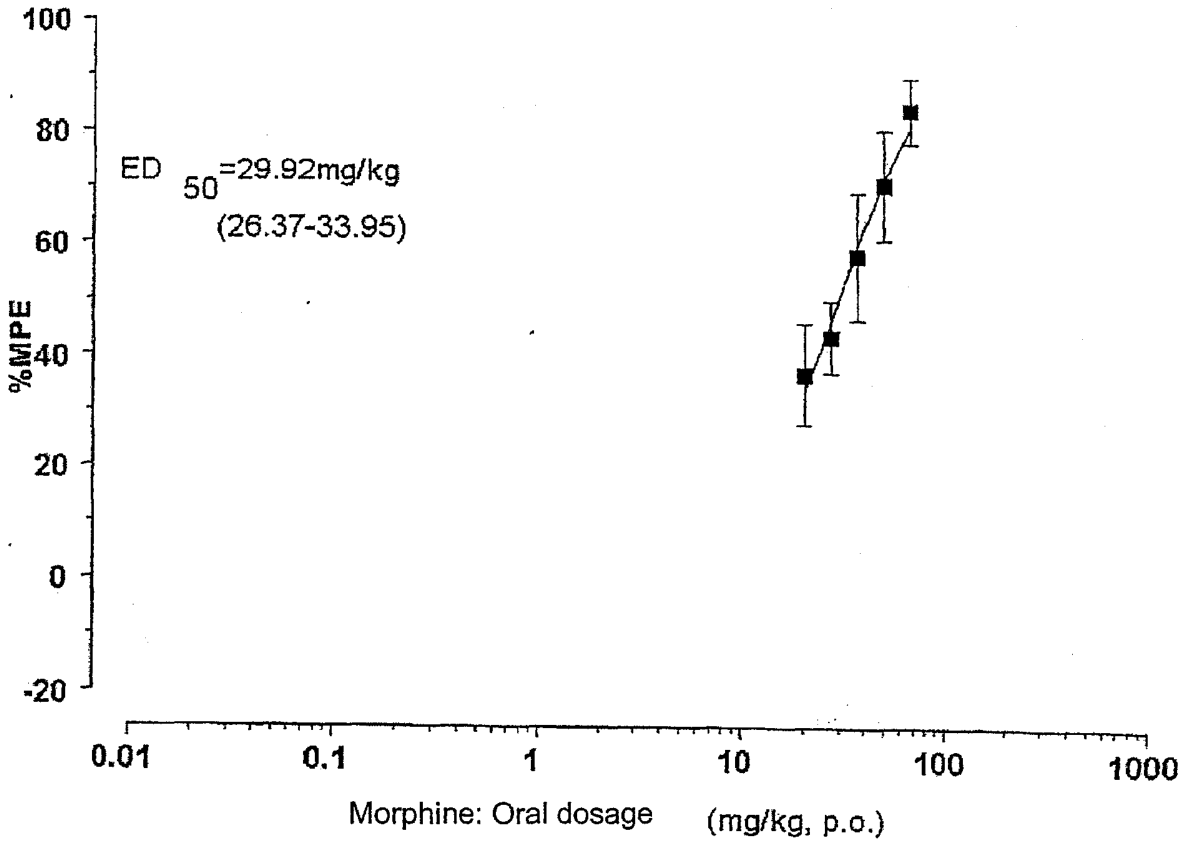
100
80
60
40
20
0
-20
%MPE
ED 50=29.92mg/kg
(26.37-33.95)
0.01
0.1
1
10
100
1000
Morphine: Oral dosage (mg/kg, p.o.)

Fig. 29

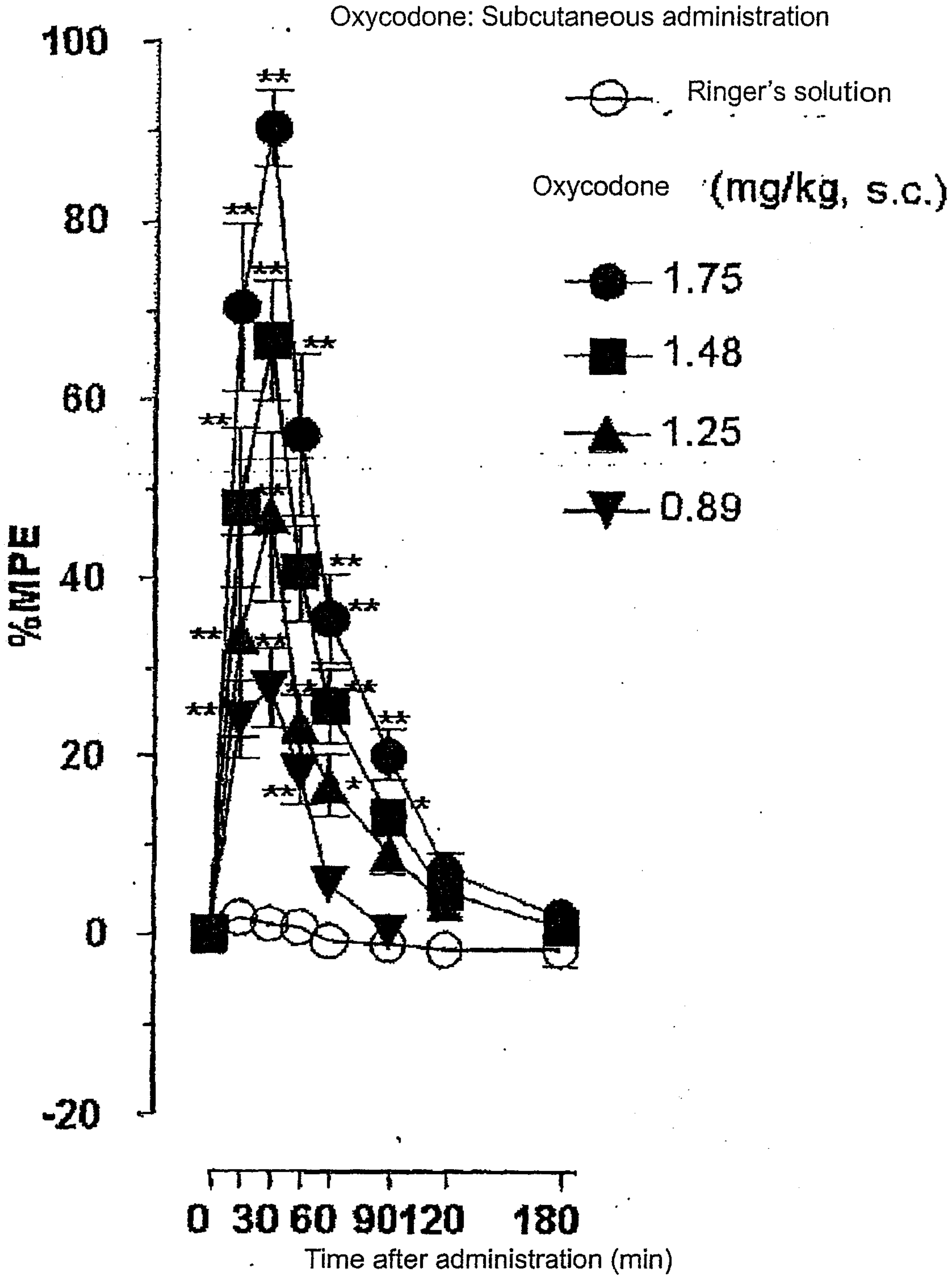
Oxycodone: Subcutaneous administration
Ringer's solution
Oxycodone (mg/kg, s.c.)
1.75
1.48
1.25
0.89
%MPE
100
80
60
40
20
0
-20
0 30 60 90120 180
Time after administration (min)

Fig. 30

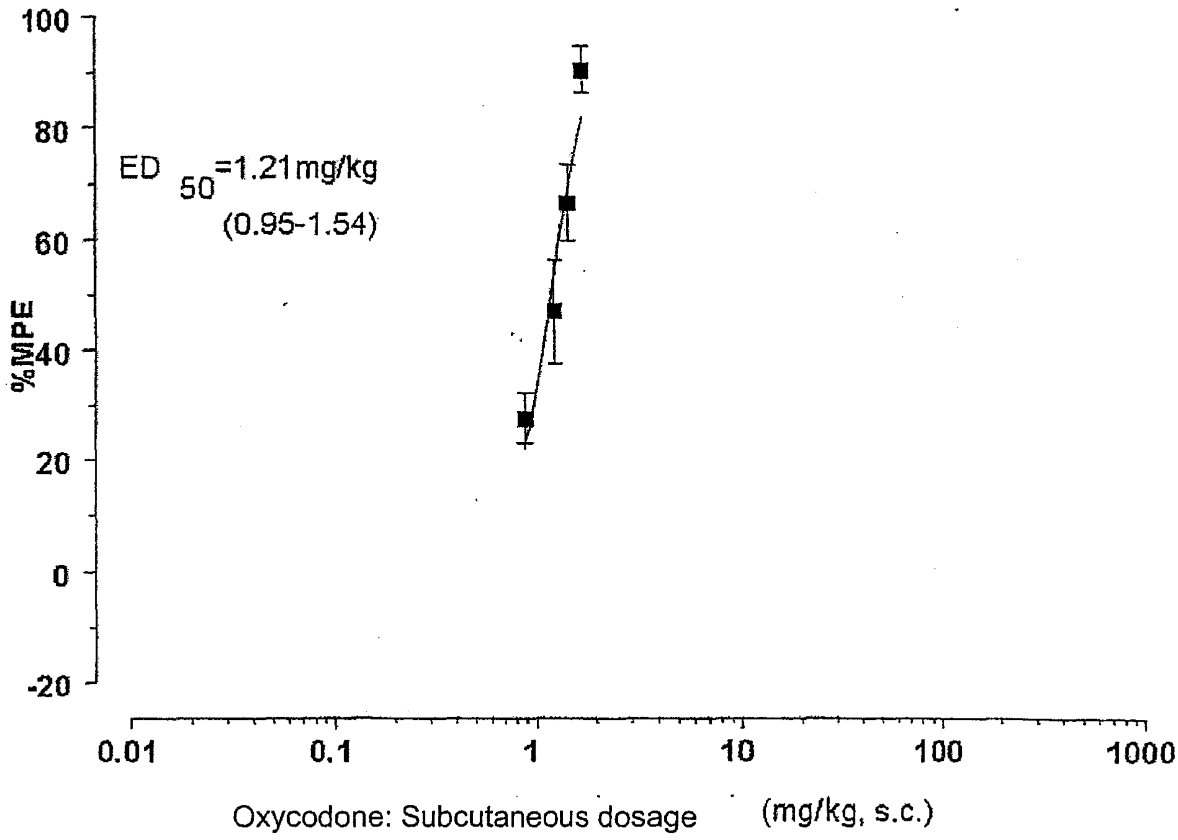
100
80
60
40
20
0
-20
%MPE
ED 50=1.21mg/kg
(0.95-1.54)
0.01
0.1
1
10
100
1000
Oxycodone: Subcutaneous dosage (mg/kg, s.c.)

Fig. 31

Oxycodone: Oral administration

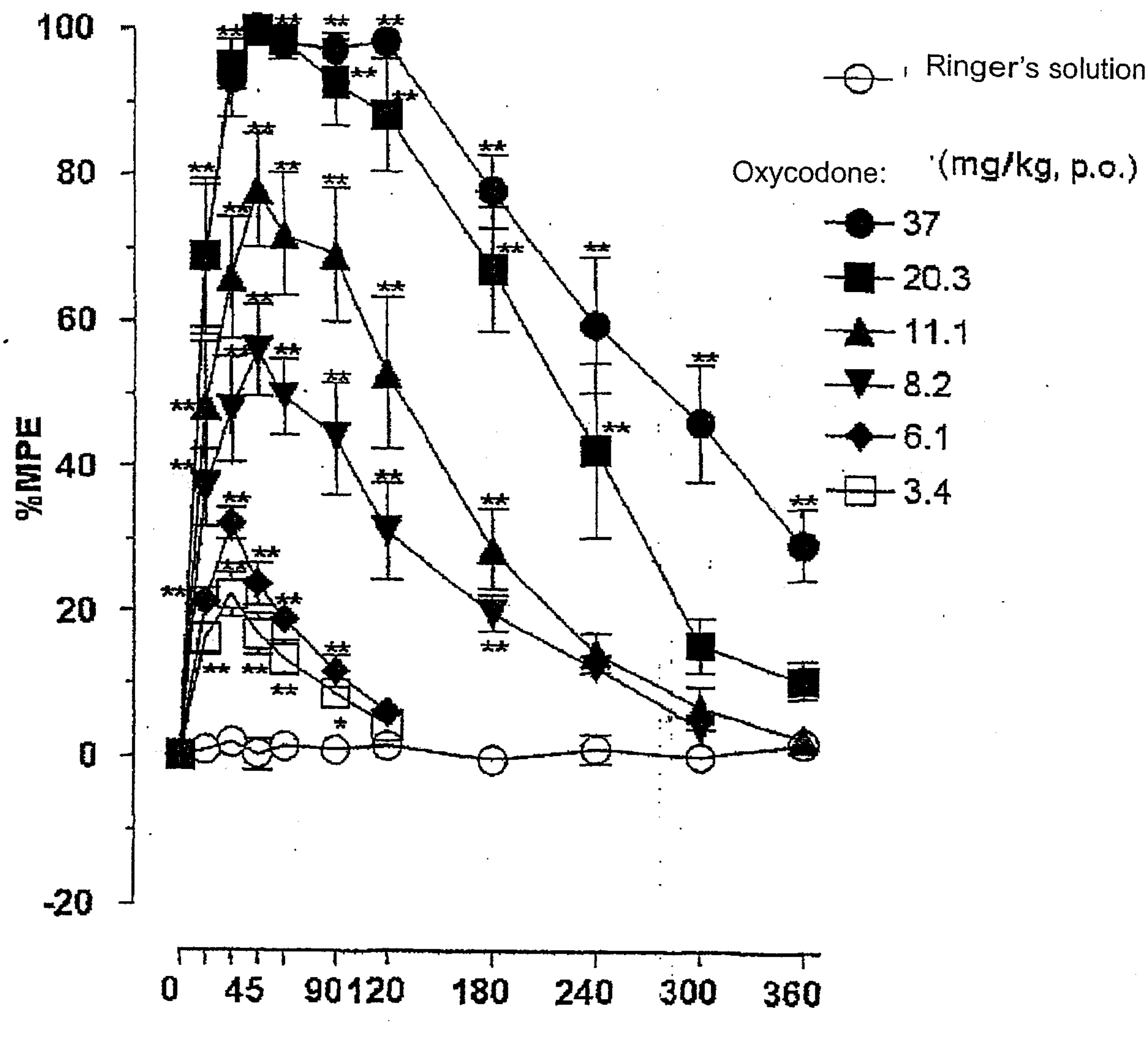

Fig. 32

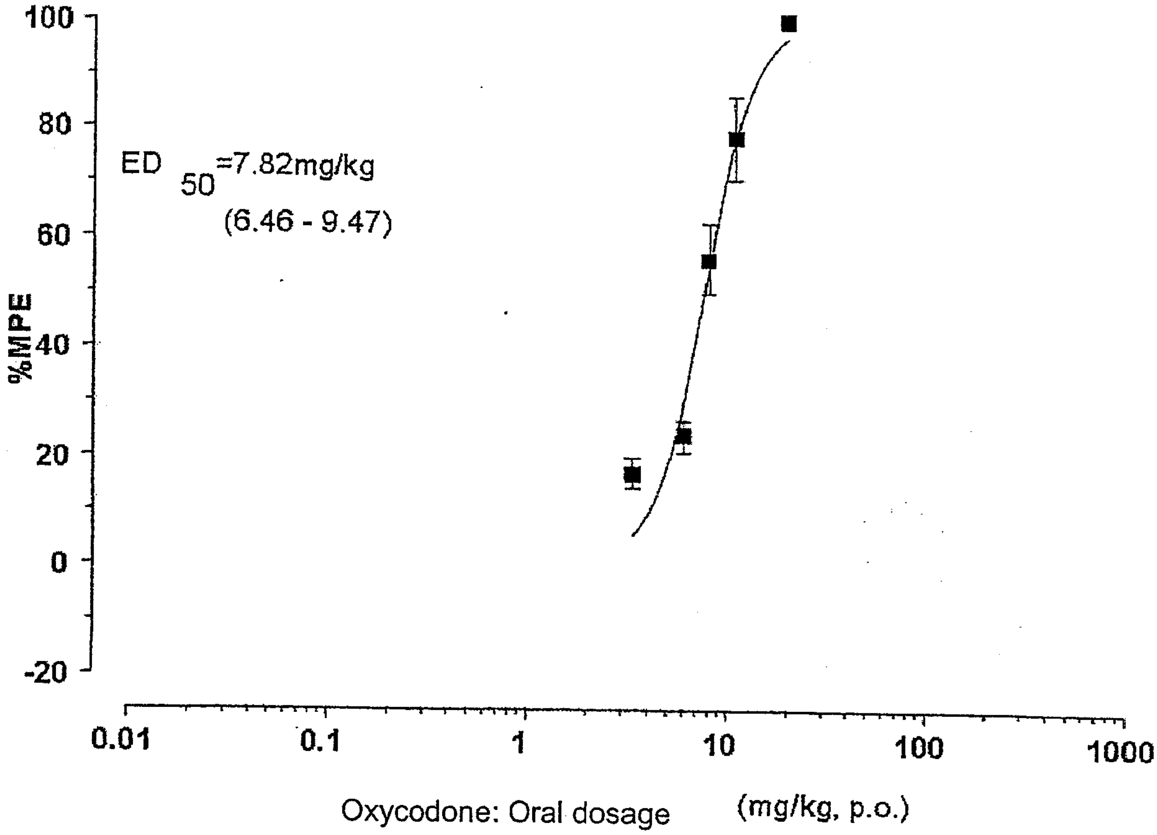
100
80
60
40
20
0
-20
%MPE
ED 50=7.82mg/kg
(6.46 - 9.47)
0.01
0.1
1
10
100
1000
Oxycodone: Oral dosage (mg/kg, p.o.)

Fig. 33

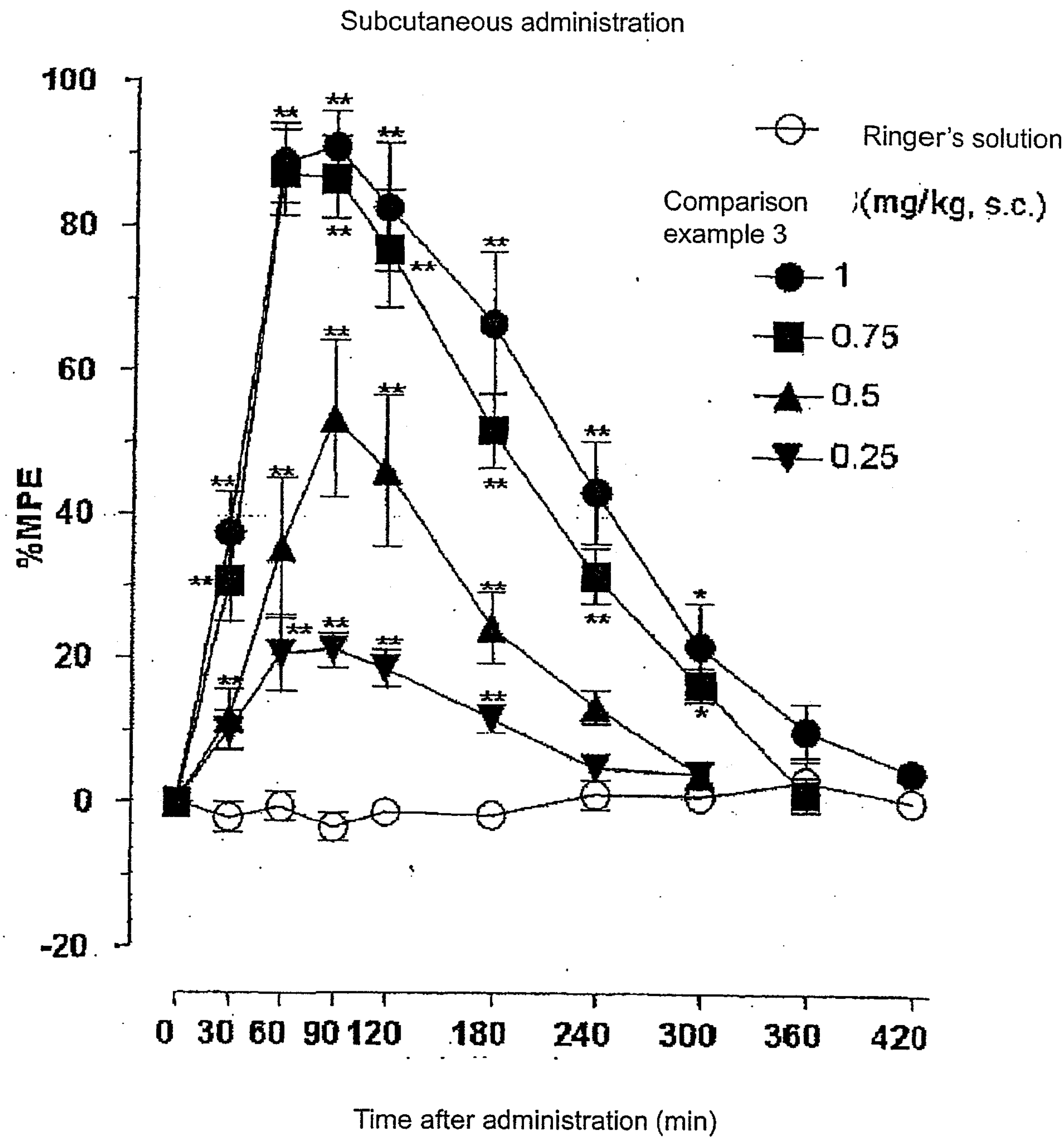
Subcutaneous administration
%MPE
100
80
60
40
20
0
-20
Ringer's solution
Comparison example 3
(mg/kg, s.c.)
1
0.75
0.5
0.25
0 30 60 90 120 180 240 300 360 420
Time after administration (min)

Fig. 34

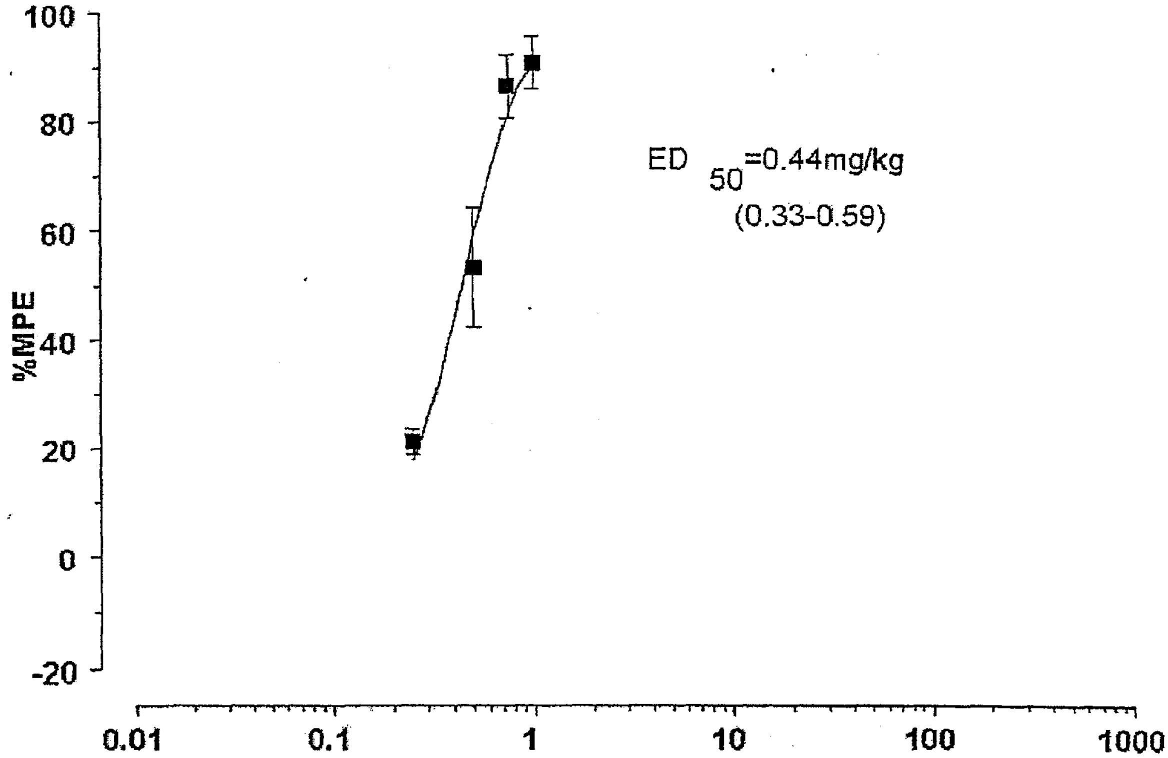


Comparison example 3: Subcutaneous dosage

Fig. 35

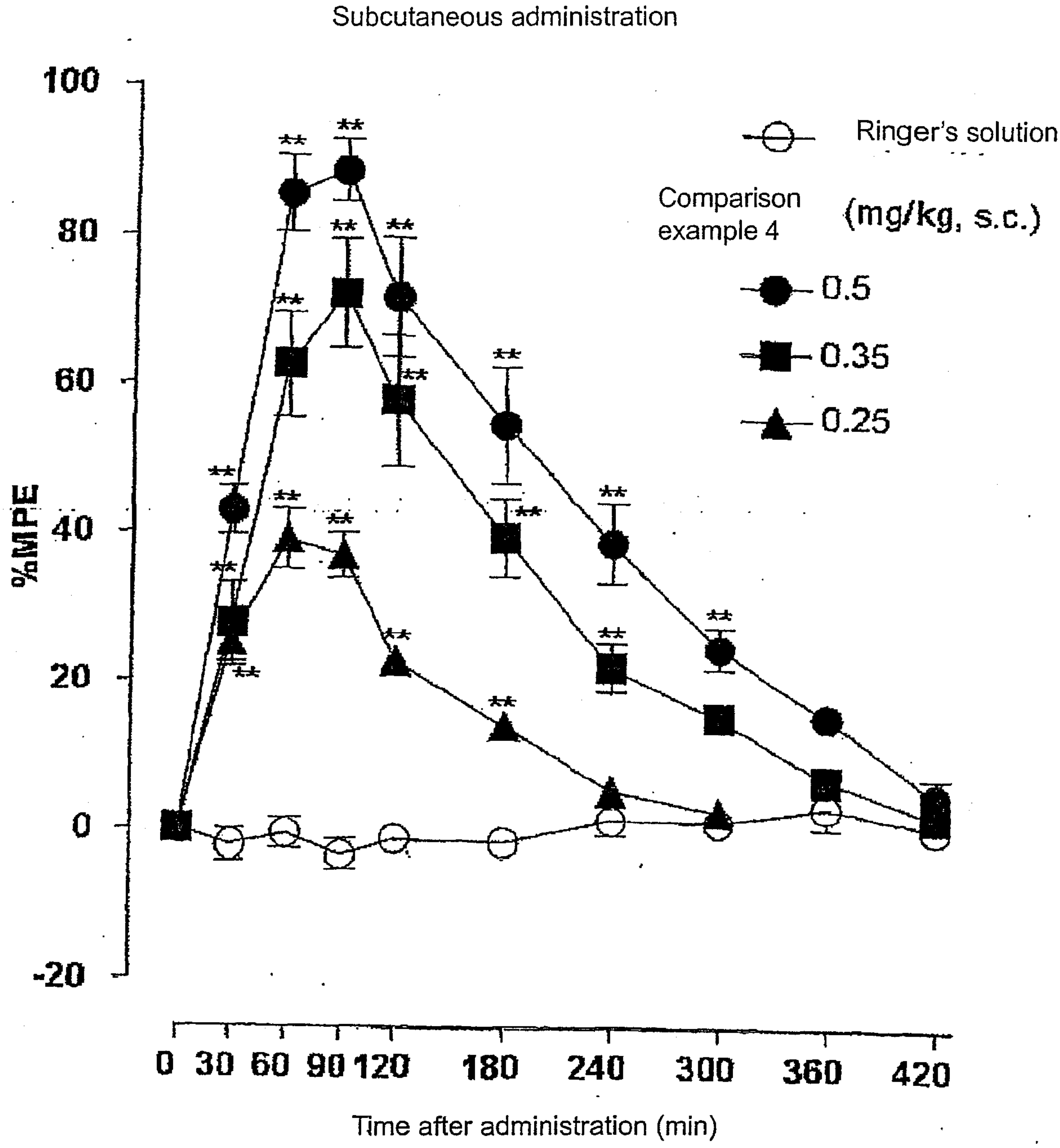
Subcutaneous administration
%MPE
100
80
60
40
20
0
-20
Ringer's solution
Comparison example 4
(mg/kg, s.c.)
0.5
0.35
0.25
0 30 60 90 120 180 240 300 360 420
Time after administration (min)

Fig. 36

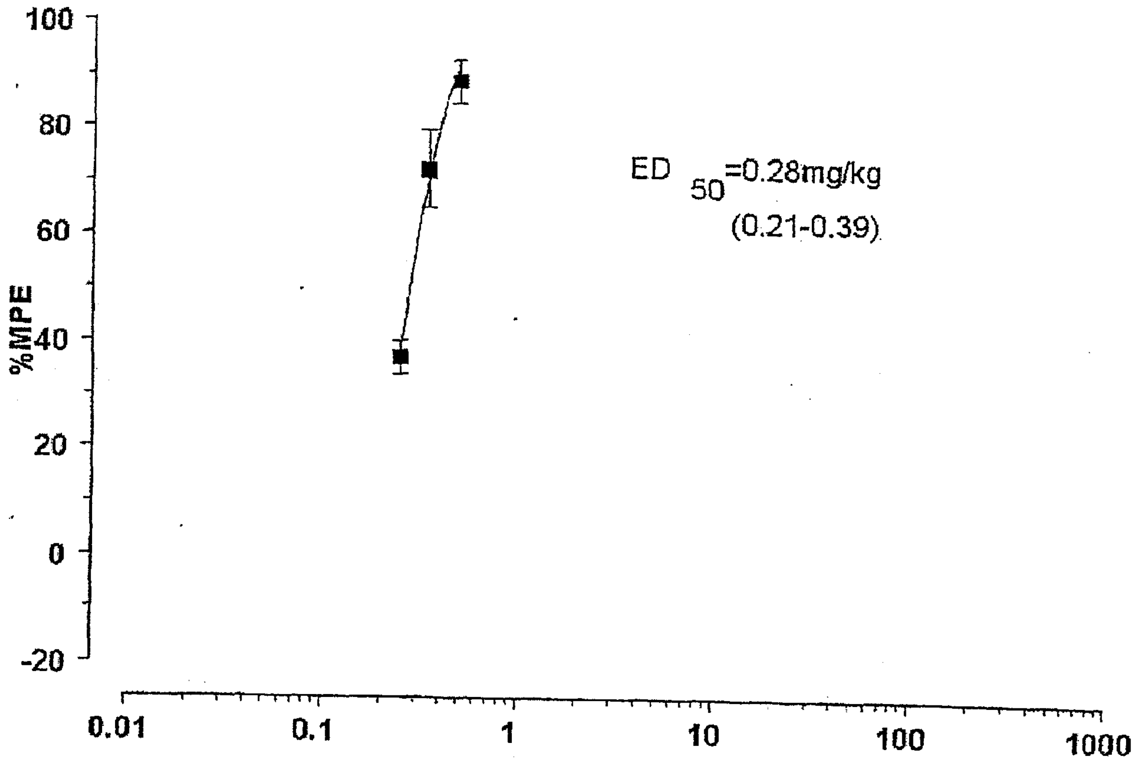


Comparison
example 4: Subcutaneous dosage

Fig. 37

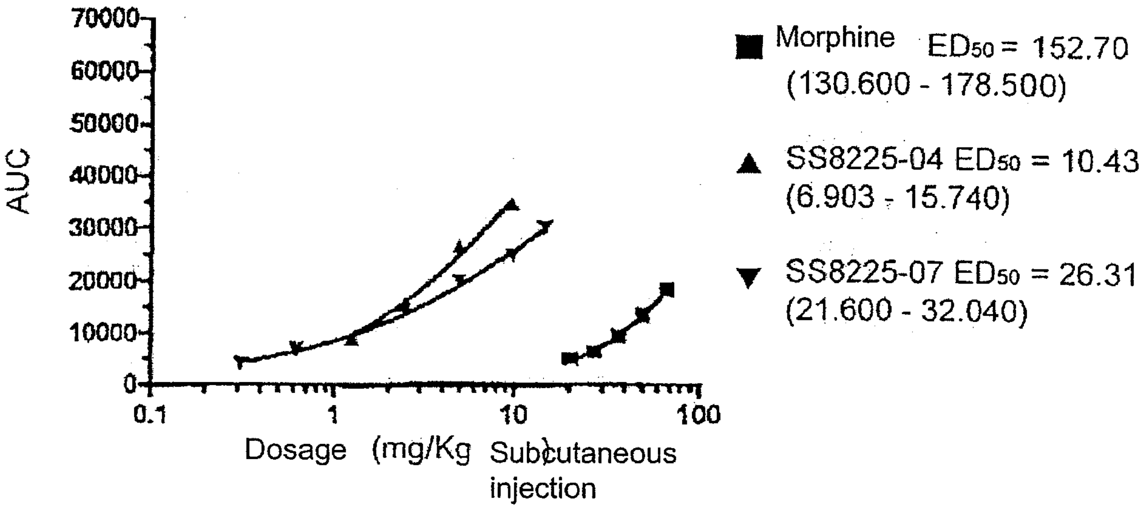
70000
60000
50000
40000
30000
20000
10000
0
AUC
0.1
1
10
100
Dosage (mg/Kg Subcutaneous injection)
Morphine ED50 = 152.70
(130.600 - 178.500)
SS8225-04 ED50 = 10.43
(6.903 - 15.740)
SS8225-07 ED50 = 26.31
(21.600 - 32.040)

Fig. 38

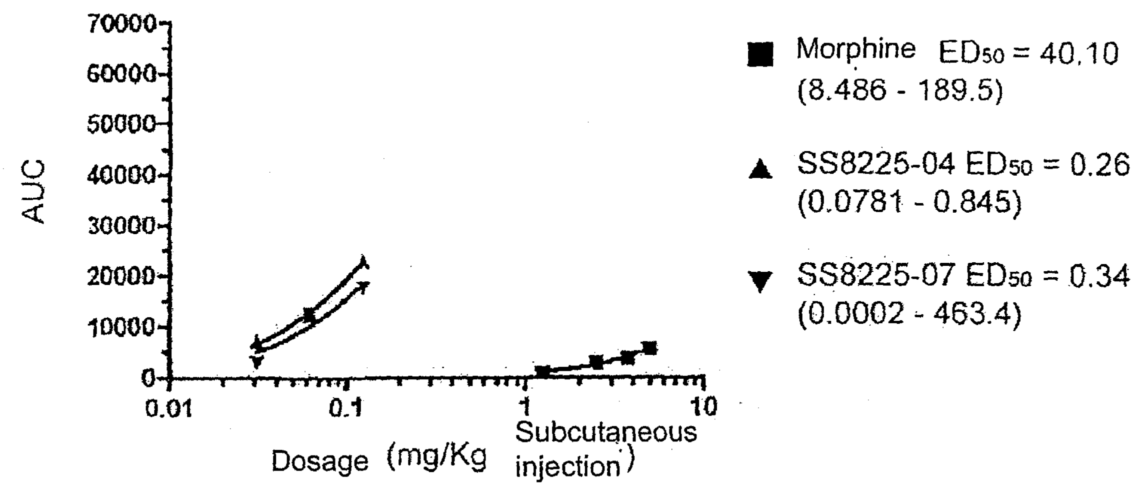
AUC
70000
60000
50000
40000
30000
20000
10000
0
0.01
0.1
1
10
Dosage (mg/Kg Subcutaneous injection)
Morphine ED50 = 40.10 (8.486 - 189.5)
SS8225-04 ED50 = 0.26 (0.0781 - 0.845)
SS8225-07 ED50 = 0.34 (0.0002 - 463.4)

Fig. 39

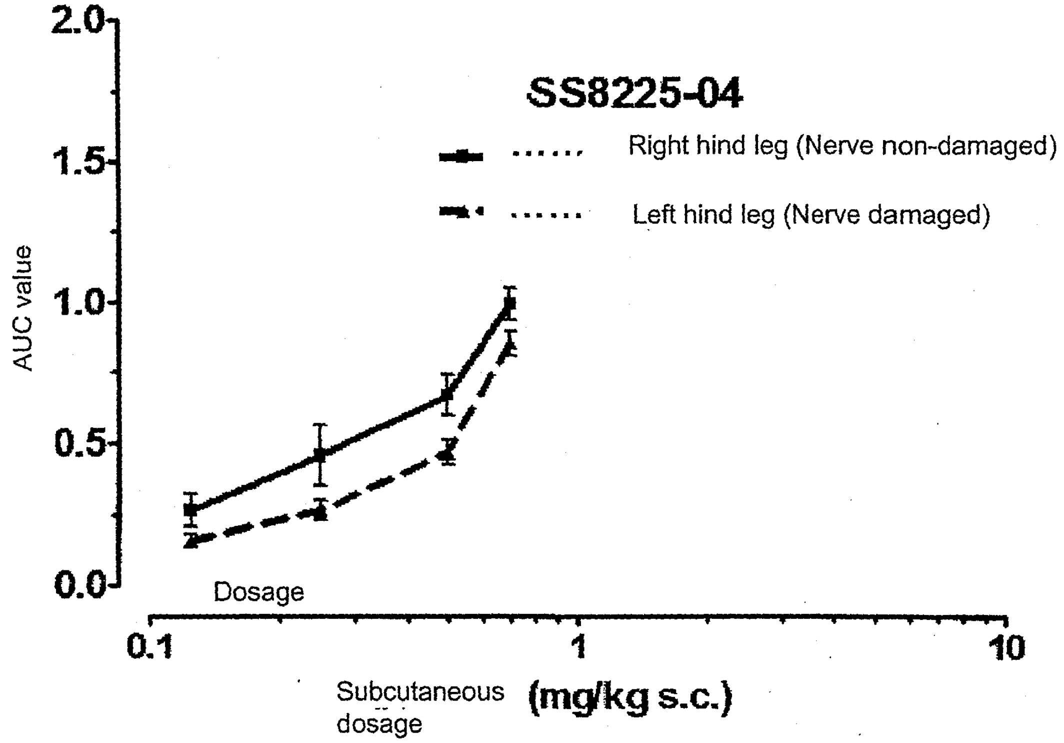
SS8225-04
Right hind leg (Nerve non-damaged)
Left hind leg (Nerve damaged)
AUC value
2.0
1.5
1.0
0.5
0.0
Dosage
0.1
1
10
Subcutaneous dosage
(mg/kg s.c.)

Fig. 40

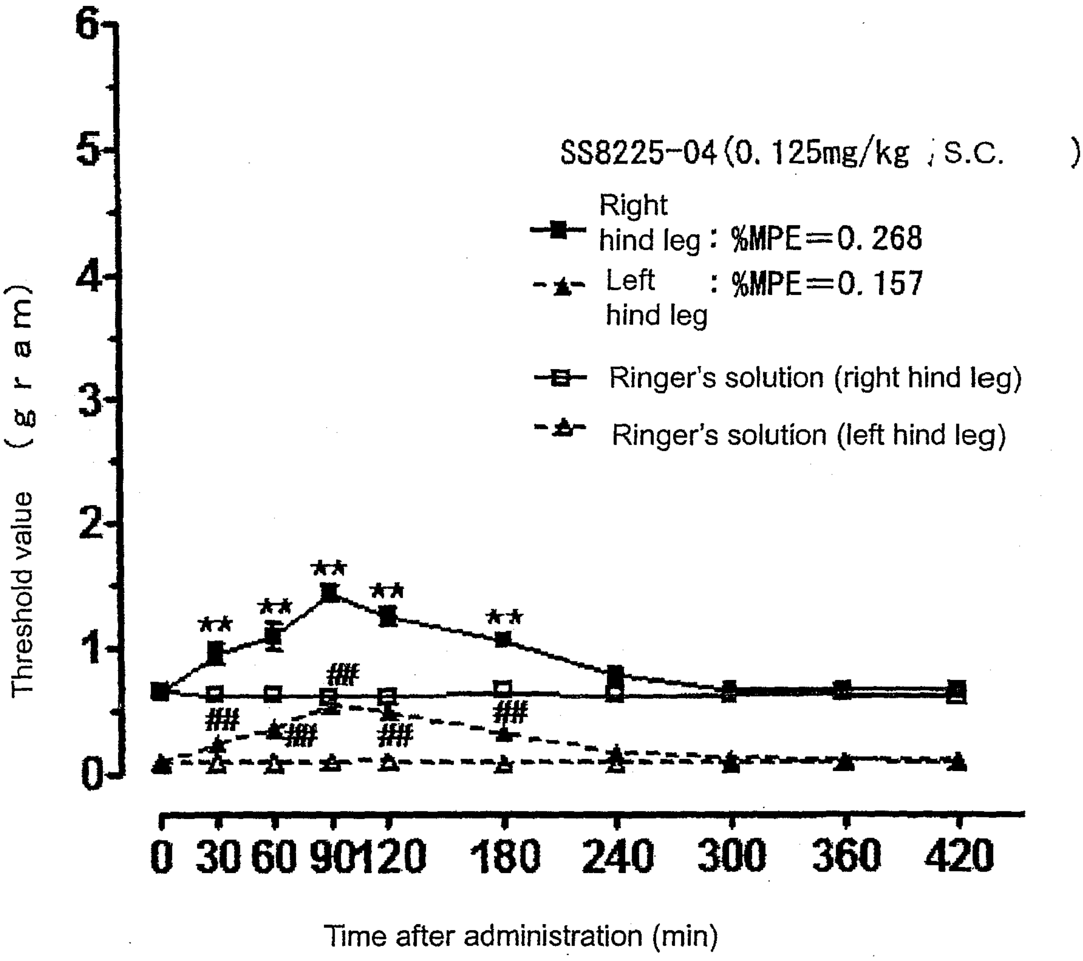
SS8225-04 (0. 125mg/kg ; S.C. )
Right hind leg : %MPE=0. 268
Left hind leg : %MPE=0. 157
Ringer's solution (right hind leg)
Ringer's solution (left hind leg)
Threshold value (g r a m)
0
1
2
3
4
5
6
0 30 60 90 120 180 240 300 360 420
Time after administration (min)
**
###

Fig. 41

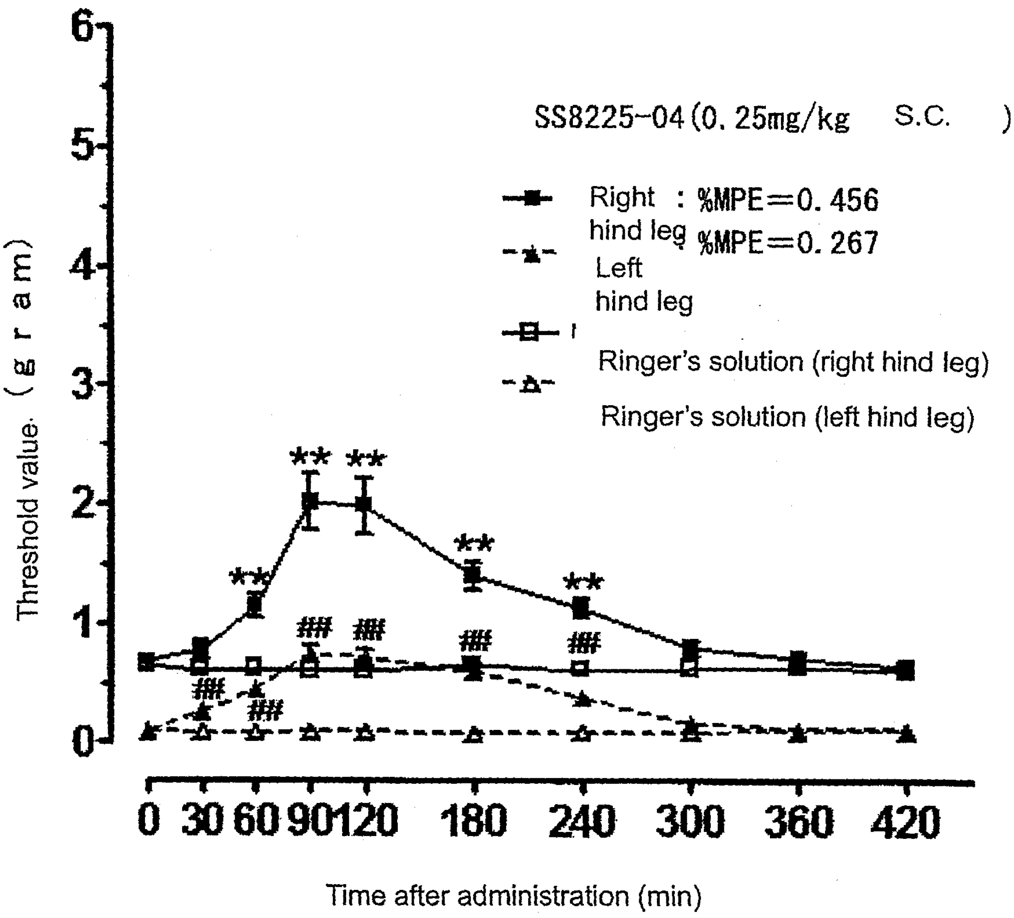
SS8225-04 (0.25mg/kg S.C. )
Right hind leg : %MPE=0.456
Left hind leg %MPE=0.267
Ringer's solution (right hind leg)
Ringer's solution (left hind leg)
Threshold value (gram)
6
5
4
3
2
1
0
0 30 60 90 120 180 240 300 360 420
Time after administration (min)

Fig. 42

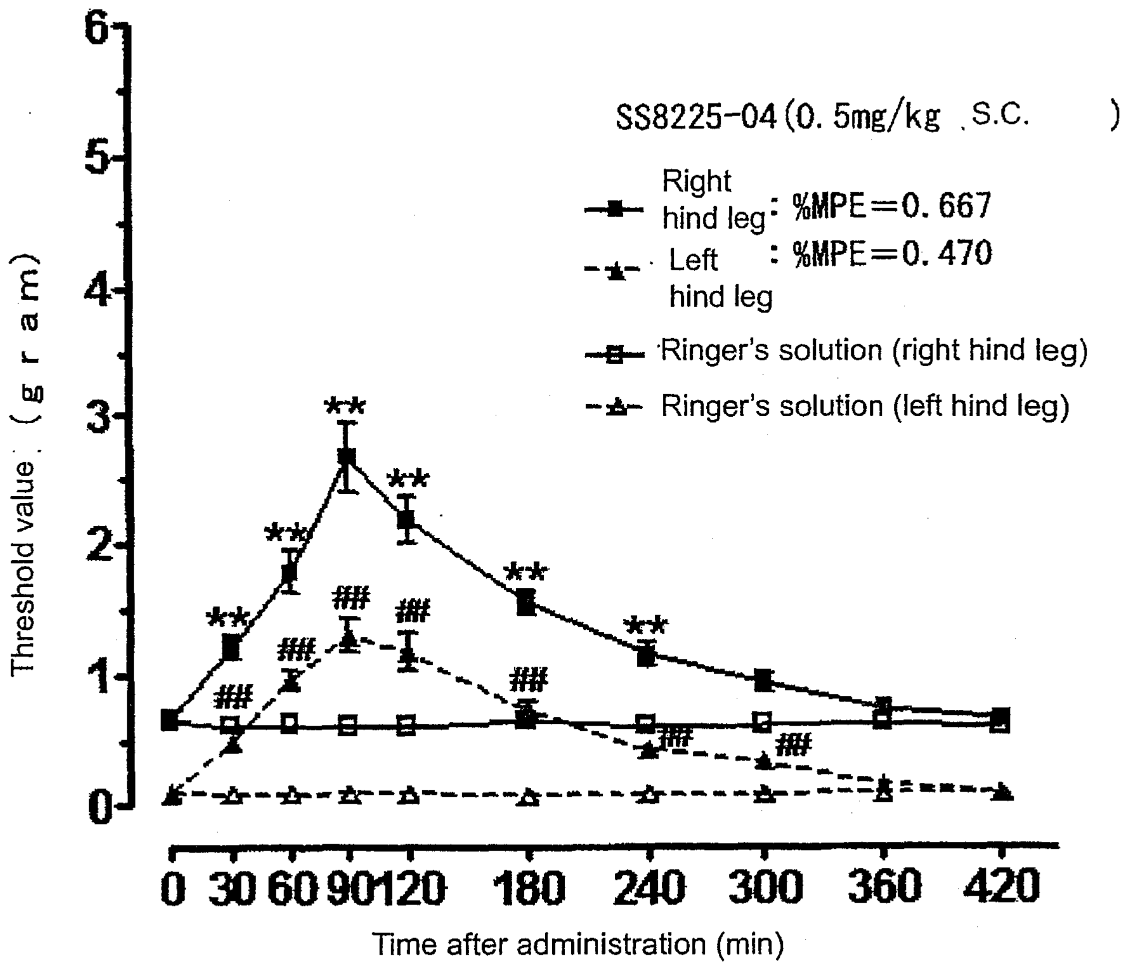
SS8225-04 (0. 5mg/kg .S.C. )
Right hind leg: %MPE=0. 667
Left hind leg : %MPE=0. 470
Ringer's solution (right hind leg)
Ringer's solution (left hind leg)
Threshold value (g r a m)
0
1
2
3
4
5
6
**
##
###
0 30 60 90 120 180 240 300 360 420
Time after administration (min)

Fig. 43

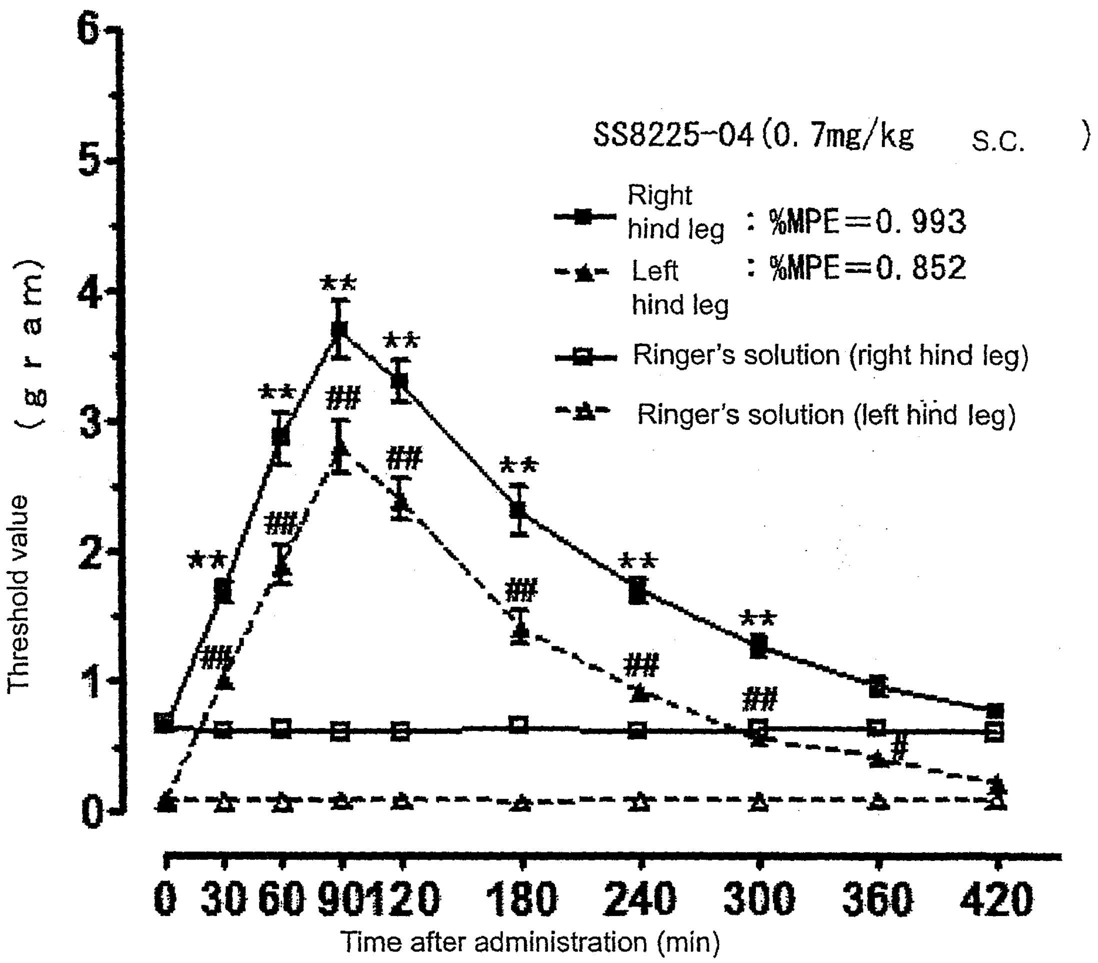
SS8225-04 (0. 7mg/kg s.c. )
Right hind leg : %MPE=0. 993
Left hind leg : %MPE=0. 852
Ringer's solution (right hind leg)
Ringer's solution (left hind leg)
Threshold value (gram)
0
1
2
3
4
5
6
0 30 60 90 120 180 240 300 360 420
Time after administration (min)

Fig. 44

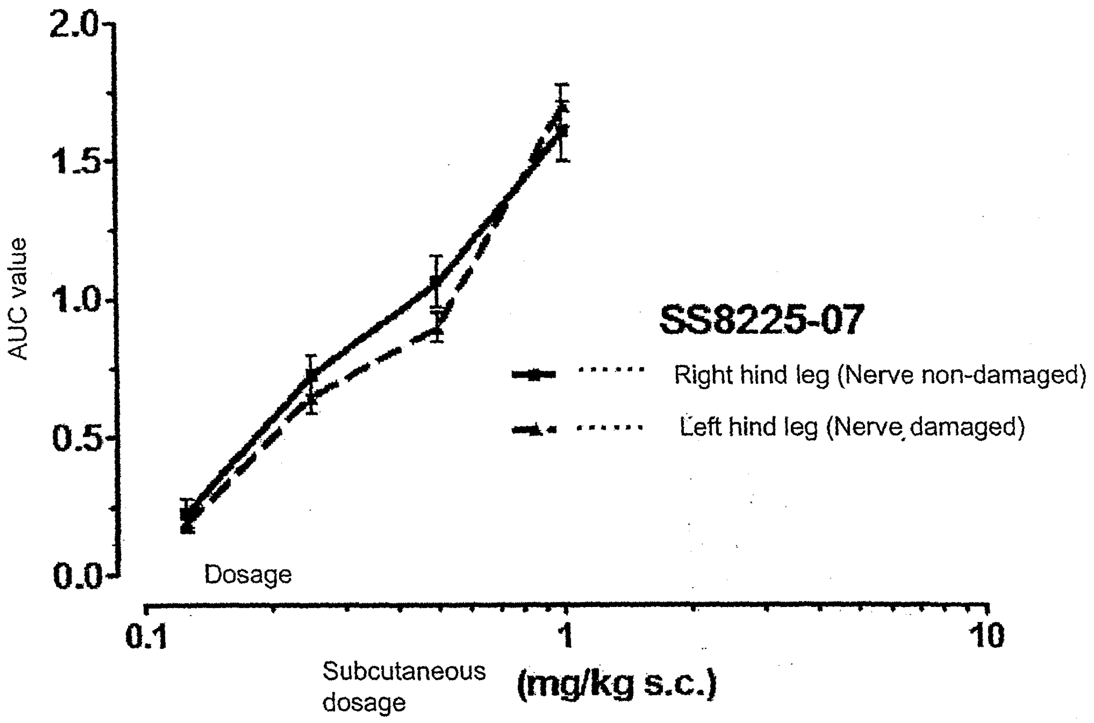
2.0
1.5
1.0
0.5
0.0
AUC value
SS8225-07
Right hind leg (Nerve non-damaged)
Left hind leg (Nerve damaged)
Dosage
0.1
1
10
Subcutaneous dosage
(mg/kg s.c.)

Fig. 45

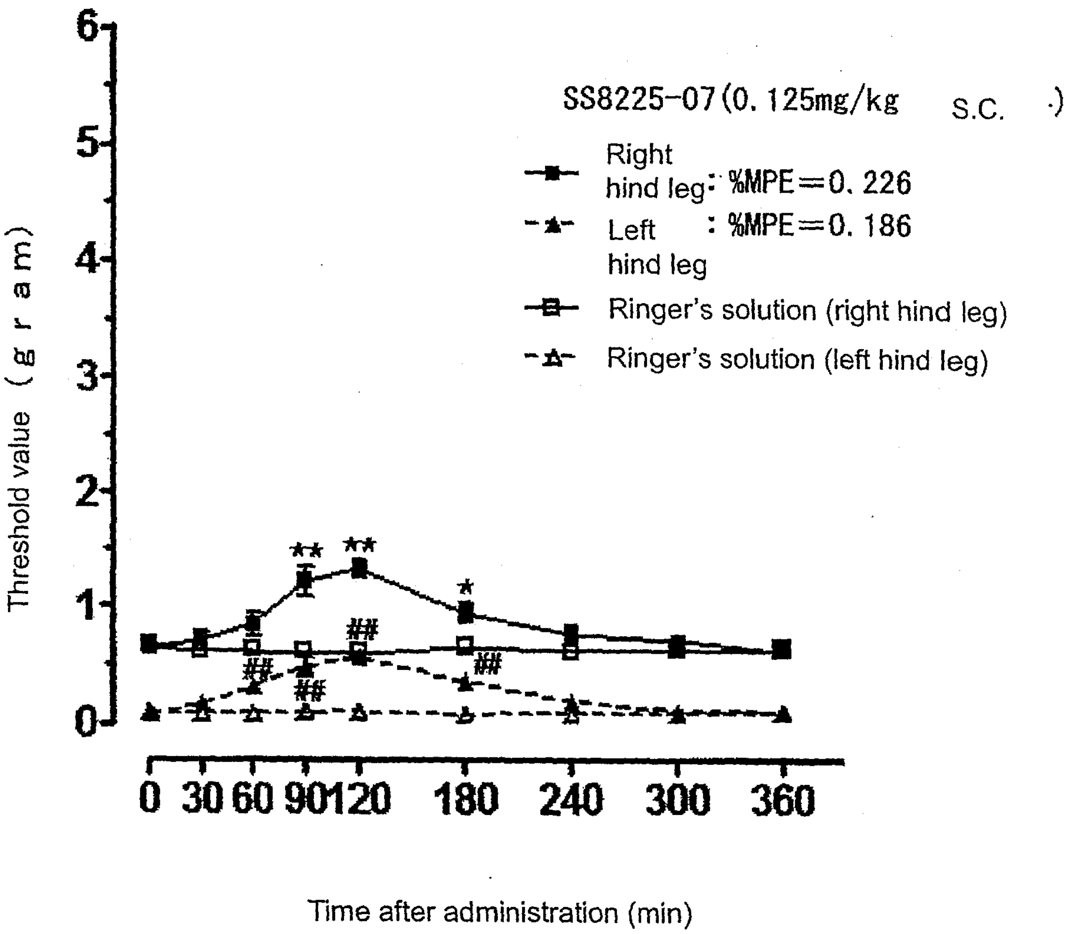
SS8225-07(0.125mg/kg S.C. )
Right hind leg: %MPE=0.226
Left hind leg : %MPE=0.186
Ringer's solution (right hind leg)
Ringer's solution (left hind leg)
Threshold value (gram)
Time after administration (min)
0 30 60 90 120 180 240 300 360
0 1 2 3 4 5 6
** ** *
## ## ## ##

Fig. 46

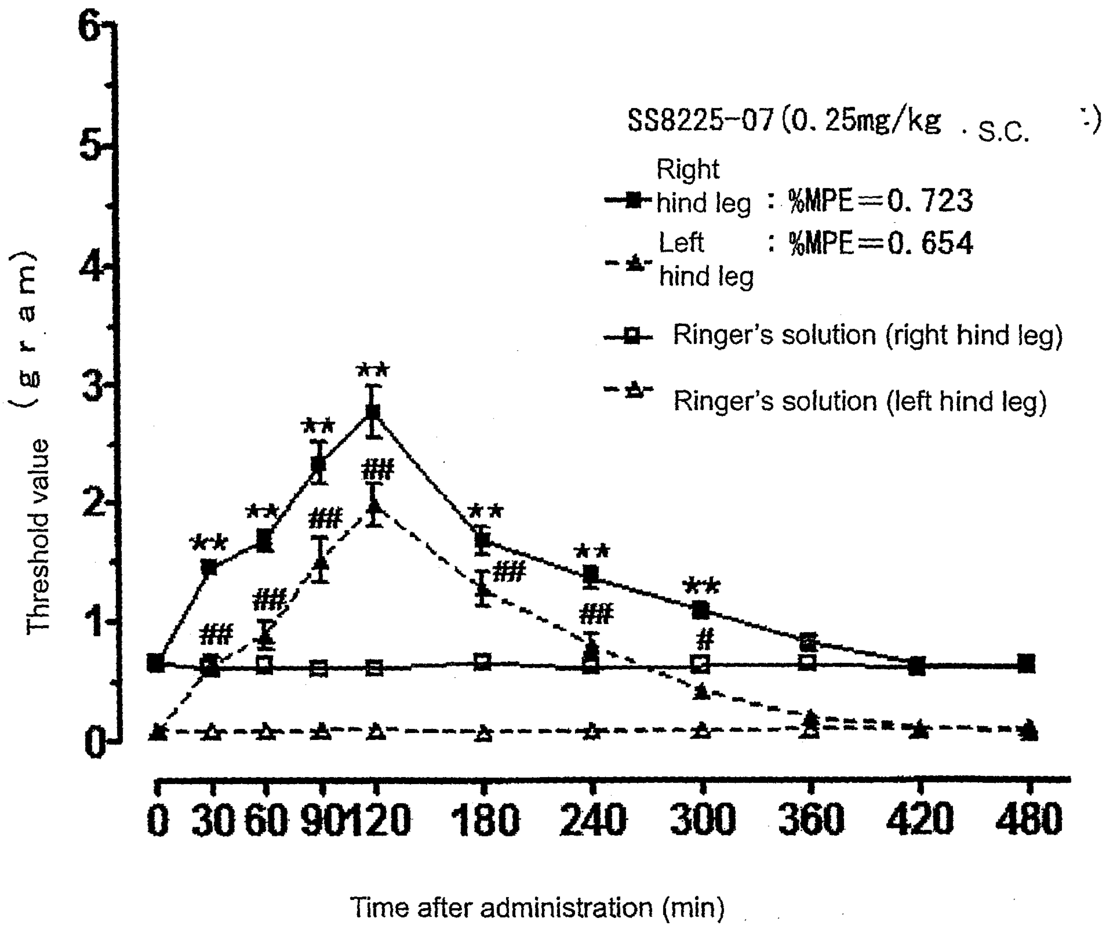
SS8225-07 (0.25mg/kg . s.c.)
Right hind leg : %MPE=0.723
Left hind leg : %MPE=0.654
Ringer's solution (right hind leg)
Ringer's solution (left hind leg)
Threshold value (gram)
0
1
2
3
4
5
6
0 30 60 90 120 180 240 300 360 420 480
Time after administration (min)
**
##
#

Fig. 47

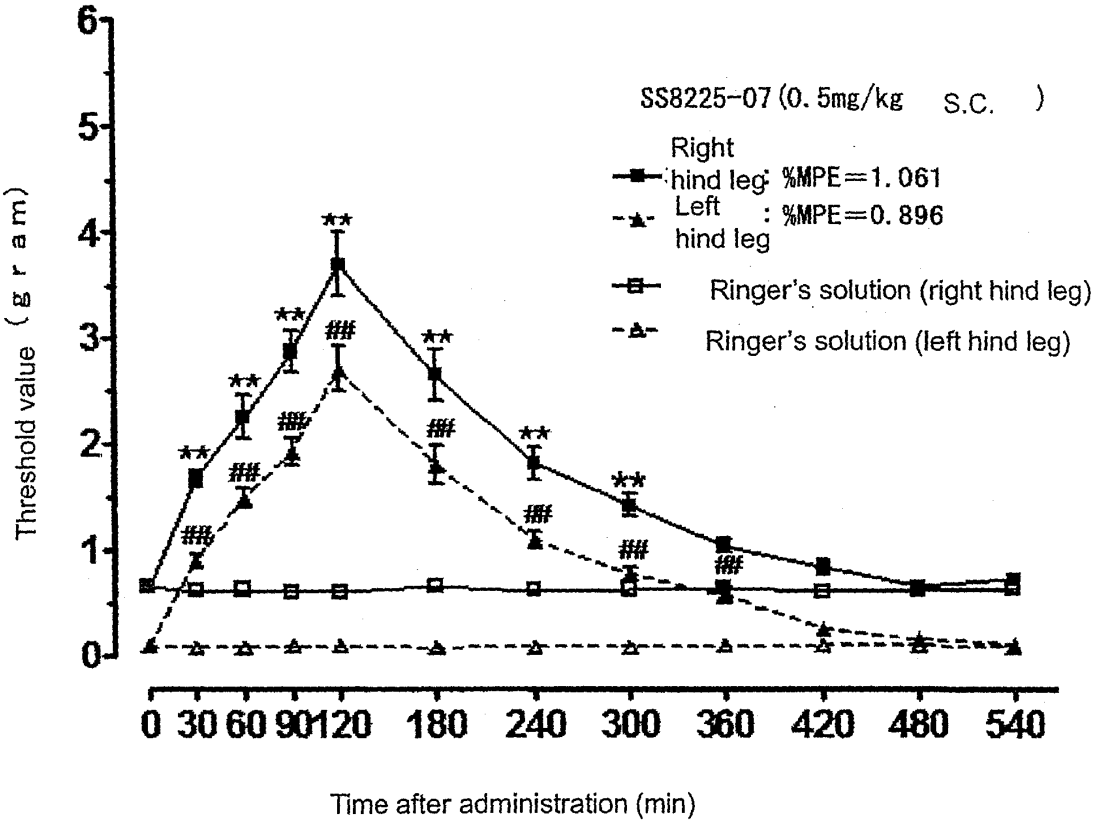
SS8225-07 (0.5mg/kg S.C. )
Right hind leg: %MPE=1.061
Left hind leg : %MPE=0.896
Ringer's solution (right hind leg)
Ringer's solution (left hind leg)
Threshold value (gram)
Time after administration (min)
0 30 60 90 120 180 240 300 360 420 480 540
0 1 2 3 4 5 6
**
##

Fig. 48

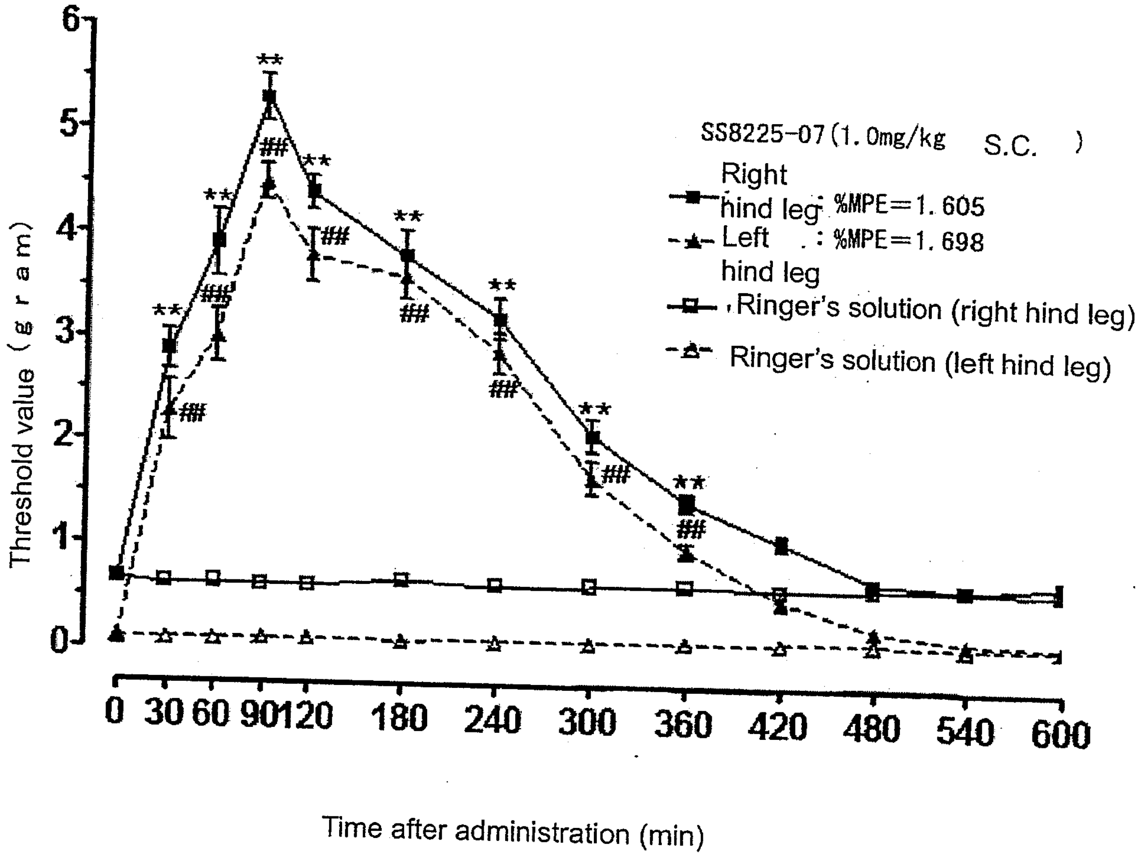
6
5
4
3
2
1
0
Threshold value（g r a m)
**
##
SS8225-07(1.0mg/kg S.C. )
Right hind leg : %MPE=1.605
Left hind leg : %MPE=1.698
Ringer's solution (right hind leg)
Ringer's solution (left hind leg)
0 30 60 90 120 180 240 300 360 420 480 540 600
Time after administration (min)

Fig. 49

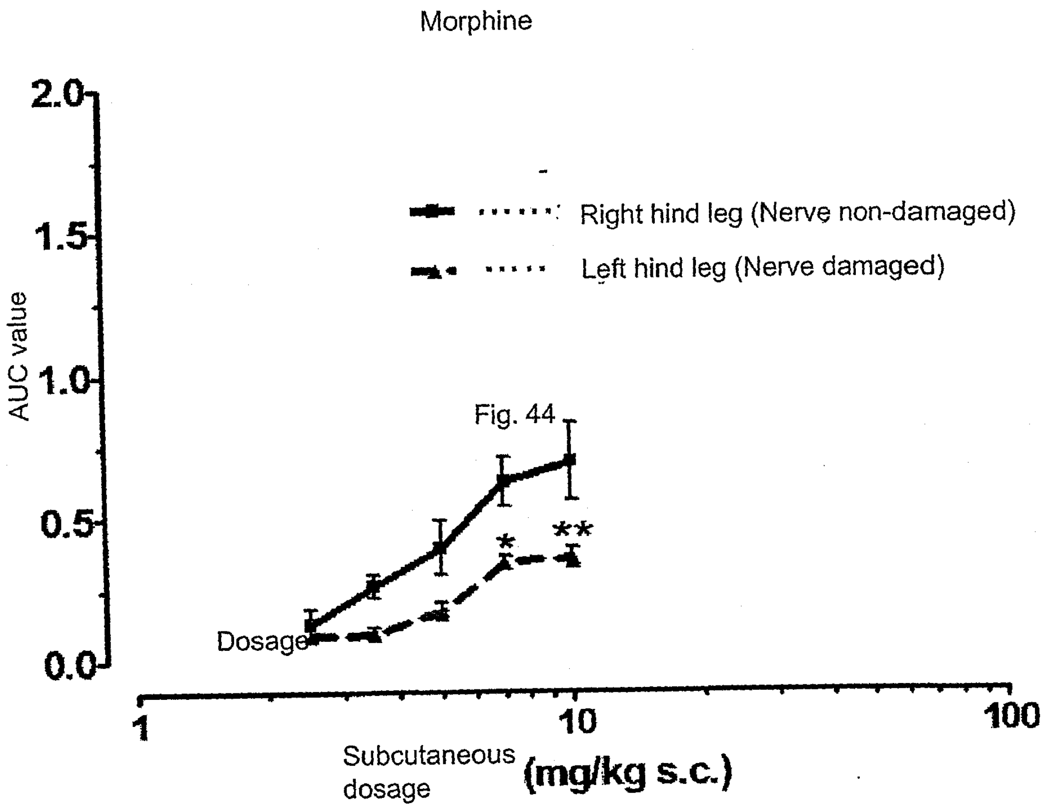
Morphine
Right hind leg (Nerve non-damaged)
Left hind leg (Nerve damaged)
AUC value
2.0
1.5
1.0
0.5
0.0
Fig. 44
Dosage
*
**
1
10
100
Subcutaneous dosage
(mg/kg s.c.)

Fig. 50

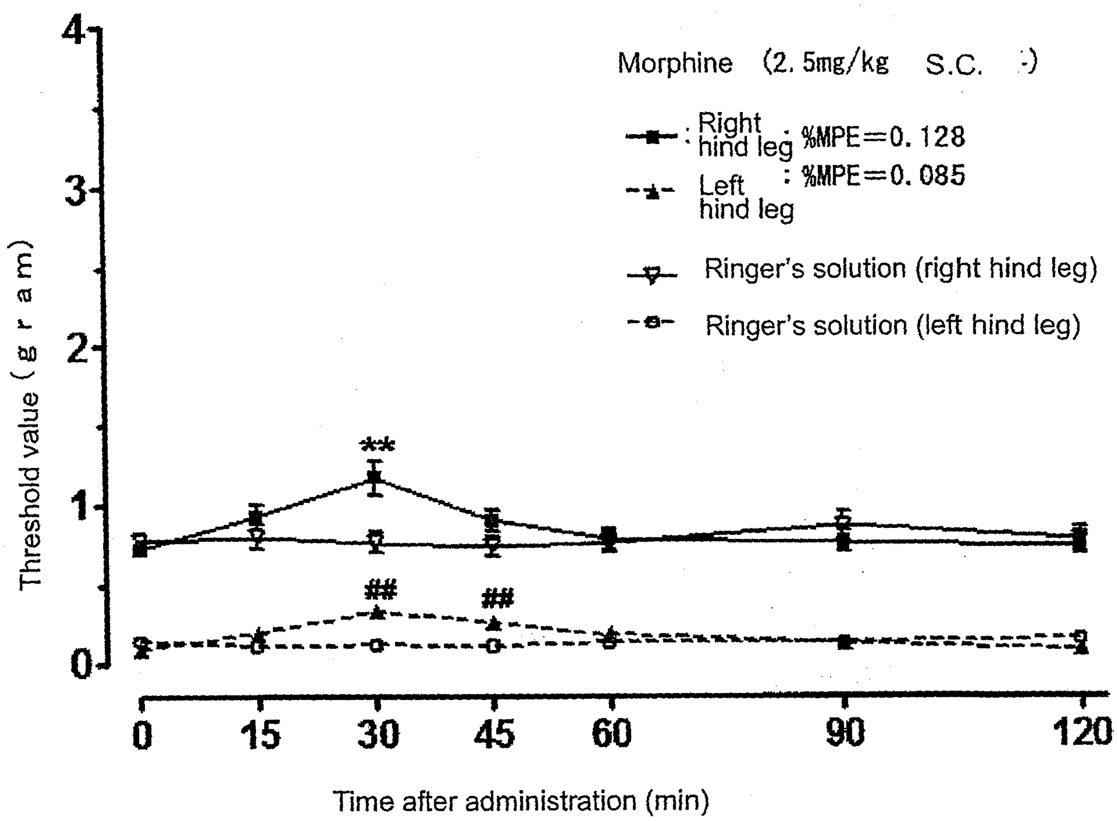
Morphine (2. 5mg/kg S.C. )
Right hind leg : %MPE=0. 128
Left hind leg : %MPE=0. 085
Ringer's solution (right hind leg)
Ringer's solution (left hind leg)
Threshold value (g r a m)
4
3
2
1
0
**
##
##
0
15
30
45
60
90
120
Time after administration (min)

Fig. 51

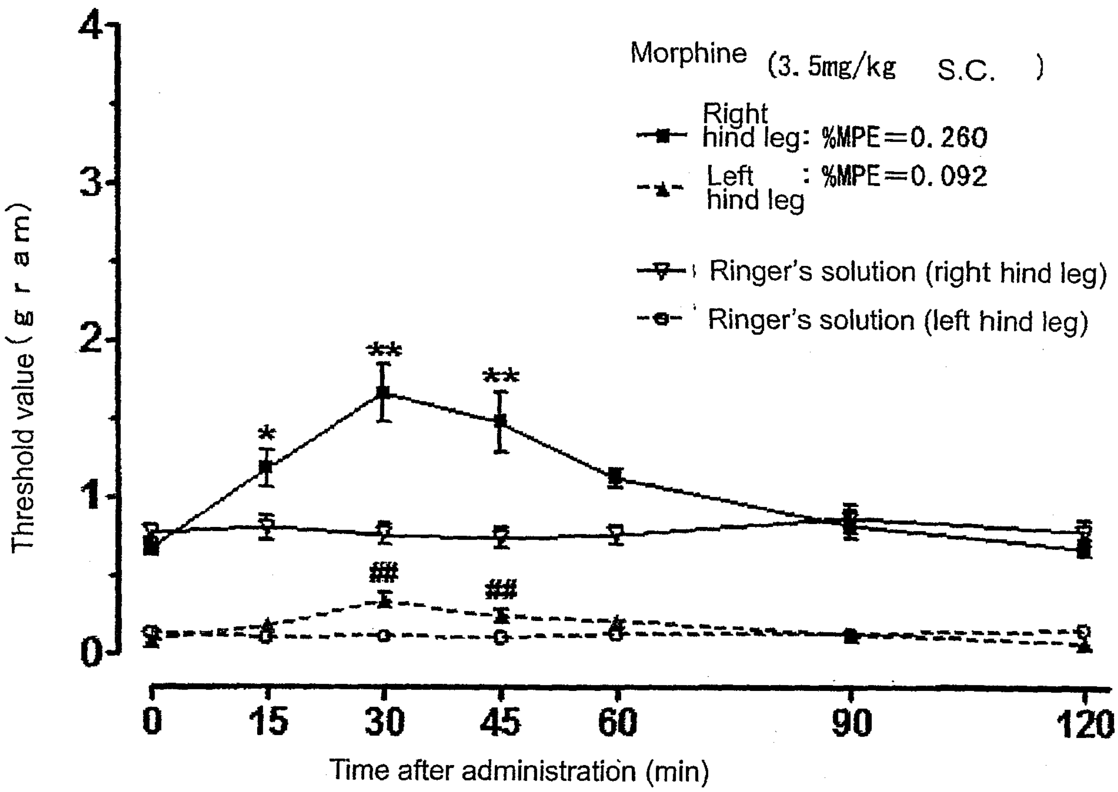
Morphine (3.5mg/kg S.C. )
Right hind leg: %MPE=0.260
Left hind leg : %MPE=0.092
Ringer's solution (right hind leg)
Ringer's solution (left hind leg)
Threshold value (gram)
Time after administration (min)
0
1
2
3
4
0
15
30
45
60
90
120
*
**
**
##
##

Fig. 52

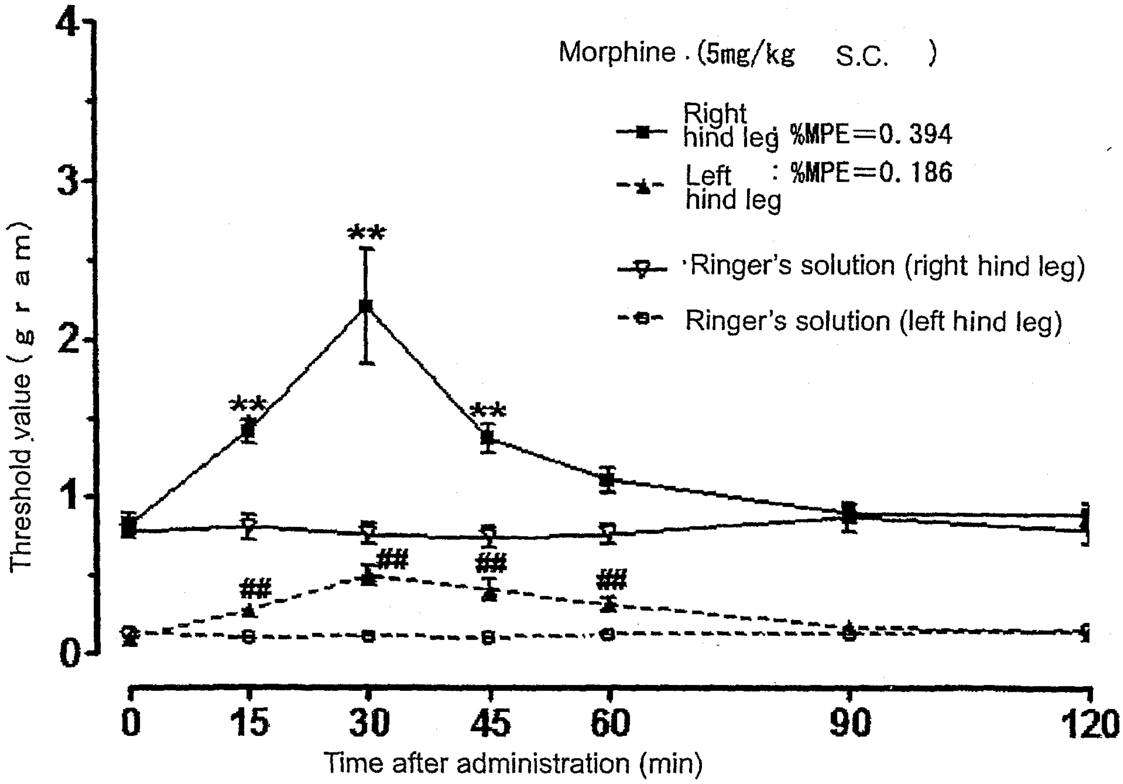
Morphine (5mg/kg S.C. )
Right hind leg : %MPE=0. 394
Left hind leg : %MPE=0. 186
Ringer's solution (right hind leg)
Ringer's solution (left hind leg)
Threshold value (g r a m)
Time after administration (min)
4
3
2
1
0
0
15
30
45
60
90
120
**
**
**
##
##
##
##

Fig. 53

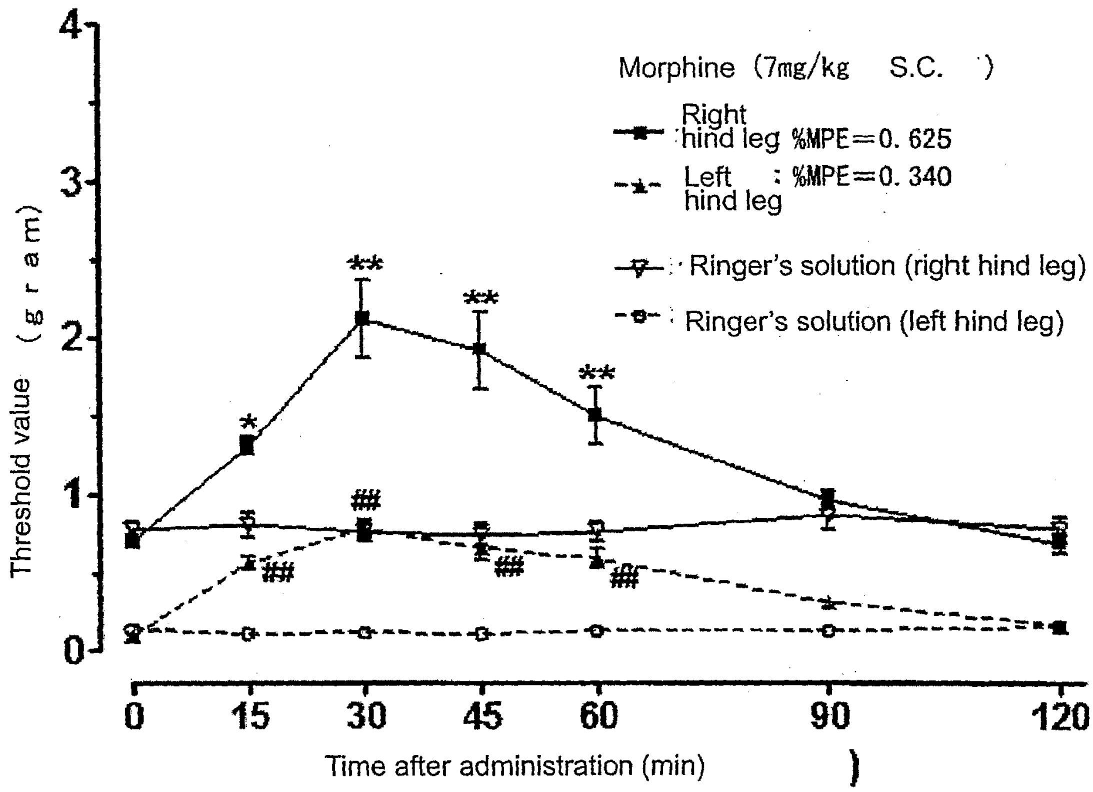
Morphine (7mg/kg S.C. )
Right hind leg %MPE=0. 625
Left hind leg : %MPE=0. 340
Ringer's solution (right hind leg)
Ringer's solution (left hind leg)
Threshold value (gram)
0
1
2
3
4
*
**
**
**
##
##
##
##
0
15
30
45
60
90
120
Time after administration (min)

Fig. 54

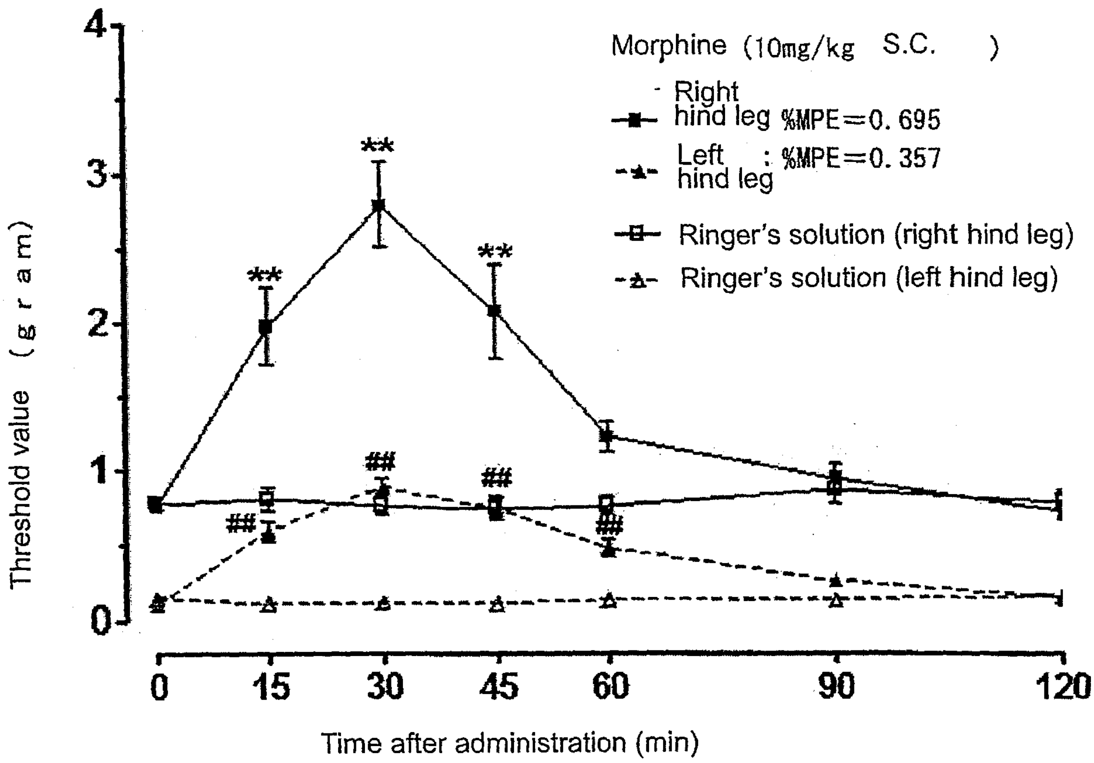

Morphine (10mg/kg S.C. )
Right hind leg %MPE=0. 695
Left hind leg : %MPE=0. 357
Ringer's solution (right hind leg)
Ringer's solution (left hind leg)
Threshold value (g r a m)
0
1
2
3
4
**
**
**
##
##
##
##
0
15
30
45
60
90
120
Time after administration (min)

Fig. 55

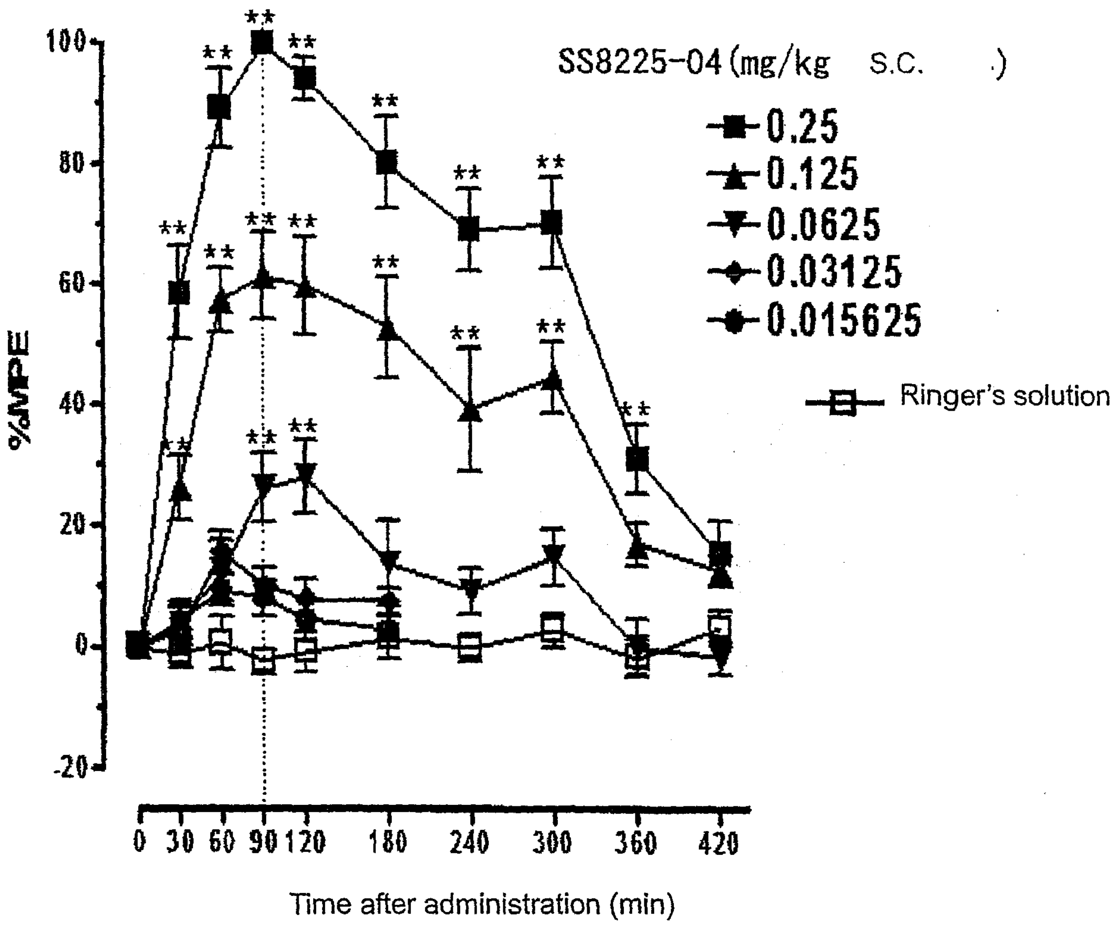
SS8225-04 (mg/kg s.c.)
0.25
0.125
0.0625
0.03125
0.015625
Ringer's solution
%MPE
100
80
60
40
20
0
-20
**
0 30 60 90 120 180 240 300 360 420
Time after administration (min)

Fig. 56

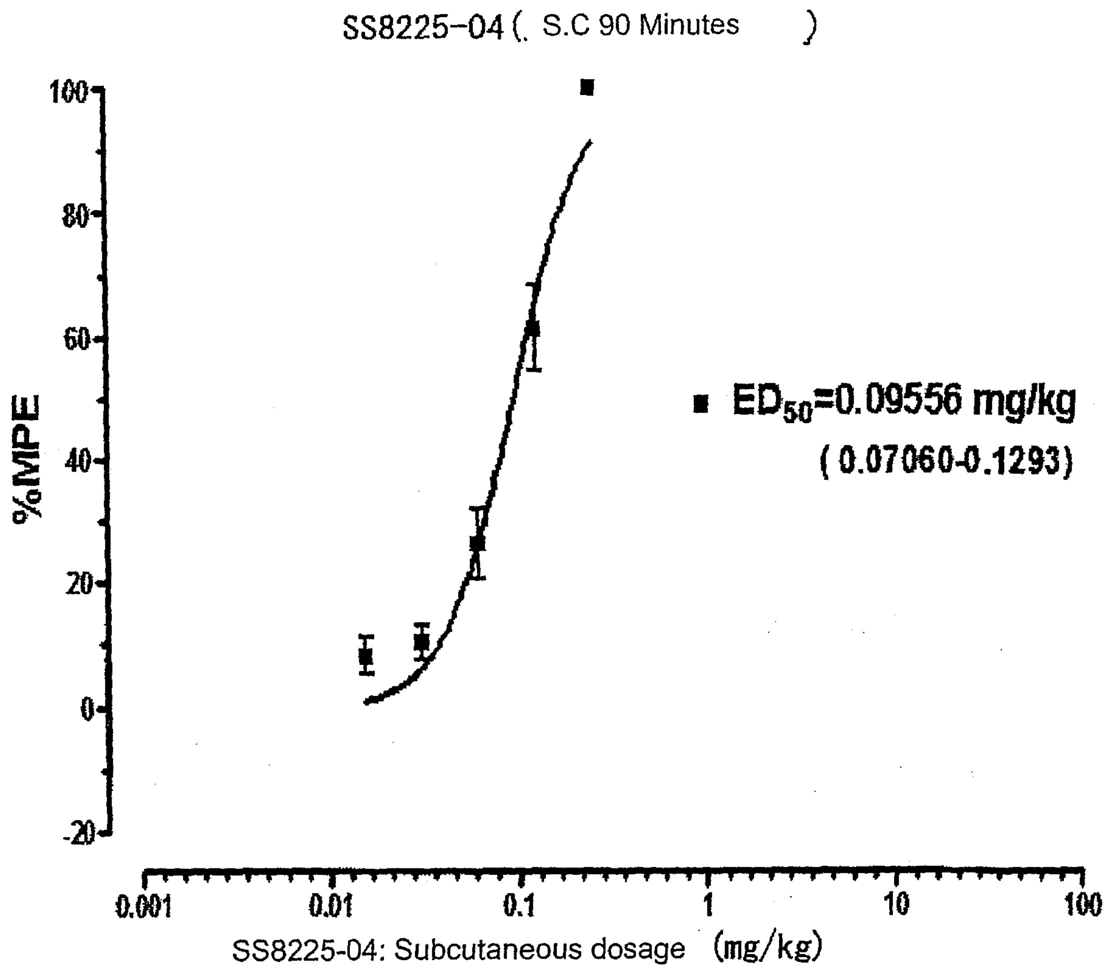
SS8225-04 (. S.C 90 Minutes )
%MPE
100
80
60
40
20
0
-20
■ $ED_{50}$=0.09556 mg/kg
( 0.07060-0.1293)
0.001
0.01
0.1
1
10
100
SS8225-04: Subcutaneous dosage (mg/kg)

Fig. 57

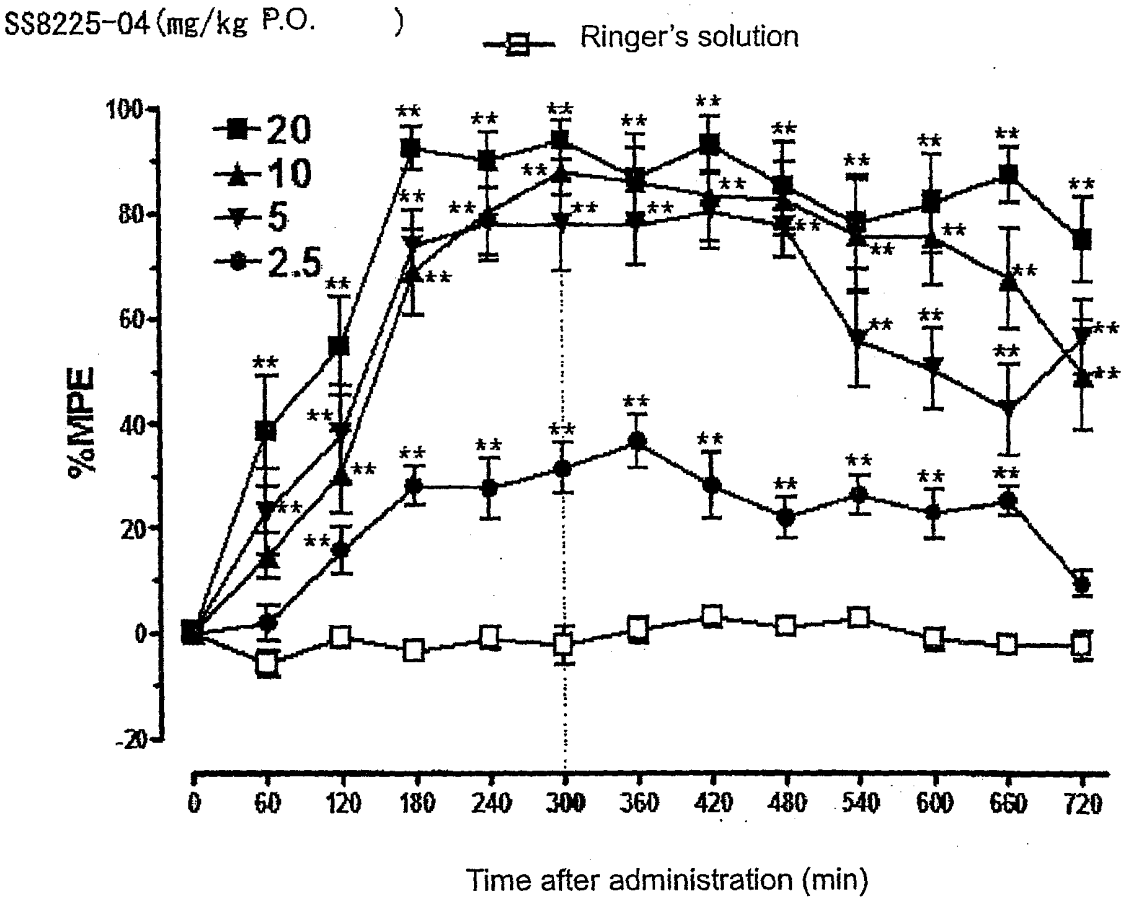
SS8225-04 (mg/kg P.O. )
Ringer's solution
20
10
5
2.5
%MPE
Time after administration (min)

Fig. 58

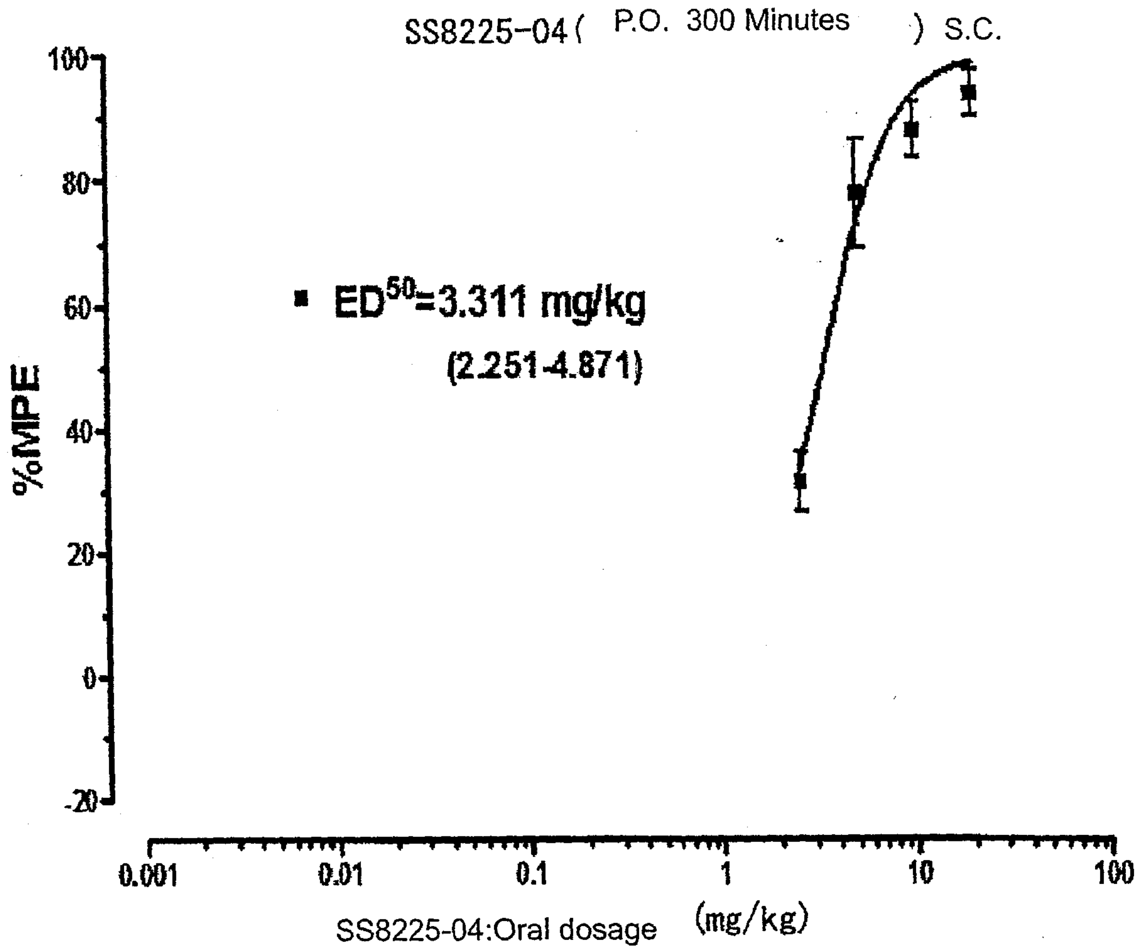
SS8225-04 ( P.O. 300 Minutes ) S.C.
100
80
60
40
20
0
-20
%MPE
ED50=3.311 mg/kg
(2.251-4.871)
0.001
0.01
0.1
1
10
100
SS8225-04:Oral dosage (mg/kg)

Fig. 59

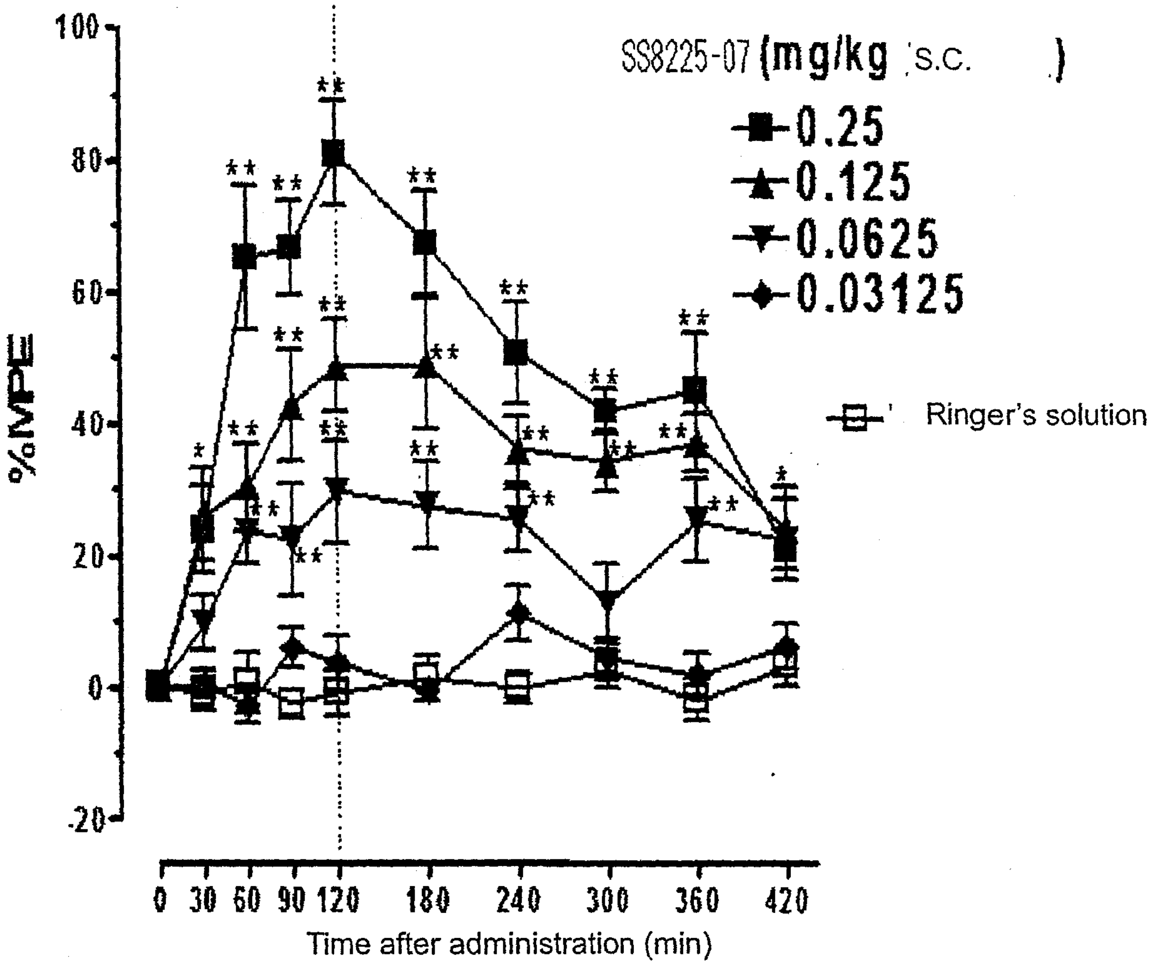
SS8225-07 (mg/kg s.c.)
0.25
0.125
0.0625
0.03125
Ringer's solution
%MPE
100
80
60
40
20
0
-20
0
30
60
90
120
180
240
300
360
420
Time after administration (min)

Fig. 60

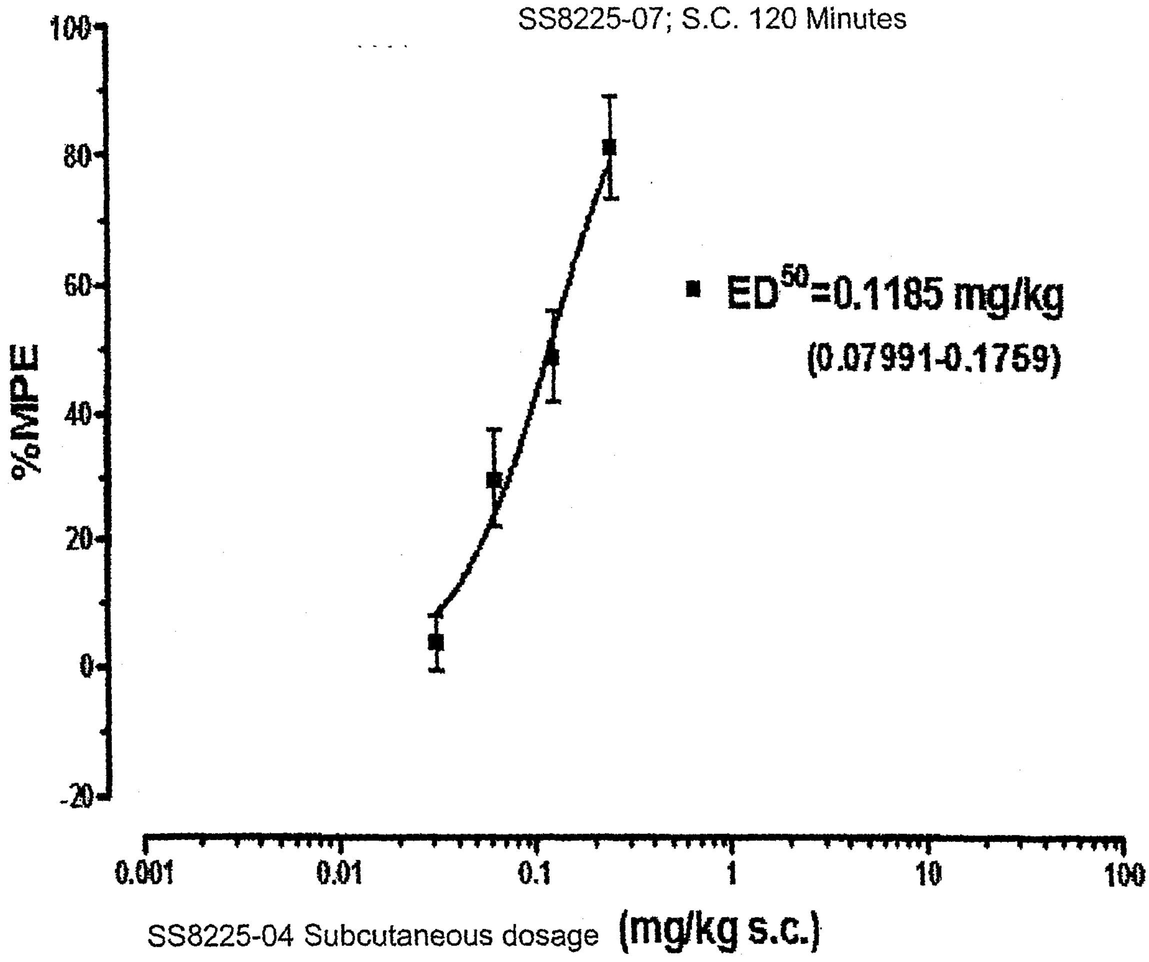
SS8225-07; S.C. 120 Minutes
%MPE
100
80
60
40
20
0
-20
ED50=0.1185 mg/kg
(0.07991-0.1759)
0.001
0.01
0.1
1
10
100
SS8225-04 Subcutaneous dosage (mg/kg s.c.)

Fig. 61

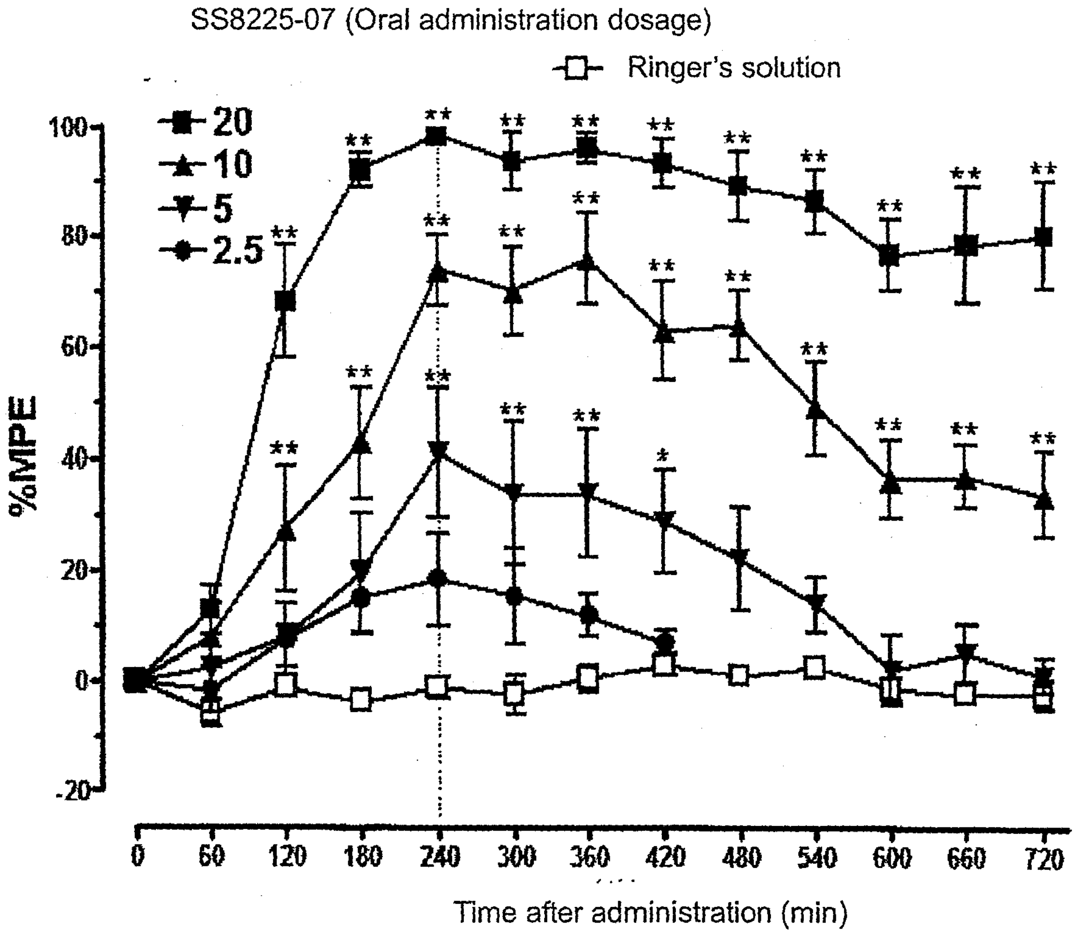
SS8225-07 (Oral administration dosage)
Ringer's solution
20
10
5
2.5
%MPE
100
80
60
40
20
0
-20
0
60
120
180
240
300
360
420
480
540
600
660
720
Time after administration (min)

Fig. 62

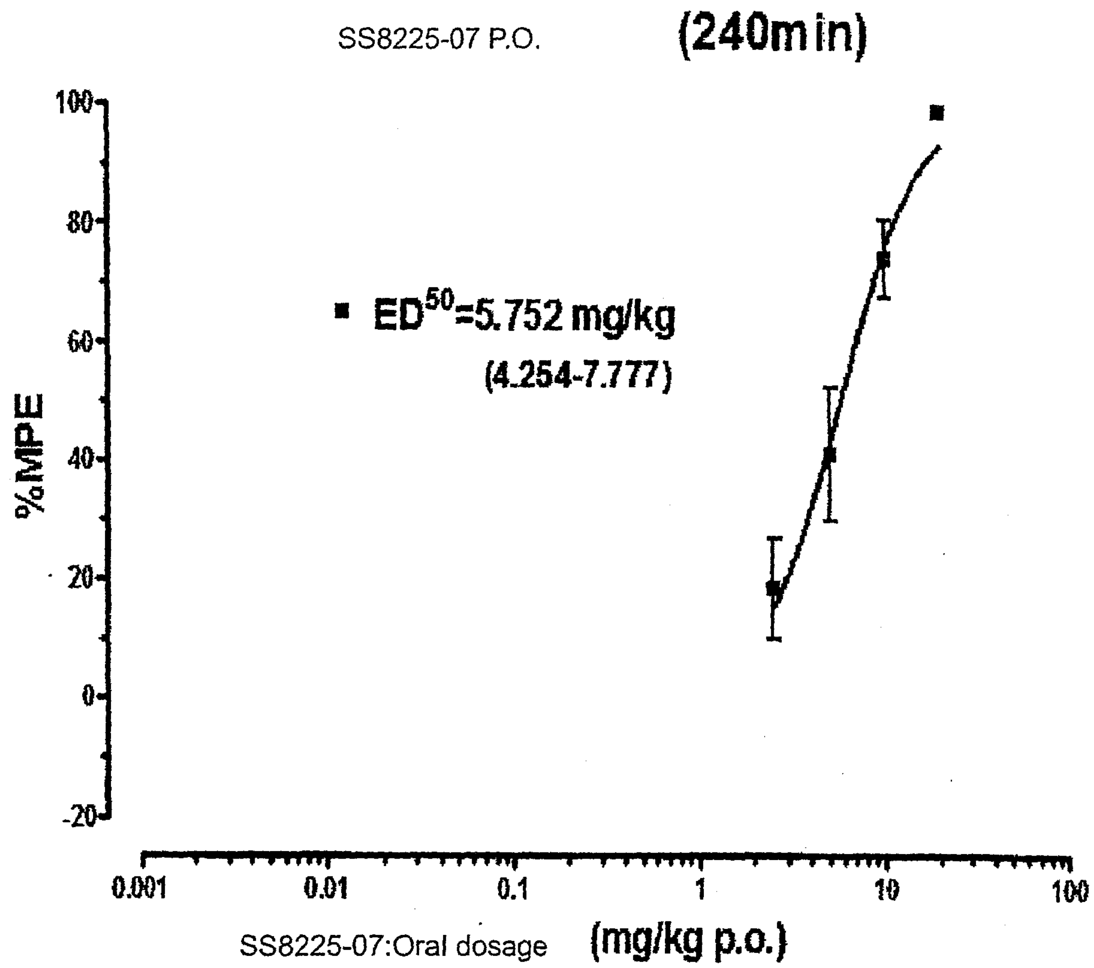
SS8225-07 P.O.
(240min)
100
80
60
40
20
0
-20
%MPE
ED50=5.752 mg/kg
(4.254-7.777)
0.001
0.01
0.1
1
10
100
SS8225-07:Oral dosage
(mg/kg p.o.)

Fig. 63

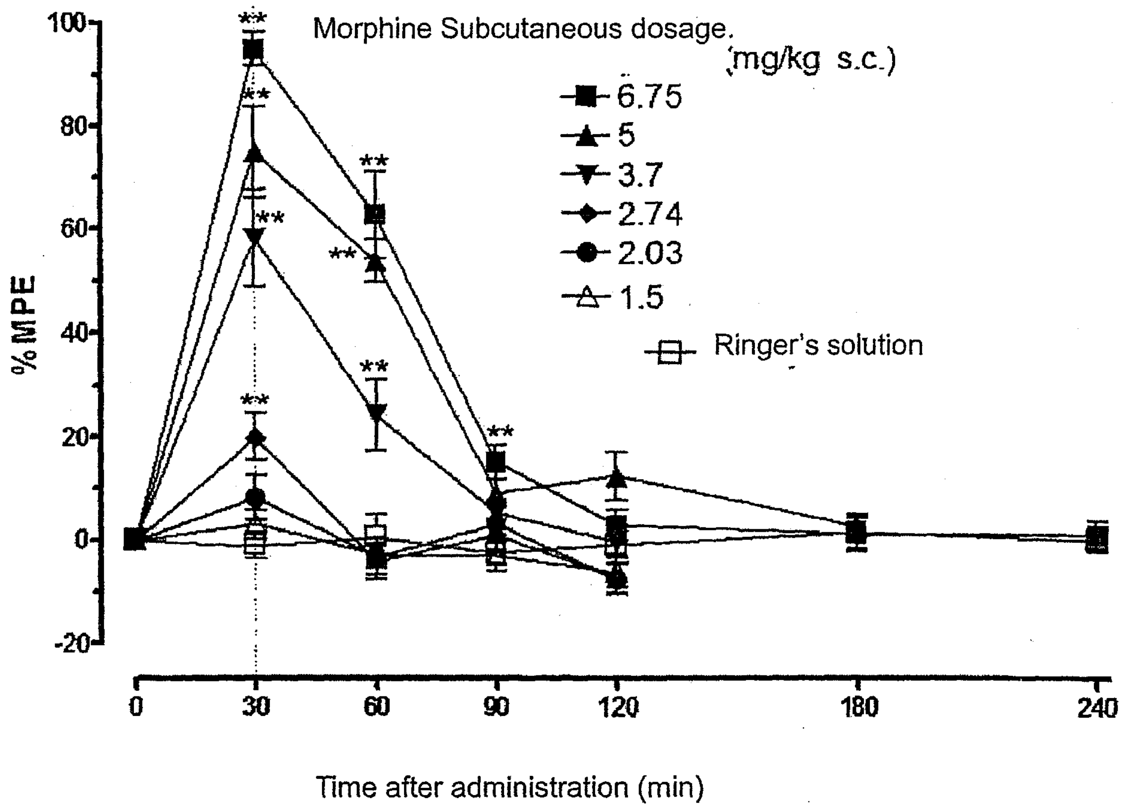
Morphine Subcutaneous dosage.
(mg/kg s.c.)
6.75
5
3.7
2.74
2.03
1.5
Ringer's solution
% MPE
100
80
60
40
20
0
-20
**
0
30
60
90
120
180
240
Time after administration (min)

Fig. 64

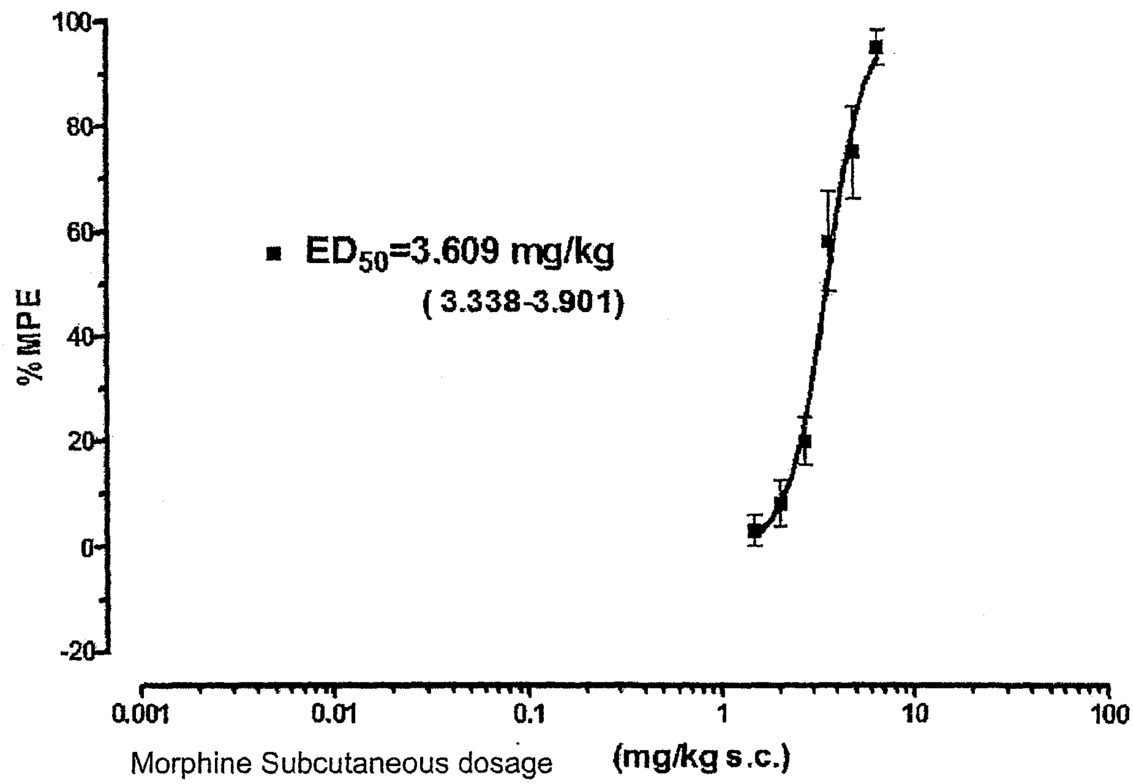

100
80
60
40
20
0
-20
% MPE
■ $ED_{50}$=3.609 mg/kg
( 3.338-3.901)
0.001
0.01
0.1
1
10
100
Morphine Subcutaneous dosage
(mg/kg s.c.)

Fig. 65

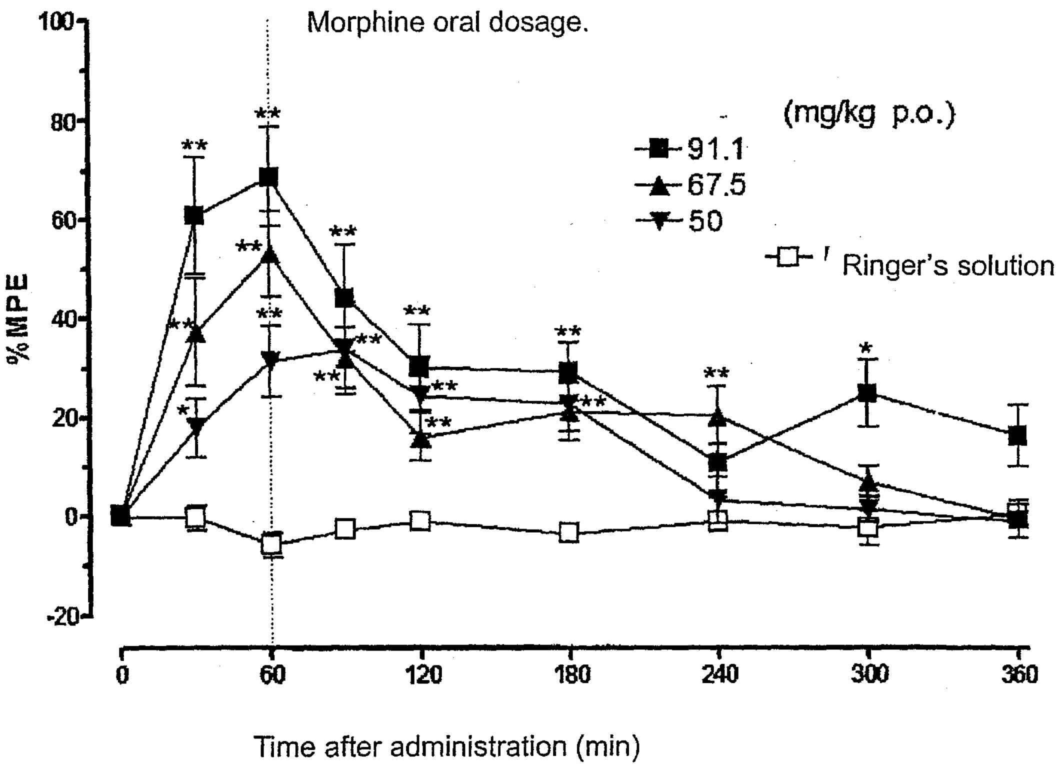
Morphine oral dosage.
(mg/kg p.o.)
91.1
67.5
50
Ringer's solution
%MPE
100
80
60
40
20
0
-20
0
60
120
180
240
300
360
Time after administration (min)

Fig. 66

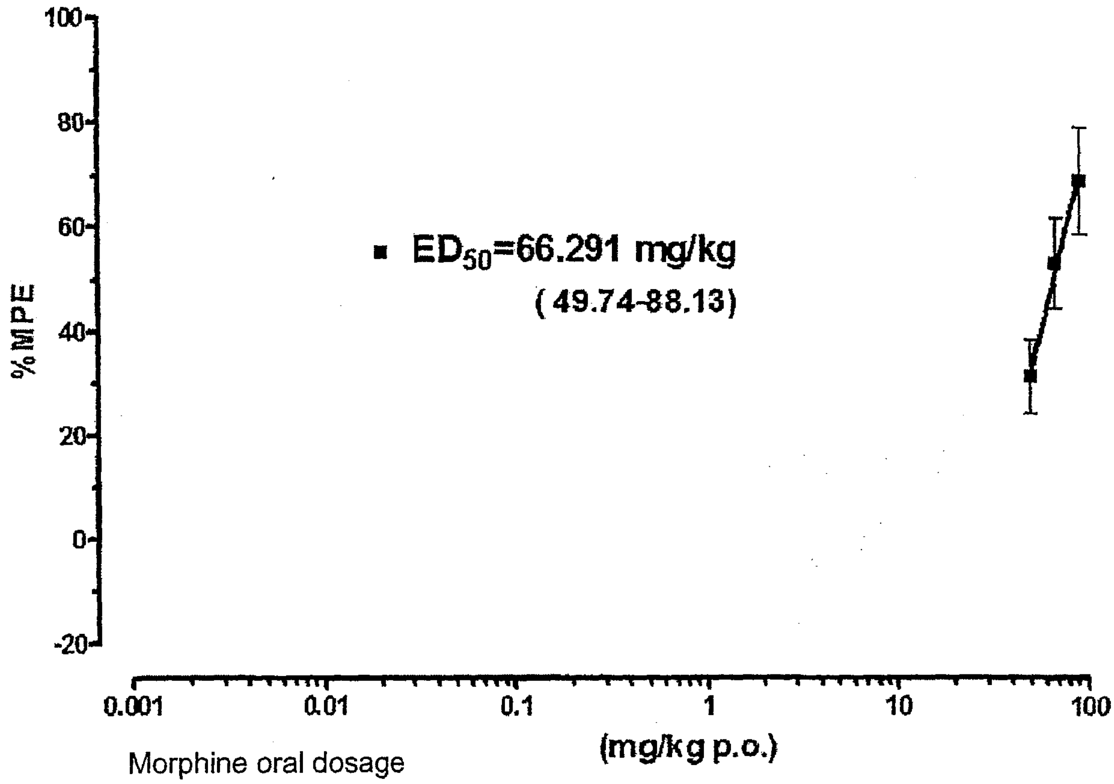


Morphine oral dosage

Fig. 67

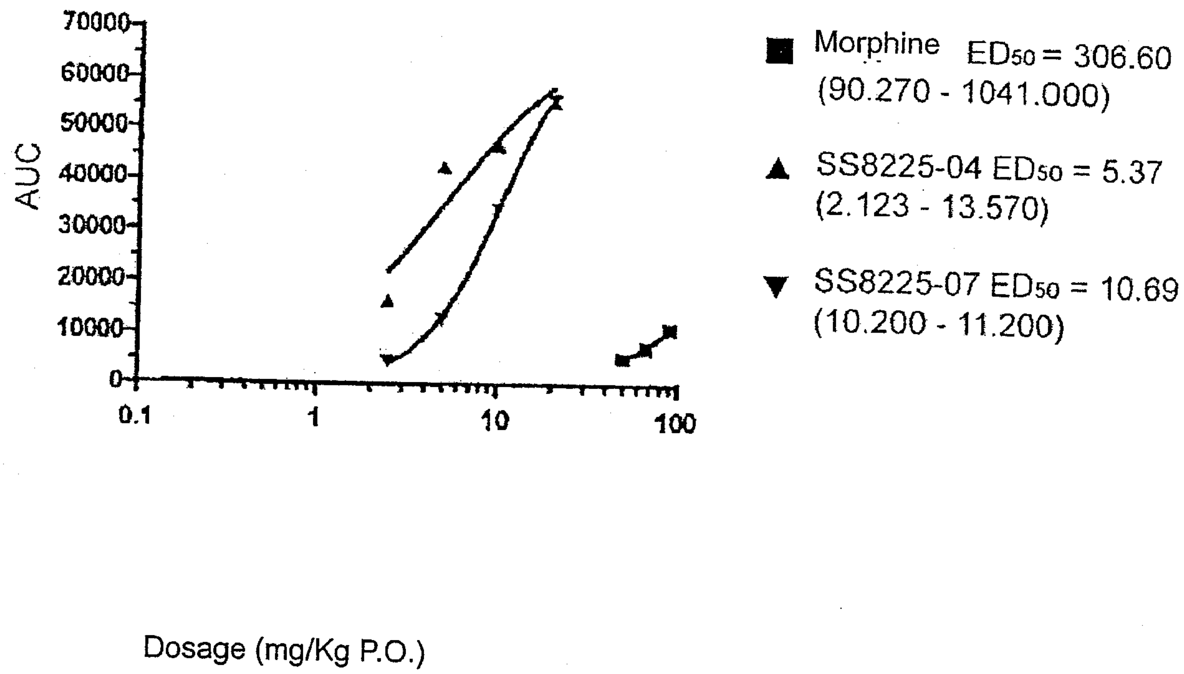
AUC
70000
60000
50000
40000
30000
20000
10000
0
0.1
1
10
100
Morphine ED50 = 306.60
(90.270 - 1041.000)
SS8225-04 ED50 = 5.37
(2.123 - 13.570)
SS8225-07 ED50 = 10.69
(10.200 - 11.200)
Dosage (mg/Kg P.O.)

Fig. 68

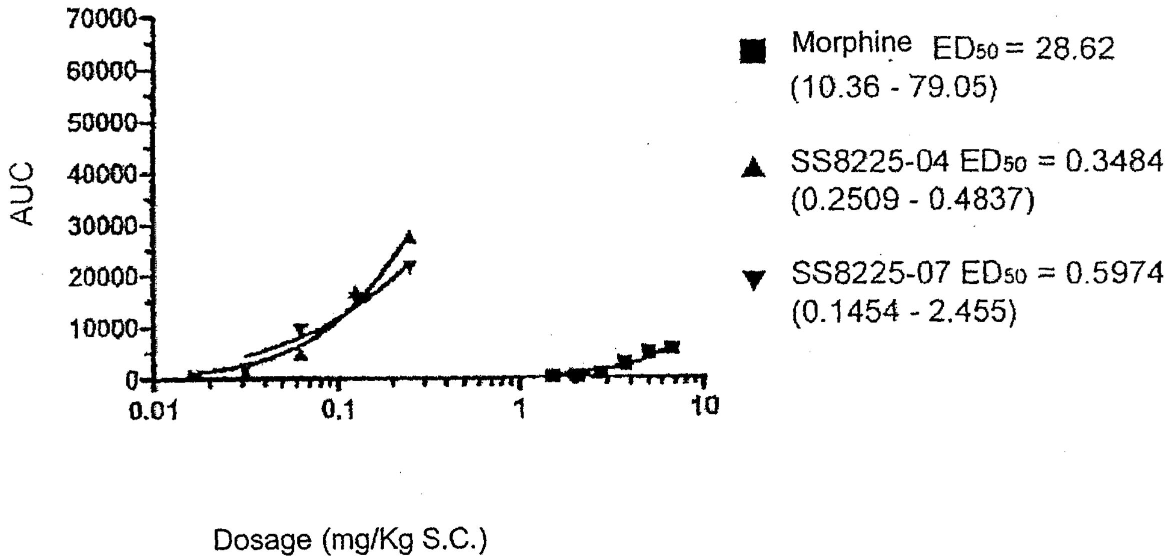
70000
60000
50000
40000
30000
20000
10000
0
AUC
0.01
0.1
1
10
Dosage (mg/Kg S.C.)
Morphine ED50 = 28.62
(10.36 - 79.05)
SS8225-04 ED50 = 0.3484
(0.2509 - 0.4837)
SS8225-07 ED50 = 0.5974
(0.1454 - 2.455)

Fig. 69

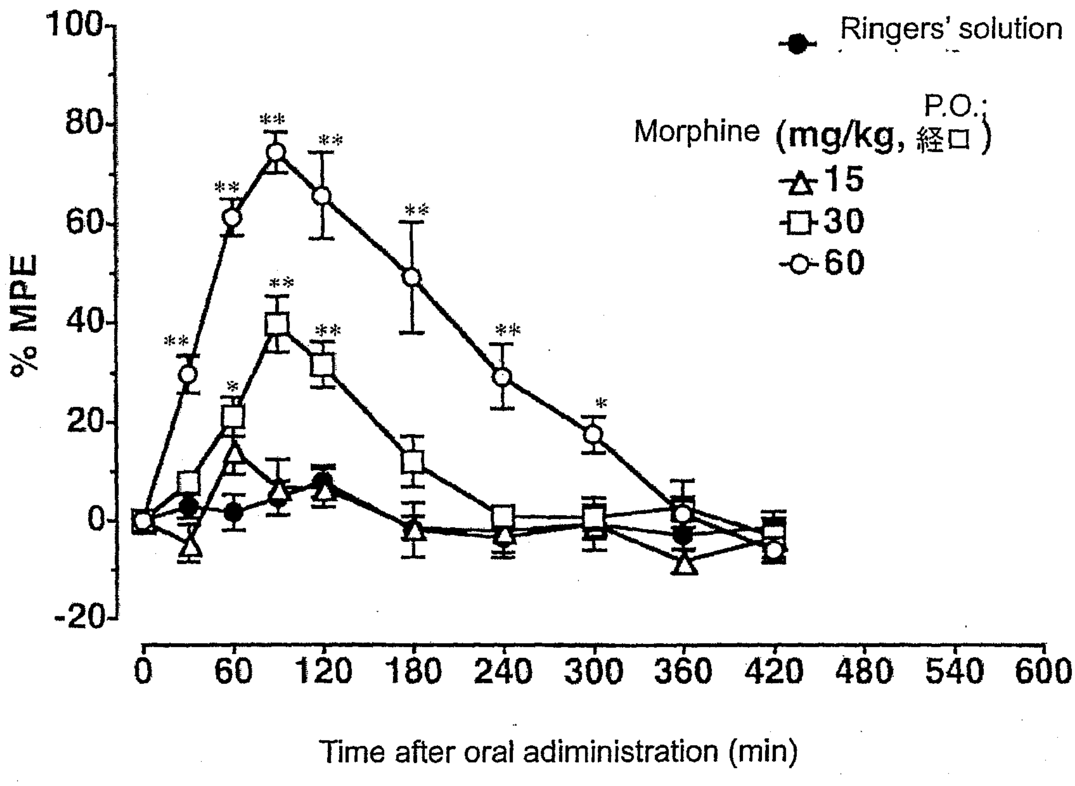
Ringers' solution
Morphine (mg/kg, P.O.; 経口)
15
30
60
% MPE
100
80
60
40
20
0
-20
**
*
0
60
120
180
240
300
360
420
480
540
600
Time after oral adiministration (min)

Fig. 70

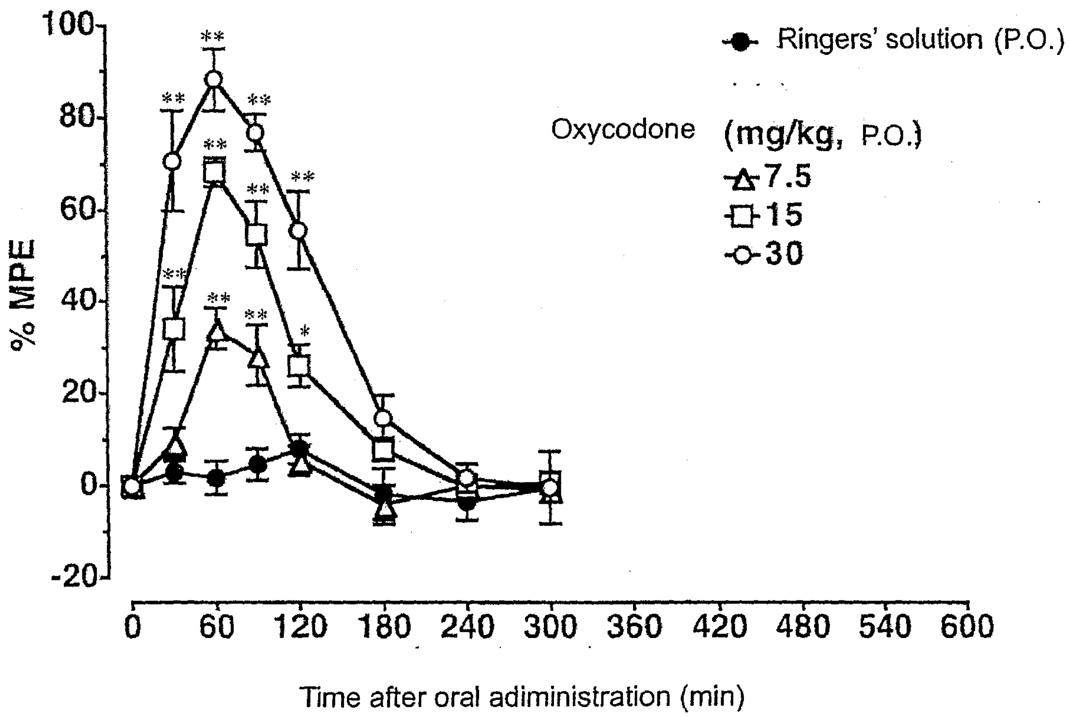
% MPE
100
80
60
40
20
0
-20
**
**
**
**
**
**
**
**
**
*
Ringers' solution (P.O.)
Oxycodone (mg/kg, P.O.)
7.5
15
30
0 60 120 180 240 300 360 420 480 540 600
Time after oral adiministration (min)

Fig. 71

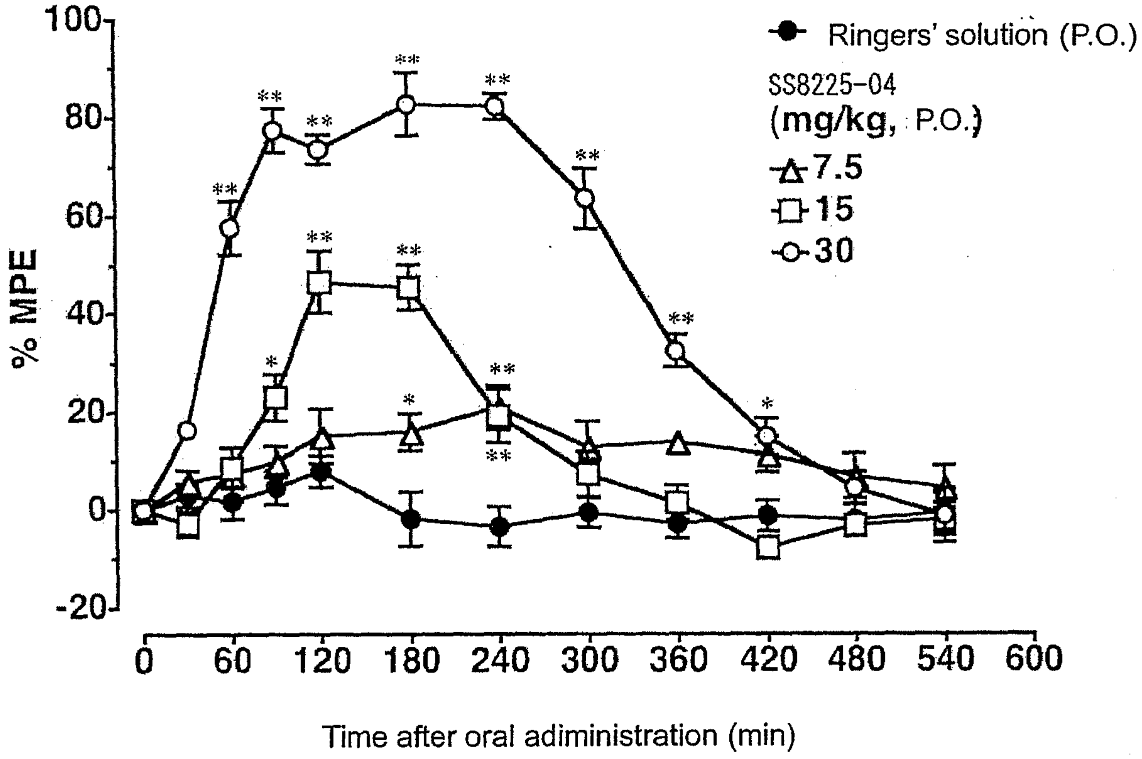
% MPE
100
80
60
40
20
0
-20
0
60
120
180
240
300
360
420
480
540
600
Time after oral adiministration (min)
Ringers' solution (P.O.)
SS8225-04
(mg/kg, P.O.)
7.5
15
30
**
*

Fig. 72

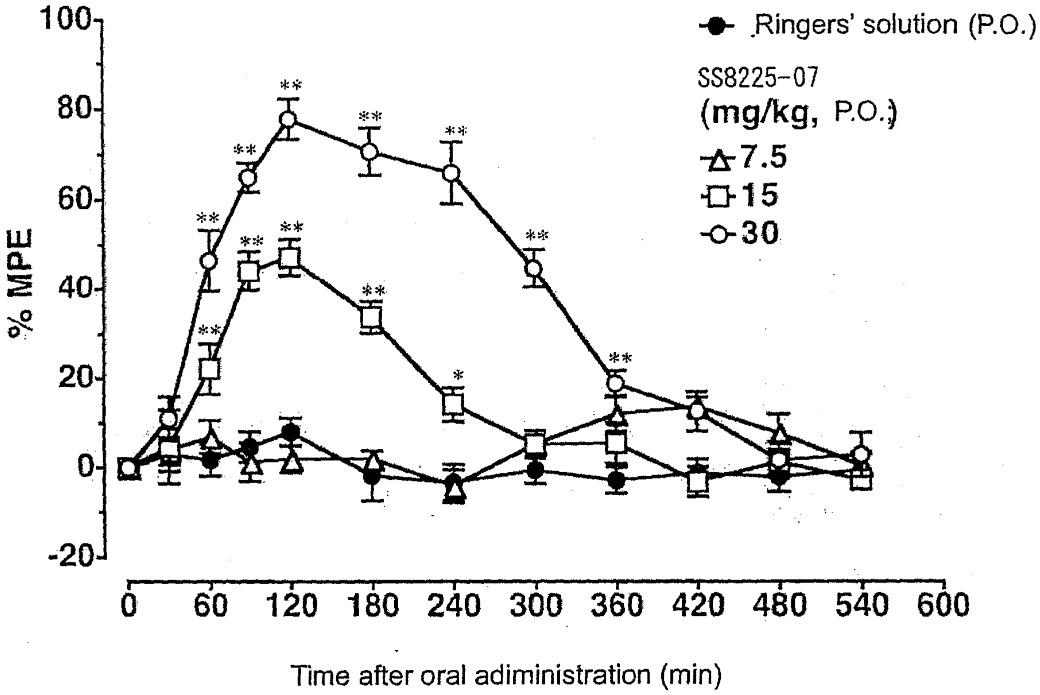

% MPE
100
80
60
40
20
0
-20
0 60 120 180 240 300 360 420 480 540 600
Time after oral adiministration (min)
Ringers' solution (P.O.)
SS8225-07
(mg/kg, P.O.)
7.5
15
30

Fig. 73

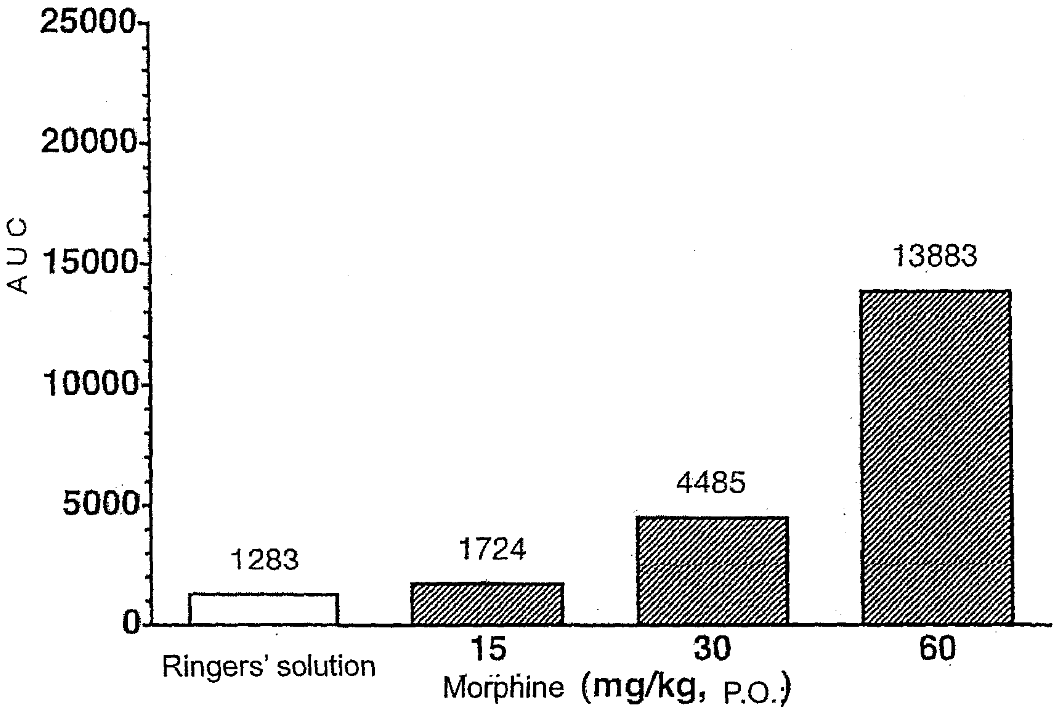
25000
20000
15000
10000
5000
0
A U C
1283
1724
4485
13883
Ringers' solution
15
30
60
Morphine (mg/kg, P.O.)

Fig. 74

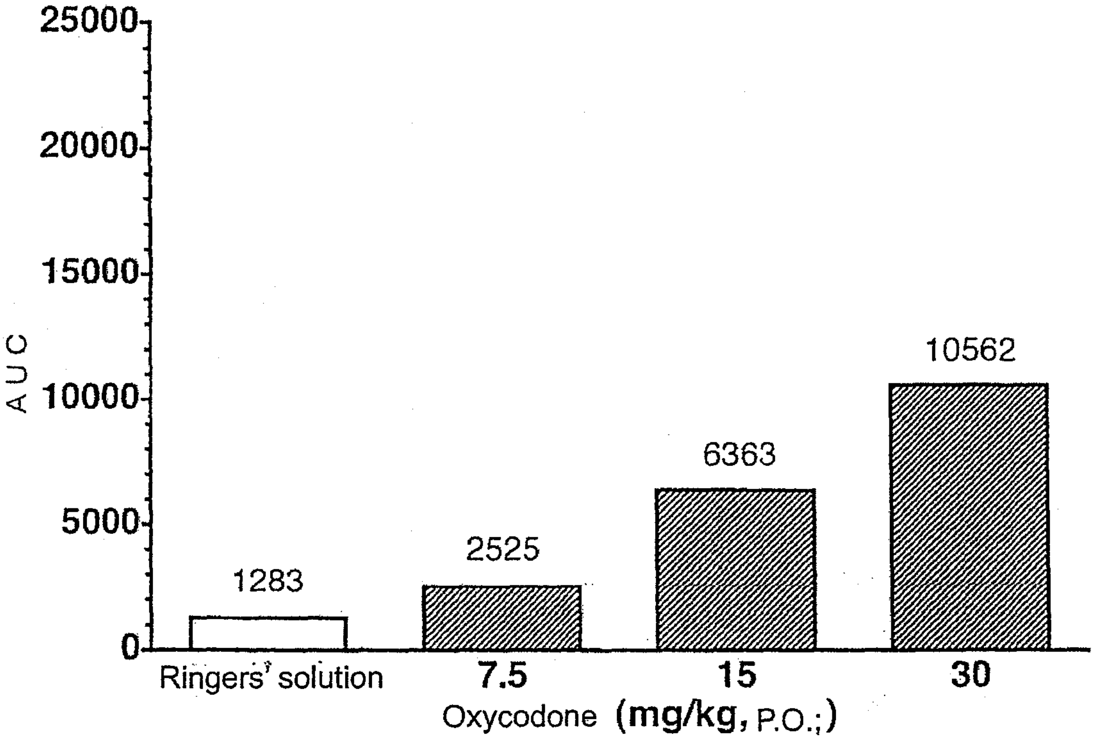
25000
20000
15000
10000
5000
0
AUC
1283
2525
6363
10562
Ringers' solution
7.5
15
30
Oxycodone (mg/kg, P.O.;)

Fig. 75

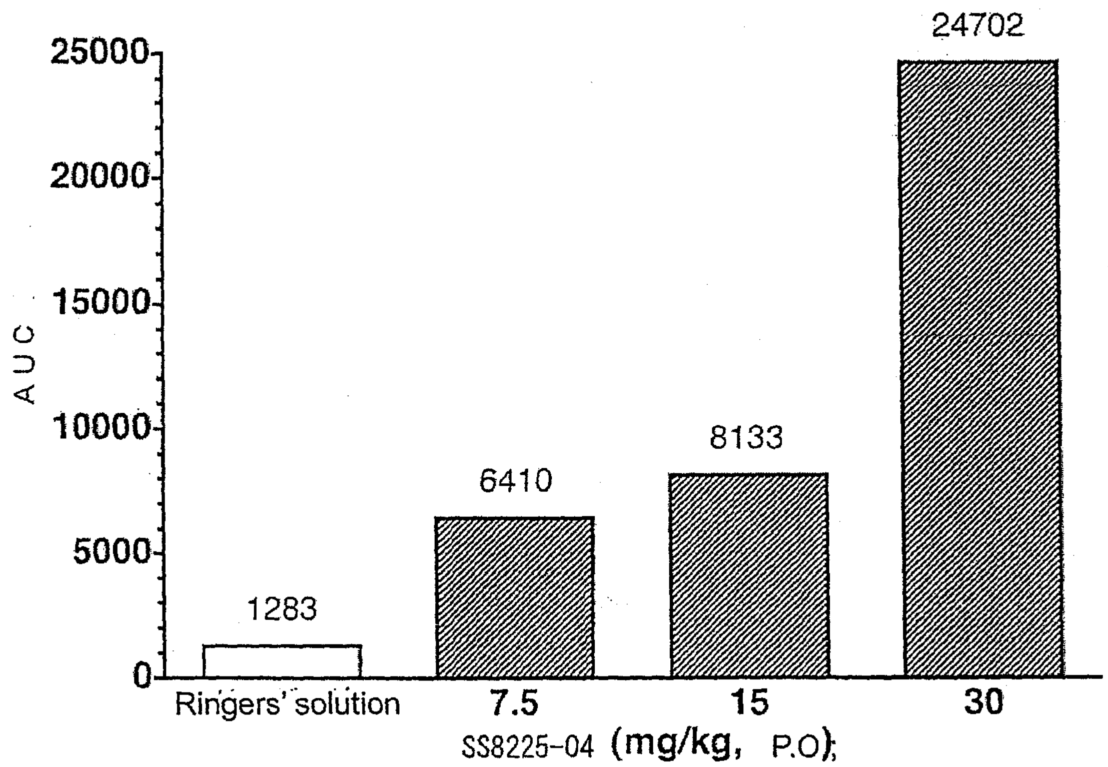
24702
25000
20000
15000
AUC
10000
8133
6410
5000
1283
0
Ringers' solution
7.5
15
30
SS8225-04 (mg/kg, P.O);

Fig. 76

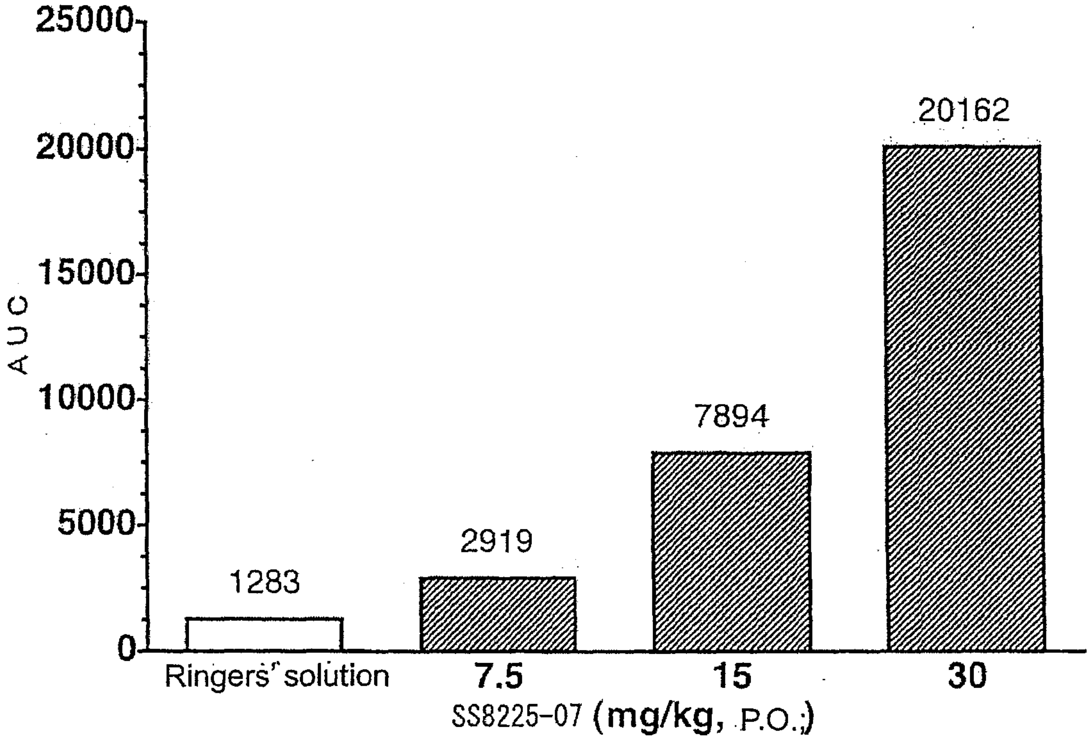
25000
20000
15000
10000
5000
0
AUC
1283
2919
7894
20162
Ringers' solution
7.5
15
30
SS8225-07 (mg/kg, P.O.)

Fig. 77

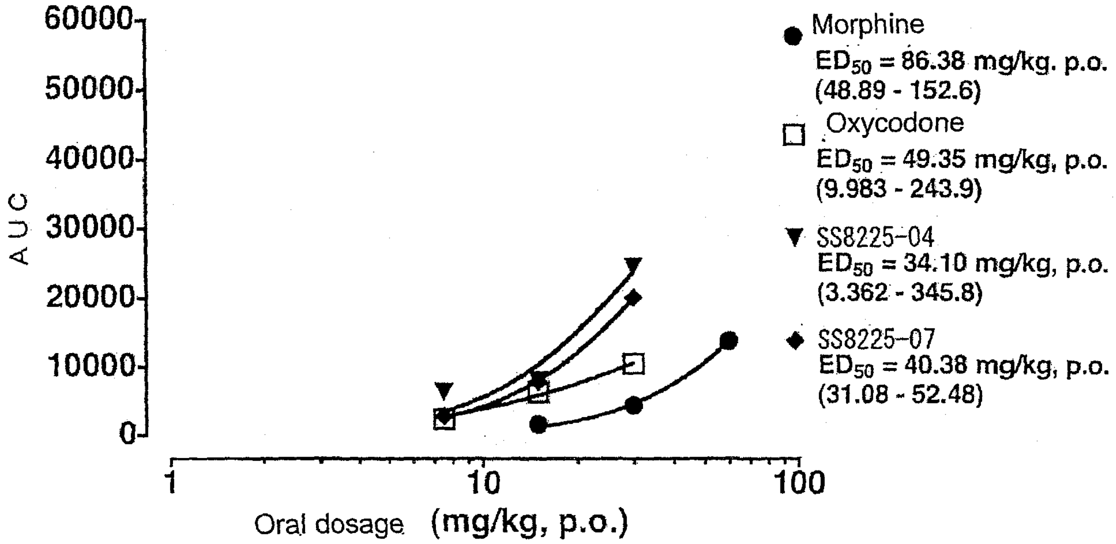
60000
50000
40000
30000
20000
10000
0
A U C
1
10
100
Oral dosage (mg/kg, p.o.)
Morphine
ED50 = 86.38 mg/kg. p.o.
(48.89 - 152.6)
Oxycodone
ED50 = 49.35 mg/kg, p.o.
(9.983 - 243.9)
SS8225-04
ED50 = 34.10 mg/kg, p.o.
(3.362 - 345.8)
SS8225-07
ED50 = 40.38 mg/kg, p.o.
(31.08 - 52.48)

Fig. 78

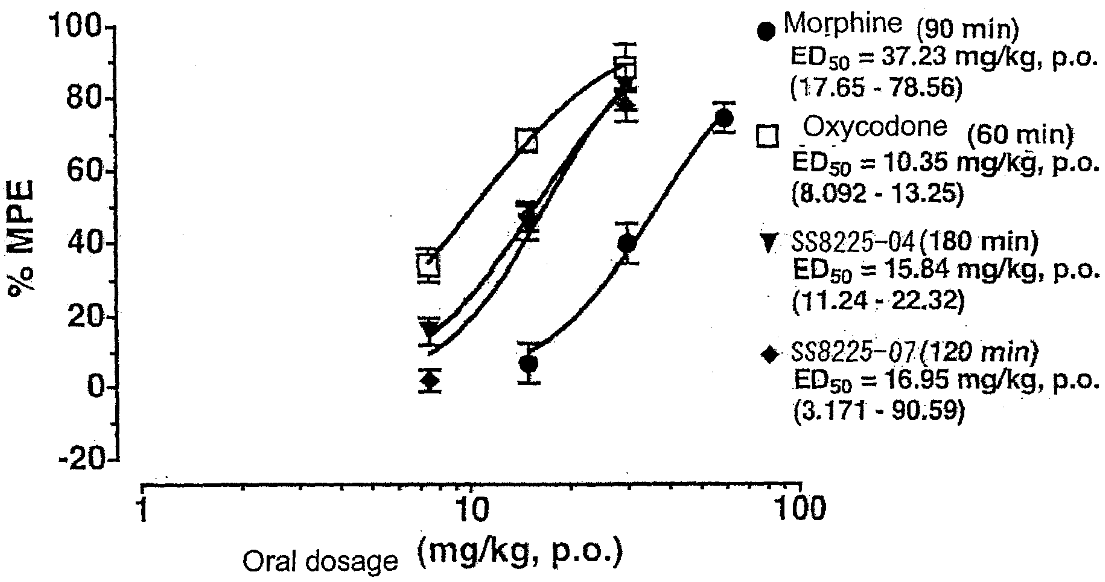
100
80
60
40
20
0
-20
% MPE
1
10
100
Oral dosage (mg/kg, p.o.)
Morphine (90 min)
ED50 = 37.23 mg/kg, p.o.
(17.65 - 78.56)
Oxycodone (60 min)
ED50 = 10.35 mg/kg, p.o.
(8.092 - 13.25)
SS8225-04 (180 min)
ED50 = 15.84 mg/kg, p.o.
(11.24 - 22.32)
SS8225-07 (120 min)
ED50 = 16.95 mg/kg, p.o.
(3.171 - 90.59)

Fig. 79

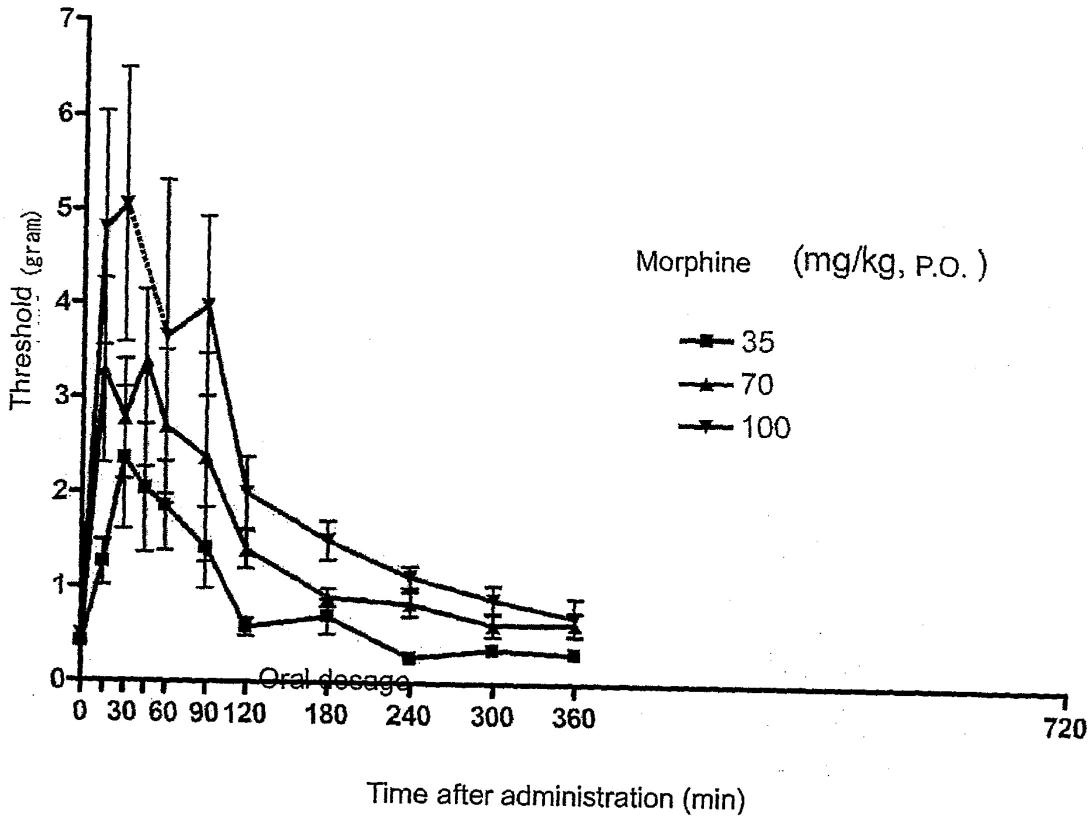

Threshold (gram)
7
6
5
4
3
2
1
0
Oral dosage
0
30
60
90
120
180
240
300
360
720
Time after administration (min)
Morphine (mg/kg, P.O.)
35
70
100

Fig. 80

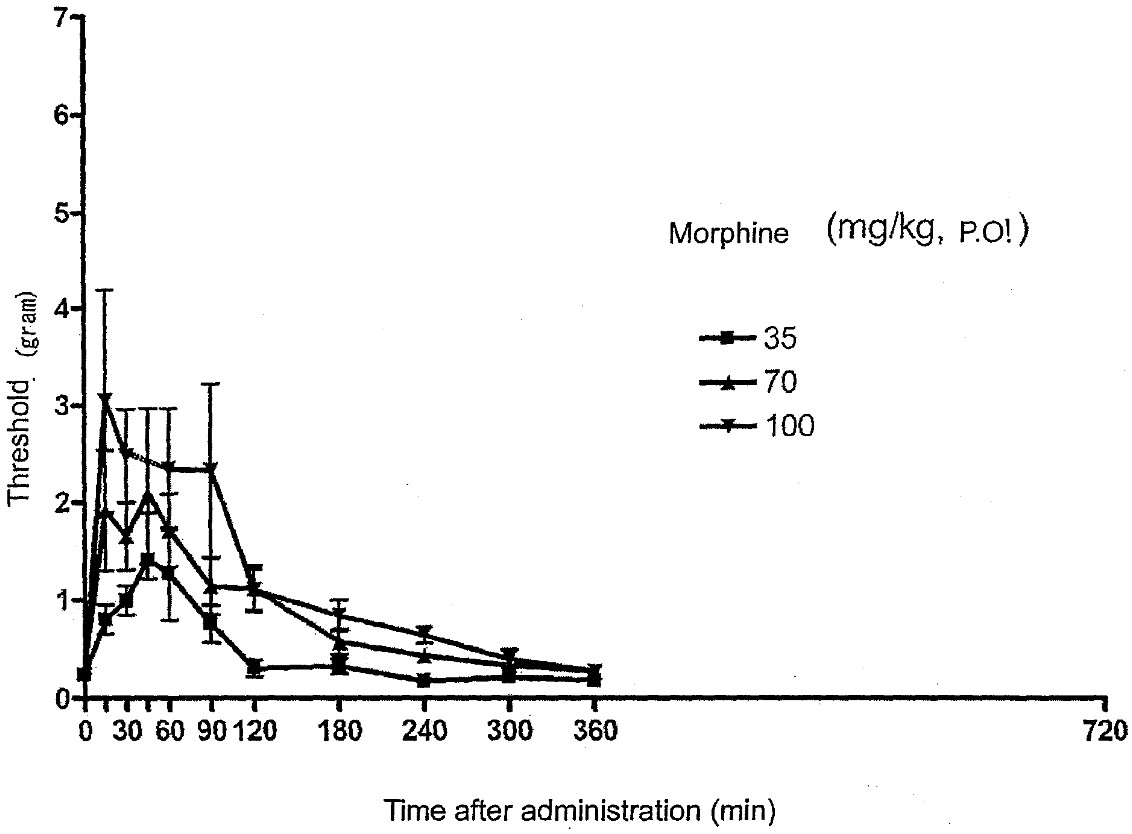
Morphine (mg/kg, P.O!)
35
70
100
Threshold (gram)
0
1
2
3
4
5
6
7
0 30 60 90 120 180 240 300 360 720
Time after administration (min)

Fig. 81

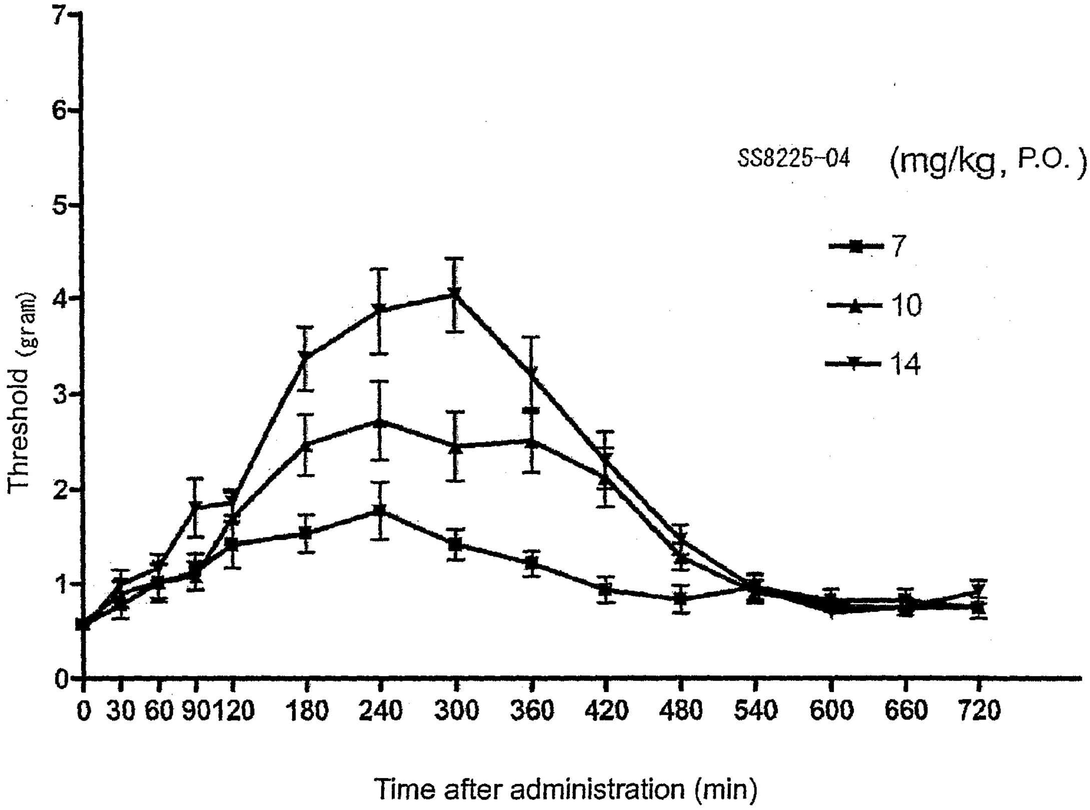
SS8225-04 (mg/kg, P.O.)
7
10
14
Threshold (gram)
0
1
2
3
4
5
6
7
0 30 60 90120 180 240 300 360 420 480 540 600 660 720
Time after administration (min)

Fig. 82

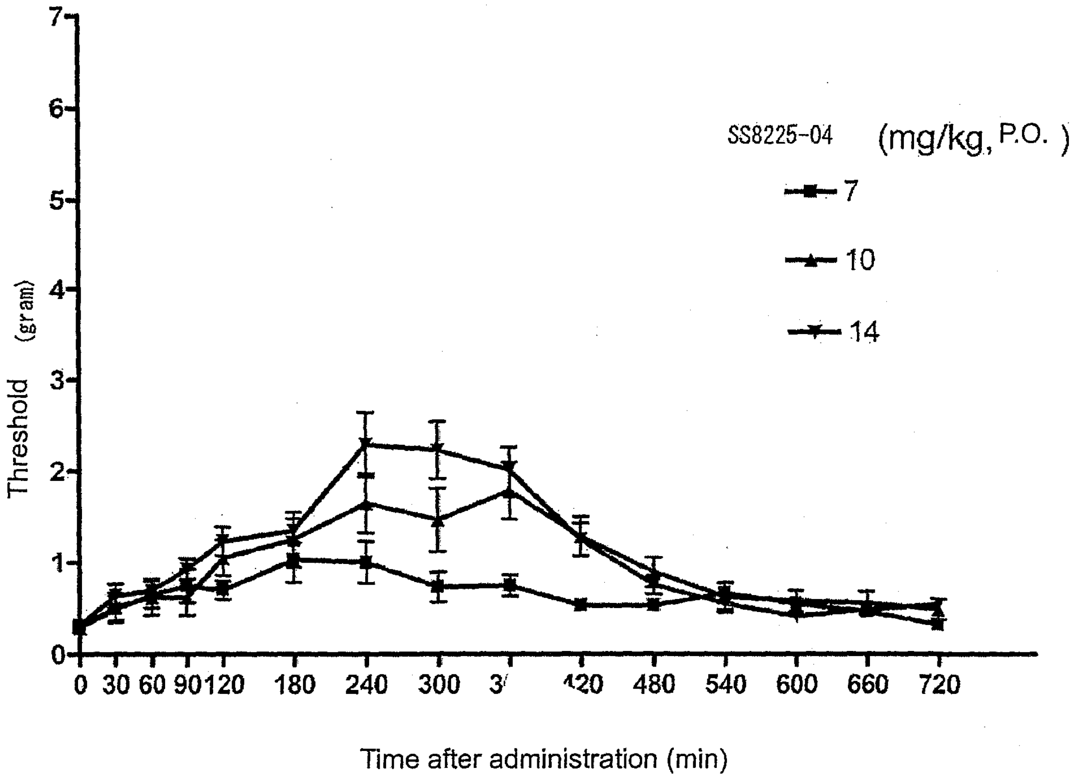
SS8225-04 (mg/kg, P.O. )
7
10
14
Threshold (gram)
0
1
2
3
4
5
6
7
0 30 60 90 120 180 240 300 3 420 480 540 600 660 720
Time after administration (min)

Fig. 83

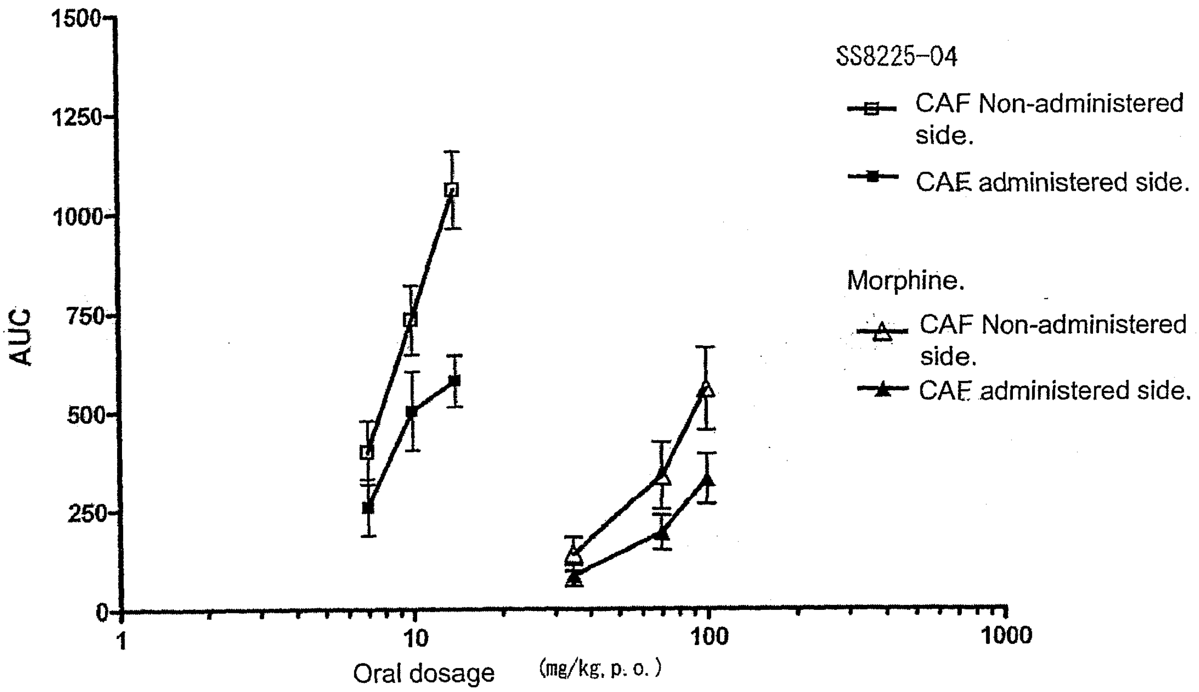
1500
1250
1000
750
500
250
0
AUC
1
10
100
1000
Oral dosage (mg/kg, p. o. )
SS8225-04
CAF Non-administered side.
CAF administered side.
Morphine.
CAF Non-administered side.
CAF administered side.

Fig. 84

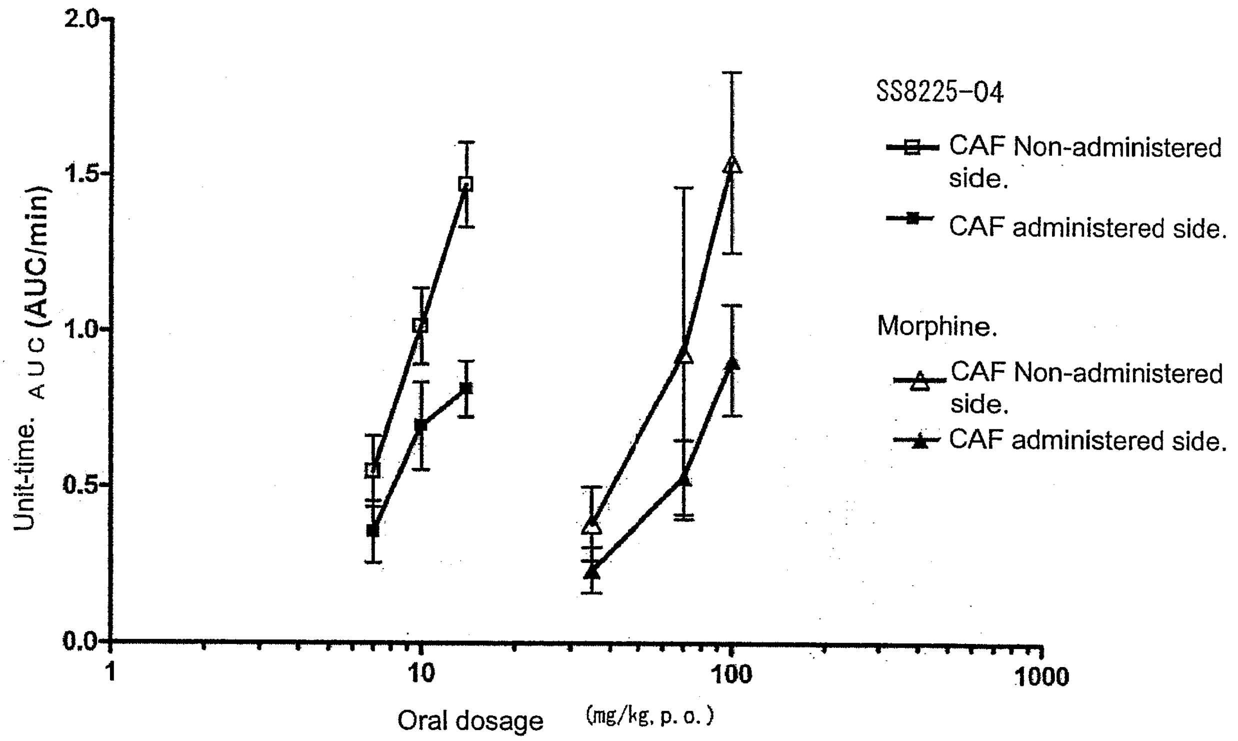
2.0
1.5
1.0
0.5
0.0
Unit-time. A U C (AUC/min)
1
10
100
1000
Oral dosage (mg/kg, p. o.)
SS8225-04
CAF Non-administered side.
CAF administered side.
Morphine.
CAF Non-administered side.
CAF administered side.

**INTERNATIONAL SEARCH REPORT**

International application No.
PCT/JP2007/061809

A. CLASSIFICATION OF SUBJECT MATTER
*C07K5/02*(2006.01)i, *A61K38/00*(2006.01)i, *A61P25/04*(2006.01)i

According to International Patent Classification (IPC) or to both national classification and IPC

B. FIELDS SEARCHED

Minimum documentation searched (classification system followed by classification symbols)
C07K5/02, A61K38/00, A61P25/04

Documentation searched other than minimum documentation to the extent that such documents are included in the fields searched

Electronic data base consulted during the international search (name of data base and, where practicable, search terms used)
CA(STN), REGISTRY(STN), WPIDS(STN)

C. DOCUMENTS CONSIDERED TO BE RELEVANT

| Category* | Citation of document, with indication, where appropriate, of the relevant passages | Relevant to claim No. |
|---|---|---|
| A | WO 99/33864 A1 (Daiichi Pharmaceutical Co., Ltd.),<br>08 July, 1999 (08.07.99),<br>Full text<br>& TW 474945 B | 1-24 |
| A | WO 97/35579 A1 (MERCK & CO., INC.),<br>02 October, 1997 (02.10.97),<br>Claim 2<br>& AU 9723409 A | 1-24 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>28 June, 2007 (28.06.07) | Date of mailing of the international search report<br>10 July, 2007 (10.07.07) |
| Name and mailing address of the ISA/<br>Japanese Patent Office<br><br>Facsimile No. | Authorized officer<br><br>Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006326144 W
- JP 2006162379 A
- WO 9524421 A
- WO 9710261 A
- WO 9710262 A
- WO 9933864 A


### Non-patent literature cited in the description


- *Int. J. Peptide Protein Res.,* 1981, vol. 17, 275
- *Br. J. Pharmacol.,* 1988, vol. 95, 15
- *Peptides,* 1990, vol. 11, 139
- *Neuropharmacol.,* 1993, vol. 32, 689
- *Pharmacol. Biochem. Behav.,* 1988, vol. 24, 27
- *Chem. Pharm. Bull.,* 2002, vol. 50, 771-780
- *J. Med. Chem.,* 2002, vol. 45, 5081-5089
- *Chem. Pharm. Bull.,* 2003, vol. 51, 759-771
- **SEIKAGAKU JITEN.** *Tokyo Kagaku Doujin,* 08 October 1998
- Guideline for Notation of Base Sequence or Amino Acid Sequence in the Description. Japanese Patent Office, July 2002
- Protein Technology - Basic and Application. Maruzen, 1992
- **M. BONDANSZKY.** Peptide Synthesis. John Wiley & Sons, 1976
- **M. STEWART ; D. J. YOUNG.** Solid Phase Peptide Synthesis. W. H. Freeman and Co, 1969